# EUROPEAN PATENT SPECIFICATION

(11) **EP 3 897 735 B1**
(45) Date of publication and mention of the grant of the patent: **22.10.2025**
(21) Application number: 19817689.3
(22) Date of filing: 09.12.2019
(51) Int. Cl.: A61K 47/54, A61K 47/68, A61K 47/55, A61K 47/59, A61K 47/64, A61K 47/60, A61P 35/00, C07H 15/256, C07J 63/00, C07K 16/32, A61K 39/00

(54) **BIOLOGICALLY ACTIVE CLUSTER OF MOLECULES**
BIOLOGISCH AKTIVE CLUSTER VON MOLEKÜLEN
GROUPE BIOLOGIQUEMENT ACTIF DE MOLÉCULES

(30) Priority: 21.12.2018 NL 2022283; 10.07.2019 NL 2023468; 25.07.2019 NL 2023568
(43) Date of publication of application: 27.10.2021
(60) Divisional application: 25203109.1
(73) Proprietor: Sapreme Technologies B.V., 3584 CM Utrecht (NL)
(72) Inventor: POSTEL, Ruben, 3584 CM Utrecht (NL); FUCHS, Hendrik, 13353 Berlin (DE)
(74) Representative: Nederlandsch Octrooibureau
(86) International application number: PCT/EP2019/084290
(87) International publication number: WO 2020/126626

(56) References cited:
- WO-A2-2006/033766
- US-A1- 2004 242 502
- CHOPDEY PRASHANT K ET AL: "Glycyrrhizin Conjugated Dendrimer and Multi-Walled Carbon Nanotubes for Liver Specific Delivery of Doxorubicin", JOURNAL OF NANOSCIENCE AND NANOTECHNOLOGY, AMERICAN SCIENTIFIC PUBLISHERS, US, vol. 15, no. 2, 31 January 2015 (2015-01-31), pages 1088 - 1100, XP009519412, ISSN: 1533-4880, DOI: 10.1166/JNN.2015.9039
- DANIEL PULIDO ET AL: "Controlling Multivalency and Multimodality: Up to Pentamodal Dendritic Platforms Based on Diethylenetriaminepentaacetic Acid Cores", ORGANIC LETTERS, vol. 16, no. 5, 14 February 2014 (2014-02-14), pages 1318 - 1321, XP055677348, ISSN: 1523-7060, DOI: 10.1021/ol500022n
- STEFFENSEN M B ET AL: "Synthesis and manipulation of orthogonally protected dendrimers: building blocks for library synthesis", ANGEWANDTE CHEMIE, INTERNATIONAL EDITION, WILEY-VCH, DE, vol. 43, no. 39, 4 October 2004 (2004-10-04), pages 5177 - 5180, XP002469507, ISSN: 1433-7851, DOI: 10.1002/ANIE.200460031
- HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), pages 14, XP055429235, DOI: 10.3390/biomedicines5020014
- SIMKO SAMA: "Untersuchung von Saponinen als neuartige Verstärker der Transfektion", 6 June 2018 (2018-06-06), XP055608281, Retrieved from the Internet <URL:https://refubium.fu-berlin.de/bitstream/handle/fub188/22176/Dissertation_Publikation_Online.pdf?sequence=3&isAllowed=y> [retrieved on 20190724]

## Description

### TECHNICAL FIELD

The invention relates to a molecular scaffold suitable for covalently binding at least one biologically active molecule such as a therapeutic molecule to a carrier molecule. In particular, the invention relates to monoclonal antibody-based antibody-drug conjugates with improved therapeutic window of the drug due to covalent linkage of (a cluster of) potentiator molecules, e.g. a payload such as a protein toxin or oligonucleotide to the ADC, or alternatively, due to co-administration of an ADC and a cell-targeting conjugate comprising (a cluster of) potentiator molecules to a patient in need thereof. The invention also relates to a method for producing a scaffold suitable for covalently binding at least one biologically active molecule to a carrier molecule, wherein such biologically active molecule is covalently bound to the scaffold.

### BACKGROUND

Molecules with a therapeutic biological activity are in many occasions in theory suitable for application as an effective therapeutic drug for the treatment of a disease such as a cancer in human patients in need thereof. A typical example are small-molecule biologically active moieties. However, many if not all potential drug-like molecules and therapeutics currently used in the clinic suffer from at least one of a plethora of shortcomings and drawbacks. When administered to a human body, therapeutically active molecules may exert off-target effects, in addition to the biologically activity directed to an aspect underlying a to-be-treated disease or health problem. Such off-target effects are undesired and bear a risk for induction of health- or even life-threatening side effects of the administered molecule. It is the occurrence of such adverse events that cause many drug-like compounds and therapeutic moieties to fail phase III clinical trials or even phase IV clinical trials (post-market entry follow-up). Therefore, there is a strong desire to provide drug molecules such as small-molecule therapeutics, wherein the therapeutic effect of the drug molecule should, e.g., (1) be highly specific for a biological factor or biological process driving the disease, (2) be sufficiently safe, (3) be sufficiently efficacious, (4) be sufficiently directed to the diseased cell with little to no off-target activity on non-diseased cells, (5) have a sufficiently timely mode of action (e.g. the administered drug molecule should reach the targeted site in the human patient within a certain time frame and should remain at the targeted site for a certain time frame), and/or (6) have sufficiently long lasting therapeutic activity in the patient's body, amongst others. Unfortunately, to date, 'ideal' therapeutics with many or even all of the beneficial characteristics here above outlined, are not available to the patients, despite already long-lasting and intensive research and despite the impressive progress made in several areas of the individually addressed encountered difficulties and drawbacks.

Chemotherapy is one of the most important therapeutic options for cancer treatment. However, it is often associated with a low therapeutic window because it has no specificity towards cancer cells over dividing cells in healthy tissue. The invention of monoclonal antibodies offered the possibility of exploiting their specific binding properties as a mechanism for the targeted delivery of cytotoxic agents to cancer cells, while sparing normal cells. This can be achieved by chemical conjugation of cytotoxic effectors (also known as payloads or warheads) to antibodies, to create antibody-drug conjugates (ADCs). Typically, very potent payloads such as emtansine (DM1) are used which have a limited therapeutic index (a ratio that compares toxic dose to efficacious dose) in their unconjugated forms. The conjugation of DM1 to trastuzumab (ado-trastuzumab emtansine), also known as Kadcycla, enhances the tolerable dose of DM1 at least two-fold in monkeys. In the past few decades tremendous efforts and investments have been made to develop therapeutic ADCs. However, it remains challenging to bring ADCs into the clinic, despite promising preclinical data. The first ADC approved for clinical use was gemtuzumab ozogamicin (Mylotarg, CD33 targeted, Pfizer/Wyeth) for relapsed acute myelogenous leukemia (AML) in 2000. Mylotarg was however, withdrawn from the market at the request of the Federal Drug Administration (FDA) due to a number of concerns including its safety profile. Patients treated with Mylotarg were more often found to die than patients treated with conventional chemotherapy. Mylotarg was admitted to the market again in 2017 with a lower recommended dose, a different schedule in combination with chemotherapy or on its own, and a new patient population. To date, only five ADCs have been approved for clinical use, and meanwhile clinical development of approximately fifty-five ADCs has been halted. However, interest remains high and approximately eighty ADCs are still in clinical development in nearly six-hundred clinical trials at present.

Despite the potential to use toxic payloads that are normally not tolerated by patients, a low therapeutic index (a ratio that compares toxic dose to efficacious dose) is a major problem accounting for the discontinuance of many ADCs in clinical development, which can be caused by several mechanisms such as off-target toxicity on normal cells, development of resistance against the cytotoxic agents and premature release of drugs in the circulation. A systematic review by the FDA of ADCs found that the toxicity profiles of most ADCs could be categorized according to the payload used, but not the antibody used, suggesting that toxicity is mostly determined by premature release of the payload. Of the approximately fifty-five ADCs that were discontinued, it is estimated that at least twenty-three were due to a poor therapeutic index. For example, development of a trastuzumab tesirine conjugate (ADCT-502, HER-2 targeted, ADC therapeutics) was recently discontinued due to a narrow therapeutic index, possibly due to an on-target, off-tissue effect in pulmonary tissue which expresses considerable levels of HER2. In addition, several ADCs in phase 3 trials have been discontinued due to missing primary endpoint. For example, phase 3 trials of a depatuxizumab mafodotin conjugate (ABT-414, EGFR targeted, AbbVie) tested in patients with newly diagnosed glioblastoma, and a mirvetuximab soravtansine conjugate (IMGN853, folate receptor alpha (FRα) targeted, ImmunoGen) tested in patients with platinum-resistant ovarian cancer, were recently stopped, showing no survival benefit. It is important to note that the clinically used dose of some ADCs may not be sufficient for its full anticancer activity. For example, ado-trastuzumab emtansine has an MTD of 3.6 mg/kg in humans. In preclinical models of breast cancer, ado-trastuzumab emtansine induced tumor regression at dose levels at or above 3 mg/kg, but more potent efficacy was observed at 15 mg/kg. This suggests that at the clinically administered dose, ado-trastuzumab emtansine may not exert its maximal potential anti-tumor effect.

ADCs are mainly composed of an antibody, a cytotoxic moiety such as a payload, and a linker. Several novel strategies have been proposed and carried out in the design and development of new ADCs to overcome the existing problems, targeting each of the components of ADCs. For example, by identification and validation of adequate antigenic targets for the antibody component, by selecting antigens which have high expression levels in tumor and little or no expression in normal tissues, antigens which are present on the cell surface to be accessible to the circulating ADCs, and antigens which allows internalizing of ADCs into the cell after binding; and alternative mechanisms of activity; design and optimize linkers which enhance the solubility and the drug-to-antibody ratio (DAR) of ADCs and overcome resistance induced by proteins that can transport the chemotherapeutic agent out of the cells; enhance the DAR ratio by inclusion of more payloads, select and optimize antibodies to improve antibody homogeneity and developability. In addition to the technological development of ADCs, new clinical and translational strategies are also being deployed to maximize the therapeutic index, such as, change dosing schedules through fractionated dosing; perform biodistribution studies; include biomarkers to optimize patient selection, to capture response signals early and monitor the duration and depth of response, and to inform combination studies.

An example of ADCs with clinical potential are those ADCs such as brentuximab vedotin, inotuzumab ozogamicin, moxetumomab pasudotox, and polatuzumab vedotin, which are evaluated as a treatment option for lymphoid malignancies and multiple myeloma. Polatuzumab vedotin, binding to CD79b on (malignant) B-cells, and pinatuzumab vedotin, binding to CD22, are tested in clinical trials wherein the ADCs each were combined with co-administered rituximab, a monoclonal antibody binding to CD20 and not provided with a payload [B. Yu and D. Liu, Antibody-drug conjugates in clinical trials for lymphoid malignancies and multiple myeloma; Journal of Hematology & Oncology (2019) 12:94*].* Combinations of monoclonal antibodies such as these examples are yet a further approach and attempt to arrive at the 'magic bullet' which combines many or even all of the aforementioned desired characteristics of ADCs.

Meanwhile in the past few decades, nucleic acid-based therapeutics are under development. Therapeutic nucleic acids can be based on deoxyribonucleic acid (DNA) or ribonucleic acid (RNA), Anti-sense oligonucleotides (ASOs, AONs), and short interfering RNAs (siRNAs), MicroRNAs, and DNA and RNA aptamers, for approaches such as gene therapy, RNA interference (RNAi). Many of them share the same fundamental basis of action by inhibition of either DNA or RNA expression, thereby preventing expression of disease-related abnormal proteins. The largest number of clinical trials is being carried out in the field of gene therapy, with almost 2600 ongoing or completed clinical trials worldwide but with only about 4% entering phase 3. This is followed by clinical trials with ASOs. Similarly to ADCs, despite the large number of techniques being explored, therapeutic nucleic acids share two major issues during clinical development: delivery into cells and off-target effects. For instance, ASOs such as peptide nucleic acid (PNA), phosphoramidate morpholino oligomer (PMO), locked nucleic acid (LNA) and bridged nucleic acid (BNA), are being investigated as an attractive strategy to inhibit specifically target genes and especially those genes that are difficult to target with small molecules inhibitors or neutralizing antibodies. Currently, the efficacy of different ASOs is being studied in many neurodegenerative diseases such as Huntington's disease, Parkinson's disease, Alzheimer's disease, and amyotrophic lateral sclerosis and also in several cancer stages. The application of ASOs as potential therapeutic agents requires safe and effective methods for their delivery to the cytoplasm and/or nucleus of the target cells and tissues. Although the clinical relevance of ASOs has been demonstrated, inefficient cellular uptake, both in vitro and in vivo, limit the efficacy of ASOs and has been a barrier to therapeutic development. Cellular uptake can be < 2% of the dose resulting in too low ASO concentration at the active site for an effective and sustained outcome. This consequently requires an increase of the administered dose which induces off-target effects. Most common side-effects are activation of the complement cascade, the inhibition of the clotting cascade and toll-like receptor mediated stimulation of the immune system.

Chemotherapeutics are most commonly small molecules, however, their efficacy is hampered by the severe off-target side toxicity, as well as their poor solubility, rapid clearance and limited tumor exposure. Scaffold-small-molecule drug conjugates such as polymer-drug conjugates (PDCs) are macromolecular constructs with pharmacologically activity, which comprises one or more molecules of a small-molecule drug bound to a carrier scaffold (e.g. polyethylene glycol (PEG)).

Such conjugate principle has attracted much attention and has been under investigation for several decades. The majority of conjugates of small-molecule drugs under pre-clinical or clinical development are for oncological indications. However, up-to-date only one drug not related to cancer has been approved (Movantik, a PEG oligomer conjugate of opioid antagonist naloxone, AstraZeneca) for opioid-induced constipation in patients with chronic pain in 2014, which is a non-oncology indication. Translating application of drug-scaffold conjugates into treatment of human subjects provides little clinical success so far. For example, PK1 (N-(2-hydroxypropyl)methacrylamide (HPMA) copolymer doxorubicin; development by Pharmacia, Pfizer) showed great anti-cancer activity in both solid tumors and leukemia in murine models, and was under clinical investigation for oncological indications. Despite that it demonstrated significant reduction of nonspecific toxicity and improved pharmacokinetics in man, improvements in anticancer efficacy turned out to be marginal in patients, and as a consequence further development of PK1 was discontinued.

The failure of scaffold-small-molecule drug conjugates is at least partially attributed to its poor accumulation at the tumor site. For example, while in murine models PK1 showed 45-250 times higher accumulation in the tumor than in healthy tissues (liver, kidney, lung, spleen, and heart), accumulation in tumor was only observed in a small subset of patients in the clinical trial.

A potential solution to the aforementioned problems is application of nanoparticle systems for drug delivery such as liposomes. Liposomes are sphere-shaped vesicles consisting of one or more phospholipid bilayers, which are spontaneously formed when phospholipids are dispersed in water. The amphiphilicity characteristics of the phospholipids provide it with the properties of self-assembly, emulsifying and wetting characteristics, and these properties can be employed in the design of new drugs and new drug delivery systems. Drug encapsulated in a liposomal delivery system may convey several advantages over a direct administration of the drug, such as an improvement and control over pharmacokinetics and pharmacodynamics, tissue targeting property, decreased toxicity and enhanced drug activity. An example of such success is liposome-encapsulated form of a small molecule chemotherapy agent doxorubicin (Doxil: a pegylated liposome-encapsulated form of doxorubicin; Myocet: a non-pegylated liposomal doxorubicin), which have been approved for clinical use.

Chopdey et al. (Glycyrrhizin Conjugated Dendrimer and Multi-Walled Carbon Nanotubes for Liver Specific Delivery of Doxorubicin. J Nanosci Nanotechnol. 2015 Feb;15(2):1088-100) reports the results of an investigation into the drug targeting potential of glycyrrhizin (GL) conjugated dendrimers (GLP-PPI) and multi walled carbon nanotubes (GL-MWCNTs) towards liver targeting of doxorubicin, as a model anti-cancer agent.

US2004/242502 discloses certain saponin derivatives for use with nucleic acids that induce an immune response when administered to animals and humans. The novel saponin derivatives disclosed comprise (a) a saponin aglycone core, wherein the aglycone core is covalently linked to one or more oligosaccharide chains; (b) a positively charged cationic chain, and optionally (c) a naturally occurring or synthetic lipophilic chain.

A solution still needs to be found that allows for drug therapies such as anti-tumor therapies, applicable for non-systemic use when desired, wherein the drug has for example an acceptable safety profile, little off-target activity, sufficient efficacy, sufficiently low clearance rate from the patient's body, etc.

### SUMMARY

For an embodiment of the present invention, it is a first goal to provide an improved biologically active compound or composition comprising such improved biologically active compound.

It is one of several objectives of embodiments of the current invention to provide a solution to the problem of non-specificity, encountered when administering small-molecule therapeutically active compounds to a human patient in need thereof. It is one of several objectives of embodiments of the current invention to provide a solution to the problem of drugs with non-optimal specificity for a biological factor or biological process driving a disease. It is one of several objectives of embodiments of the current invention to provide a solution to the problem of insufficient safety characteristics of current drugs, when administered to human patients in need thereof. It is one of several objectives of embodiments of the current invention to provide a solution to the problem of current drugs being less efficacious than desired, when administered to human patients in need thereof. It is one of several objectives of embodiments of the current invention to provide a solution to the problem of current drugs being not sufficiently directed to the diseased cell with little to no off-target activity on non-diseased cells, when administered to human patients in need thereof. It is one of several objectives of embodiments of the current invention to provide a solution to the problem that current drugs do not have a sufficiently timely mode of action (e.g. the administered drug molecule should reach the targeted site in the human patient within a certain time frame and should remain at the targeted site for a certain time frame), when administered to human patients in need thereof. It is one of several objectives of embodiments of the current invention to provide a solution to the problem that current drugs have not sufficiently long lasting therapeutic activity in the patient's body, when administered to human patients in need thereof.

At least one of the above objectives of embodiments of the invention is achieved by providing a molecular scaffold suitable for covalently binding at least one biologically active molecule such as a therapeutic molecule to a carrier molecule as defined in the claims.

The present invention will be described with respect to particular embodiments but the invention is not limited thereto but only by the claims. The embodiments of the invention described herein can operate in combination and cooperation, unless specified otherwise.

An aspect of the current invention relates to a scaffold, as defined in the claims, suitable for covalently binding at least one biologically active molecule to a carrier molecule, the scaffold comprising a polymeric or oligomeric structure and at least one of said biologically active molecules covalently bound to said polymeric or oligomeric structure, wherein the scaffold further comprises a first chemical group for covalently coupling of the scaffold to the carrier molecule.

An embodiment is the scaffold of the invention, wherein the at least one biologically active molecule has a molecular mass of 3.000 Dalton or less, preferably 2.500 Dalton or less, more preferably 2.300 Dalton or less, most preferably, 2.000 Dalton or less, such as 1.700 Dalton - 1.950 Dalton.

An embodiment is the scaffold of the invention, wherein the at least one biologically active molecule is a single specific molecule or is a mixture of different molecules, when more than one biologically active molecules are covalently bound to the polymeric or oligomeric structure comprised by the scaffold.

An embodiment is the scaffold of the invention, wherein the saponin is a saponin that can be isolated from *a Gypsophila* species and/or a *Saponaria* species and/or an *Agrostemma* species and/or *a Quillaja* species such as *Quillaja saponaria* or is a single specific saponin or is a mixture of two or more different saponins, such as one or more of the saponins in Table A1 or Scheme I, SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, or any of their stereomers and/or any combinations thereof, preferably the saponin is SO1861 and/or GE1741 and/or SA1641 and/or QS-21 and/or saponin with a quillaic acid aglycon core, a Gal-(1→2)-[Xyl-(1→3)]-GlcA carbohydrate substituent at the C-3beta-OH group and a Glc-(1→3)-Xyl-(1→4)-Rha-(1→2)-[Xyl-(1→3)-4-OAc-Qui-(1→4)]-Fuc carbohydrate substituent at the C-28-OH group, and/or is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4-OAc-beta-D-quinovopyranosyl-(1→4)]-beta-D-fucopyranoside, more preferably the saponin is SO1861 and/or QS-21.

An embodiment is the scaffold of the invention, wherein the at least one biologically active molecule is covalently bound to the polymeric or oligomeric structure of the scaffold via a cleavable bond, wherein said cleavable bond is subject to cleavage *in vivo* under acidic conditions as present in endosomes and/or lysosomes of mammalian cells, preferably human cells, preferably at pH 4.0 - 6.5, and more preferably at pH ≤ 5.5.

An embodiment is the scaffold of the invention, wherein the at least one biologically active molecule is a defined number of glycoside molecules or a defined range of glycoside molecules, preferably 1-128 or at least 2, 3, 4, 5, 6, 8, 10, 16, 32, 64 or 128 glycoside molecules, or any number of glycoside molecules therein between, such as 7, 9, 12 glycoside molecules.

An embodiment is the scaffold of the invention, wherein the polymeric or oligomeric structure comprises a linear, branched and/or cyclic polymer, oligomer, dendrimer, dendron, dendronized polymer, dendronized oligomer, a DNA, a polypeptide, a poly-lysine, a poly-ethylene glycol, or an assembly of these polymeric or oligomeric structures which assembly is preferably built up by covalent cross-linking.

An embodiment is the scaffold of the invention, wherein the carrier molecule comprises or consists of an immunoglobulin, at least one binding domain of an immunoglobulin and/or at least one binding fragment of an immunoglobulin, such as an antibody, an IgG, a molecule comprising or consisting of a Vhh domain or Vh domain, a Fab, an scFv, an Fv, a dAb, an F(ab)₂, Fcab fragment, or comprises or consists of at least one non-proteinaceous ligand and/or at least one proteinaceous ligand for binding to a cell-surface molecule such as EGF or a cytokine.

An embodiment is the scaffold of the invention, wherein the carrier molecule comprises or consists of at least one effector molecule, or wherein the carrier further comprises at least one effector molecule when the carrier also comprises e.g. an immunoglobulin, wherein the effector molecule is at least one of an active pharmaceutical substance, such as any one or more of a payload, a toxin, a drug, a polypeptide, an oligonucleotide, a nucleic acid, a xeno nucleic acid, an enzyme such as urease and Cre-recombinase, a protein toxin, a ribosome-inactivating protein.

An aspect of the invention relates to a method for producing a scaffold suitable for covalently binding at least one biologically active molecule to a carrier molecule as defined in the claims, the method comprising: a) providing a polymeric or oligomeric structure comprising a first chemical group for covalently coupling of the polymeric structure or the oligomeric structure to the carrier molecule and comprising at least one of a second chemical group different from the first chemical group, wherein each second chemical group is for covalently coupling one of the at least one biologically active molecules to the oligomeric or polymeric structure; and b) covalently coupling at least one biologically active molecule to said polymeric or oligomeric structure via the second chemical group(s), wherein preferably the biologically active molecule(s) is/are any one of the biologically active molecules of the invention such as a saponin, a triterpenoid saponin, a bisdesmosidic triterpene, more preferably SO1861 and/or GE1741 and/or SA1641 and/or QS-21, therewith providing the scaffold.

An aspect of the invention relates to a method for producing a scaffold covalently bound to a carrier molecule as defined in the claims, the scaffold comprising at least one covalently bound biologically active molecule, the method comprising: a) providing a scaffold comprising at least one biologically active molecule covalently bound to a polymeric or oligomeric structure in said scaffold, preferably providing a scaffold according to the invention or the scaffold obtainable by the method of the invention or the scaffold obtained with the method of the invention; and b) covalently coupling the scaffold of a) to a carrier molecule according to the invention, therewith providing the scaffold covalently bound to a carrier molecule, the scaffold comprising at least one covalently bound biologically active molecule.

An embodiment is the scaffold according to the invention or the method according to the invention, wherein the scaffold is able to augment endosomal escape and/or lysosomal escape of the effector molecule according to the invention when either said effector molecule is covalently bound to the scaffold and contacted with a mammalian cell, or when said effector molecule is contacted with a mammalian cell in the presence of the scaffold.

### DEFINITIONS

The term "linker" has its regular scientific meaning, and here refers to a chemical moiety or a linear stretch of amino-acid residues complexed through peptide bonds, which attaches a molecule or an atom to another molecule, e.g. to a ligand or to an effector molecule or to a scaffold. Typically, the linker comprises a chain of atoms linked by chemical bonds. Any linker molecule or linker technology known in the art can be used in the present disclosure. Where indicated, the linker is a linker for covalently binding of molecules through a chemical group on such a molecule suitable for forming a covalent linkage or bond with the linker. The linker may be a non-cleavable linker, e.g., the linker is stable in physiological conditions. The linker may be a cleavable linker, e.g. a linker that is cleavable, in the presence of an enzyme or at a particular pH range or value, or under physiological conditions such as intracellular conditions in the endosomes such as the late endosomes and the lysosomes of mammalian cells such as human cells. Exemplary linkers that can be used in the context of the present disclosure includes N-ε-maleimidocaproic acid hydrazide (EMCH), succinimidyl 3-(2-pyridyldithio)propionate or 3-(2-Pyridyldithio)propionic acid N-hydroxysuccinimide ester (SPDP), and 1-[Bis(dimethylamino)methylene]-1H-1,2,3-triazolo[4,5-b]pyridinium 3-oxid hexafluorophosphate (HATU).

The term "tri-functional linker" has its regular scientific meaning, and here refers to a linker which attaches three molecules via a chemical group on each of the three molecules. The skilled person is able to design such tri-functional linkers, based on the present disclosure and the common general knowledge. Such tri-functional linker can exhibit, for instance, a maleimido group that can be used for conjugation to targeting ligands that exhibit thiol groups to perform a thiol-ene reaction. In addition, the tri-functional linker could exhibit a dibenzocyclooctyne (DBCO) group to perform the so-called strainpromoted alkyne-azide cycloaddition (SPAAC, click chemistry) with an azido bearing saponin. Finally, the tri-functional linker could obtain a third functional group such as a trans-cyclooctene (TCO) group to perform the so-called inverse electron demand Diels-Alder (IEDDA) reaction with a tetrazine (Tz) bearing effector molecule. The skilled person will appreciate that the chemical groups of the tri-functional linker can be all three the same, or different, or the linker may comprise two of the same chemical groups for linking a molecule to the tri-functional linker. The formed bonds between the tri-functional linker can be covalent or non-covalent, and covalent bonds are preferred. The formed bonds between the tri-functional linker and the one or two or three bound molecules via respective chemical groups, can be cleavable (labile) bonds, such as cleavable under acidic conditions inside cells such as endosomes and lysosomes of mammalian cells such as human cells, or can be non-cleavable bonds. Of course, the tri-functional linker may encompass one or two chemical groups for forming covalent bonds while the further two or one chemical group(s), respectively, are/is for forming a non-covalent bond. Of course, the tri-functional linker may encompass one or two chemical groups for forming cleavable bonds while the further two or one chemical group(s), respectively, are/is for forming a non-cleavable bond.

The term "cleavable", such as used in the term "cleavable linker" or "cleavable bond" has its regular scientific meaning, and here refers to being subject to cleavage under conditions such as acidic conditions, reductive conditions, enzymatic conditions or light-induced conditions. For example, a cleavable linker may be subject to cleavage under acidic conditions, preferably said cleavable linker is subject to cleavage in vivo under acidic conditions as present in endosomes and/or lysosomes of mammalian cells, preferably human cells, preferably at pH 4.0 - 6.5, and more preferably at pH ≤ 5.5. As another example, a cleavable linker may be subject to cleavage by an enzyme, e.g. by cathepsin such as Cathepsin B. Furthermore, an example of a covalent bond cleavable under reductive conditions is a disulphide bond.

The terms "oligomer" and "polymer" in the context of an oligomeric or polymeric scaffold has its regular scientific meaning. A polymer here refers to a substance which has a molecular structure built up chiefly or completely from a large number of equal or similar units bonded together; an oligomer here refers to a polymer whose molecules consist of relatively few repeating units. For example, a structure comprising 5-10 or less equal or similar units, may be called an oligomeric structure, whereas a structure comprising 10-50 monomeric units or more may be called a polymeric structure, whereas a structure of 10 monomeric units may be called either oligomeric or polymeric.

The term "binding site" has its regular scientific meaning, and here refers to a region or an epitope on a molecule, e.g. a protein, DNA or RNA, to which another molecule can bind.

The term "scaffold" has its regular scientific meaning, and here refers to an oligomeric or polymeric template or a carrier or a base (base molecule or base structure), to which one or more molecules, e.g. ligand molecule, effector molecule, can be covalently bound, either directly, or via a linker, such as a cleavable linker. A scaffold may have a structurally ordered formation such as a polymer, oligomer, dendrimer, dendronized polymer, or dendronized oligomer or have an assembled polymeric structure such as a hydrogel, microgel, nanogel, stabilized polymeric micelle or liposome, but excludes structures that are composed of non-covalent assemblies of monomers such as cholesterol/phospholipid mixtures. A scaffold may comprise a polymeric or oligomeric structure, such as poly- or oligo(amines), e.g., polyethylenimine and poly(amidoamine); or structures such as polyethylene glycol, poly- or oligo(esters), such as poly(lactids), poly(lactams), polylactide-co-glycolide copolymers; or poly(dextrin), poly- or oligosaccharides, such as cyclodextrin or polydextrose; or structures such as natural and/or artificial poly- or oligoamino acids such as poly-lysine or a peptide or a protein, DNA oligo- or polymers, stabilized RNA polymers or PNA (peptide nucleic acid) polymers. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism.

The term "ligand" has its regular scientific meaning, and here refers to any molecule or molecules which may selectively bind to a target cell-surface molecule or target cell-surface receptor expressed at target cells, e.g. target cancer cells or target auto-immune cells. The ligand may bind to an epitope comprised by receptors or other antigens on the target cells. Preferably, the cell-binding ligands are antibodies.

The term "antibody" as used herein is used in the broadest sense, which may refer to an immunoglobulin (Ig) defined as a protein belonging to the class IgG, IgM, IgE, IgA, or IgD (or any subclass thereof), or a functional binding fragment or binding domain of an immunoglobulin. In the context of the present invention, a "binding fragment" or a "binding domain" of an immunoglobulin is defined as antigen-binding fragment or -domain or other derivative of a parental immunoglobulin that essentially maintains the antigen binding activity of such parental immunoglobulin. Functional fragments and functional domains are antibodies in the sense of the present invention even if their affinity to the antigen is lower than that of the parental immunoglobulin. "Functional fragments and -domains" in accordance with the invention include F(ab')2 fragments, Fab' fragments, Fab fragments, scFv, dsFv, single-domain antibody (sdAb), monovalent IgG, scFv-Fc, reduced IgG (rlgG), minibody, diabodies, triabodies, tetrabodies, Fc fusion proteins, nanobodies, variable V domains such as VHH, Vh, and other types of antigen recognizing immunoglobulin fragments and domains. The fragments and domains may be engineered to minimize or completely remove the intermolecular disulphide interactions that occur between the CH1 and CL domains. Functional fragment and -domains offer the advantage of greater tumor penetration because of their smaller size. In addition, the functional fragment or -domain can be more evenly distributed throughout the tumor mass as compared to whole immunoglobulin.

The antibodies (immunoglobulins) of the present invention may be bi- or multifunctional. For example, a bifunctional antibody has one arm having a specificity for one receptor or antigen, while the other arm recognizes a different receptor or antigen. Alternatively, each arm of the bifunctional antibody may have specificity for a different epitope of the same receptor or antigen of the target cell.

The antibodies (immunoglobulins) of the present invention may be polyclonal antibodies, monoclonal antibodies, human antibodies, humanized antibodies, chimeric antibodies, resurfaced antibodies, anti-idiotypic antibodies, mouse antibodies, rat antibodies, rat/mouse hybrid antibodies, llama antibodies, llama heavy-chain only antibodies, heavy-chain only antibodies, and veterinary antibodies. Preferably, the antibody (immunoglobulin) of the present invention is a monoclonal antibody. The resurfaced, chimeric, humanized and fully human antibodies are also more preferred because they are less likely to cause immunogenicity in humans. The antibodies of the ADC of the present invention preferably specifically binds to an antigen expressed on the surface of a cancer cell, an autoimmune cell, a diseased cell, an aberrant cell, while leaving any healthy cell essentially unaltered (e.g. by not binding to such normal cell, or by binding to a lesser extent in number and/or affinity to such healthy cell).

Specific antibodies that can be used for the ADCs of the present invention include anti-HER2 monoclonal antibody such as trastuzumab and pertuzumab, anti-CD20 monoclonal antibody such as rituximab, ofatumumab, tositumomab and ibritumomab, anti-CA125 monoclonal antibody such as oregovomab, anti-EpCAM (17-1A) monoclonal antibody such as edrecolomab, anti-EGFR monoclonal antibody such as cetuximab, panitumumab and nimotuzumab, anti-CD30 monoclonal antibody such brentuximab, anti-CD33 monoclonal antibody such as gemtuzumab and huMy9-6, anti-vascular integrin alpha-v beta-3 monoclonal antibody such as etaracizumab, anti-CD52 monoclonal antibody such as alemtuzumab, anti-CD22 monoclonal antibody such as epratuzumab, anti-CEA monoclonal antibody such as labetuzumab, anti-CD44v6 monoclonal antibody such as bivatuzumab, anti-FAP monoclonal antibody such as sibrotuzumab, anti-CD19 monoclonal antibody such as huB4, anti-CanAg monoclonal antibody such as huC242, anti-CD56 monoclonal antibody such huN901, anti-CD38 monoclonal antibody such as daratumumab, anti-CA6 monoclonal antibody such as DS6, anti-IGF-IR monoclonal antibody such as cixutumumab and 3B7, anti-integrin monoclonal antibody such as CNTO 95, and antisyndecan-1 monoclonal antibody such as B-B4.

Any other molecules than antibodies that bind to a cell receptor or antigen of a target cell can also be used as the cell-binding ligand for the ligand-drug conjugates of the present invention and the ligands provided with covalently bound saponin according to the invention. These ligands include proteins, polypeptides, peptides, small molecules. Examples of these non-antibody ligands are interferons (e.g. IFN-α, IFN-β, and IFN-γ), transferrins, lectins, epidermal growth factors (EGF) and EGF-like domains, gastrin-releasing peptides (GRP), platelet-derived growth factors (PDGF), transforming growth factors (TGF), vaccinia growth factor (VGF), insulin and insulin-like growth factors (IGF, e.g. IGF-1 and IGF-2), other suitable hormones such as thyrotropin releasing hormones (TRH), melanocyte-stimulating hormones (MSH), steroid hormones (e.g. estrogen and androgen), somatostatin, lymphokines (e.g. IL-2, IL-3, IL-4, and IL-6), colony-stimulating factors (CSF, e.g. G-CSF, M-CSF and GM-CSF), bombesin, gastrin, Arg-Gly-Asp or RGD, aptamers (e.g. AS-1411, GBI-10, RNA aptamers against HIV glycoprotein), small molecules (e.g. folate, anisamide phenylboronic acid), vitamins (e.g., vitamin D), carbohydrates (e.g. hyaluronic acid, galactose).

An "effector molecule" or "effector moiety" or "payload" has its regular scientific meaning and in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol.

The effector molecule or -moiety is a pharmaceutically active substance, such as a toxin such as a proteinaceous toxin, a drug, a polypeptide or a polynucleotide. A pharmaceutically active substance in this invention is an effector molecule or -moiety that is used to achieve a beneficial outcome in an organism, preferably a vertebrate, more preferably a mammal such as non-human subjects or a human being/subject. Benefits include diagnosis, prognosis, treatment, cure and prevention (prophylaxis) of diseases and/or symptoms and/or health problems. The pharmaceutically active substance may also lead to undesired and sometimes even harmful side effects (adverse events such as observed during clinical trials). In this case, pros and cons must be weighed to decide whether the pharmaceutically active substance is suitable in the particular case. If the effect of the pharmaceutically active substance inside a cell is predominantly beneficial for the organism as a whole, the cell is called a target cell. If the effect inside a cell is predominantly harmful for the organism as a whole, the cell is called an off-target cell. In artificial systems such as cell cultures and bioreactors, target cells and off-target cells depend on the purpose and are defined by the user. Examples of effector molecules and -moieties are a drug, a toxin, a polypeptide (such as an enzyme), a polynucleotide (including polypeptides and polynucleotides that comprise non-natural amino acids or nucleic acids), and any combination thereof.

An effector molecule or effector moiety that is a drug may include anti-cancer agents, antiinflammatory agents, and anti-infective (e.g., anti-fungal, antibacterial, anti-parasitic, anti-viral) agents. Preferably, the drug molecule of the present invention is an anti-cancer agent or an anti-auto-immune agent. Suitable anti-cancer agents include alkylating agents, antimetabolites, spindle poison plant alkaloids, cytotoxic/antitumor antibiotics, topoisomerase inhibitors, photosensitizers, and kinase inhibitors. Also included in the definition of "anti-cancer agent" are: e.g. (i) anti-hormonal agents that act to regulate or inhibit hormone action on tumors such as anti-estrogens and selective estrogen receptor modulators; (ii) aromatase inhibitors that inhibit the enzyme aromatase, which regulates estrogen production in the adrenal glands; (iii) anti-androgens; (iv) protein kinase inhibitors; (v) lipid kinase inhibitors; (vi) antisense oligonucleotides, particularly those which inhibit expression of genes in signaling pathways implicated in aberrant cell proliferation; (vii) ribozymes such as VEGF expression inhibitors and HER2 expression inhibitors; (viii) vaccines such as gene therapy vaccines; topoisomerase 1 inhibitors; (ix) anti-angiogenic agents; and pharmaceutically acceptable salts, acids, solvates and derivatives of any of the above.

An effector molecule or -moiety that is a toxin may include proteinaceous toxins (e.g. bacterial-derived toxins, and plant-derived toxins), toxins targeting tubulin filaments, toxins targeting DNA, toxins targeting RNA. Examples of proteinaceous toxins are saporin, dianthin, ricin, modeccin, abrin, volkensin, viscumin, shiga toxin, shiga-like toxin, pseudomonas exotoxin (PE, also known as exotoxin A), diphtheria toxin (DT), and cholera toxin. Examples of tubulin filaments-targeting toxins are maytansinoids (e.g. DM1 and DM4), auristatins (e.g. Monomethyl auristatin E (MMAE) and Monomethyl auristatin F (MMAF)), toxoids, tubulysins, cryptophycins, rhizoxin. Examples of DNA-targeting toxins are calicheamicins: N-Acetyl- γ-calicheamicin, CC-1065 analogs, duocarmycins, doxorubicin, methotrexate, benzodiazepines, camptothecin analogues, and anthracyclines. Examples of DNA-targeting toxins are amanitins, spliceostatins, and thailanstatins. A toxin, as used in this invention, is defined as a pharmaceutically active substance that is able to kill or inactivate a cell. Preferably, a targeted toxin is a toxin that is only, or at least predominantly, toxic for target cells but not for off-target cells. The net effect of the targeted toxin is preferably beneficial for the organism as a whole.

An effector molecule or -moiety that is a polypeptide may be, e.g., a polypeptide that recover a lost function, such as for instance enzyme replacement, gene regulating functions, or a toxin. Examples of polypeptides as effector molecules are, e.g., Cas9; toxins (e.g. saporin, dianthin, gelonin, (de)bouganin, agrostin, ricin (toxin A chain); pokeweed antiviral protein, apoptin, diphtheria toxin, pseudomonas exotoxin) metabolic enzymes (e.g. argininosuccinate lyase, argininosuccinate synthetase), enzymes of the coagulation cascade, repairing enzymes; enzymes for cell signaling; cell cycle regulation factors; gene regulating factors (transcription factors such as NF-κB or gene repressors such as methionine repressor).

An effector molecule or an effector moiety that is a polynucleotide may, e.g., be a polynucleotide that comprises coding information, such as a gene or an open reading frame encoding a protein. It may also comprise regulatory information, e.g. promotor or regulatory element binding regions, or sequences coding for micro RNAs. Such polynucleotide may comprise natural and artificial nucleic acids. Artificial nucleic acids include, e.g. peptide nucleic acid (PNA), Morpholino and locked nucleic acid (LNA), as well as glycol nucleic acid (GNA) and threose nucleic acid (TNA). Each of these is distinguished from naturally occurring DNA or RNA by changes to the backbone of the molecule. Examples of nucleotides as effector molecules are, e.g., DNA: single stranded DNA (e.g. DNA for adenine phosphoribosyltransferase); linear double stranded DNA (e.g. clotting factor IX gene); circular double stranded DNA (e.g. plasmids); RNA: mRNA (e.g. TAL effector molecule nucleases), tRNA, rRNA, siRNA, miRNA, antisense RNA; anti-sense oligonucleotides (ASOs, AONs e.g. PNA, PMO, LNA and BNA).

The term "proteinaceous", used in e.g. "proteinaceous molecule" and "proteinaceous toxin", are molecules and toxins comprising at least a string of amino acid residues that can be obtained as an expression product from a single mRNA. Such a molecule or toxin may further comprise any post-translational modifications, a carbohydrate such as an N- or O-linked carbohydrate, disulphide bonds, phosphorylations, sulphatations, etc., as a result of any post-translational modification, and/or may further comprise any other modification such as those resulting from chemical modifications (e.g., linking of effector moieties, saponin, scaffolds, ligands, etc., either directly to e.g. an amino-acid side chain, or via at least one linker (covalently) bound to the molecule for chemically modifying the proteinaceous molecule, and chemically bound (covalently) to the proteinaceous molecule). The term "proteinaceous" also encompasses and includes assemblies of such molecules, e.g. homodimers, heterotrimers, heterohexamers or complex assemblies such as ribosomes.

The terms "specific" and "specifically", in the context of for example "specific binding" and "receptor or molecular target specifically present or expressed at the surface of a tumor cell" and the like, have their normal scientific meaning known in the art, and here refer to e.g. a binding interaction of a first molecule with a second molecule which occurs with a higher affinity relative to any putative binding of the first molecule to a further molecule different from the second molecule, or e.g. to the expression or expression to a higher extent when e.g. the number of receptors or molecular targets is considered, of a cell-surface receptor or molecular target on the surface of a first type of cell such as a tumor cell, autoimmune cell, diseased cell, aberrant cell, relative to the extent of expression of the same receptor or molecular target at a second type of cell such as a healthy cell, etc., wherein expression at the second type of cell can be fully absent or very low, relative to any extent of expression on the tumor cell, etc. Furthermore, the term "specific", for example in "specific binding", has its normal scientific meaning known in the art, and here has the meaning of indicating a molecule that can have an interaction with another molecule with higher binding affinity than background interactions between molecules. Similarly, the term "specificity" refers to an interaction, for example, between two molecules or between a cell and a molecule, which has higher binding affinity than background interactions between molecules. Binding molecules such as immunoglobulins bind via their binding site such as immunoglobulin variable regions of the immunoglobulin, to binding sites on molecules, such as epitopes, cell-surface receptors, etc., with a higher binding affinity than background interactions between molecules. In the context of the invention, background interactions are typically interactions with an affinity lower than a K_{D} of 10E-4 M. Similarly, "specific binding domains" are domains that preferentially bind to binding sites on molecules, such as epitopes, cell-surface receptors, etc., with a higher binding affinity than background interactions between molecules. In the context of the invention, "background interactions" are typically interactions with an affinity lower than a K_{D} of 10E-4 M. Preferably, specific binding domains bind with an affinity higher than a K_{D} of about 10E-5 M.

The term "binding" is defined as interactions between molecules that can be distinguished from background interactions.

Throughout the specification, the term "fragment" refers to an amino acid sequence which is part of a protein domain or which builds up an intact protein domain. Binding fragments according to the invention must have binding specificity for the respective target such as a cell-surface receptor, e.g. on the surface of a diseased cell such as a tumor cell.

The term "ADC" or "antibody-drug conjugate" has its regular scientific meaning known to the skilled person, and here refers to a class of biopharmaceutical drugs designed as a targeted therapy for treating e.g. cancer. Unlike chemotherapy, ADCs are intended to target and kill tumor cells while sparing healthy cells. ADCs are composed of an antibody linked to a biologically active cytotoxic (anticancer) payload or drug. ADCs combine the targeting capabilities of monoclonal antibodies with the cancerkilling ability of cytotoxic drugs. They are designed with the intention to discriminate between healthy cells and diseased tissue such as tumor cells in a tumor.

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillajasaponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (~63%), QS-21 A-xylo (~32%), QS-21 B-apio (~3.3%), and QS-21 B-xylo (~1.7%).

Similarly, "QS-21A" has its regular scientific meaning and here refers to a mixture of QS-21 A-apio (~65%) and QS-21 A-xylo (~35%).

Similarly, "QS-21B" has its regular scientific meaning and here refers to a mixture of QS-21 B-apio (~65%) and QS-21 B-xylo (~35%).

The term "Quil-A" refers to a commercially available semi-purified extract from Quillaja saponaria and contains variable quantities of more than 50 distinct (water-soluble) saponins, many of which incorporate the triterpene-trisaccharide substructure Gal-(1→2)-[Xyl-(1→3)]-GlcA- at the C-3beta-OH group found in QS-7, QS-17, QS18, and QS-21. The saponins found in Quil-A are listed in van Setten (1995), Table 2 [Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillajasaponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et al. (2019) in Table 1, row no. 28 [Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171*].* The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms first, second, third in the description and in the claims, are used for distinguishing between similar elements and not necessarily for describing a sequential or chronological order. The terms are interchangeable under appropriate circumstances. The embodiments of the invention can operate in other sequences than described or illustrated herein.

Furthermore, the various embodiments, although referred to as "preferred" or "e.g." or "for example" or "in particular" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a pharmaceutical composition comprising A and B" should not be limited to a pharmaceutical composition consisting only of components A and B, rather with respect to the present invention, the only enumerated components of the pharmaceutical composition are A and B, and further the claim should be interpreted as including equivalents of those components. Similarly, the scope of the expression "a method comprising step A and step B" should not be limited to a method consisting only of steps A and B, rather with respect to the present invention, the only enumerated steps of the method are A and B, and further the claim should be interpreted as including equivalents of those steps.

In addition, reference to a feature by the indefinite article "a" or "an" does not exclude the possibility that more than one of the features such as for example a component, excipient, saponin, etc. are present, unless the context clearly requires that there is one and only one of the features. The indefinite article "a" or "an" thus usually means "at least one".

### BRIEF DESCRIPTION OF THE DRAWINGS

Figure 1: *in vivo* HSP27 expression in A431 xenograph 'nude' mouse tumor model treated with 30mg/kg cetuximab-Cys-(SO1861-L-trifunctional linker-L-HSP27BNA), 25 mg/kg Cetuximab-(Lys-L-HSP27BNA)⁴ or 25 mg/kg Cetuximab-(Cys-L-SO1861).
Figure 2: *in vitro* enhanced HSP27 gene silencing in EGFR expressing A431 cells by treatment with cetuximab-Cys-(SO1861-L-trifunctional linker-L-HSP27BNA), Cetuximab-(Lys-L-HSP27BNA)⁴ or Cetuximab-(Cys-L-SO1861).

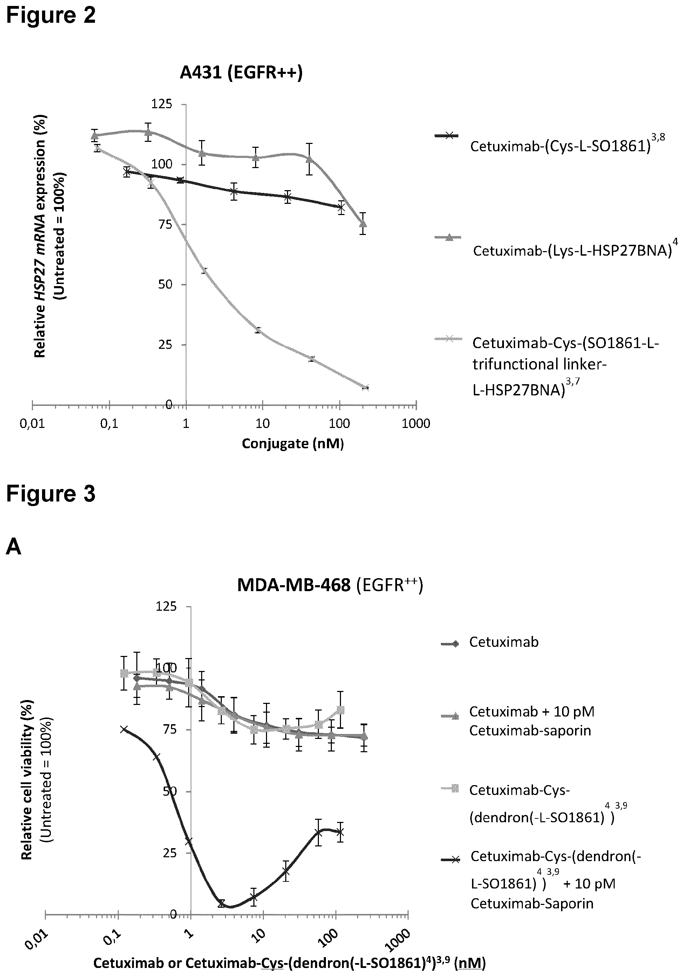

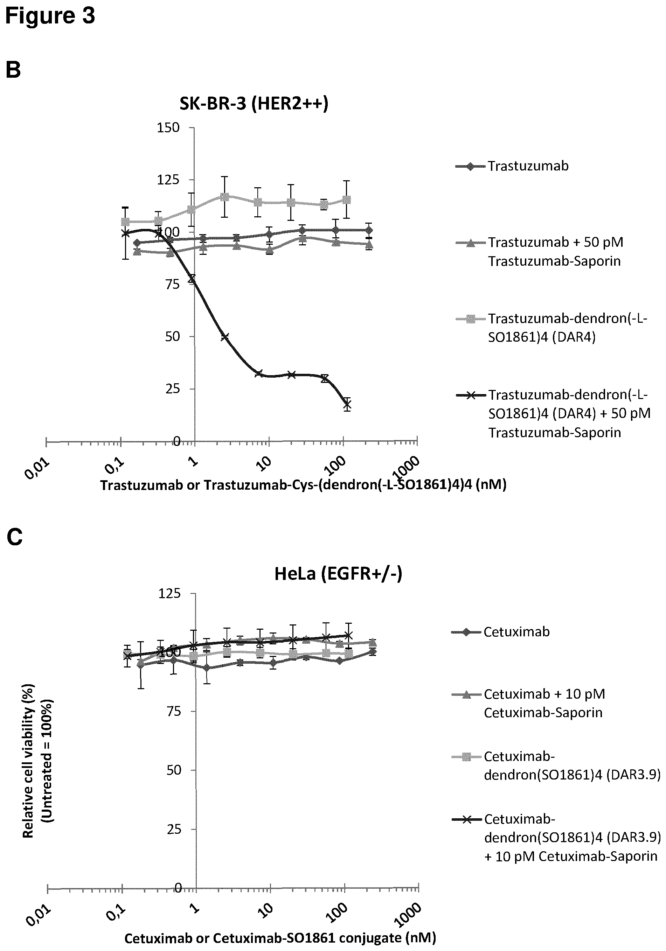

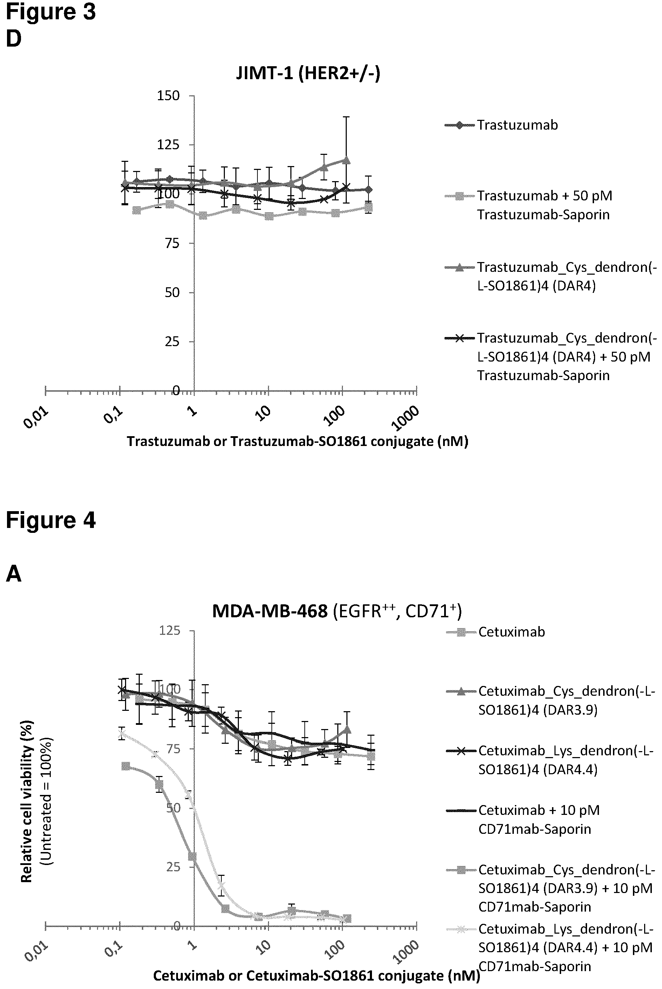

Figure 3: The legends and axes for Figures A, B, C and D are the same. **A.** cell killing activity in EGFR expressing cells (MDA-MB-468) by cetuximab, cetuxamib + 10 pM cetuximab-saporin, cetuximab-Cys-(dendron(-L-SO1861)⁴)^{3,9} and cetuximab-Cys-(dendron(-L-SO1861)⁴)^{3,9} + 10pM cetuximab-saporin. **B.** cell killing activity in HER2 expressing cells (SK-BR-3) by trastuzumab, trastuzumab + 50 pM trastuzumab-saporin, Trastuzumab-Cys-(dendron(-L-SO1861)⁴)⁴ and Trastuzumab-Cys-(dendron(-L-SO1861)⁴)⁴ + 50 pM trastuzumab-saporin. **C.** cell killing activity in EGFR expressing cells (HeLa) by cetuximab, cetuxamib + 10 pM cetuximab-saporin, cetuximab-Cys-(dendron(-L-SO1861)⁴)^{3,9} and cetuximab-Cys-(dendron(-L-SO1861)⁴)^{3,9} + 10pM cetuximab-saporin. **D.** cell killing activity in HER2 expressing cells (JIMT-1) by trastuzumab, trastuzumab + 50 pM trastuzumab-saporin, Trastuzumab-Cys-(dendron(-L-SO1861)⁴)⁴ and Trastuzumab-Cys-(dendron(-L-SO1861)⁴)⁴ + 50 pM trastuzumab-saporin.

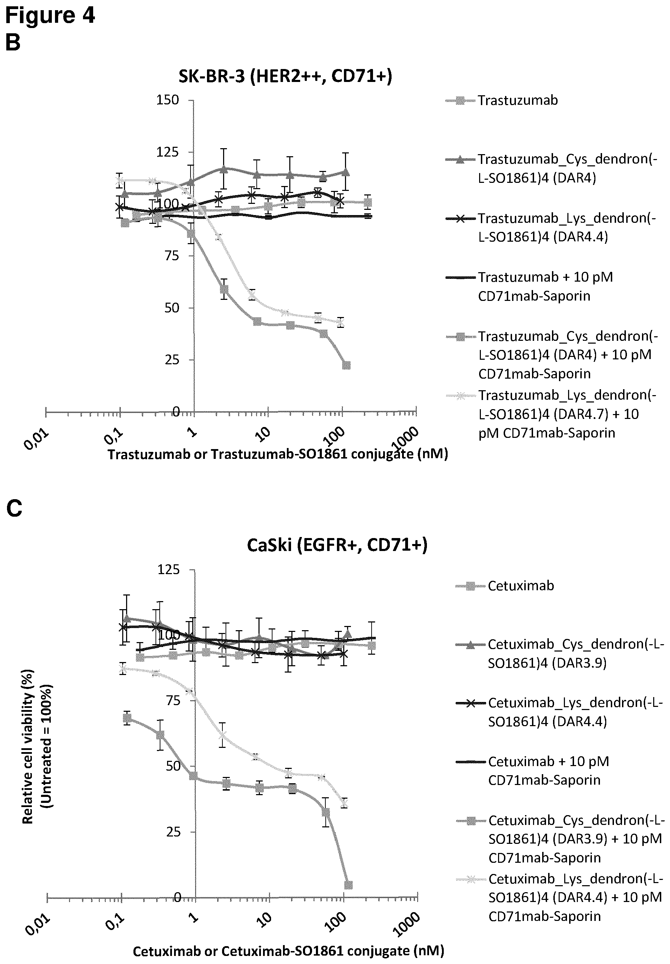

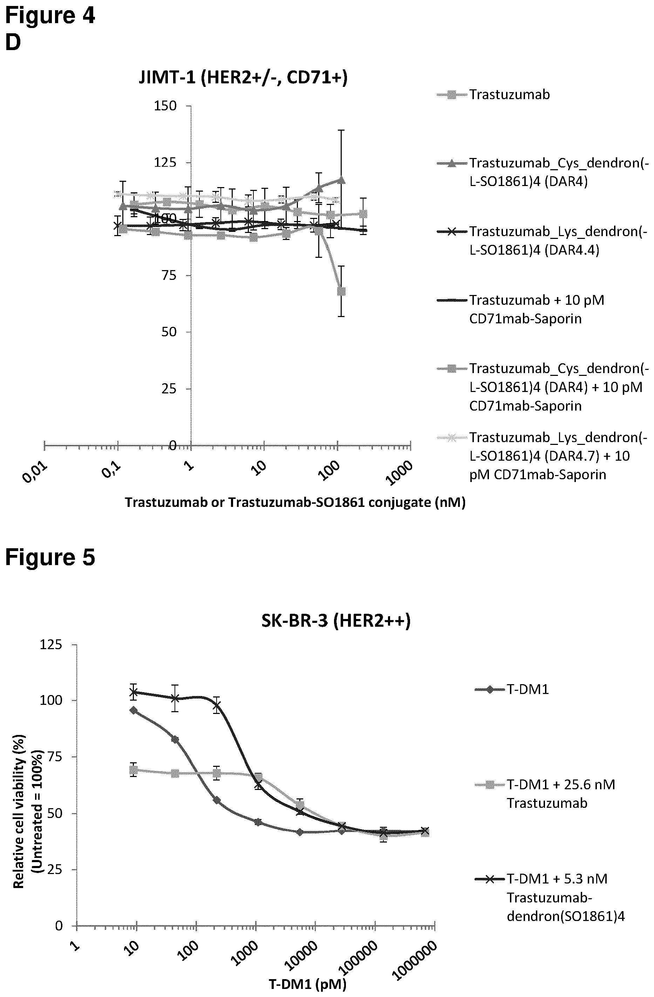

Figure 4: The legends and axes for Figures A, B, C and D are the same. **A.** cell killing activity in EGFR⁺⁺/CD71⁺ cells (MDA-MB-468) of cetuximab, cetuximab + 10 pM CD71mab-saporin , Cetuximab-Cys-(dendron(-L-SO1861)⁴)^{3,9}, Cetuximab-Cys-(dendron(-L-SO1861)⁴)^{3,9} + 10 pM CD71mab-saporin , Cetuximab-Lys-(dendron(-L-SO1861)⁴)^{4,4} or Cetuximab-Lys-(dendron(-L-SO1861)⁴)^{4,4} + 10 pM CD71mab-saporin. **B.** cell killing activity in HER2⁺⁺/CD71⁺ (SK-BR-3) cell lines of trastuzumab, trastuzumab + 10 pM CD71mab-saporin , trastuzumab-Cys-(dendron(-L-SO1861)⁴)⁴, trastuzumab-Cys-(dendron(-L-SO1861)⁴)⁴ + 10 pM CD71mab-saporin , trastuzumab-Lys-(dendron(-L-SO1861)⁴)^{4,7} or trastuzumab-Lys-(dendron(-L-SO1861)⁴)^{4,7} + 10 pM CD71mab-saporin. C. cell killing activity in EGFR⁺/CD71⁺ cells (CaSki) of cetuximab, cetuximab + 10 pM CD71mab-saporin , Cetuximab-Cys-(dendron(-L-SO1861)⁴)^{3,9}, Cetuximab-Cys-(dendron(-L-SO1861)⁴)^{3,9} + 10 pM CD71mab-saporin , Cetuximab-Lys-(dendron(-L-SO1861)⁴)^{4,4} or Cetuximab-Lys-(dendron(-L-SO1861)⁴)^{4,4} + 10 pM CD71mab-saporin. **D.** cell killing activity in HER2⁺¹⁻/CD71⁺ cells (JIMT-1) of trastuzumab, trastuzumab + 10 pM CD71mab-saporin , trastuzumab-Cys-(dendron(-L-SO1861)⁴)⁴, trastuzumab-Cys-(dendron(-L-SO1861)⁴)⁴ + 10 pM CD71mab-saporin , trastuzumab-Lys-(dendron(-L-SO1861)⁴)^{4,7} or trastuzumab-Lys-(dendron(-L-SO1861)⁴)^{4,7} + 10 pM CD71mab-saporin.
Figure 5: cell killing activity in HER2 expressing cells (SK-BR-3) of T-DM1, T-DM1 + 25.6 nM trastuzumab or T-DM1 + 5.3 nM trastuzumab-Cys-(dendron(-L-SO1861)⁴)⁴.
Figure 6: HSP27 gene silencing activity of HSP27BNA-dendron(-L-SO1861)⁴ compared to the HSP27BNA alone.
Figure 7: **A.** Schematic representation of release of SO1861 from dendron(-L-SO1861)⁴ under acidic conditions. **B.** UPLC UV-traces (PDA) of dendron(-L-SO1861)⁴ itself (top), reaction mixture comprising 50 µL of solution containing water/acetonitrile/TFA and dendron(-L-SO1861)⁴ after 30 minutes (middle) and the reaction mixture after 1.5 hours (bottom). **C.** interpretation of the observed m/z values of Figure 7B by LRMS.
Figure 8: The legends and axes for Figures A and B are the same. **A.** cell killing activity in EGFR expressing A431 cells by the 'naked' dendron (Dendron(NEM)⁴), Dendron(NEM)⁴ + 10 pM EGFdianthin, dendron(-L-SO1861)⁴ or dendron(L-SO1861)⁴ + 10 pM EGFdianthin. **B.** cell killing activity in EGFR expressing HeLa cells by the 'naked' dendron (Dendron(NEM)⁴), Dendron(NEM)⁴ + 10 pM EGFdianthin, dendron(-L-SO1861)⁴ or dendron(L-SO1861)⁴ + 10 pM EGFdianthin.

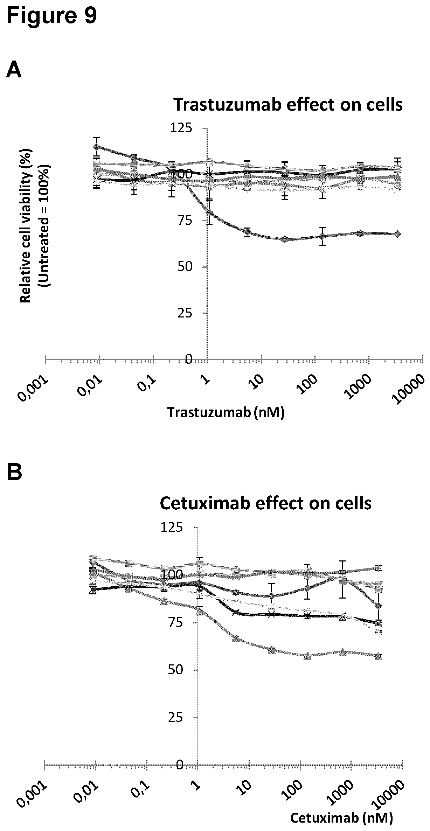

Figure 9: The legends and axes for Figures A, B and C are the same. **A.** Effect of trastuzumab on the cell viability of a range of cancer cells. **B.** Effect of cetuximab on the cell viability of a range of cancer cells. **C.** Effect of T-DM1 on the cell viability of a range of cancer cells.
Figure 10: Schematic representation of the monoclonal antibody-(SO1861-scaffold-antisense BNA oligo) conjugate.
Figure 11: Schematic representation of the 1-target 2-component system using a monoclonal antibody bound to a toxin and the same monoclonal antibody bound to a scaffold comprising saponin.
Figure 12: Schematic representation of the 2-target 2-component system using a monoclonal antibody bound to a toxin and a different monoclonal antibody with a different target bound to a scaffold comprising a saponin.
Figure 13: Reaction scheme of SO1861-EMCH synthesis.
Figure 14: Reaction scheme of of Dendron(-L-SO1861)⁴ synthesis.
Figure 15: Reaction scheme of Dendron(-L-SO1861)⁸ synthesis.
Figure 16: Reaction scheme of the synthesis of the SO1861-L-trifunctional linker-L-HSP27BNA.
Figure 17: Reaction scheme of the synthesis of the Dendron(SO1861)⁴-HSP27BNA oligo conjugate.
Figure 18: Reaction scheme of Dendron(NEM)⁴ ("naked" Dendron) synthesis.
Figure 19: Model scaffold consisting of four molecular arms for saponin binding via a Schiff base (imine) and one arm for click chemistry. The polymeric structure is a pentavalent polyethylene glycolbased dendrimer of the first generation.
Figure 20: The inset shows the theoretically expected mass spectrum of the model scaffold with SA1641 saponin obtained from a calculation with the isotope pattern calculator enviPat Web 2.0. The experimental data obtained by LC-MS/ESI-MS show almost exactly the same peaks at *m*/*z* 758-760 Da proving successful saponin coupling.
Figure 21: Cell viability of HER14 cells after treatment with a pentameric dendrimer (pentrimer), the pentrimer in the presence of SA1641, dianthin-EGF, dianthin-EFG in the presence of SA1641, the pentrimer in presence of dianthin-EGF, and the pentrimer in presence of dianthin-EGF as well as SA1641.
Figure 22: **A.** H-NMR spectrum of SO1861. **B.** H-NMR spectrum of SO1861-EMCH ((EMCH = N-ε-maleimidocaproic acid hydrazide) conjugate.
Figure 23: **A.** MALDI-TOF-MS spectrum of SO1861-EMCH conjugate. **B.** MALDI-TOF-MS spectrum of SO1861-EMCH-mercaptoethanol conjugate.
Figure 24: SO1861 structure with highlighted chemical groups for conjugation of endosomal escape enhancing saponins to a polymeric structure. Highlighted groups are aldehyde (black circle), carboxylic acid (dashed circle), alkene (dashed pentagon), and alcohol (dashed box).
Figure 25: **A.** Schematic representation of the production of stable 'ready-to conjugate' endosomal escape enhancer saponins. **B.** Schematic representation of the production of cleavable 'ready-to conjugate' endosomal escape enhancer saponins.
Figure 26: Hydrolysis of the hydrazone bond of SO1861-EMCH under acidic conditions.
Figure 27: **A.** Standard molecular structure of SO-1861-EMCH conjugate. Maleimide group is marked with a circle. **B.** 3D model of SO1861-EMCH conjugate. Maleimide group is marked with a circle.
Figure 28: **A.** synthesis scheme of SO1861-EMCH. **B.** MALDI-TOF-MS spectrum of SO1861 in negative reflector mode. TFA: trifluoroacetic acid, r.t: room temperature, h: hours, and MW: molecular weight. **C.** MALDI-TOF-MS spectrum of SO1861-EMCH in negative reflector mode. TFA: trifluoroacetic acid, r.t: room temperature, h: hours, and MW: molecular weight.
Figure 29: **A.** MALDI-TOF-MS spectrum of SO1861-EMCH before hydrolysis in HCl solution at pH 3. **B.** MALDI-TOF-MS spectrum of SO1861-EMCH after hydrolysis in HCl solution at pH 3.
Figure 30: Reaction scheme of SO1861-EMCH conjugation to any amine-bearing polymeric structure.
Figure 31: **A.** MALDI-TOF-MS spectrum of BSA-SO1861. **B.** MALDI-TOF-MS spectrum of BSA.
Figure 32: **A.** Reaction scheme of SO1861-EMCH conjugation to a cyanine 3 dye labeled polyamidoamine (PAMAM) G5 dendrimer. **B.** (B) Reaction scheme of SO1861-HATU (HATU = 1-[Bis(dimethylamino)methylene]-1H-1,2,3-triazolo[4,5-b]pyridinium 3-oxid hexafluorophosphate) conjugation to a cyanine 3 dye labeled polyamidoamine (PAMAM) G5 dendrimer.
Figure 33: **A.** MALDI-TOF-MS spectrum of Cy3-PAMAM. **B.** MALDI-TOF-MS spectrum of Cy3-PAMAM-SO1861 with 5 SO1861 attached per PAMAM. **C.** MALDI-TOF-MS spectrum of Cy3-PAMAM-SO1861 with 13 SO1861 attached per PAMAM. **D.** MALDI-TOF-MS spectrum of Cy3-PAMAM-SO1861 51 SO1861 attached per PAMAM.
Figure 34: **A.** MALDI-TOF-MS spectrum of Cy3-PAMAM-SO1861 with 5 equivalents feed SO1861-EMCH. **B.** MALDI-TOF-MS spectrum of Cy3-PAMAM-SO1861 with 30 equivalents feed SO1861-EMCH.
Figure 35: MALDI-TOF-MS spectra of Cy3-PAMAM-NC-SO1861 (NC = stable bond ("non-cleavable").
Figure 36: **A.** Reaction scheme of Cy3-PAMAM-NC-SO1861- Dibenzocyclooctyne (DBCO). **B.** MALDI-TOF MS spectrum of Cy3-PAMAM-NC-SO1861- Dibenzocyclooctyne (DBCO). C. MALDI-TOF-MS spectrum of Cy3-PAMAM-(SO1861)₅-DBCO. D. MALDI-TOF-MS spectrum of Cy3-PAMAM-(SO1861)₂₇-DBCO.
Figure 37: **A.** Reaction scheme of dianthin-EGF-Alexa488. **B.** Reaction scheme of dianthin-EGF-Alexa488-SS-PEG-N₃. **C.** MALDI-TOF-MS spectrum of dianthin-EGF. **D.** MALDI-TOF-MS spectrum of dianthin-EGF-Alexa488. **E.** MALDI-TOF-MS spectrum of dianthin-EGF-Alexa488-SS-PEG-N₃.
Figure 38: **A.** Reaction scheme of dianthin-Alexa488. **B.** Reaction scheme of dianthin-Alexa488-SS-PEG-N₃. **C.** MALDI-TOF-MS spectrum of Dianthin. **D.** MALDI-TOF-MS spectrum of dianthin-Alexa488. **E.** MALDI-TOF-MS spectrum of dianthin-Alexa488-SS-PEG-N₃.
Figure 39: Fluorescence images of SDS-PAGE gel performed on a VersaDoc imaging system. M = marker, P = Cy3-PAMAM-(SO1861)₂₇-DBCO, D = dianthin-EGF-Alexa488-SS-PEG-N₃, C1 = Cy3-PAMAM-(SO1861)₅-Dianthin-EGF-Alexa488, C2 = Cy3-PAMAM-NC-SO1861-Dianthin-EGF-Alexa488, and C3 = Cy3-PAMAM-(SO1861)₂₇-Dianthin-EGF-Alexa488.
Figure 40: **A.** Synthesis scheme of Cy3-PAMAM-NC-SO1861 via reductive amination. **B.** MALDI-TOF-MS spectrum of Cy3-PAMAM-NC-SO1861 synthesized via reductive amination with 10 SO1861 per PAMAM. **C.** MALDI-TOF-MS spectrum of Cy3-PAMAM-NC-SO1861 synthesized via reductive amination with 30 SO1861 per PAMAM.
Figure 41: Reaction scheme for the generation of poly(SO1861) using SO1861-EMCH as monomer, the APS / TMEDA system as polymerization initiator, and aminopropanethiol as radical quencher.
Figure 42: **A.** MALDI-TOF-MS spectrum of poly(SO1861) reaction batch of SO1861-EMCH at 60 °C. **B.** MALDI-TOF-MS spectrum of poly(SO1861) reaction batch of SO1861-EMCH + 11⁻³ equivalents APS at 60 °C. **C.** MALDI-TOF-MS spectrum of poly(SO1861) reaction batch of SO1861-EMCH + 11⁻³ equivalents APS / TMEDA at 60 °C.
Figure 43: Schematic representation of the DNA approach. Usage of the principle of DNA-origami to generate a DNA based scaffold that is able to conjugate and release glycoside molecules. In addition, one of the DNA strands obtains a click chemistry moiety that can be used for conjugation to a targeted toxin to form a functionalized scaffold. bp: base pair.
Figure 44: Schematic representation of the poly(peptide-SO1861) approach. Usage of a peptide sequence that can conjugate and release glycoside molecules and which can react with itself to form a poly(peptide-SO1861) construct. The poly(peptide) chain endings can be further modified with click chemistry moieties (e.g., BCN-NHS linker) that can be used for conjugation to a toxin.
Figure 45: **A.** MALDI-TOF-MS spectrum of native peptide. **B.** MALDI-TOF-MS spectrum of peptide-SO1861 conjugate.
Figure 46: Molecular structure of G4-dendron with protected amino groups.
Figure 47: Synthesis scheme for the generation of dendron based scaffolds.
Figure 48: **A.** Reaction scheme for partial dye labeling and deprotection of the G4-dendron. **B.** MALDI-TOF-MS spectrum of deprotected and partially dye labeled G4-dendron.
Figure 49: **A.** MALDI-TOF-MS spectrum of G4-dendron-SO1861 scaffold with 22 feed equivalents of SO1861-EMCH. **B.** MALDI-TOF-MS spectrum of G4-dendron-SO1861 scaffold with 10 feed equivalents of SO1861-EMCH. C. MALDI-TOF-MS spectrum of G4-dendron-SO1861 scaffold with 3 feed equivalents of SO1861-EMCH.

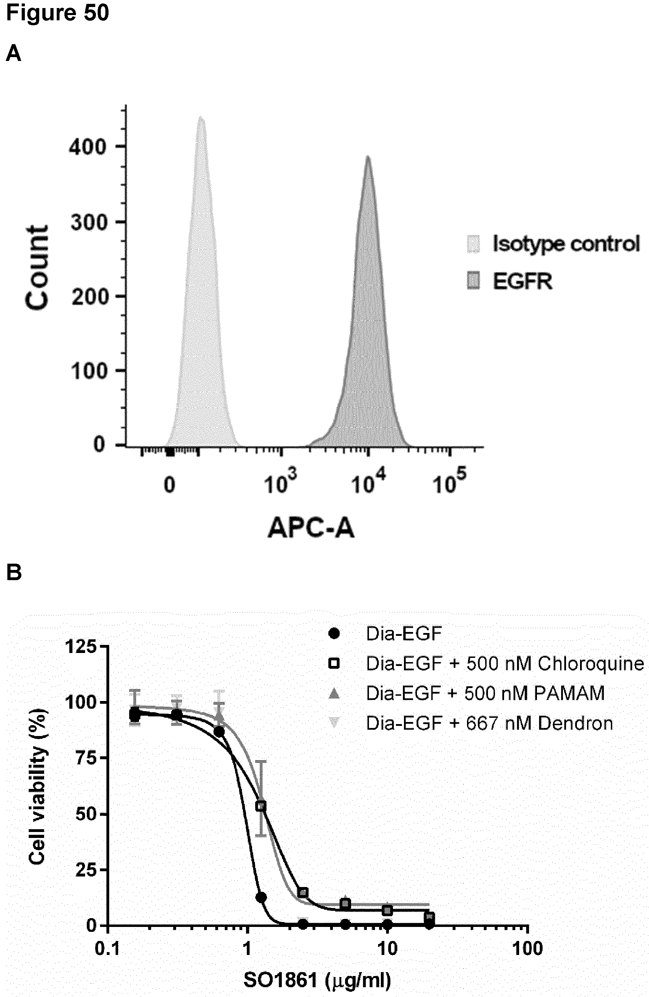

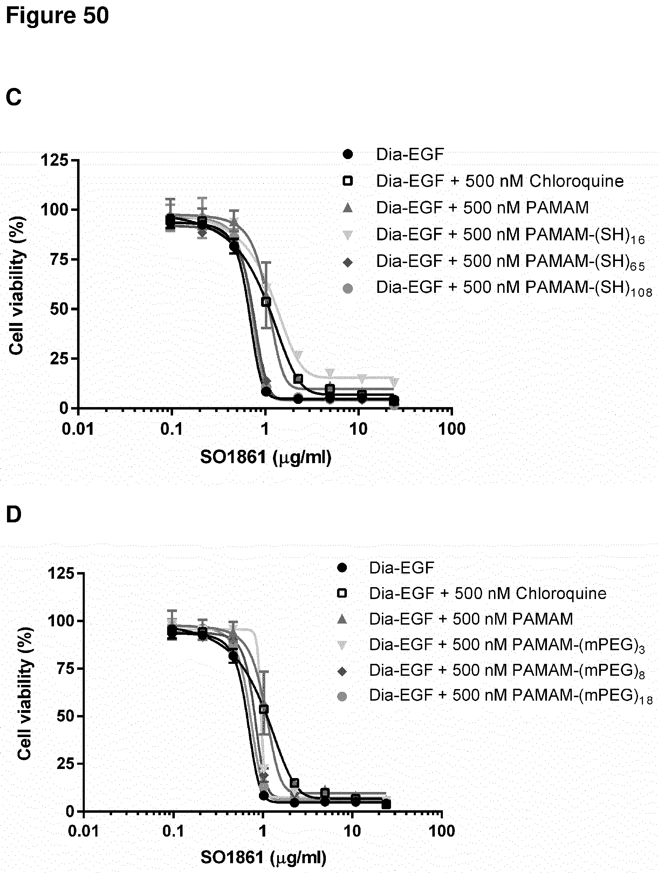

Figure 50: **A.** EGFR cell surface expression as determined by FACS analyses of HeLa cells. **B.** Cell viability of HeLa cells treated with SO1861 + dianthin-EGF (Dia-EGF), SO1861 + dianthin-EGF + 500 nM chloroquine, SO1861 + dianthin-EGF + 500 nM PAMAM, SO1861 + dianthin-EGF + 667 nM dendron. C. Cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(SH)₁₆, SO1861 + dianthin-EGF + 500 nM PAMAM-(SH)₆₅, SO1861 + dianthin-EGF + 500 nM PAMAM-(SH)₁₀₈.**D**. Cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(mPEG)₃, SO1861 + dianthin-EGF + 500 nM PAMAM-(mPEG)₈, SO1861 + dianthin-EGF + 500 nM PAMAM-(mPEG)₁₈.
Figure 51: **A.** Reaction scheme of the thiolation of PAMAM using the thiolation reagent 2-iminothiolane. **B.** MALDI-TOF-MS spectrum of native PAMAM. **C.** MALDI-TOF-MS spectrum of thiolated PAMAM-(SH)₁₆. **D.** MALDI-TOF-MS spectrum of thiolated PAMAM-(SH)₆₅. **E.** MALDI-TOF-MS spectrum of thiolated PAMAM-(SH)₁₀₈.
Figure 52: **A.** Reaction scheme of the PEGylation of PAMAM using the PEGylating reagent mPEG₂ₖ-NHS. **B.** MALDI-TOF-MS spectrum of native PAMAM**.C.** MALDI-TOF-MS spectrum of PEGylated PAMAM-(mPEG₂ₖ)₃. **D**.MALDI-TOF-MS spectrum of PEGylated PAMAM-(mPEG₂ₖ)₈. **E.** MALDI-TOF-MS spectrum of PEGylated PAMAM-(mPEG₂ₖ)₁₈.
Figure 53: Schematic representation of a basic scaffold with click chemistry function to link any desired effector molecule.
Figure 54: Schematic representation of a functionalized scaffold with pre-bound effector molecule and click chemistry function to link any desired ligand. Optionally, a pH-sensitive linkage can be provided to release the effector molecule from the scaffold after reaching the endosomes.

### DETAILED DESCRIPTION

In order for a bioactive molecule to work, the molecule must be able to engage with its target, e.g. in the blood serum, on the outside of the cell surface or inside a cell or an organelle. The active moiety of almost all protein-based targeted toxins, e.g., must enter the cytosol of the target cell to mediate its target modulatory effect. In many constellations the toxin remains ineffective since (1) the targeting moiety is poorly internalized and remains bound to the outside of the cells, (2) is recycled back to the cell surface after internalization or (3) transported to the endolysosomes where it is degraded. Although these fundamental issues are known for decades and more than 500 targeted toxins have been investigated in the past decades, the problems have not been solved yet and only one antibody-targeted protein toxin, moxetumomab pasudotox-tdfk (LUMOXITI^{®}, AstraZeneca Pharmaceuticals LP), has been approved for relapsed or refractory hairy cell leukemia by the FDA to date.

To overcome these problems, many strategies have been described including approaches to redirect the toxins to endogenous cellular membrane transport complexes of the biosynthetic pathway in the endoplasmic reticulum and techniques to disrupt or weaken the membrane integrity of endosomes, i.e. the compartments of the endocytic pathway in a cell, and thus facilitating the endosomal escape. This comprises the use of lysosomotropic amines, carboxylic ionophores, calcium channel antagonists, various cell-penetrating peptides of viral, bacterial, plant, animal, human and synthetic origin, other organic molecules and light-induced techniques. Although the efficacy of the targeted toxins was typically augmented in cell culture hundred- or thousand-fold, in exceptional cases more than million-fold, the requirement to co-administer endosomal escape enhancers with other substances harbors new problems including additional side effects, loss of target specificity, difficulties to determine the therapeutic window and cell type-dependent variations.

All strategies, including physicochemical techniques, require enhancer molecules that interact more or less directly with membranes and comprise essentially small chemical molecules, secondary metabolites, peptides and proteins. A common feature of all these substances is that they are *per se* not target cell-specific and distribute with other kinetics than the targeted toxins. This is one major drawback of the current approaches.

The present invention will be described with respect to particular embodiments but the invention is not limited thereto but only by the claims. The embodiments of the invention described herein can operate in combination and cooperation, unless specified otherwise.

While the invention has been described in terms of several embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

An aspect of the invention is a scaffold suitable for covalently binding at least one biologically active molecule to a carrier molecule as defined in the claims, the scaffold comprising a polymeric or oligomeric structure and at least one of said biologically active molecules covalently bound to said polymeric or oligomeric structure, wherein the scaffold further comprises a first chemical group for covalently coupling of the scaffold to the carrier molecule.

An embodiment is the scaffold according to the invention, wherein the at least one biologically active molecule has a molecular mass of 3.000 Dalton or less, preferably 2.500 Dalton or less, more preferably 2.300 Dalton or less, most preferably, 2.000 Dalton or less, such as 1.700 Dalton - 1.950 Dalton.

In accordance with the invention, the at least one biologically active molecule is an amphiphilic molecule. Such amphiphilic molecules, such as saponins, can typically be part or form layers such as bi-layers and lipid layers, e.g. cell membranes, and/or can be part of vesicles, e.g. inside cells.

An embodiment is the scaffold according to the invention, wherein the at least one biologically active molecule is a single specific molecule or is a mixture of different molecules, when more than one biologically active molecules are covalently bound to the polymeric or oligomeric structure comprised by the scaffold. Typically, the biologically active molecule is obtained or derived from a natural source, and/or typically the biologically active molecule comprises one, two or more of near-identical or similar molecules. An example is the water-soluble fraction comprising saponins, obtained from Quillaja saponaria ('Quil-A'), which encompasses a series of quillaja saponins such as QS-21, QS-18, QS-7. Another example is a mixture of QS-21xyl and QS-21api.

In accordance with the invention, the at least one biologically active molecule is a bisdesmosidic triterpene saponin, most preferably a bisdesmosidic triterpene saponin belonging to the type of a 12,13-dehydrooleanane with an aldehyde function in position C-23 and optionally comprising a glucuronic acid function in a carbohydrate substituent at the C-3beta-OH group of the saponin.

Before the present invention it was not possible to guide a single or multiple glycoside molecules to a (target) cell. In particular, it was not possible to specifically guide an effector molecule, for example as part of an ADC, and a particular number or range of glycoside molecules per effector molecule at the same time to the cytosol of cells, such as via the endocytic pathway of a cell. A solution provided for by the invention immobilizes and even polymerizes the glycoside molecules and enables re-monomerization at the intracellular site where the mode of action of the glycoside is desired, e.g. after endocytosis. "Polymerizes" in this context means the reversible and/or irreversible multiple conjugation of glycoside molecules to a polymeric or oligomeric structure to form a scaffold or the reversible and/or irreversible multiple conjugation of (modified) glycoside molecules thereby forming a polymeric or oligomeric structure to form a scaffold. "Re-monomerization" in this context means the cleavage of the glycoside molecules from the scaffold for example after endocytosis and regaining the (native) chemical state of the unbound glycoside molecules, which unbound glycoside molecules may or may not comprise additional chemical groups such as a chemical group for linking the glycoside to the scaffold, and/or a (chemical) linker. Due to the complex chemistry of the glycoside molecule the 'polymerization' of glycoside molecules and their 're-monomerization' at a desired location such as intracellularly e.g. after endocytosis, was a challenging task. In particular, the chemical reactions used for providing the scaffold of the invention comprising covalently linked triterpenoid saponins (polymerization of the glycosides), normally occur in water-free organic solvents, but glycoside molecules and biocompatible polymers are water-soluble molecules. The chemical properties of the unmodified glycoside molecule further prohibited polymerization by itself and, one other possible solution, to bind multiple glycoside molecules (directly) to the effector molecule was estimated not to be very promising, as an effector molecule (drug, toxin, polypeptide or polynucleotide) does typically not provide sufficient binding sites and because the coupling product would become quite heterogeneous and/or coupling biologically active molecules such as a glycoside and e.g. a peptide, a toxin, a nucleic acid together bears the risk for influencing and hampering the activity of one or even both molecules bound together in such glycoside-comprising conjugate. Further, there was a considerable risk that the effector molecule loses its function after coupling. The present invention solves at least one of these drawbacks.

An embodiment is the scaffold according to the invention, wherein the at least one biologically active molecule is a saponin that can be isolated from a *Gypsophila* species and/or a *Saponaria* species and/or an *Agrostemma* species and/or a *Quillaja* species such as *Quillaja saponaria* or is a single specific saponin or is a mixture of two or more different saponins, such as one or more of the saponins in Table A1 or Scheme I, SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, or any of their stereomers and/or any combinations thereof, preferably the saponin is SO1861 and/or GE1741 and/or SA1641 and/or QS-21 and/or saponin with a quillaic acid aglycon core, a Gal-(1→2)-[Xyl-(1→3)]-GlcA carbohydrate substituent at the C-3beta-OH group and a Glc-(1→3)-Xyl-(1→4)-Rha-(1→2)-[Xyl-(1→3)-4-OAc-Qui-(1→4)]-Fuc carbohydrate substituent at the C-28-OH group, and/or is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4-OAc-beta-D-quinovopyranosyl-(1→44)]-beta-D-fucopyranoside, more preferably the saponin is SO1861 and/or QS-21.

Table A1 and Scheme I and the above embodiment summarize a series of saponins that have been identified for their endosomal escape enhancing activity when contacted to mammalian cells such as human cells in free form together with a second molecule (e.g. an effector moiety or effector molecule). Indeed, in cell-based bioassays it was established for the saponins tabulated in Table A1 and those in Scheme I, that under influence of these saponins a second molecule such as a nucleic acid and/or a toxin such as a protein toxin (e.g. one or more of the protein toxins listed in Table A5), is released intracellularly from the (late) endosomes and lysosomes with increased efficiency and/or efficacy. That is to say, endosomal and/or lysosomal escape of such second molecules (i.e. effector moieties, effector molecules), e.g. nucleic acids and/or toxins, is less efficient in the absence of the saponin.

Surprisingly, the inventors now demonstrate that a water-soluble saponin fraction from Quillaja saponaria, comprising QS-21 and its family members QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, QS-18, and also referred to as 'Quil-A', also exhibits the ability to potentiate a biological effect in vitro of e.g. a nucleic acid bound to a monoclonal antibody or a protein toxin bound to a monoclonal antibody, when co-administered to tumor cells of a mammalian species (human) as a single covalent conjugate comprising a monoclonal antibody, the effector molecule (the aforementioned second molecule) and comprising at least one glycoside such as the QS-21 and its family member saponins encompassed by such QS-21 preparation, wherein the effector molecule and the glycoside, e.g. QS-21, SO1861, SA1641, GE1741, are covalently bound to the polymeric or oligomeric scaffold, either directly or via at least one linker, or wherein the glycoside is covalently bound to the effector molecule and the effector molecule is covalently bound to the polymeric or oligomeric scaffold. Alternatively, the glycoside and the effector molecule are separately covalently bound to the scaffold, wherein a monoclonal antibody is also separately bound to this conjugate of saponin, toxin, scaffold, wherein the scaffold is a tri-functional linker encompassing the polymeric or oligomeric structure, such as for example the scaffold outlined in Scheme II and Structure B. Without wishing to be bound by any theory, the observed stimulation or potentiation of for example saporin- or dianthin mediated tumor-cell killing in the presence of saponins derived from Quillaja saponaria may (also) relate to activation of the inflammasome in the tumor cell by the saponins, for example resulting in tumor cell pyroptosis.

QS-21, and also the water-soluble saponins fraction of Quillaja saponaria, is already for a long time known and previously intensively applied for its immune-potentiating abilities, e.g. as an adjuvant in e.g. sub-unit vaccines. For example, QS-21 is applied in two phase III clinical trials with human patients, who were vaccinated with a sub-unit vaccine mixed with an adjuvant comprising QS-21 (Glaxo-Smith-Kline, MAGRIT trial, DERMA study), wherein the sub-unit was MAGE-A3 protein, which is specifically expressed and presented by tumor cells. The anti-tumor vaccinations, potentiated with QS-21, aimed for extension of disease-free survival of the cancer patients (melanoma; non-small cell lung cancer). In addition, QS-21 has been tested as an adjuvant in clinical trials for developing anti-cancer vaccine treatment, for vaccines for HIV-1 infection, in development of a vaccine against hepatitis B, and for anti-malaria vaccine development using QS-21 comprising adjuvants AS01 and AS02 of Glaxo-Smith-Kline. Previous studies revealed an immune response elicited against MAGE-A3 peptides presented at the cancer cell surface, under influence of the QS-21 saponin comprising adjuvant (AS15; GSK). To the surprise of the inventors, the saponins in the Quillaja saponaria fraction, comprising QS-21, potentiate the anti-tumor cell activity of e.g. a payload such as a protein toxin (dianthin). The saponins are for example covalently coupled to cetuximab and toxic effects of dianthin in several human tumor cell lines is established when a ligand-dianthin conjugate is exposed to the tumor cells in the presence of cetuximab-saponins. Further details are outlined in the Examples section.

Similarly, saponins potentiate anti-tumor cell activity of a nucleic acid such as the oligonucleotide BNA (HSP27) for silencing HSP27 expression in (tumor) cells. The inventors show that a tumor-cell targeting monoclonal antibody, such as cetuximab, provided with covalently coupled antisense BNA (HSP27) and provided with covalently coupled saponin (here SO1861), both the BNA and the saponin coupled to the antibody via a cleavable bond (here a hydrazone bond) through which both the BNA and the SO1861 are coupled to a tri-functional linker (here the tri-functional oligomeric scaffold of Scheme II), is capable of silencing HSP27 in vivo in tumors, compared to control and compared to ADC only, without coupled saponin. Conjugating an ADC with a saponin thus results in a conjugate comprising the BNA and the saponin, which combination endows the ADC with anti-tumor cell activity not seen with the ADC at the same dose. Noteworthy, the ADC and the monoclonal antibody with covalently coupled saponin increase HSP27 expression in tumor cells, when administered to tumor-bearing mice separately in separate groups of mice, compared to a control group (vehicle administered, only). Only the ADC comprising the scaffold of the invention with covalently coupled saponin and effector molecule, displays reduced HSP27 expression when compared to controls. The antisense BNA (HSP27) was BNA with oligo nucleic acid sequence 5'-GGCacagccagtgGCG-3' according to Zhang et al. (2011) [*Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18*, 326-333]. Noteworthy, to the best of the knowledge of the inventors, BNA is designed for application as a free nucleic acid. The inventors are now the first to demonstrate that the antisense BNA can be covalently coupled through a (non-)cleavable linker with a ligand or an antibody, in a way that gene-silencing activity is retained in vitro and more importantly in vivo in the tumor cells of a tumor-bearing animal. This approach opens new ways to administer targeted BNA to human (cancer) patients in need thereof.

An embodiment is the scaffold according to the invention, wherein the at least one biologically active molecule is a bisdesmosidic saponin having a molecular mass of at least 1.500 Dalton and comprising an oleanan-type triterpene containing an aldehyde group at the C-23 position and optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units, optionally containing at least one acetyl residue such as two acetyl residues and/or optionally comprising deoxy carbohydrates and/or optionally comprising quinovose and/or optionally comprising glucose and/or optionally comprising 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond, or wherein the at least one saponin is QS-21 or any one or more of QS-21A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, protonated QS1861 (QS1862), Quil-A. Such saponins are further exemplified in Table A1 and Scheme I. The inventors demonstrate here that covalently coupling saponins such as QS-21, SA1641, SO1861, water-soluble saponins fraction of Quillaja saponaria, to a scaffold, such as a dendron or a tri-functional linker, e.g. the tri-functional linker of Scheme II and Structure B, the scaffold further comprising covalently bound effector molecule(s) such as a proteinaceous toxin such as dianthin or saporin, and/or a (tumor) cell-surface molecule targeting ligand or moiety such as a monoclonal antibody for binding to a (tumor) cell-surface receptor, results in improved cell toxicity exerted by the toxin, under influence of the covalently coupled saponin.

An embodiment is the scaffold of the invention comprising as the biologically active molecule a saponin comprising one or several or all of the indicated structural features of the saponin of Structure A in Scheme I, and/or a saponin selected from any one or more of the further saponins in Scheme I:

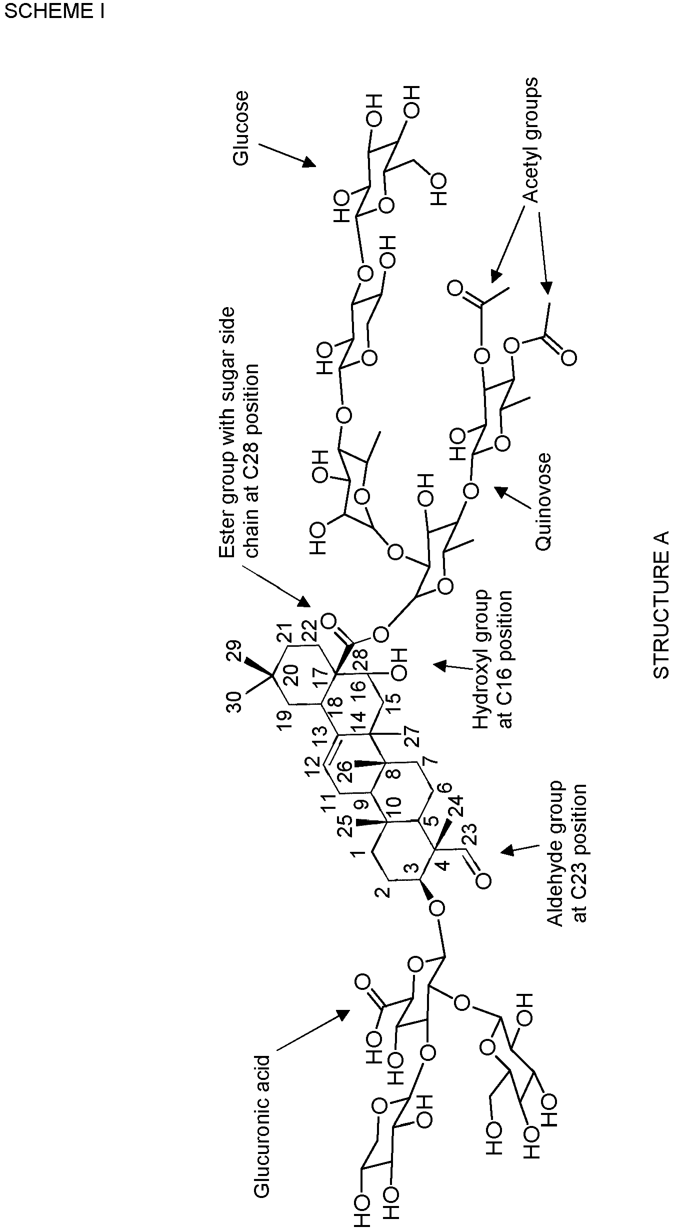

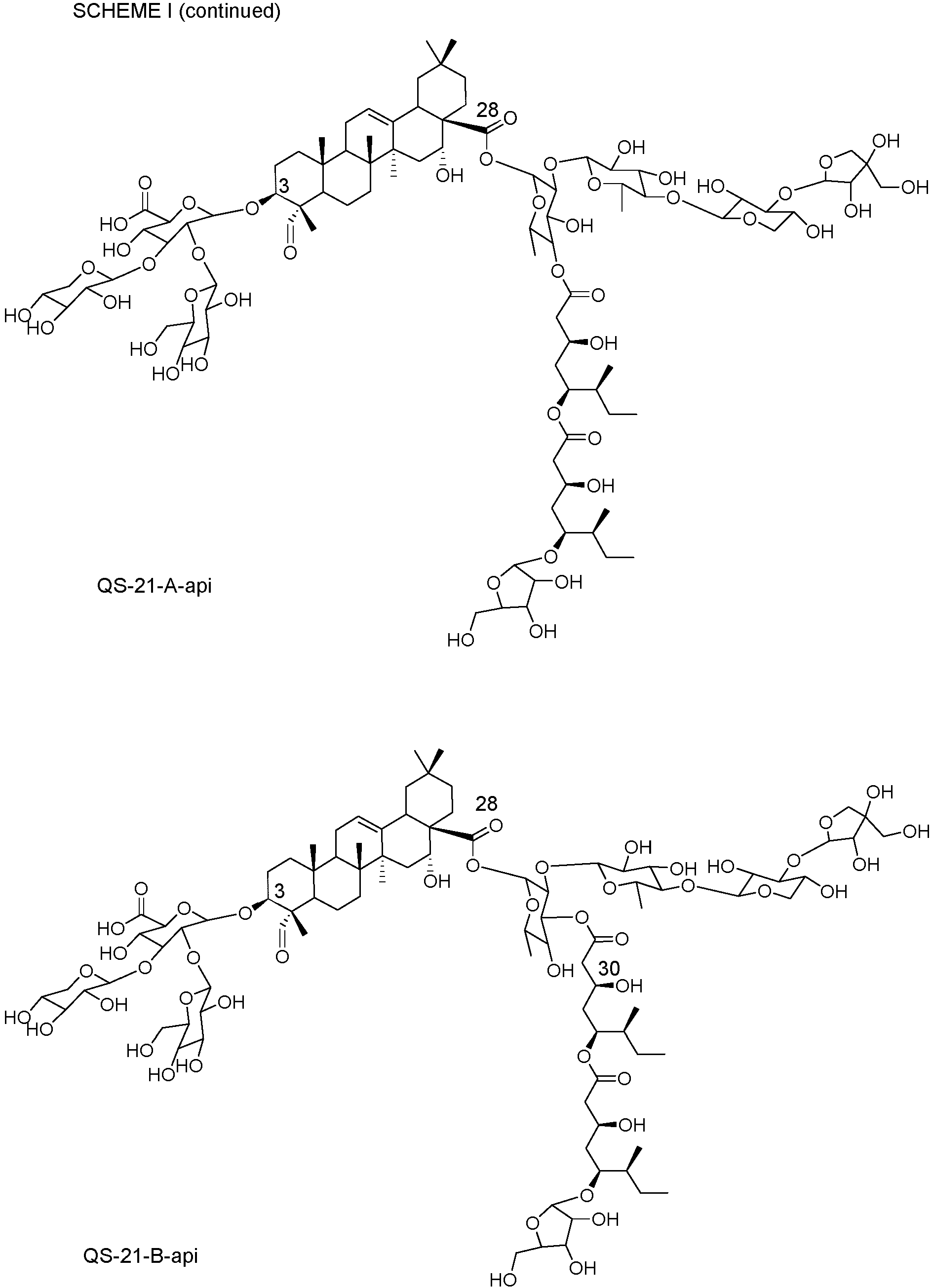

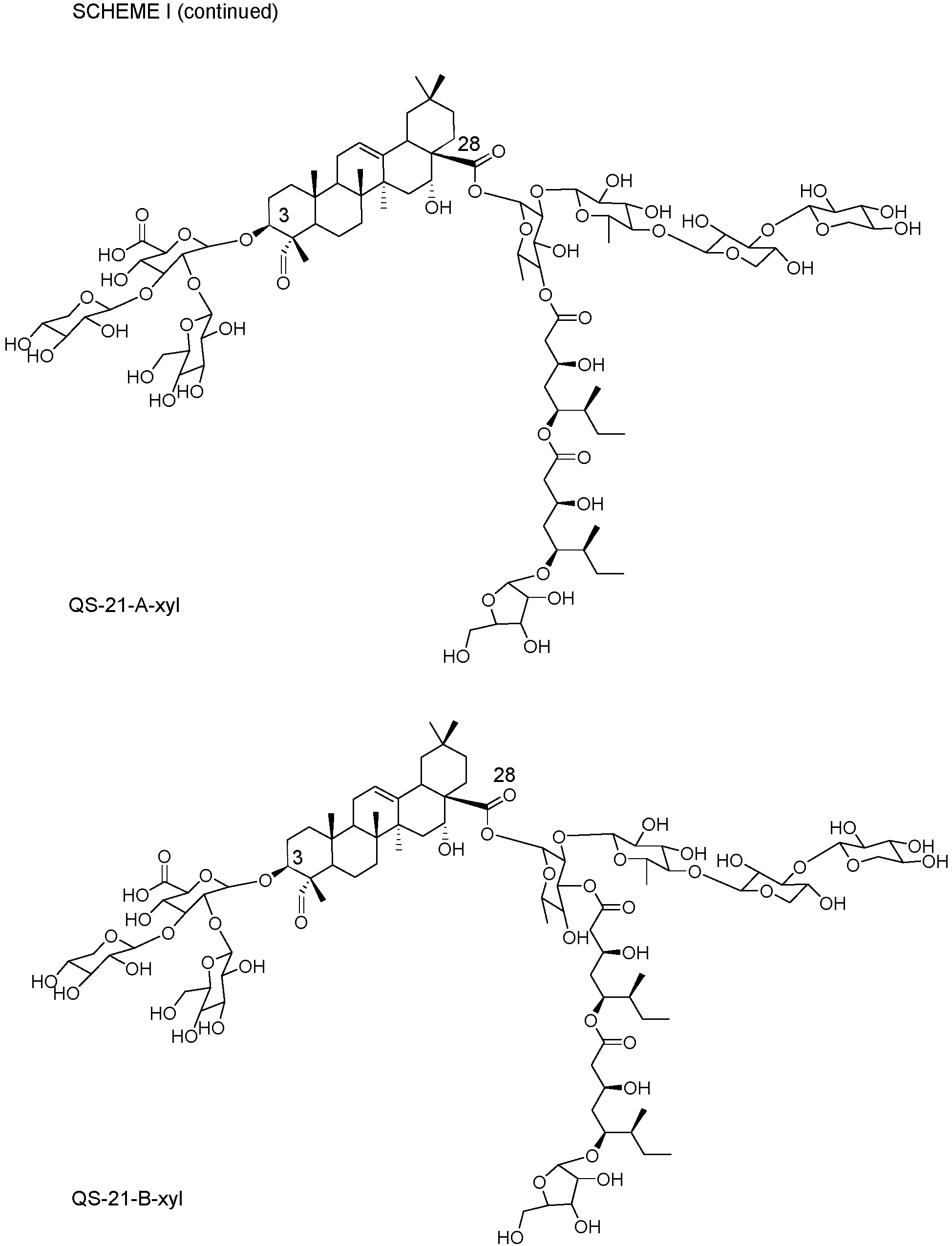

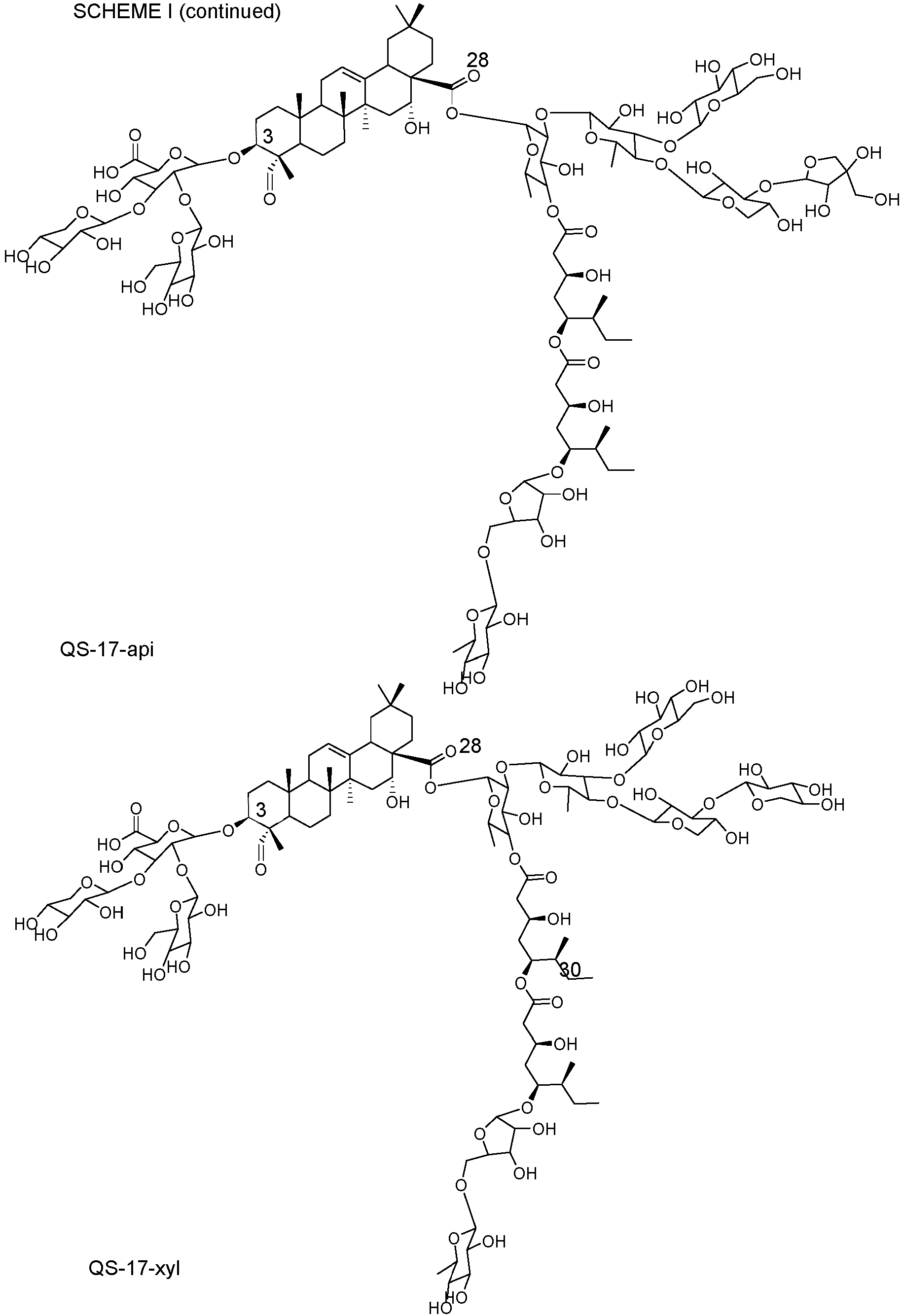

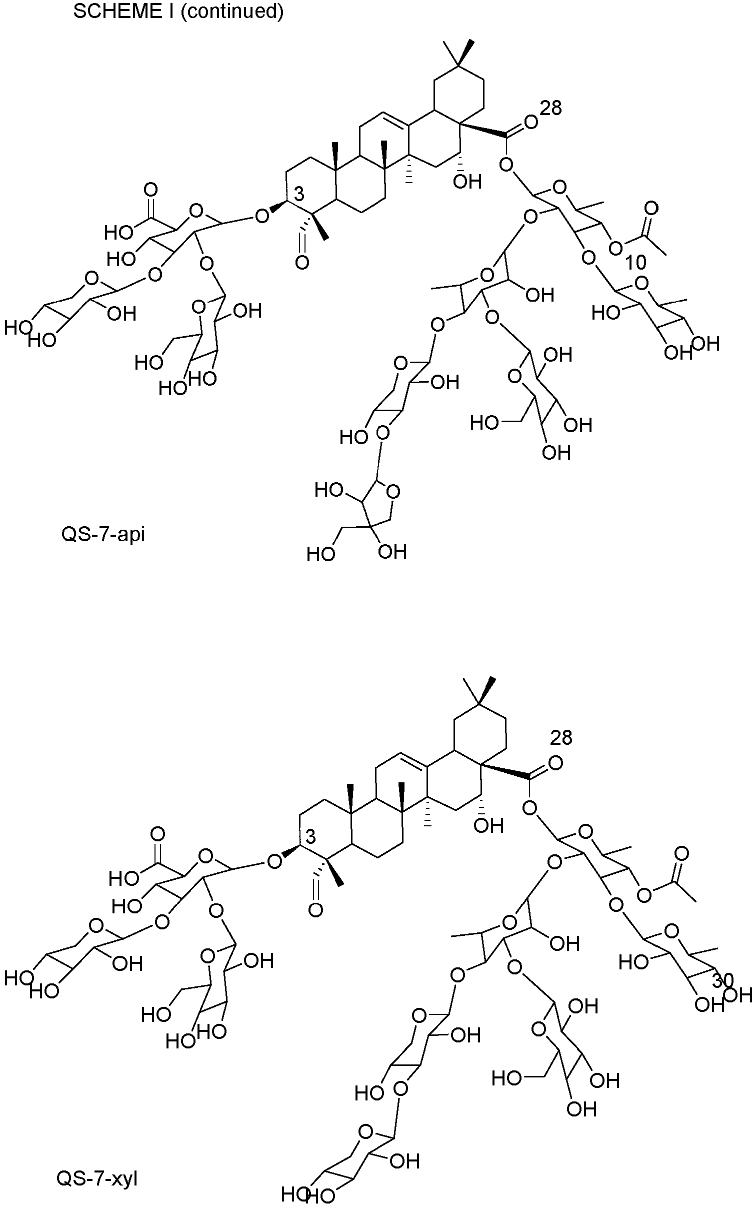

According to the invention, a biologically active molecule which has the 'ideal' structure for the purpose of enhancing endosomal escape of an effector molecule bound to the scaffold of the invention as a carrier molecule is a bisdesmosidic saponin according to Structure A of Scheme I, having a molecular mass of at least 1.500 Dalton and comprising an oleanan-type triterpene containing an aldehyde group at the C-23 position and optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units, optionally containing at least one acetyl residue such as two acetyl residues and/or optionally comprising deoxy carbohydrates and/or optionally comprising quinovose and/or optionally comprising glucose and/or optionally comprising 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond.

SO1861 is different from the *"ideal structure"* displayed in Scheme I, Structure A, only in having only one acetyl residue at the quinovose and having an additional xylose. The "ideal structure" of a saponin for enhancing endosomal escape of an effector molecule or effector moiety, is a saponin which preferably has the Structure A of Scheme I, and saponins which display the endosomal escape enhancing activity have one or more of the structural features displayed in Structure A of Scheme I. Without wishing to be bound by any theory, the inventors belief that the Structure A of Scheme I represents an "ideal saponin" (and not a minimum requirement saponin) for endosomal escape enhancing activity, which means that not all of the structures can or must be present in each saponin with at least sufficient endosomal escape enhancing activity to promote accumulation of the effector moiety in the cytosol, and which means that some saponins might have other structure elements such as acyl chains, and/or for yet other saponins that display endosomal escape enhancing activity, the sugars can be different than the sugars displayed in Scheme I. For example, the QS-21 saponin and some of the saponins in the water soluble fraction of Quillaja saponaria (Quillaja saponins; Quil-A) differ in the carbohydrate modification at C-28 when the ideal structure of Structure A in Scheme I is considered: presence of an acyl chain in QS-21 for example. In the water soluble fraction of Quillaja saponaria, saponins such as QS-7, QS1862, are similar to the ideal Structure A, and are similar to SO1861.

To explain the invention in more detail, the process of cellular uptake of substances (although the inventors do not wish to be bound by any theory) and the used terminology in this invention is described. The uptake of extracellular substances into a cell by vesicle budding is called endocytosis. Said vesicle budding can be characterized by (1) receptor-dependent ligand uptake mediated by the cytosolic protein clathrin, (2) lipid-raft uptake mediated by the cholesterol-binding protein caveolin, (3) unspecific fluid uptake (pinocytosis), or (4) unspecific particle uptake (phagocytosis). All types of endocytosis run into the following cellular processes of vesicle transport and substance sorting called the endocytic pathways. The endocytic pathways are complex and not fully understood. Without wishing to be bound by any theory, organelles may be formed *de novo* and mature into the next organelle along the endocytic pathway. It is however, now hypothesized that the endocytic pathways involve stable compartments that are connected by vesicular traffic. A compartment is a complex, multifunctional membrane organelle that is specialized for a particular set of essential functions for the cell. Vesicles are considered to be transient organelles, simpler in composition, and are defined as membraneenclosed containers that form *de novo* by budding from a preexisting compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles. The endocytic vesicle, which arises at the plasma membrane, most prominently from clathrin-coated pits, first fuses with the early endosome, which is a major sorting compartment of approximately pH 6.5. A large part of the cargo and membranes internalized are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of an endocytosed molecule occurs inside an endolysosome or lysosome. Endosomal escape is the active or passive release of a substance from the inner lumen of any kind of compartment or vesicle from the endocytic pathway, preferably from clathrin-mediated endocytosis, or recycling pathway into the cytosol. Endosomal escape thus includes release from endosomes, endolysosomes or lysosomes, including their intermediate and hybrid organelles. Unless specifically indicated otherwise and in particular when relating to the endosomal escape mechanism of the glycoside molecule, whenever the word "endosome" or "endosomal escape" is used herein, it also includes the endolysosome and lysosome, and escape from the endolysosome and lysosome, respectively. After entering the cytosol, said substance might move to other cell units such as the nucleus. In formal terms, a glycoside is any molecule in which a sugar group is bound through its anomeric carbon to another group via a glycosidic bond. Glycoside molecules in the context of the invention are such molecules that are further able to enhance the effect of an effector molecule, without wishing to be bound by any theory, in particular by facilitating the endosomal escape of the effector molecule. Without wishing to be bound by any theory, the glycoside molecules interact with the membranes of compartments and vesicles of the endocytic and recycling pathway and make them leaky for said effector molecules resulting in augmented endosomal escape. With the term "the scaffold is able to augment endosomal escape of the effector molecule" is meant that the at least one glycoside molecule, which is coupled to the polymeric or oligomeric structure of the scaffold, is able to enhance endosomal escape of an effector molecule when both molecules are within an endosome, e.g. a late endosome, optionally and preferably after the at least one glycoside is released from the polymeric or oligomeric structure, e.g., by cleavage of a cleavable bond between the at least one glycoside and the polymeric or oligomeric structure. Even though a bond between the at least one glycoside and the scaffold may be a "stable bond", that does not mean that such bond cannot be cleaved in the endosomes by, e.g., enzymes. For instance, the glycoside, together with a linker or a part of the polymeric structure may be cleaved off the remaining polymeric structure. It could, for instance be that a protease cuts a proteinaceous polymeric structure, e.g., albumin, thereby releasing the at least one glycoside. It is, however, preferred that the glycoside molecule is released in an active form, preferably in the original form that it had before it was (prepared to be) coupled to the scaffold; thus the glycoside has its natural structure after such cleavage or the glycoside has (part of) a chemical group or linker bound thereto, after such cleavage, while glycoside biological activity, e.g. endosomal/lysosomal escape enhancing activity towards an effector molecule present in the same endosome or lysosome, is maintained or restored upon said cleavage of the bound between the glycoside and the carrier molecule, e.g. the scaffold of the invention. With regard to the present invention the term "stable" with respect to bonds between saponins, polymeric or oligomeric structures (of the scaffold), ligands, (monoclonal) immunoglobulins or binding domains or -fragments thereof, and/or effectors (effector moieties, effector molecules), is meant that the bond is not readily broken or at least not designed to be readily broken by, e.g., pH differences, salt concentrations, or UV-light. With regard to the present invention the term "cleavable" with respect to bonds between saponins, polymeric or oligomeric structures of the scaffold of the invention, ligands, antibodies and/or effectors, is meant that the bond is designed to be readily broken by, e.g., pH differences, salt concentrations, under reductive conditions, and the like. The skilled person is well aware of such cleavable bonds and how to prepare them.

An effector molecule, or effector moiety, in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol. Cytosolic delivery of an effector molecule in the context of the invention preferably means that the effector molecule is able to escape the endosome (and/or lysosome), which, as defined previously, also includes escaping the endolysosome and the lysosome, and is preferably able to reach the effector molecule target as described herein. The invention is a new type of molecule, referred to as scaffold that serves to bring both an effector molecule and at least one glycoside molecule in an endosome at the same time in a pre-defined ratio. Within the context of the present invention, the polymeric or oligomeric structure of the scaffold is a structurally ordered formation such as a polymer, oligomer, dendrimer, dendronized polymer, or dendronized oligomer or it is an assembled polymeric structure such as a hydrogel, microgel, nanogel, stabilized polymeric micelle or liposome, but excludes structures that are composed of non-covalent assemblies of monomers such as cholesterol/phospholipid mixtures. The terms "polymer, oligomer, dendrimer, dendronized polymer, or dendronized oligomer" have their ordinary meaning. In particular a polymer is a substance which has a molecular structure built up chiefly or completely from a large number of equal or similar units bonded together and an oligomer is a polymer whose molecules consist of relatively few repeating units. There is no consensus about one specific cut-off for "many" and "a few" as used in the above definition of polymer and oligomer, respectively. However, as the scaffold may comprise a polymeric or an oligomeric structure, or both, the full range of numbers of similar units bonded together applies to such structure. i.e. from 2 monomeric units to 100 monomeric units, 1000 monomeric units, and more. A structure comprising 5 or less, for instance maybe called an oligomeric structure, whereas a structure comprising 50 monomeric units maybe called a polymeric structure. A structure of 10 monomeric units maybe called either oligomeric or polymeric. A scaffold as defined herein, further comprises at least one glycoside molecule. A scaffold includes a polymeric or oligomeric structure such as poly- or oligo(amines), e.g., polyethylenimine and poly(amidoamine), and biocompatible structures such as polyethylene glycol, poly- or oligo(esters), such as poly(lactids), poly(lactams), polylactide-co-glycolide copolymers, and poly(dextrin), poly- or oligosaccharides, such as cyclodextrin or polydextrose, and poly- or oligoamino acids, such as poly-lysine or a peptide or a protein, or DNA oligo- or polymers. An assembled polymeric structure as defined herein comprises at least one scaffold and, optionally, other individual polymeric or oligomeric structures. Other individual polymeric or oligomeric structures of said assembly may be (a) scaffolds (thus comprising at least one glycoside molecule), (b) functionalized scaffolds (thus comprising at least one glycoside molecule, and a ligand, antibody, etc. and/or an effector molecule, (c) polymeric or oligomeric structures comprising at least one ligand, antibody, etc. and/or at least one effector, or (d) polymeric or oligomeric structures without a glycoside molecule, without a ligand, antibody, etc., and without an effector molecule. A functionalized assembled polymeric structure is an assembled polymeric structure that contains (a) at least one functionalized scaffold or (b) at least one scaffold and at least one polymeric structure comprising at least one ligand, antibody, etc. and/or at least one effector. Polymeric or oligomeric structures within an assembled polymeric structure that do not comprise any of the above mentioned molecules (i.e. no glycosides, ligands, antibodies, or effectors) are in particular added as structural components of the assembled structures, which help to build up or to stabilize the assembled structure ("glue-like"). The inventors have found that in particular scaffolds that, after coupling to the glycoside molecules, comprise polymeric structures with many primary, secondary and/or tertiary amine groups, do not particularly enhance endosomal escape. Particularly non-preferred in this respect are polymeric and oligomeric structures comprising secondary amine groups. Without wishing to be bound by any theory, the acidic environment seems to be a prerequisite for the synergistic action between glycoside and effector and it is believed that such amine groups disturb the acidic environment in the late endosomes. Therefore, a scaffold that is able to disturb the acidic environment, e.g., because of the presence of amine groups, is preferably not encompassed by a scaffold according to the invention. However, primary amine groups can, of course, be blocked or shielded, e.g. by thiolation or PEGylation. After appropriate blocking or shielding of the primary amine groups, which method is known by the skilled person, such scaffold is encompassed by the claims.

In a particularly preferred embodiment, the invention provides a scaffold that does not substantially inhibit endosomal (and lysosomal) escape of the effector molecule and, preferably, comprises less than 10, more preferably less than 5, more preferably less than 2, most preferably no primary, secondary or tertiary amine group. For sake of clarity it is emphasized that a primary amine group that is blocked by, e.g., thiolation or PEGylation, is no longer called an amine group.

Whether or not a scaffold is able to disturb the acidic environment and inhibit the endosomal escape function of the at least one glycoside can be easily determined with an assay as described in Example 4 and as known in the art. The inhibition is described as "fold amount increases of glycoside necessary to induced 50% cell killing". It is preferred that the scaffold does not lead to an increase that is at least the increase in glycoside molecules necessary to obtain 50% cell killing observed when using Chloroquine as a positive control. Alternatively, and preferably, the scaffold does not lead to an at least 4-fold increase of glycoside molecules to induce 50% cell killing, more preferably does not lead to an at least 2-fold increase. The fold increase is to be measured in assay, essentially as described in Example 4, wherein Chloroquine, as a positive control, induces a 2-fold increase in glycoside amount to observe 50% cell killing.

With the term "improving or enhancing an effect of an effector molecule" is meant that the glycoside molecule increases the functional efficacy of that effector molecule (e.g. the therapeutic index of a toxin or a drug or an oligonucleotide such as a BNA; the metabolic efficacy of a modifier in biotechnological processes; the transfection efficacy of genes in cell culture research experiments), preferably by enabling or improving its target engagement. Acceleration, prolongation, or enhancement of antigen-specific immune responses are preferably not included. Therapeutic efficacy includes a stronger therapeutic effect, preferably with lower dosing and/or with less side effects. "Improving an effect of an effector molecule" can also mean that an effector molecule, which could not be used because of lack of effect (and was e.g. not known as being an effector molecule), becomes effective when used in combination with the present invention. Any other effect, which is beneficial or desired and can be attributed to the combination of effector molecule and a scaffold, as provided by the invention is considered to be "an improved effect". In an embodiment, the scaffold enhances an effect of the effector molecule which effect is intended and/or desired. In case of a proteinaceous scaffold, the proteinaceous polymeric structure as such may have, for instance, an effect on colloid osmotic pressure in the blood stream. If such effect is not the intended or desired effect of the ultimate functionalized scaffold, the proteinaceous structure of the scaffold is not the effector molecule as defined in the invention. Or, for instance in case of a DNA- or RNA-based scaffold, parts of that DNA or RNA may have an (unintended) function, e.g., by interfering with expression. If such interference is not the intended or desired effect of the ultimate functionalized scaffold, the DNA- or RNA polymeric structure of the scaffold is not the effector molecule as defined in the invention.

A number of preferred features can be formulated for endosomal escape enhancers, i.e. a glycoside according to the invention: (1) they are preferably not toxic and do not invoke an immune response, (2) they preferably do not mediate the cytosolic uptake of the effector molecule into off-target cells, (3) their presence at the site of action is preferably synchronized with the presence of the effector molecule, (4) they are preferably biodegradable or excretable, and (5) they preferably do not substantially interfere with biological processes of the organism unrelated to the biological activity of the effector molecule with which the endosomal escape enhancer is combined with, e.g. interact with hormones. Examples of glycoside molecules that fulfill the before mentioned criteria, at least to some extent, are bisdesmosidic triterpenes, preferably bisdesmosidic triterpene saponins, such as SO1861, SA1641, QS-21, GE1741.

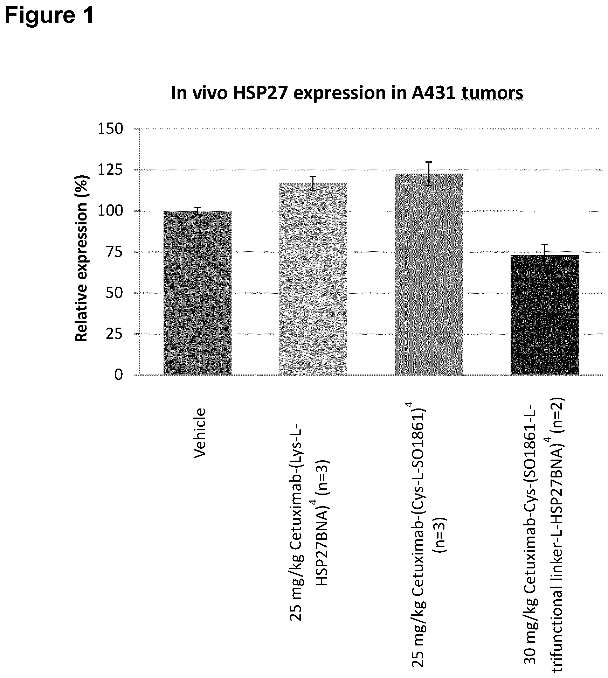

In accordance with the invention the at least one biologically active molecule is covalently bound to the polymeric or oligomeric structure via a hydrazone bond. According to the invention, the biologically active molecule is a saponin (see also Table A1, Scheme I). It has been proven beneficial for the activity of the saponin, e.g. the endosomal escape enhancing activity inside cells when the entry into the cell and the accumulation inside the cytosol of an effector molecule covalently coupled to the same scaffold as the saponin, is considered, when the saponin is covalently coupled to the scaffold involving a hydrazone bond,. Such bond types readily cleave under the acidic conditions inside (late) endosomes and lysosomes of mammalian cells, e.g. human cells, and/or under the reductive conditions. Alternatively, the inventors also demonstrate that covalent coupling of saponin to a carrier molecule via a bond that is not readily cleavable under the physiological conditions inside cells, e.g. (late) endosomes, lysosomes, cytosol, is also beneficial to the potentiating activity of the saponin on the biological effect of e.g. an effector moiety such as a nucleic acid (e.g. BNA silencing HSP27) and a proteinaceous toxin such as saporin. Throughout the application, including the claims, the term 'cleavable linker', 'cleavable bond', etc., is also referred to as 'labile linker' ('L') and 'labile bond', for example in the context of cleavage of such a bond or linker in the (late) endosome and/or lysosome when a conjugate of the invention, e.g. a scaffold with saponins coupled to the scaffold via hydrazone bonds, is referred to. For example, Figure 1 shows the in vivo HSP27 gene silencing in tumors of mice. The tumor-bearing mice were treated with a scaffold according to the invention, comprising an oligomeric tri-functional linker (see Scheme II and Structure B) with saponin SO1861 covalently bound to it via a hydrazone bond, an antisense BNA for silencing the HSP27 gene in the tumor cells, covalently coupled to the scaffold via a hydrazone bond, and monoclonal anti-EGFR antibody cetuximab covalently coupled to the scaffold via a disulphide bond, wherein the hydrazone bonds and the disulphide bond are referred to as cleavable, and thus labile bonds. That is to say, without wishing to be bound by any theory, the hydrazone bonds and the disulphide bond are cleaved in the (late) endosomes and/or lysosomes of the targeted tumor cells that express EGFR at the cell surface, once the conjugate comprising the scaffold of the invention is internalized by e.g. endocytosis. Cleavage of the bonds likely contributes to the endosomal escape enhancing activity of the saponin when the entry of the BNA from the endosome and/or lysosome into the cytosol is considered, although such cleavage is not a necessity for observing the gene silencing effect of the cetuximab(saponin)(BNA) conjugate comprising the tri-functional linker, i.e. the scaffold of the invention.

The skilled person will appreciate that such a tri-functional linker is a scaffold of the invention suitable for covalently coupling one, two or three saponin moieties. For the tri-functional linker covalent coupling of one or two saponin moieties is preferred. The second and/or third binding site is for example suitable for covalent coupling an effector moiety such as a payload, e.g. a protein toxin, a small molecule toxin, a nucleic acid. The second or third binding site of the tri-functional linker is for example also suitable for covalent coupling of a non-proteinaceous ligand for targeting cells such as tumor cells or autoimmune cells, and/or for coupling a proteinaceous ligand. Typical proteinaceous ligands are EGF for targeting (tumor) cells expressing EGFR at the cell surface, and cytokines for targeting tumor cells or autoimmune cells. Moreover, the second or third binding site of the tri-functional linker is suitable for covalent coupling of an immunoglobulin such as a monoclonal antibody, for binding to a cell surface molecule such as a tumor cell surface molecule, preferably a tumor-cell specific molecule, more preferably a tumor cell receptor that is specifically (over-)expressed at the surface of the tumor cell.

Similarly, the immunoglobulin, or any fragment(s) and/or domain(s) thereof which encompass the binding specificity of the immunoglobulin, is suitable for binding to a cell surface molecule such as a receptor, expressed at the surface of an autoimmune cell. Thus, in an embodiment, the tri-functional linker comprises or consists of a covalently bound saponin, e.g. QS-21, SO1861, a covalently bound effector moiety such as a toxin or an oligonucleotide such as a BNA, and a covalently bound cell targeting moiety such as a ligand or an antibody for (specific) binding to a tumor cell, an auto-immune cell, a diseased cell, an aberrant cell, a non-healthy cell, a B-cell disease.

An embodiment is the scaffold of the invention, comprising the oligomeric tri-functional linker as the scaffold core structure, according to Scheme II: 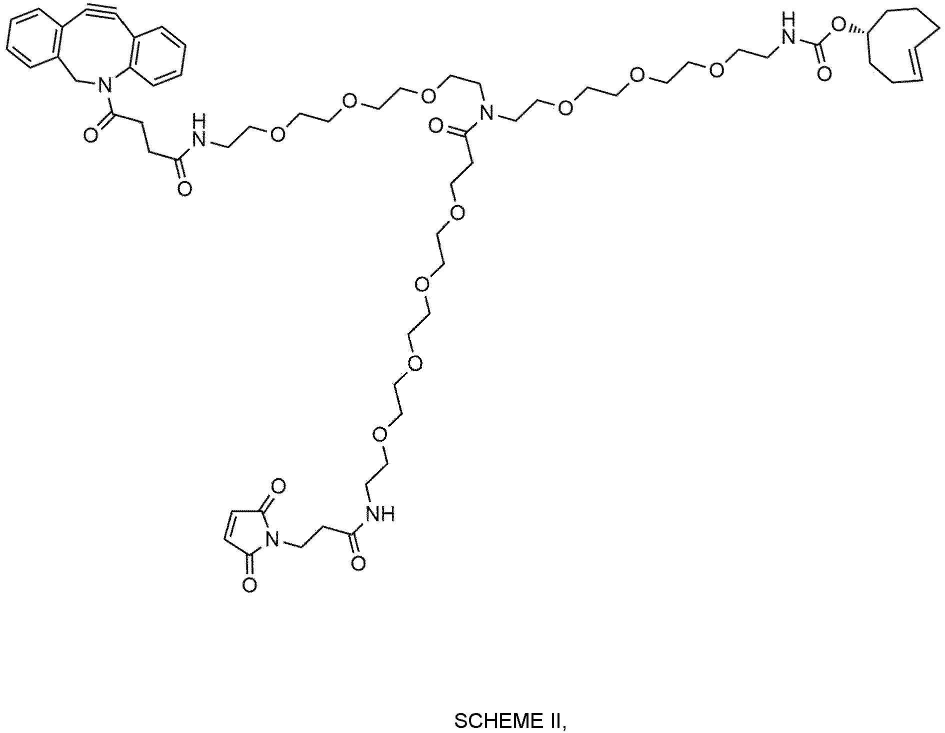 wherein the saponin and here as an example the effector moiety are covalently bound to the tri-functional linker scaffold via labile, cleavable hydrazone linkers (acid sensitive) and/or via a maleimidecomprising bond, when the effector moiety is considered, with optionally cysteines in the carrier molecule, whereas the (optional) binding of the scaffold to the carrier molecule such as an antibody or a linker is established via labile, cleavable hydrazone linkers (acid sensitive) and/or via a maleimidecomprising bond with cysteines in the carrier molecule, such as 1, 2, 3 or 4 cysteines therewith forming Structure B: 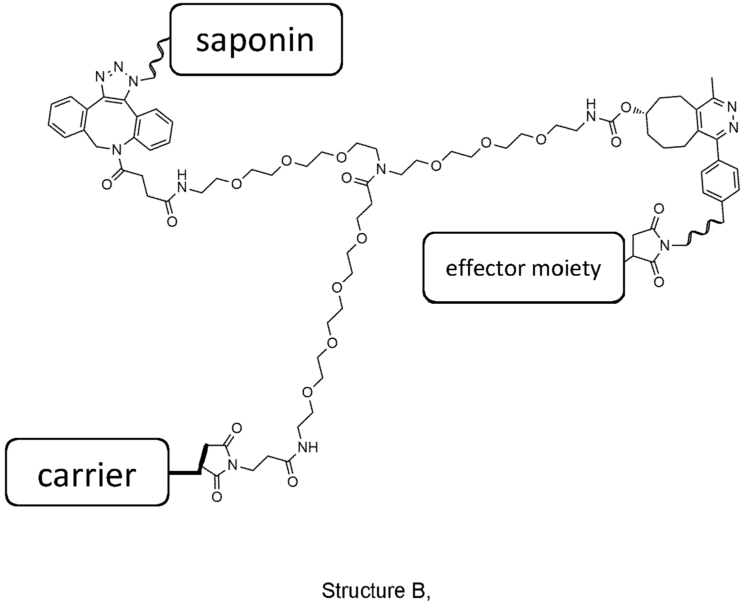 such that 1-4 scaffolds are covalently bound to a single carrier molecule such as a monoclonal antibody. Thus, a single carrier molecule such as an antibody, a ligand, an effector moiety, can covalently conjugate with a single scaffold such as the tri-functional linker of Scheme II and Structure B, or with two or more scaffolds, according to the invention, the two or more scaffolds being the same or different, and the two or more scaffolds optionally comprising the same (number) or different (numbers of) covalently bound saponins.

An embodiment is the scaffold according to the invention, wherein the at least one biologically active molecule is covalently bound to the polymeric or oligomeric structure via a cleavable bond, wherein said cleavable bond is subject to cleavage *in vivo* under acidic conditions as present in endosomes and/or lysosomes of mammalian cells, preferably human cells, preferably at pH 4.0 - 6.5, and more preferably at pH ≤ 5.5. Such a cleavable bond facilitates the release of the biologically active molecule such as one or more saponins, when the scaffold is in such an intracellular acidic environment, e.g. late endosomes. Without wishing to be bound by any theory, free saponin inside endosomes and/or lysosomes may contribute to the endosomal escape enhancing activity of the saponin when endosomal escape of an effector moiety present inside the endosome or lysosome is considered, such endosomal escape enhancing activity being higher or more efficient compared to the activity of saponins bound to e.g. the scaffold. The inventors established that saponins bound to scaffolds and carrier molecules via bonds such as hydrazone bonds, amide bonds, disulphide bonds, are all capable of improving the cytotoxic effect of an effector moiety or molecule inside (tumor) cells, such as payloads as diverse as proteinaceous toxins and oligonucleotides such as antisense BNA.

An embodiment is the scaffold according to the invention, wherein the at least one biologically active molecule is covalently bound to the polymeric or oligomeric structure of the scaffold via a hydrazone bond preferably via at least one linker,. The inventors demonstrated that covalently coupling of e.g. saponins to a scaffold using such bonds results in enhancement of the effect and activity of an effector moiety when cells such as tumor cells are exposed to both a scaffold, such as an oligomeric tri-functional linker or a dendron, carrying the covalently coupled saponins and an effector moiety such as antisense BNA or saporin.

According to the invention, the aldehyde function in position C-23 of the at least one saponin is involved in the covalent bonding to the polymeric or oligomeric structure of the scaffold.

An embodiment is the scaffold according to the invention, wherein the aldehyde function in position C-23 of the at least one saponin is covalently coupled to linker N-ε-maleimidocaproic acid hydrazide, which linker is covalently coupled via a thio-ether bond to a sulfhydryl group in the polymeric or oligomeric structure of the scaffold, such as a sulfhydryl group of a cysteine.

According to the invention the glycoside molecule is a saponin and the linkage between saponin and polymeric or oligomeric structure within the scaffold occurs via an acid-labile bond that is stable at pH 7.4 and, preferably releases the saponin below pH 6.5, more preferably between pH 6.5 and 5.0. This is realized via hydrazones, requiring hydrazides and hydroxyl groups as the functional group of the polymeric or oligomeric structure, respectively. The saponin is attached to the polymeric or oligomeric structure of the scaffold via the aldehyde function in position 23.

An embodiment is the scaffold according to the invention, wherein the chemical group for covalently coupling of the scaffold to the carrier molecule is a click chemistry group.

An embodiment is the scaffold according to the invention, wherein the click chemistry group is a tetrazine, an azide, an alkene or an alkyne, or a cyclic derivative of any of these groups, preferably an azide.

An embodiment is the scaffold according to the invention, wherein the scaffold further comprises a click chemistry group for coupling to the effector molecule and/or to a ligand, antibody, binding domain or -fragment thereof. A click chemistry group is a functional chemical group suitable for click chemistry, which is defined as a reaction that is modular, wide in scope, gives very high yields, generates only inoffensive byproducts, offers high selectivity, and high tolerance over different functional groups, and is stereospecific. The required process characteristics include simple reaction conditions, readily available starting materials and reagents, the use of no solvent or a solvent that is benign (such as water) or easily removed, and simple product isolation. The click chemistry group is preferably a tetrazine, azide, alkene, or alkyne, or reactive derivates of them such as methyl-tetrazine or maleimide (alkene), more preferably an alkyne, or a cyclic derivative of these groups, such as cyclooctyne (e.g. aza-dibenzocyclooctyne, difluorocyclooctyne, bicyclo[6.1.0]non-4-yne, dibenzocyclooctyne).

A scaffold according to the invention thus comprises at least one glycoside molecule. With "at least one" in this context is meant that the scaffold comprises one glycoside molecule but may also comprise a couple (e.g. two, three or four) of glycoside molecules or a multitude (e.g. 10, 20 or 100) of glycoside molecules. Depending on the application, a scaffold may be designed such that it comprises a defined number of glycoside molecules. Preferably, a scaffold according to the invention comprises a defined number or range of glycoside molecules, rather than a random number. This is especially advantageous for drug development in relation to marketing authorization. A defined number in this respect means that a scaffold preferably comprises a previously defined number of glycoside molecules. This is, e.g., achieved by designing a polymeric structure with a certain number of possible moieties for the glycoside(s) to attach. Under ideal circumstances, all of these moieties are coupled to a glycoside molecule and the scaffold than comprises the prior defined number of glycoside molecules. It is envisaged to offer a standard set of scaffolds, comprising, e.g., two, four, eight, sixteen, thirty-two, sixtyfour, etc., glycoside molecules so that the optimal number can be easily tested by the user according to his needs. An embodiment is the scaffold of the invention, wherein the glycoside is present in a defined range as, e.g., under non-ideal circumstances, not all moieties present in a polymeric structure bind a glycoside molecule. Such ranges may for instance be 2 - 4 glycoside molecules per scaffold, 3 - 6 glycoside molecules per scaffold, 4 - 8 glycoside molecules per scaffold, 6 - 8 glycoside molecules per scaffold, 6 - 12 glycoside molecules per scaffold and so on. In such case, a scaffold according to the invention thus comprises 2, 3 or 4 glycoside molecules if the range is defined as 2 - 4.

An embodiment is the scaffold according to the invention, wherein the number of monomers of the polymeric or oligomeric structure is an exactly defined number or range. The polymeric or oligomeric structure comprises structures such as poly(amines), e.g., polyethylenimine and poly(amidoamine), or structures such as polyethylene glycol, poly(esters), such as poly(lactides), poly(lactams), polylactide-co-glycolide copolymers, poly(dextrin), or a peptide or a protein, or structures such as natural and/or artificial polyamino acids, e.g. poly-lysine, DNA polymers, stabilized RNA polymers or PNA (peptide nucleic acid) polymers, either appearing as linear, branched or cyclic polymer, oligomer, dendrimer, dendron, dendronized polymer, dendronized oligomer or assemblies of these structures, either sheer or mixed. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism. Assemblies can be built up by covalent cross-linking or non-covalent bonds and/or attraction. They can therefore also form nanogels, microgels, or hydrogels, or they can be attached to carriers such as inorganic nanoparticles, colloids, liposomes, micelles or particle-like structures comprising cholesterol and/or phospholipids. Said polymeric or oligomeric structures preferably bear an exactly defined number or range of coupling moieties for the coupling of glycoside molecules (and/or effector molecules and/or carrier molecules such as a ligand, monoclonal antibody or a fragment thereof). Preferably at least 50%, more preferably at least 75%, more preferably at least 85%, more preferably at least 90%, more preferably at least 95%, more preferably at least 98%, more preferably at least 99%, most preferably 100% of the exactly defined number or range of coupling moieties in the polymeric or oligomeric structure is occupied by a glycoside molecule in a scaffold according to the invention.

Preferably, a dendron is a branched, clearly defined tree-like polymer with a single chemically addressable group at the origin of the tree, called the focal point. A dendrimer is a connection of two or more dendrons at their focal point. A dendronized polymer is a connection of the focal point of one or more dendrons to a polymer. In a preferred embodiment, a scaffold according to the invention is provided, wherein the polymeric or oligomeric structure comprises a linear, branched or cyclic polymer, oligomer, dendrimer, dendron, dendronized polymer, dendronized oligomer or assemblies of these structures, either sheer or mixed, wherein assemblies can be built up by covalent cross-linking or non-covalent attraction and can form nanogels, microgels, or hydrogels, and wherein, preferably, the polymer is a derivative of a poly(amine), e.g., polyethylenimine and poly(amidoamine), and structures such as polyethylene glycol, poly(esters), such as poly(lactids), poly(lactams), polylactide-co-glycolide copolymers, and poly(dextrin), and structures such as natural and/or artificial polyamino acids such as poly-lysine, or a peptide or a protein (e.g. a ligand or an antibody such as a monoclonal antibody of any of the Tables A2-A4) or DNA polymers, stabilized RNA polymers or PNA (peptide nucleic acid) polymers. Preferably, the polymeric or oligomeric structures are biocompatible.

An embodiment is the scaffold according to the invention, wherein said effector molecule is a pharmaceutically active substance, such as a toxin, a drug, a polypeptide and/or a polynucleotide. An embodiment is the scaffold of the invention wherein the effector molecule is a toxin, a micro RNA, or a polynucleotide encoding a protein. Typically, the carrier molecule encompasses a proteinaceous molecule for targeting the scaffold to e.g. a tumor cell or an auto-immune cell or a cell related to a B-cell disease. Preferably the carrier molecule comprises an immunoglobulin or at least one or more binding domains and/or binding fragments thereof, such immunoglobulin preferably selected from any of the monoclonal antibodies of any of Tables A2-A4, such as cetuximab, OKT-9, trastuzumab.

A pharmaceutically active substance in this invention is an effector molecule that is used to achieve a beneficial outcome in an organism, preferably a vertebrate, more preferably a human being such as a cancer patient or an auto-immune patient. Benefit includes diagnosis, prognosis, treatment, cure and/or prevention of diseases and/or symptoms. The pharmaceutically active substance may also lead to undesired harmful side effects. In this case, pros and cons must be weighed to decide whether the pharmaceutically active substance is suitable in the particular case. If the effect of the pharmaceutically active substance inside a cell is predominantly beneficial for the whole organism, the cell is called a target cell. If the effect inside a cell is predominantly harmful for the whole organism, the cell is called an off-target cell. In artificial systems such as cell cultures and bioreactors, target cells and off-target cells depend on the purpose and are defined by the user.

An effector molecule that is a polypeptide may be, e.g., a polypeptide that recover a lost function, such as for instance enzyme replacement, gene regulating functions, or a toxin.

An embodiment is the scaffold according to the invention, wherein the scaffold is a tri-functional linker comprising a second chemical group with at least one biologically active molecule covalently bound thereto, comprising a third chemical group for covalent binding to a molecule and comprising the first chemical group for covalent binding to the carrier, preferably the tri-functional linker is the tri-functional linker of Scheme II and Structure B.

An embodiment is the scaffold according to the invention, wherein the at least one biologically active molecule is a defined number of glycoside molecules or a defined range of glycoside molecules, preferably 1-128 or at least 2, 3, 4, 5, 6, 8, 10, 16, 32, 64 or 128 glycoside molecules, or any number of glycoside molecules therein between, such as 7, 9, 12 glycoside molecules.

An embodiment is the scaffold according to the invention, wherein the polymeric or oligomeric structure comprises a linear, branched and/or cyclic polymer, oligomer, dendrimer, dendron, dendronized polymer, dendronized oligomer, a DNA, a polypeptide, a poly-lysine, a poly-ethylene glycol, or an assembly of these polymeric or oligomeric structures which assembly is preferably built up by covalent cross-linking.

The scaffold is fundamentally independent of the type of an effector molecule covalently bound to the scaffold. Thus, the scaffold is the basis product for a new platform technology. Since the at least one covalently bound glycoside mediates intracellular delivery, the scaffold technology according to the invention is the first system known that mediates controlled intracellular effector molecule delivery by glycosides.

Synchronization is the missing link between a very successful delivery strategy for mice and its application in humans. Indeed, the inventors established in a series of in vivo mouse tumor models that separately administering to the mice a dose of free saponin and a dose of an ADC did not result in any desired anti-tumor activity such as delayed tumor growth, tumor regression, diminished and slower tumor growth, compared to control animals not treated with the ADC and free saponin. The free saponin was administered using various routes of administration and using various time points of administering the free saponin compared to the moment of administering the ADC (administering free saponin before, during and after administering the ADC). The ADC tested in in vivo tumor models was cetuximabdianthin (with free SO1861), or trastuzumab-saporin (with free SO1861). Varying the dose of free saponin did not provide for an efficacious anti-tumor activity. The ADCs referred to were administered at a dose that in itself did not inflict any beneficial anti-tumor effect on the tumor-bearing animals. Surprisingly, the inventors now established that beneficial anti-tumor activity in various in vitro mammalian cell-based bioassays and/or in various in vivo animal tumor models can be achieved by treating the animals with conjugates according to the invention, comprising a scaffold according to the invention. The scaffold for example being a dendron with up to four covalently bound saponin molecules such as SO1861 via cleavable linkers. The scaffold for example being a tri-functional linker with a covalently bound saponin (e.g. SO1861, QS-21) via a cleavable or non-cleavable linkage, and with a covalently bound effector moiety (e.g. dianthin, silencing BNA (HSP27) via a non-cleavable bond or a cleavable bond, and with a covalently bound monoclonal antibody such as cetuximab, trastuzumab, OKT-9, or the scaffold being a dendron, such as a dendron to which for example four moieties can bind such as four saponin molecules, or a dendron for binding for example two saponins and two effector molecules, the dendron comprising a chemical group for (covalent) coupling to a ligand or an antibody or fragment or domain thereof. Reference is made to the Examples section, exemplifying various of these scaffolds according to the invention, showing in vivo and/or in vitro anti-tumor cell activity when cell toxicity exerted by e.g. a proteinaceous toxin is considered or when gene silencing in the tumor cell is considered.

The scaffold of the invention provides an optimized and functionally active unit that can be linked to the effector molecule and/or to a ligand, an antibody, etc., at a single and defined position, preferably via covalent bonds and when desired for the intended purpose, via cleavable covalent bonds.

Without wishing to be bound by any theory, in view of the failures observed when treatment of tumor-bearing animals with an ADC together with free saponin is considered, it is preferred to synchronize the presence of both, the at least one glycoside, preferably a saponin, and the effector molecule, preferably a toxin or an oligonucleotide such as a BNA, in compartments or vesicles of the endocytic pathway of the target cell, e.g. a tumor cell or an auto-immune cell. With ADC and free saponin, synchronizing the presence of the molecules in the late endosomes, in order to obtain the synergistic effects *in vivo* was not beneficially obtainable according to the numerous attempts of the inventors. In one aspect, the invention preferably solves at least one of the following problems with respect to combining the effector molecule and the glycoside molecules in one compound, comprising the scaffold of the invention: (1) the number of required glycoside molecules per effector molecule is a defined number or range (e.g., preferably 1 or more, preferably at least 2, more preferably at least 3, more preferably at least 5, more preferably at least 6, more preferably at least 10, more preferably at least 15, more preferably at least 20, more preferably at least 25, more preferably at least 27, most preferably at least 30 or more) so that a simple chemical linkage is not expedient; (2) the only reasonable chemical group within, e.g., the saponins that can be used for (covalent), in particular single and cleavable, retainable coupling is required for the endosomal escape activity; (3) the effector molecule may not possess a suitable counter group for coupling; and (4) glycosides may lose their necessary potential to interact with cholesterol when not freely diffusible. All these restrictions are most likely the reason why glycosides have not been used in combination with pharmaceutically active substances in clinical investigations other than the application of saponins in vaccination regimes wherein the use of an immune-potentiating adjuvant substance was implied, although the striking endosomal escape enhancer effect of, e.g., saponins listed in Table A1 and Scheme I is known for more than 10 years. A scaffold according to the present invention solves these difficulties, at least in part. Surprisingly, the saponins previously applied for their immune-potentiating activity in the vaccination context involving saponins as adjuvant component, are now also suitably for (covalent) coupling to the scaffold of the invention, for implication in a conjugate comprising the scaffold bearing the saponin and further having an effector molecule and optionally a cell-targeting moiety such as a ligand or antibody, (covalently) bound thereto, for anti-tumor activity in vitro and in vivo.

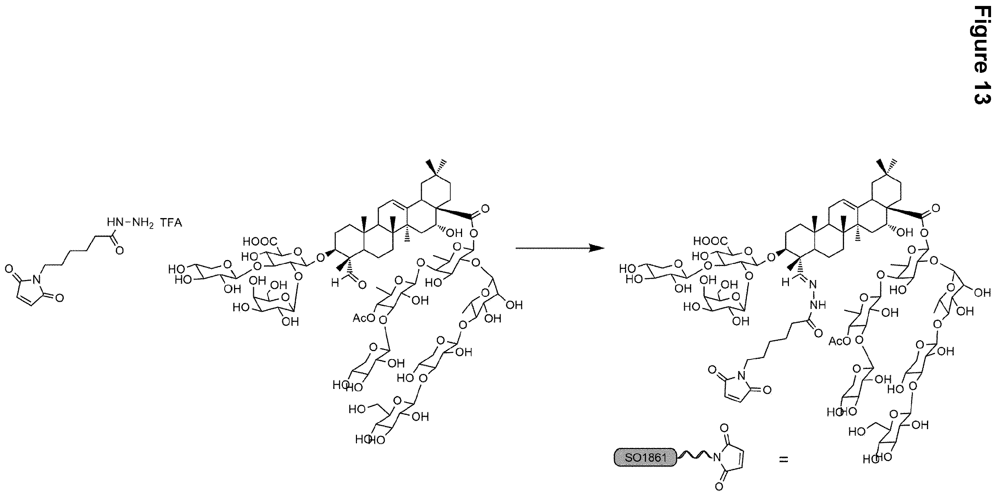

In its basic form, the scaffold comprises a polymeric and/or oligomeric structure that bears at least one glycoside molecules as defined in the claims, i.e. saponins, such as SO1861 (Table A1, Fig. 13). As defined in the claims, a scaffold is provided, wherein the glycoside molecules are bound to the polymeric or oligomeric structure via a hydrazone bond.

An embodiment is the scaffold according to the invention, wherein the glycoside molecules are SA1641, SO1861, GE1741, QS-21 or any of their stereomers such as any of their diastereomers.

An embodiment is the scaffold according to the invention, wherein the carrier molecule comprises or consists of any of a proteinaceous molecule, a protein, a peptide, a nucleic acid, an oligonucleotide, a lipid, a fat, a fatty acid, a nanoparticle, a carbohydrate, or any covalently bound conjugate or covalently bound complex of combinations thereof.

An embodiment is the scaffold according to the invention, wherein the carrier molecule comprises or consists of an immunoglobulin, at least one binding domain of an immunoglobulin and/or at least one binding fragment of an immunoglobulin, such as an antibody, an IgG, a molecule comprising or consisting of a Vhh domain or Vh domain, a Fab, an scFv, an Fv, a dAb, an F(ab)₂, Fcab fragment, or comprises or consists of at least one non-proteinaceous ligand and/or at least one proteinaceous ligand for binding to a cell-surface molecule such as EGF or a cytokine.

An embodiment is the scaffold according to the invention, wherein the carrier molecule comprises or consists of at least one binding domain and/or at least one binding fragment for binding to a cell-surface receptor such as a tumor-cell specific cell-surface receptor selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably selected from CD71, EGFR, HER2.

An embodiment is the scaffold according to the invention, wherein the carrier molecule comprises or consists of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell receptor binding-fragment thereof and/or at least one tumor-cell receptor binding-domain thereof, such as at least one tumor-cell specific receptor binding-fragment thereof and/or at least one tumor-cell specific receptor binding-domain thereof.

An embodiment is the scaffold according to the invention, wherein the scaffold is suitable for forming a covalent bond with the carrier molecule, said covalent bond preferably involving a cysteine side-chain of the carrier molecule and/or a lysine side-chain of the carrier molecule when the carrier molecule comprises at least a cysteine and/or a lysine.

An embodiment is the scaffold according to the invention, wherein the carrier molecule comprises or consists of at least one effector molecule, or wherein the carrier further comprises at least one effector molecule when also comprising an immunoglobulin, a binding fragment, a binding domain thereof, etc., etc., according to the invention, wherein the effector molecule is at least one of an active pharmaceutical substance, such as any one or more of a payload, a toxin, a drug, a polypeptide, an oligonucleotide, a nucleic acid, a xeno nucleic acid, an enzyme such as urease and Cre-recombinase, a protein toxin, a ribosome-inactivating protein.

An embodiment is the scaffold according to the invention, wherein the protein toxin comprises or consists of any one or more of a protein toxin selected from Table A5 and/or a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin.

An embodiment is the scaffold according to the invention, wherein the oligonucleotide, the xeno nucleic acid or the nucleic acid comprises or consists of any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, preferably a BNA, for example a BNA for silencing HSP27 protein expression.

With the scaffold of the invention it has now become possible to design and manufacture a one-component, non-viral clinically applicable gene delivery technology. For example, the scaffold of the invention allows for development of non-viral based gene delivery technology, which enhances therapeutic efficacy with lower therapeutic dose thereby improving the health of patients. The scaffold of the invention, in particular when covalently bound to a carrier molecule such as a monoclonal antibody for binding to a (tumor, auto-immune) cell-surface specific molecule, and when bound to a carrier molecule such as an oligonucleotide for example a BNA, allows for overcoming a longstanding and major bottleneck in the field of gene delivery, namely efficient, safe and cost-effective transfer of gene therapeutic products across the endosomal membrane into the cytosol/nucleosol. Indeed, gene therapy is one of the most promising treatment options for future advanced therapies in a broad range of diseases. Successful gene delivery requires the recognition of target cells as well as cytosolic and nucleosolic uptake of the gene. One of the major problems in the field of non-viral gene therapy is the inefficient and insufficiently safe delivery of genetic material for therapeutic use in patients.

Thus, when applying the scaffold of the invention, comprising a cell-targeting carrier molecule such as a ligand or preferably an antibody (fragment, domain thereof) and comprising an oligonucleotide such as an antisense BNA, the inventors now made it possible to overcome a longstanding and major bottleneck in the field of gene delivery: safe transfer of gene therapeutic products across the endosomal membrane into the cytosol/nucleosol. The scaffold of the invention represents technology designed for allowing targeting of any addressable cell type with all known genetic agents, thereby ensuring better patient therapy not limited to inherited disorders, but also for cancer therapy and therefore of importance for large patient groups. The technology based on the scaffold of the invention is a polymeric or oligomeric scaffold that serves as a carrier for endosomal escape enhancers (EEEs), such as the saponins of Table A1 and Scheme I and any of the embodiments according to the invention, for the targeting ligand or (monoclonal) (tumor-cell specific) antibody, and for the effector moiety, here an effector gene such as an LNA or BNA. Use of the scaffold of the invention, e.g. comprising a cell-targeting antibody (fragment) and an oligonucleotide such as a BNA, has potential to bring any kind of biological macromolecules into the cytosol and the nucleus. Development of new targeting ligands and monoclonal (human, humanized) antibodies is under continuous investigation by numerous research groups and companies worldwide. The same for the oligonucleotides that are aimed for delivery in the cytosol of diseases cells such as cancer cells. The scaffold of the invention thus provides a molecular interface for linking present and future targeting ligands and antibodies and present and future therapeutic oligonucleotides (as well as payloads such as protein toxins) to the oligomeric or polymeric scaffold module of the invention by click chemistry, allowing for customized drug applications and for future developments in the field of tissue and cell targeting techniques. The scaffold of the invention can be combined with antibodies and ligands as the covalently coupled carrier molecule. The worldwide market of gene therapeutics is rapidly growing and is covering potential treatments for a wide range of disease areas such as, cancer, cardiovascular diseases, Parkinson's, Alzheimer, HIV and many rare (monogenetic) diseases. The current viral vector-based gene therapeutic technologies have significant challenges, such as safety, manufacturing logistics, and associated high costs. The scaffold of the invention allows for use in a technology platform which represents an alternative for a current viral gene delivery technology. Therefore, the scaffold of the invention is suitable for implementing in approaches for developing non-viral gene treatments for diseases such as cancers, cardiovascular diseases, Parkinson's disease, Alzheimer's disease, HIV infection and many rare (monogenetic) diseases. The scaffold of the invention is suitable for developing novel treatments for transforming the field of antibody-drug conjugates (ADCs) and oligonucleotide-based therapeutics by making non-viral vector based gene therapeutics such as based on targeted antisense BNA. The application of the scaffold of the invention, in particular in a covalent conjugate with an antibody and an oligonucleotide such as a BNA is one of the many beneficial approaches made possible due to the present invention. For example, use of the scaffold of the invention now allows for exploitation of the endocytic pathway of mammalian cells. Endocytosis is exploited for the delivery of therapeutics, wherein the scaffold of the invention contributes to improved uptake and endosomal escape of e.g. siRNAs which are conjugated with the scaffold. The scaffold of the invention is suitably used together with small molecules that act as delivery enhancers for e.g. payloads, oligonucleotides. Herewith, the scaffold of the invention bearing the covalently coupled oligonucleotide such as a BNA and bearing the covalently coupled cell targeting moiety such as a ligand and preferably an antibody (domain or fragment), provides a solution for the current problem seen with current endosomal escape enhancers and gene therapeutic product, relating to their application as two components, thus complicating therapeutic approval and clinical applicability, since such a scaffold of the invention is a single-conjugate therapeutic molecule encompassing the saponin, gene product such as a BNA and the (tumor) cell targeting moiety such as a (monoclonal) antibody. Thus the invention provides a non-viral gene delivery technology where endosomal escape enhancers (e.g. the glycosides of Table A1, Scheme I, embodiments of the invention), gene therapeutic product (oligonucleotides according to the invention such as a BNA) and targeting ligand or antibody (according to e.g. Table A2, A3, A4, embodiments of the invention) are all bound to one molecular scaffold of the invention. Such a scaffold of the invention thus provides therapeutic opportunities for current and future macromolecule drugs for a broad range of diseases and large patient groups. With the application of such a scaffold of the invention comprising at least one saponin, at least one oligonucleotide and at least one specific cell-targeting moiety such as an immunoglobulin, the problem is addressed which is apparent for current methods of applying endosomal escape enhancers and gene therapeutic product separately, which current methods do not ensure that both compounds are at the same time at the site of interaction. This problem is now overcome by using the scaffold of the invention. That is to say, such a scaffold of the invention provides a non-viral gene delivery technology with increased synchronization (in time and place) of both compounds, i.e. the saponin and the gene product such as a BNA.

Gene therapies could help with hereditary, previously incurable diseases such as cystic fibrosis, chorea, Huntington's disease or hemophilia. However, currently some problems have not been overcome: for example, the therapeutic genes must precisely reach specific target cells in the body. On the other hand, the therapeutic genes should be absorbed by the targeted cells, but the therapeutic genes should not be destroyed. The current gene therapy approaches use viruses as a ferry for genes. However, these procedures involve considerable risks and cannot be transferred to the introduction of other biomolecules. An embodiment is the scaffold of the invention comprising (plant-derived) glycosides for use a platform technology that allows not only delivery of genes when bound to the scaffold as the carrier molecule, but also allows for the delivery of different therapeutic biomolecules to be introduced into target cells. Therefore, the scaffold of the invention is used for developing treatments based on nucleic acids for cystic fibrosis, chorea, Huntington's disease or hemophilia. Herewith, with the scaffold of the invention, a new gene therapy strategy is available for improving the health of patients with genetic diseases, including those patients with cystic fibrosis, Huntington's disease, and hemophilia. As part of the invention, a non-viral gene delivery technology is developed that combines plant-derived endosomal escape enhancers (glycosides), gene therapeutic products, and a targeting ligand that are all bound to a single molecular scaffold. The resulting non-viral gene therapy based on the scaffold of the invention displays about 40 times increased delivery efficiency at a lower dosage over currently available strategies. Herewith, the scaffold of the invention is for use in clinical applications such as for the repair or replacement of defective genes, like in cystic fibrosis patients, and for the targeted delivery of specific genes, for instance, to destroy cancer cells. In fact, the scaffold of the invention is suitable for application in treatment regimens for any disease caused by a genetic defect - such as cystic fibrosis, Huntington's disease and hemophilia and which are currently incurable. Gene therapy which makes use of the scaffold of the invention helps in overcoming two current problems: Firstly, it is possible with the scaffold of the invention to deliver therapeutic genes to specific target cells in the body; Secondly, the therapeutic genes enter the interior of these cells, but are not destroyed, due to the presence of saponin(s), the oligonucleotide product and a targeting moiety such as an antibody for binding a target cell, all covalently linked to the oligomeric or polymeric scaffold of the invention.

An embodiment is the scaffold according to the invention, wherein the effector molecule comprises or consists of at least one payload, preferably selected from any one or more of a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

An embodiment is the scaffold according to the invention, wherein the carrier molecule comprises or consists of a covalently linked combination of an effector molecule and a monoclonal antibody, preferably selected from Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3.

An aspect of the invention relates to a method for producing a scaffold suitable for covalently binding at least one biologically active molecule to a carrier molecule as defined in the claims, the method comprising: a) providing a polymeric or oligomeric structure comprising a first chemical group for covalently coupling of the polymeric structure or the oligomeric structure to the carrier molecule and comprising at least one of a second chemical group different from the first chemical group, wherein each second chemical group is for covalently coupling one of the at least one biologically active molecules to the oligomeric or polymeric structure; and b) covalently coupling at least one biologically active molecule to said polymeric or oligomeric structure via the second chemical group(s), wherein preferably the biologically active molecule(s) is/are any one of the biologically active molecules of the invention, more preferably SO1861 and/or GE1741 and/or SA1641 and/or QS-21 and/or any saponin of Table A1 and/or Scheme I, therewith providing the scaffold.

The polymeric or oligomeric structure is any of the polymeric or oligomeric structures according to the invention defined in the claims. Similarly, the first and second chemical groups for covalent coupling are chemical groups according to embodiments of the invention. The biologically active molecules are preferably any of the biological active molecules of the aspects and embodiments of the invention. Preferably, the biologically active molecule is a saponin according to the invention, and also preferably the saponin is covalently coupled to the scaffold via a linker such as a cleavable linker according to the invention. Typical scaffolds of the invention are a tri-functional linker and a dendron.

An embodiment is the method of the invention, wherein the at least one biologically active molecule is any one of the glycosides and saponins of the previous aspects and embodiments of the invention; and/or wherein the at least one biologically active molecule is coupled to the scaffold through a linker according to any of the previous aspects and embodiments of the invention; and/or wherein the saponin is linked to the scaffold through a covalent bond according to any one of the previous embodiments and aspects of the invention; and/or wherein the saponin is coupled to the scaffold through a cleavable bond of any one of the previous embodiments of the invention.

An aspect of the invention relates to a method for producing a scaffold covalently bound to a carrier molecule, the scaffold comprising at least one covalently bound biologically active molecule, as defined in the claims, the method comprising: a) providing a scaffold comprising at least one biologically active molecule covalently bound to a polymeric or oligomeric structure in said scaffold, preferably providing a scaffold according to the invention or the scaffold obtainable by the method of the invention or the scaffold obtained with the method of the invention; and b) covalently coupling the scaffold of a) to a carrier molecule according to the invention, therewith providing the scaffold covalently bound to a carrier molecule, the scaffold comprising at least one covalently bound biologically active molecule, preferably the scaffold according to the invention.

An embodiment is the method of the invention, wherein the at least one biologically active molecule is any one of the glycosides and saponins of the previous aspects and embodiments of the invention; and/or wherein the at least one biologically active molecule is coupled to the scaffold through a linker according to any of the previous aspects and embodiments of the invention; and/or wherein the saponin is linked to the scaffold through a covalent bond according to any one of the previous embodiments and aspects of the invention; and/or wherein the saponin is coupled to the scaffold through a cleavable bond of any one of the previous embodiments of the invention; and/or wherein the carrier molecules is or comprises any one of the payloads and effector molecules according to any of the previous embodiments and aspects of the invention and/or is or comprises any one of the ligands and immunoglobulins, monoclonal antibodies and/or any binding domain or -fragment thereof according to any one of the previous aspects and embodiments of the invention. Typically the biologically active molecule is any of SO1861, SA1641, GE1741, QS-21. Typically, the carrier molecule comprises or is cetuximab, trastuzumab, OKT-9. Typically, the effector molecule or payload is a BNA, a protein toxin, dianthin, saporin, an oligonucleotide. A preferred biologically active molecule of the invention is the saponin fraction from Quillaja saponaria, e.g. the water-soluble saponins fraction of Quillaja saponaria.

An embodiment is the scaffold according to the invention or the method according to the invention, in particular when the saponin is SO1861, SA1641, GE1741 and/or QS-21, wherein the scaffold is able to augment (late) endosomal escape and/or lysosomal escape of the effector molecule according to the invention when either said effector molecule is covalently bound to the scaffold and contacted with a mammalian cell, in particular a tumor cell e.g. in a human subject, or when said effector molecule is contacted with a mammalian cell, in particular a tumor cell e.g. in a human subject, in the presence of the scaffold of the invention.

Examples of polypeptides (proteinaceous molecules) as effector molecules are, e.g., Cas9; toxins (e.g. saporin, dianthin, gelonin, (de)bouganin, agrostin, ricin (toxin A chain); pokeweed antiviral protein, apoptin, diphtheria toxin, pseudomonas exotoxin), metabolic enzymes (e.g. argininosuccinate lyase, argininosuccinate synthetase), enzymes of the coagulation cascade, repairing enzymes; enzymes for cell signaling; cell cycle regulation factors; gene regulating factors (transcription factors such as NF-κB or gene repressors such as methionine repressor).

An effector molecule that is a polynucleotide may, e.g., be a polynucleotide that comprises coding information, such as a gene or an open reading frame encoding a protein. It may also comprise regulatory information, e.g. promotor or regulatory element binding regions, or sequences coding for micro RNAs. Such polynucleotide may comprise natural and artificial nucleic acids. Artificial nucleic acids include peptide nucleic acid (PNA), Morpholino and locked nucleic acid (LNA), as well as glycol nucleic acid (GNA) and threose nucleic acid (TNA). Each of these is distinguished from naturally occurring DNA or RNA by changes to the backbone of the molecule. Examples of nucleotides as effector molecules are, e.g., DNA: single stranded DNA (e.g. DNA for adenine phosphoribosyltransferase); linear double stranded DNA (e.g. clotting factor IX gene); circular double stranded DNA (e.g. plasmids); RNA: mRNA (e.g. TAL effector molecule nucleases), tRNA, rRNA, siRNA, miRNA, antisense RNA.

A toxin in this invention is a pharmaceutically active substance that is able to kill a cell. Preferably, a targeted toxin is a toxin that is only or at least predominantly toxic for target cells but not for off-target cells.

An effector molecule useful in the present invention preferably relies on late endosomal escape for exerting its effect. Some effectors, such as, e.g., a pseudomonas exotoxin, are rerouted to other organelles prior to the "late endosomal stage" and, thus, would normally not benefit from a scaffold according to the present invention. However, such toxin may be adapted for use with the present invention, e.g., by deleting the signal peptide responsible rerouting. In particular toxins that are highly toxic and would require only one molecule to escape the endosomes to kill a cell maybe modified to be less potent. It is preferred to use a toxin that kills a cell if at least 2, more preferably at least 5, more preferably at least 10, more preferably at least 20, more preferably at least 50, most preferably at least 100 toxin molecules escape the endosome. It is further preferred that a functionalized scaffold, i.e. a scaffold of the invention comprising covalently bound effector molecule(s) and/or a ligand and/or a monoclonal antibody, etc., for targeting the scaffold at a target cell such as a tumor cell or an autoimmune cell, comprises a ratio of at least 2 : 1, more preferably at least 5 : 1, more preferably at least 10 : 1, more preferably at least 20 : 1, most preferably at least 50 : 1 glycoside molecules for each effector molecule covalently bound to the scaffold. In particular in a functionalized scaffold comprising an assembled polymeric structure, wherein the glycoside molecules and the effector molecules are attached to different polymeric structures within said assembly, it is preferred to have a ratio of at least 10 : 1, more preferably at least 20 : 1, more preferably at least 50 : 1, more preferably at least 100 : 1, most preferably at least 200 : 1 glycoside molecules with respect to effector molecule in such assembly. Further, in order to reduce off-target toxicity, cell membrane non-permeable small molecule toxins are preferred effector molecules over cell membrane permeable toxins.

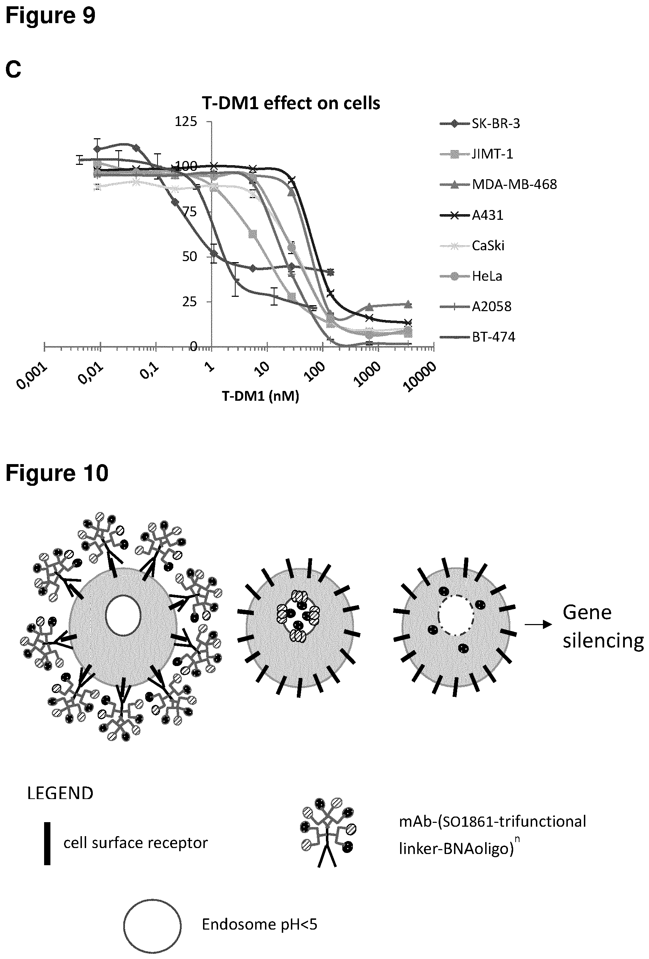

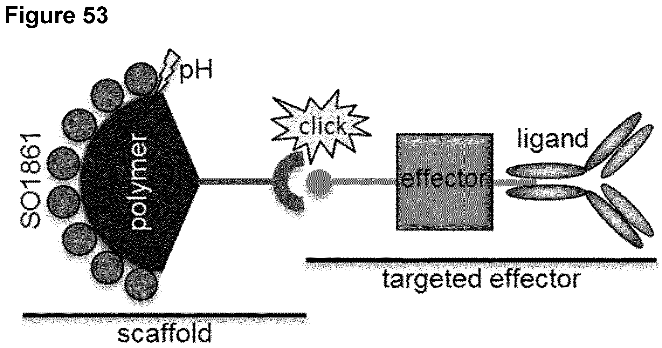

The invention further provides a functionalized scaffold comprising at least one scaffold according to the invention, coupled to either a) at least one effector molecule, b) at least one ligand, c) at least one effector molecule and in addition at least one ligand (Figs. 10-12, 54), d) at least one effector molecule that itself bears at least one ligand (Fig. 53), or e) at least one ligand that itself bears at least one effector molecule. Such coupling in a) - e) maybe achieved through a cleavable (labile) or stable (non-cleavable) bond. Preferably, coupling in a) - e) independently occurs via click chemistry bonds. Preferably, the functionalized scaffold is able to enhance endosomal escape of the effector. An embodiment is the scaffold of the invention wherein the scaffold is a functionalized scaffold according to the invention, wherein said at least one effector molecule is a pharmaceutically active substance, such as a toxin, a drug, a polypeptide and/or a polynucleotide. An embodiment is the functionalized scaffold of the invention wherein the effector molecule is a toxin or a polynucleotide coding for a protein.

The term "ligand" as used in this invention has its ordinary meaning and preferably means a molecule or structure that is able to bind another molecule or structure on the cell surface of a target cell, wherein said molecule or structure on the cell surface can be endocytosed and is preferably absent or less prominent on off-target cells. Preferably, said molecule or structure on the cell surface is constitutively endocytosed. More preferably a ligand in this invention induces endocytosis of said molecule or structure on the cell surface of target cells after binding to said molecule or structure. This is for instance the case for Epidermal Growth Factor Receptor (EGFR), present on the surface of a variety of cancer cells. Examples of molecules or structures on the cell surface of target cells that are constitutively endocytosed, are for instance Claudin-1 or major histocompatibility complex class II glycoproteins. A ligand can, e.g., be an antibody, a growth factor or a cytokine. Combining in a carrier molecule a toxin with a ligand is one possibility to create a targeted toxin. A toxin that is only toxic in a target cell because it interferes with processes that occur in target cells only can also be seen as a targeted toxin (as in off-target cells it cannot exert its toxic action, e.g. apoptin). Preferably, a targeted toxin is a toxin that is combined with a ligand or e.g. a monoclonal antibody in order to be active in target cells and not in off-target cells (as it is only bound to and endocytosed by target cells). In a functionalized scaffold comprising a carrier molecule comprising a ligand and an effector molecule, the ligand or the monoclonal antibody guides the effector molecule and scaffold to the target cells. After internalization, the at least one glycoside, i.e. the saponin, mediates the endosomal escape of the effector molecule. The saponin is typically a saponin listed in Table A1 and Scheme I, and preferably the saponin is SO1861 and/or QS-21, and/or SA 1641 and/or GE1741.

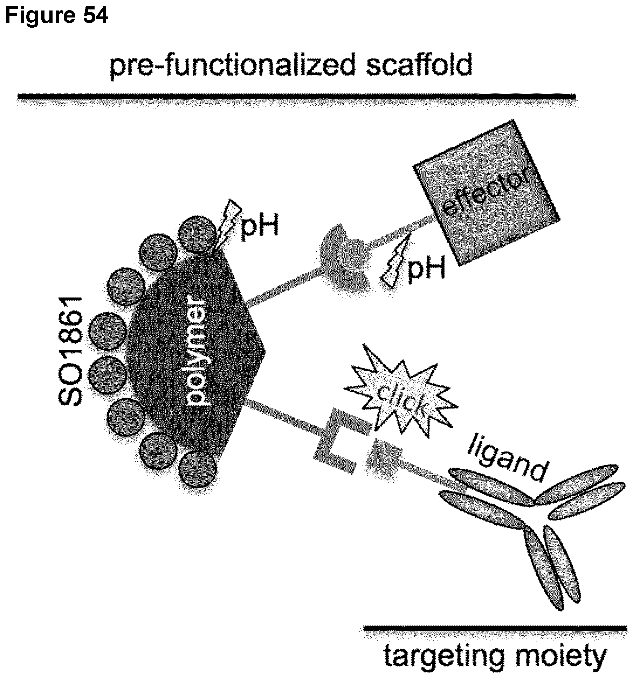

The scaffold of the invention which is not provided with a carrier molecule covalently linked to the scaffold, such as an effector molecule and/or a cell-targeting ligand or antibody, i.e., a nonfunctionalized scaffold, the provided scaffold can be supplied to, e.g., a drug manufacturer, who will then be responsible for the coupling of the effector molecule alone or effector molecule and ligand or antibody to the scaffold. The drug manufacturer can, if required, add cleavable units to release the effector molecule from the scaffold and/or ligand, antibody, e.g. by inserting disulfide bridges between effector molecule and ligand and/or effector molecule and click position. The invention also provides a (pre)functionalized version of the scaffold, wherein this functionalized scaffold already bears an effector molecule, e.g. a tumor cell-killing toxin (Fig. 54). The activity of the scaffold according to the invention, relating to endosomal effector molecule release, is preferably already included in the scaffold. The functionalized scaffold can be supplied to the pharmaceutical industry, e.g. for further development of existing and future therapeutic antibodies and to any supplier or owner of antibodies to functionalize the targeting antibody. Functionalized scaffolds can also be used by biotechnology companies or for research.

With the scaffold of the invention, comprising an effector molecule in the carrier molecule covalently bound to the scaffold, and comprising a cell-targeting moiety in the carrier molecule covalently bound to the scaffold, such as an immunoglobulin, such as listed in Table A2-A4, the inventors now for the first time provide a conjugate with covalently bound glycoside, effector molecule and monoclonal antibody, and a conjugate with covalently bound glycoside and an effector molecule, and a conjugate with covalently bound glycoside and a cell-targeting molecule such as a ligand or a monoclonal antibody (fragment, domain), for targeted delivery of effector molecules inside target diseased cells such as tumor cells. Although perhaps administered to a patient in need thereof in a systemic manner (site-directed, localized administration is preferred), the scaffold of the invention exerts its intracellular activity specifically inside target cells that bear and expose the binding partner for the ligand or the antibody, when the scaffold is provided with such a ligand or antibody which preferentially and specifically binds to the desired target cells. This way, if for example the effector molecule is also provided with the same or a different target cell specific ligand or antibody, specific for binding to the same or a different cell-surface molecule present on the same target cell such as a target tumor cell or target auto-immune cell, the effector molecule and the glycoside bound to the ligand or antibody are directed and ideally accumulate in the same (late) endosomes, lysosomes of the same targeted (diseased) cell in which the cell-killing effect of the effector molecule is intended.

The new (functionalized) scaffold provides a number of advantages:
1) Use of the functionalized scaffold, i.e. the scaffold of the invention comprising covalently bound effector molecule and ligand or antibody, results in a one-component system, i.e. the effector molecule and endosomal escape enhancer, i.e., the at least one glycoside, are delivered at the same time in a pre-defined ratio to the endosomes, due to the presence of the cell-targeting ligand or antibody, preferably a monoclonal antibody such as any one of the antibodies in Table A2-A4.
2) The at least one glycoside molecule is now also targeted by joint use of the targeting ligand or monoclonal antibody of the effector molecule; thus the glycosides are not distributed throughout the whole body and taken up randomly by cells, which aids in to reducing possible side effects and broadens the therapeutic window.
3) The number of glycoside molecules per effector molecule can be exactly defined and therefore be reduced to the required minimum; side effects by surplus glycoside molecules can be avoided. A defined number of glycoside molecules per effector molecule also facilitates marketing authorization for a specific medicament.
4) The present invention allows to offer a preformed effector molecule-loaded scaffold (functionalized scaffold) to be used with any available ligand and/or (monoclonal) antibody (or at least one binding fragment and/or -domain thereof), which makes the invention optimal for platform development.
5) If the scaffold or functionalized scaffold is attached to a carrier molecule, it is also possible that the carrier molecule bears the ligand or antibody and/or the effector molecule. In such case, the carrier molecule is considered a linker.

One other application of the present invention is, e.g., gene therapy. The efficient intracellular delivery of biological macromolecules, such as, e.g., polynucleotides, is currently still a major hurdle. In contrast to conventional unspecific DNA transfection systems, the present invention is not limited to DNA and is specific for target cells. Known viral systems are efficient and specific for target cells, however, they are only suitable for DNA. Moreover, they bear the risk of immune and inflammatory responses, possess a potential oncogenic activity and require complex and expensive procedures for preparation in each individual case. The novelty of the here presented technology is based on its fundamentality, flexibility and ease of use.

An embodiment is the scaffold of the invention wherein the scaffold is a functionalized scaffold, wherein the scaffold comprises a carrier molecule comprising at least one ligand or antibody, said at least one ligand or antibody being capable of specifically binding to a target cell specific surface molecule or structure, wherein preferably the functionalized scaffold is, after binding, endocytosed together with the surface molecule. Preferably, said target cell is a diseased or disease-related cell, preferably a tumor cell, a tumor-associated cell (e.g. tumor vascular cell), an immune cell (e.g. a T regulatory cell), or a cell with a monogenic defect. With the term "target cell specific surface molecule" is meant that the molecule is preferably expressed in the target cell and to a lesser extent in a non-target cell, either qualitatively or quantitatively. Examples of such target cell specific surface molecules are the receptor EGFR that is upregulated on tumor cells but also expressed (in a lower level) on, e.g., fibroblast in the skin, and HER2, which is overexpressed in breast cancer cells. However, many target cell specific surface molecules are known in the art and the skilled person is very well capable of choosing a target cell specific surface molecule for a specific purpose, i.e., to discriminate a target cell from a non-target cell for a specific disease or application. See also Table A2, A3 and A4 for examples of tumor-cell specific receptor binding antibodies, and see also the embodiments described here above concerning tumor-cell specific receptors suitable for targeting by carrier molecules comprised by the scaffold of the invention, e.g. monoclonal antibodies. As used herein, "monogenic defect" has its usual meaning which is a modification in a single gene occurring in substantially all cells of the body. The mutation may be present on one or both chromosomes (one chromosome inherited from each parent). Though relatively rare, monogenic defects affect millions of people worldwide. Scientists currently estimate that over 10,000 of human diseases are known to be monogenic disease. Non-limiting examples of monogenic diseases know to date are: sickle cell disease, cystic fibrosis, polycystic kidney disease, and Tay-Sachs disease.

An embodiment is the scaffold of the invention wherein the scaffold is a functionalized scaffold according to the invention, wherein the scaffold comprises a carrier molecule covalently bound thereto and comprising a ligand and/or an antibody, the at least one ligand being an antibody or a derivate or fragment thereof (e.g. VHH or scFv), a cytokine, a growth factor, or an antibody-like molecule such as an aptamer or a designed ankyrin repeat protein (DARPin). DARPins are genetically engineered antibody mimetic proteins typically exhibiting highly specific and high-affinity target protein binding. They are derived from natural ankyrin proteins, which are responsible for diverse cellular functions. They constitute a new class of potent, specific and versatile small-protein (typically 14 to 18 kDa) therapies, and are used as investigational tools in various research, diagnostic and therapeutic applications. Other non-limiting examples of antibodies or derivatives thereof known to date are: (i) a Fab' or Fab fragment, a monovalent fragment consisting of a variable light domain, a variable heavy domain, a constant light domain and a constant heavy domain 1, or a monovalent antibody as described in WO2007059782; (ii) F(ab')2 fragments, bivalent fragments comprising two Fab fragments linked by a disulfide bridge at the hinge region; (iii) an Fd fragment consisting essentially of the variable heavy domain and the constant heavy 1 domain; and (iv) a Fv fragment consisting essentially of the variable light and variable heavy domains of a single arm of an antibody. Furthermore, although the two domains of the Fv fragment, variable light and variable heavy, are coded for by separate genes, they may be joined, using recombinant methods, by a synthetic linker that enables them to be made as a single protein chain in which the variable light and variable heavy regions pair to form monovalent molecules (known as single chain antibodies or single chain Fv (scFv)).

Preferably, the effector molecule, which effect is enhanced by the glycoside molecules (e.g. saponins), detaches from the scaffold and/or ligand or antibody when endocytosed. This can be achieved by a cleavable bond that breaks, e.g. under acidic, reductive, enzymatic or light-induced conditions. an embodiment is the scaffold of the invention, therefore, which scaffold is a functionalized scaffold according to the invention, wherein said at least one effector molecule is bound to said scaffold and/or to said at least one ligand or antibody via a cleavable bond, wherein preferably said cleavable bond is subject to cleavage under acidic, reductive, enzymatic or light-induced conditions. Preferably the cleavable bond is an imine, hydrazone, oxime, 1,3-dioxolane, disulfide or ester, more preferably a disulfide or hydrazone bond.

An embodiment is the scaffold of the invention wherein the scaffold is a functionalized scaffold according to the invention, wherein said at least one effector molecule is bound to said scaffold and/or to said at least one ligand or antibody via a stable bond, e.g. through an amide coupling or amine formation. This is, e.g., realized via carbodiimide-mediated amide bond formation by an amino group of the polymeric or oligomeric structure of the scaffold and an activated carboxylic acid group on the effector molecule or ligand.

An embodiment is the scaffold or functionalized scaffold according to the invention, further comprising a carrier, such as a nanoparticle, liposome, micelle, colloid, or a particle-like structure comprising cholesterol and/or phospholipids.

As said before, the at least one glycoside molecule that is comprised within a scaffold according to the invention increases the efficacy of at least current and new effector molecules as defined in this invention. Potential side-effects will be decreased due to lowering of dosing of the effector molecule without lowering the efficacy. Therefore, the invention provides a scaffold according to the invention or a functionalized scaffold according to the invention for use in medicine or for use as a medicament. Thus, an aspect of the invention relates to a scaffold according to the invention, the scaffold comprising at least an effector molecule of the invention and/or an antibody according to the invention, preferably both the effector molecule and the antibody, for use as a medicament. Also provided is the use of a scaffold according to the invention or a functionalized scaffold according to invention for manufacturing a medicament. Especially cancer medicines, and in particular the classical chemotherapy medicaments, are notorious for their side effects. Because of targeting and synchronization in time and place of both the pharmaceutically active substance and the glycoside molecule, a scaffold or functionalized scaffold according to the invention is especially valuable for use as a medicament, in particular for use in a method of treating cancer. The invention thus provides a scaffold according to the invention or a functionalized scaffold according to the invention for use in a method of treating cancer. The invention also provides a scaffold according to the invention or a functionalized scaffold according to the invention for use in a method of treating acquired or hereditary disorders, in particular monogenic deficiency disorders. Thus, an aspect of the invention relates to a scaffold according to the invention, the scaffold comprising a covalently bound carrier molecule, which carrier molecule comprises at least an effector molecule of the invention and/or an antibody according to the invention, preferably both the effector molecule and the antibody, for use in a method for the treatment of a cancer or an auto-immune disease.

An aspect of the invention relates to the scaffold of the invention comprising an effector molecule and/or an antibody for targeting a tumor cell, for use in the treatment or prophylaxis of a cancer, wherein the scaffold is administered to a human subject in need thereof.

Also disclosed herein but not covered by the claims is the treatment of a human subject who suffers from a cancer or who is at risk of developing a cancer and who is in need of said treatment, the treatment comprising the step of administering to the human subject an effective dose of a pharmaceutical composition comprising a scaffold of the invention or comprising a functionalized scaffold of the invention, such scaffold comprising either an effector moiety together with a saponin, or a monoclonal antibody together with a saponin, or both an effector molecule and a monoclonal antibody together with the saponin. An aspect of the invention relates to a scaffold of the invention for use as a medicament. An aspect of the invention relates to a scaffold of the invention for use in a method for treatment or prophylaxis of a cancer or an auto-immune disease, in a human subject in need thereof. An aspect of the invention relates to the use of a scaffold or functionalized scaffold of the invention or a pharmaceutical composition comprising said scaffold or functionalized scaffold, for the manufacture of a medicament for anti-cancer therapy or anti-auto-immune disease therapy. The scaffold or functionalized scaffold preferably comprises at least a carrier molecule comprising an effector molecule and/or an antibody, preferably both the antibody and the effector molecule.

A further application in medicine is the substitution of intracellular enzymes in target cells that produce these enzymes in insufficient amount or insufficient functionality. The resulting disease might be hereditary or acquired. In most cases, only symptomatic treatment is possible and for a number of rare diseases, insufficient treatment options lead to a shortened life span of concerned patients. An example for such a disease is phenylketonuria, which is an inborn error of metabolism that results in decreased metabolism of the amino acid phenylalanine. The disease is characterized by mutations in the gene for the hepatic enzyme phenylalanine hydroxylase. Phenylketonuria is not curable to date. The incidence is approximately 1:10,000 with the highest known incidence in Turkey with 1:2,600. A functionalized scaffold with phenylalanine hydroxylase or with a polynucleotide that encodes phenylalanine hydroxylase can be used to target liver cells by use of a suitable ligand and to substitute the defect enzyme in hepatocytes; a scaffold covalently bound to a carrier molecule comprising phenylalanine hydroxylase or a polynucleotide that encodes phenylalanine hydroxylase can be used to target liver cells by use of a suitable ligand and to substitute the defect enzyme in hepatocytes. This is one example of use of the scaffold comprising a carrier molecule or a functionalized scaffold according to the invention for substitution or gene therapy. In a preferred embodiment, a scaffold according to the invention or a scaffold comprising a carrier molecule of the invention or a functionalized scaffold according to the invention for use in a method of gene therapy or substitution therapy is provided.

The invention can also be used for biotechnological processes. A possible application is the biomolecular engineering of intracellular switches in eukaryotes. Transcriptional switches target gene expression at the level of mRNA polymerization, translational switches target the process of turning the mRNA signal into protein, and post-translational switches control how proteins interact with one another to attenuate or relay signals. When optimized, these cellular switches can turn a cellular function "on" and "off" based on cues designated by the developer. These cues include small molecules, hormones and drugs. To apply the switch, the cue must enter the target cell. Therefore, in current applications, only small, diffusible molecules can be used that are neither specific for target cells nor do they have high specificity for the selected switch. A functionalized scaffold or a scaffold comprising a carrier molecule with a more complex and thus more specific, non-diffusible effector molecule can be used to target a particular switch and the use of a suitable ligand can restrict the effect to target cells. An embodiment is the use of a scaffold comprising a carrier molecule of the invention or a functionalized scaffold according to the invention for enhancing an effect of an effector molecule, preferably *in vitro.* Preferably, the use is for enhancing an effect of transcriptional switches *in vitro.*

Another application is the use of the invention in basic research. For functional analyses of cellular processes, it is often required to bring a protein into cells, a method called protein transfection. For instance, to investigate the molecular mechanisms of the chicken virus protein apoptin that leads to apoptosis in eukaryotic cells, it is required to bring the purified protein into the target cell. Existing protein transfection kits are, however, characterized by low efficacy, missing specificity for target cells and high toxicity and can therefore not be used for a number of applications, in particular when metabolic pathways are part of the investigation. A scaffold comprising a covalently linked carrier molecule of the invention or a functionalized scaffold, either of which with apoptin, and use of a suitable ligand can be used to conduct such investigations. An embodiment is a scaffold of the invention, a scaffold comprising a carrier molecule according to the invention or a functionalized scaffold according to the invention, for polypeptide transfection, preferably *in vitro.* Also provided is a use of a scaffold, a scaffold comprising a carrier molecule according to the invention or functionalized scaffold according to the invention for polynucleotide transfection, preferably *in vitro.*

Also disclosed herein but not covered by the claims is a method of treating cancer, the method comprising administering a medicament comprising a scaffold according to the invention or, preferably, a functionalized scaffold according to the invention, or preferably a scaffold of the invention comprising a carrier molecule comprising either an effector molecule or a monoclonal antibody, preferably both the monoclonal antibody and the effector molecule according to the invention, to a patient in need thereof, preferably administering an effective dose of said medicament to a patient in need thereof, preferably a human cancer patient.

The scaffold or functionalized scaffold stands for a platform technology that may
- Provide a widened therapeutic window for current and new ADCs, wherein the ADCs may comprise a payload such as a toxin, protein toxin, oligonucleotide, BNA
- provide highly efficient cytosolic delivery of macromolecules
- facilitate cellular research and biotechnical applications
- have a therapeutic potential in multiple diseases, such as cancers and auto-immune disease, rheumatoid arthritis
- be used to induce cellular destruction (e.g. of cancer cells)
- reduce unwanted side effects by lowering therapeutic levels required for diseased cells
- reduce the risk of an immune response to the effector molecule (as less effector molecule is needed, but, without wishing to be bound by any theory, maybe also because the route of antigen presentation through the endosomes onto MHC molecules is disrupted)
- open the possibility for highly efficient manipulation of genes
- resurrect failed drug candidates, in particular ADCs, by increasing their efficacy
- be made of biocompatible and degradable and/or excretable materials
- rely on a mild and non-hazardous effector molecule release triggered by endosomal pH

Flexibility is ensured by the possibility to use any type of a ligand (e.g. antibodies, fragments and domains thereof, or aptamers) and effector molecule. A sophisticated implementation of click chemistry may be used to provide a user-friendly interface to apply this technology to own ligands and effector molecules. The platform technology of the invention offers a variety of possibilities, such as production of the clickable scaffold as a stand-alone product, which allows the user to simply couple any of his effector molecules and/or ligands at his discretion (Fig. 53), or production of a functionalized scaffold or a scaffold comprising a carrier molecule covalently linked thereto, wherein the basic scaffold is already coupled to an effector molecule (such as a proteinaceous toxin of Table A5) and/or to a ligand such as a monoclonal antibody (such as an antibody of Table A2, A3, A4), which allows the user to couple his ligand to guide the effector molecule to the desired target cells (Fig. 54). A possible scaffold comprising a covalently coupled carrier molecule or a possible functionalized scaffold is a scaffold linked to a ribosome-inactivating protein, e.g. dianthin, saporin. This toxic enzymes with a high potential for targeted cell killing can be used to click any future antibodies or antibodies already existing on the market that are designed to specifically recognize tumor cells, such as trastuzumab, cetuximab, rituximab, gemtuzumab, OKT-9 or obinutuzumab (next generation ADC technology), or any of the antibodies listed in the previous embodiments and Tables A2-A4. As a nucleic acid effector molecule, micro-RNA (miRNA, a polynucleotide) or miRNA inhibitors, or LNA or BNA can for instance be used to create functionalized scaffolds for efficient and low dose cytosolic delivery. MiRNAs or miRNA inhibitors have high potential as novel therapeutics, capable of changing gene programs within the cell, and thereby changing cellular function.

The invention further provides a method for producing a scaffold according to the invention as defined in the claims, the scaffold comprising at least one glycoside molecule capable of improving an effect of an effector molecule, bound to a polymeric or oligomeric structure, the method comprising: providing the polymeric or oligomeric structure; and coupling the at least one glycoside molecule to said polymeric or oligomeric structure. Preferably, the at least one glycoside molecule augments endosomal escape of said effector molecule. Preferably, the glycoside is any of the saponins listed in Table A1 and Scheme I according to the invention. In particular, the thus obtained scaffold augments endosomal escape of said effector molecule.The at least one glycoside molecule is a bisdesmosidic triterpene, saponin belonging to the type of a 12,13-dehydrooleanane with an aldehyde function in position 23, more preferably, a saponin that can be isolated from *Gypsophila* or *Saponaria* species, most preferably SA1641 and/or SO1861, or any of their diastereomers.The at least one glycoside molecule is coupled to the polymeric or oligomeric structure, *via* a hydrazone bond. The saponin is attached to the scaffold via the aldehyde function in position 23.

Preferably, the scaffold further comprises a click chemistry group for coupling to the carrier molecule such as an effector molecule and/or a ligand and/or a monoclonal antibody (fragment, domain thereof), preferably to both an effector molecule and an immunoglobulin. The immunoglobulin preferably is a monoclonal antibody of Table A2, A3, A4. The monoclonal antibody and the effector molecule together preferably form an ADC according to the invention, such as an ADC of Table A4. Preferably, the click chemistry group is a tetrazine, azide, alkene, or alkyne, or a cyclic derivative of these groups, such as cyclooctyne (e.g. aza-dibenzocyclooctyne, difluorocyclooctyne, bicyclo[6.1.0]non-4-yne, or dibenzocyclooctyne).

An embodiment is the method according to the invention for producing a scaffold of the invention as defined in the claims, wherein the number of glycoside molecules is a defined number or a defined range. Preferably, the polymeric or oligomeric structure comprises a linear, branched or cyclic polymer, oligomer, dendrimer, dendron, dendronized polymer, dendronized oligomer or assemblies of these structures, either sheer or mixed, wherein assemblies can be built up by covalent cross-linking or noncovalent attraction and can form hydrogels or nanogels, and wherein, preferably, the polymer is a derivate of a polyethylenimine, polyethylene glycol, polyamino acid or DNA polymer or wherein the oligomer or polymer is a derivate of a dextran, lactic acid, nucleic acid or peptide nucleic acid. In a preferred embodiment, the effector molecule is a pharmaceutically active substance, such as a toxin, a drug, a polypeptide and/or a polynucleotide.

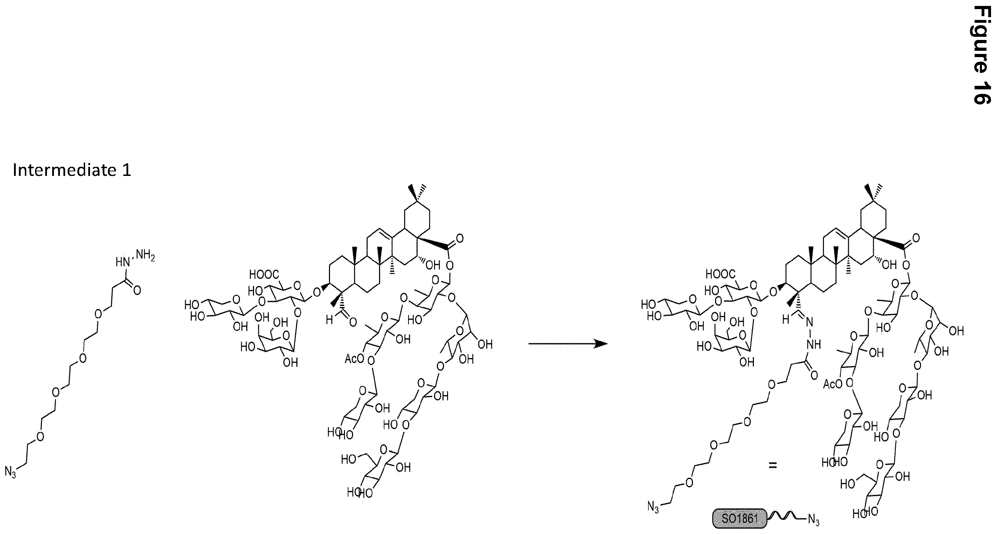

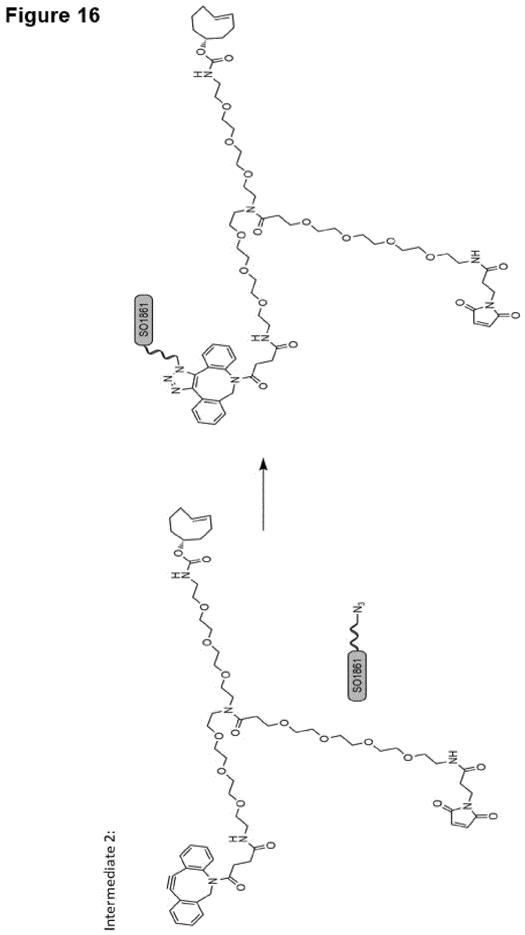

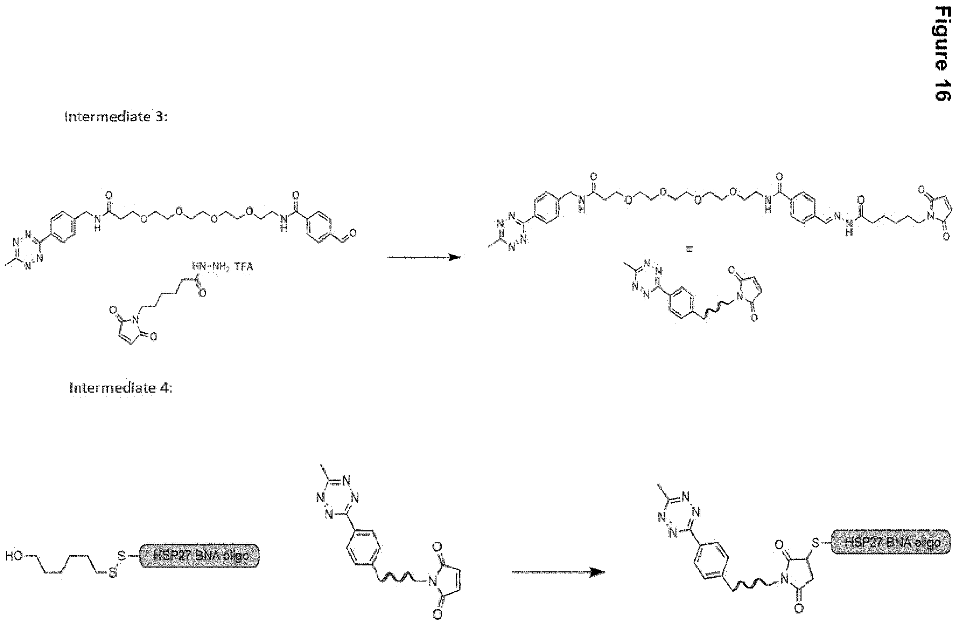

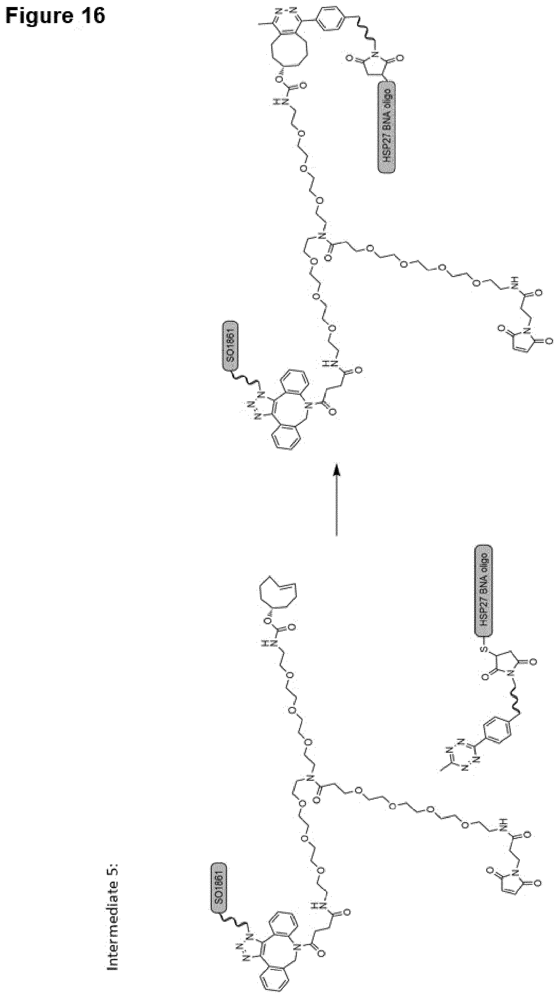

Also provided is a method for producing a scaffold comprising a carrier molecule according to the invention or a functionalized scaffold of the invention, the method comprising: providing a scaffold comprising multiple glycoside molecules and a polymeric or oligomeric structure, preferably a scaffold according to the invention or obtainable by a method according to the invention for producing a scaffold; and coupling either a) at least one effector molecule, b) at least one ligand such as a monoclonal antibody, c) at least one effector molecule and in addition at least one ligand preferably a monoclonal antibody targeting a tumor-cell receptor, d) at least one effector that itself bears at least one ligand such as a monoclonal antibody for binding to a tumor cell receptor, or e) at least one ligand, for example a tumor-cell targeting monoclonal antibody, that itself bears at least one effector, to said scaffold. Preferably, coupling in a) - e) independently occurs via click chemistry bonds. An embodiment is the method of the invention wherein in c) the scaffold is coupled to at least one effector molecule and in addition at least one ligand preferably a monoclonal antibody targeting a tumor-cell receptor, wherein the effector molecule and the ligand (e.g. monoclonal antibody) are both bound to a linker that in itself is bound to the scaffold. The skilled person is able to design such tri-functional linkers, based on the present disclosure and the common general knowledge, such as the tri-functional linker of Scheme II and Structure B (See also Figure 16). Such tri-functional linker can exhibit, for instance, a maleimido group that can be used for conjugation to targeting ligands that exhibit thiol groups to perform a thiolene reaction. In addition, the tri-functional linker could exhibit a dibenzocyclooctyne (DBCO) group to perform the so-called strain-promoted alkyne-azide cycloaddition (SPAAC, click chemistry) with an azido bearing saponin. Finally, the tri-functional linker could obtain a third functional group such as a trans-cyclooctene (TCO) group to perform the so-called inverse electron demand Diels-Alder (IEDDA) reaction with a tetrazine (Tz) bearing effector molecule. An embodiment is the method of the invention wherein said at least one effector molecule is a pharmaceutically active substance, such as a toxin, drug, polypeptide, or polynucleotide. An embodiment is the method of the invention wherein the at least one effector molecule is a toxin or a polynucleotide. Preferably, said at least one ligand, such as a monoclonal antibody for binding to a tumor-cell receptor, is capable of specifically binding to a target cell specific surface molecule or structure that is able to undergo endocytosis, preferably an antibody or fragment thereof, a cytokine, a growth factor, an aptamer or a designed ankyrin repeat protein.

Preferably, said target cell is a diseased or disease-related cell, preferably a tumor cell, a tumor-associated cell (e.g. tumor vascular cell), an immune cell (e.g. a T regulatory cell), or a cell with a monogenic defect. An embodiment is the method according to the invention, the method for producing a functionalized scaffold or a scaffold comprising a carrier molecule according to the invention, wherein said at least one effector molecule is coupled to a scaffold and/or to said at least one ligand via a cleavable bond, wherein preferably said cleavable bond is subject to cleavage under acidic, reductive, enzymatic or light-induced conditions. An embodiment is the method according to the invention for producing a scaffold or functionalized scaffold, the method further comprising coupling said scaffold or functionalized scaffold to a carrier, wherein said carrier preferably is a kind of nanoparticle, liposome, micelle, colloid or a particle-like structure comprising cholesterol and/or phospholipids.

The invention provides a pharmaceutical composition comprising a scaffold according to the invention or a functionalized scaffold according to the invention or a scaffold comprising a covalently coupled carrier molecule according to the invention, and optionally a pharmaceutical acceptable carrier. Such pharmaceutical composition is for use in the treatment of a patient, in particular for use in the treatment of cancer or acquired or hereditary disorders, in particular monogenic deficiency disorders.

Another aspect of the present invention features a pharmaceutical composition comprising a compound or combination of compounds according to the invention and a physiologically acceptable carrier. A "pharmacological composition" refers to a composition in a form suitable for administration into a mammal, preferably a human. Preferably, the pharmaceutical composition contains a sufficient amount of a compound according to the invention in a proper pharmaceutical form to exert a therapeutic effect on a human.

Considerations concerning forms suitable for administration are known in the art and include toxic effects, solubility, route of administration, and maintaining activity. For example, pharmacological compositions injected into the bloodstream should be soluble.

Suitable dosage forms, in part depend upon the use or the route of entry, for example transdermal or by injection. Such dosage forms should allow the compound to reach a target cell whether the target cell is present in a multicellular host. Other factors are known in the art, and include considerations such as toxicity and dosage form which retard the compound or composition from exerting its effect.

An embodiment is the scaffold of the invention wherein the scaffold comprises a defined number of glycosides or a defined range. An embodiment is the scaffold of the invention wherein the scaffold comprises a defined number of glycosides or a defined range, wherein the defined range is between 1 - 30 glycoside(s), preferably between 1 - 20, more preferably between 1 - 10, more preferably between 1 - 6, more preferably between 2 - 6, more preferably between 2 - 5, more preferably between 3 - 5, more preferably between 3 - 4 glycosides.

An embodiment is the scaffold of the invention wherein the scaffold comprises a covalently bound carrier molecule which carrier molecule comprises a cell-targeting binding site and is or comprises for example a (monoclonal) antibody for binding to a cell-surface receptor on a target cell. An embodiment is the scaffold of the invention wherein the scaffold comprises a covalently bound carrier molecule which carrier molecule comprises a cell-targeting binding site and is or comprises for example a (monoclonal) antibody for binding to a cell-surface receptor on a target cell, wherein the target cell is a diseased cell or a disease-related cell, preferably a tumor cell or a tumor-associated cell (e.g. tumor vascular cell), or an immune cell (e.g. a T regulatory cell), or an autoimmune cell.

According to the invention the biologically active molecule is a glycoside capable of augmenting endosomal escape of an effector molecule comprised by the carrier molecule which is covalently bound to the scaffold.

An embodiment is the scaffold of the invention wherein the scaffold is part of a pharmaceutical composition, the pharmaceutical composition further comprising at least one further active pharmaceutically ingredient in addition to the scaffold, such as a further immunoglobulin.

An embodiment is the scaffold of the invention or the pharmaceutical composition according to the invention, for use in a method of treating cancer or an autoimmune disease.

An embodiment is the scaffold of the invention for use in a method of treating cancer, the method comprising administering the scaffold of the invention to a patient in need thereof, wherein the scaffold comprises covalently bound carrier molecule comprising or consisting of an effector molecule of the invention and/or a ligand or cell-targeting antibody of the invention.

An embodiment is the scaffold of the invention for use in a method of treating cancer, the method comprising administering a pharmaceutical composition according to the invention, to a patient in need thereof.

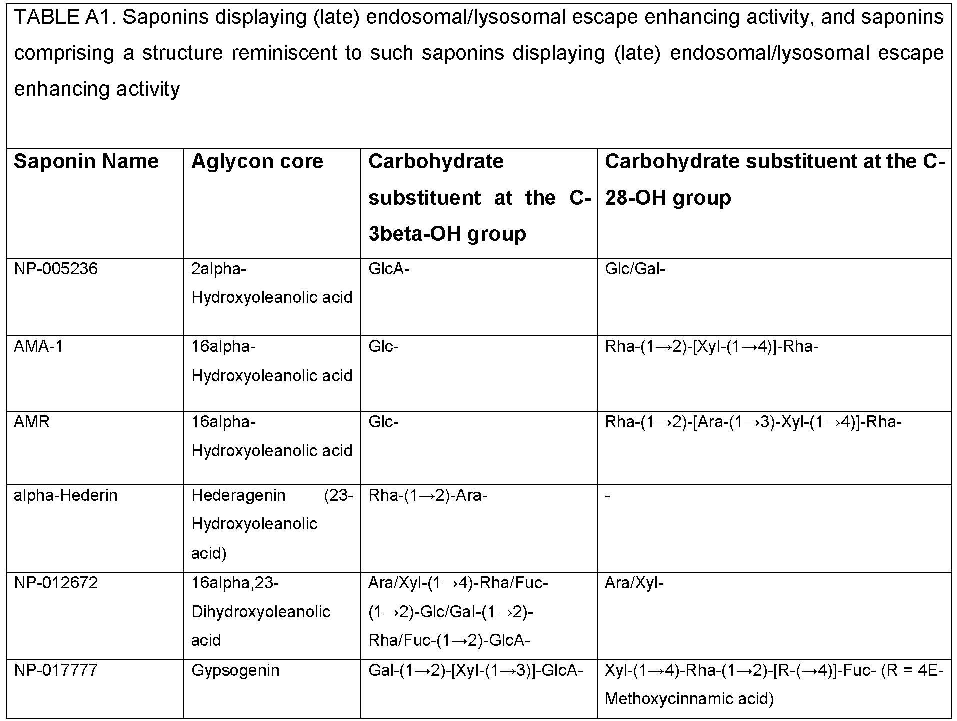

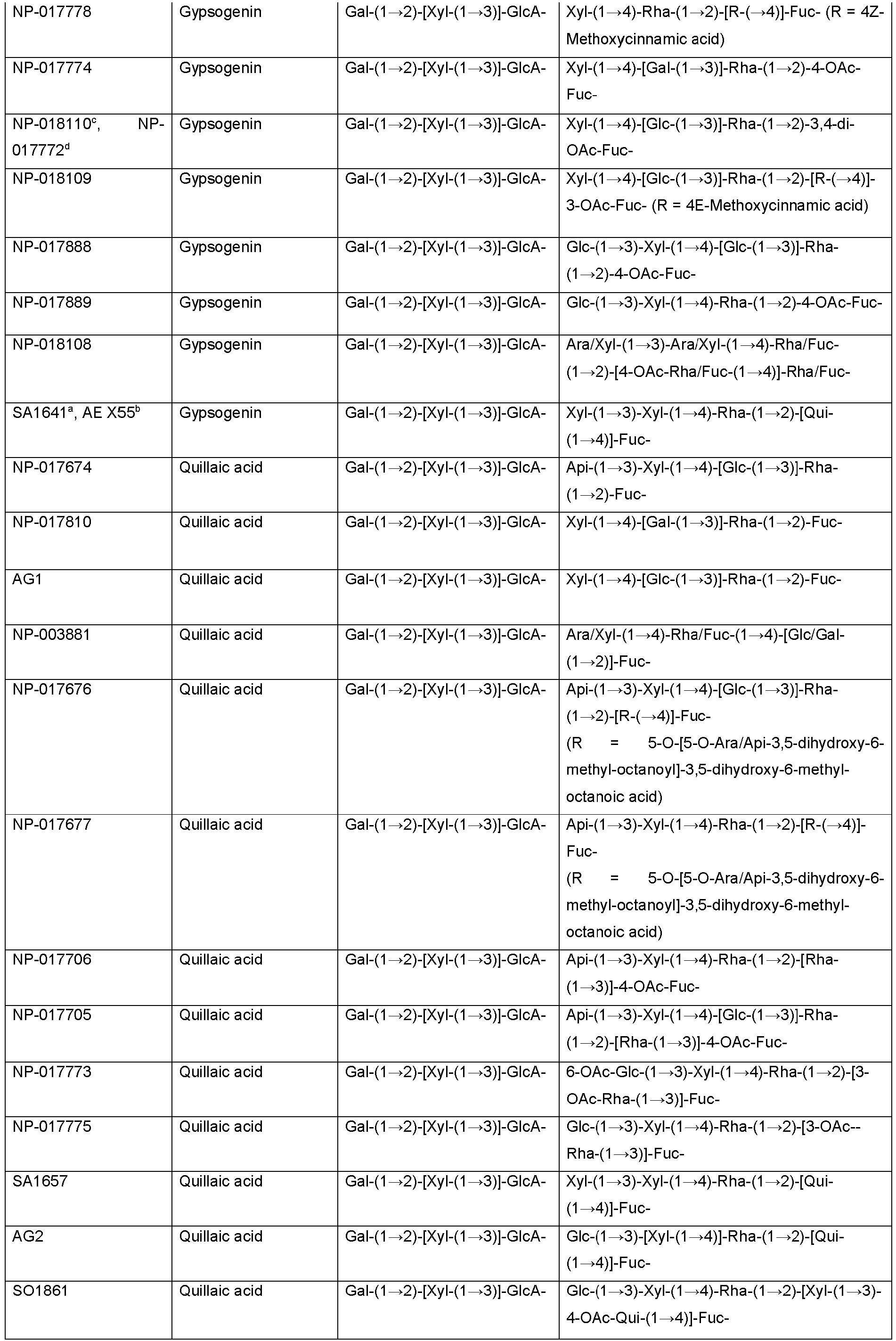

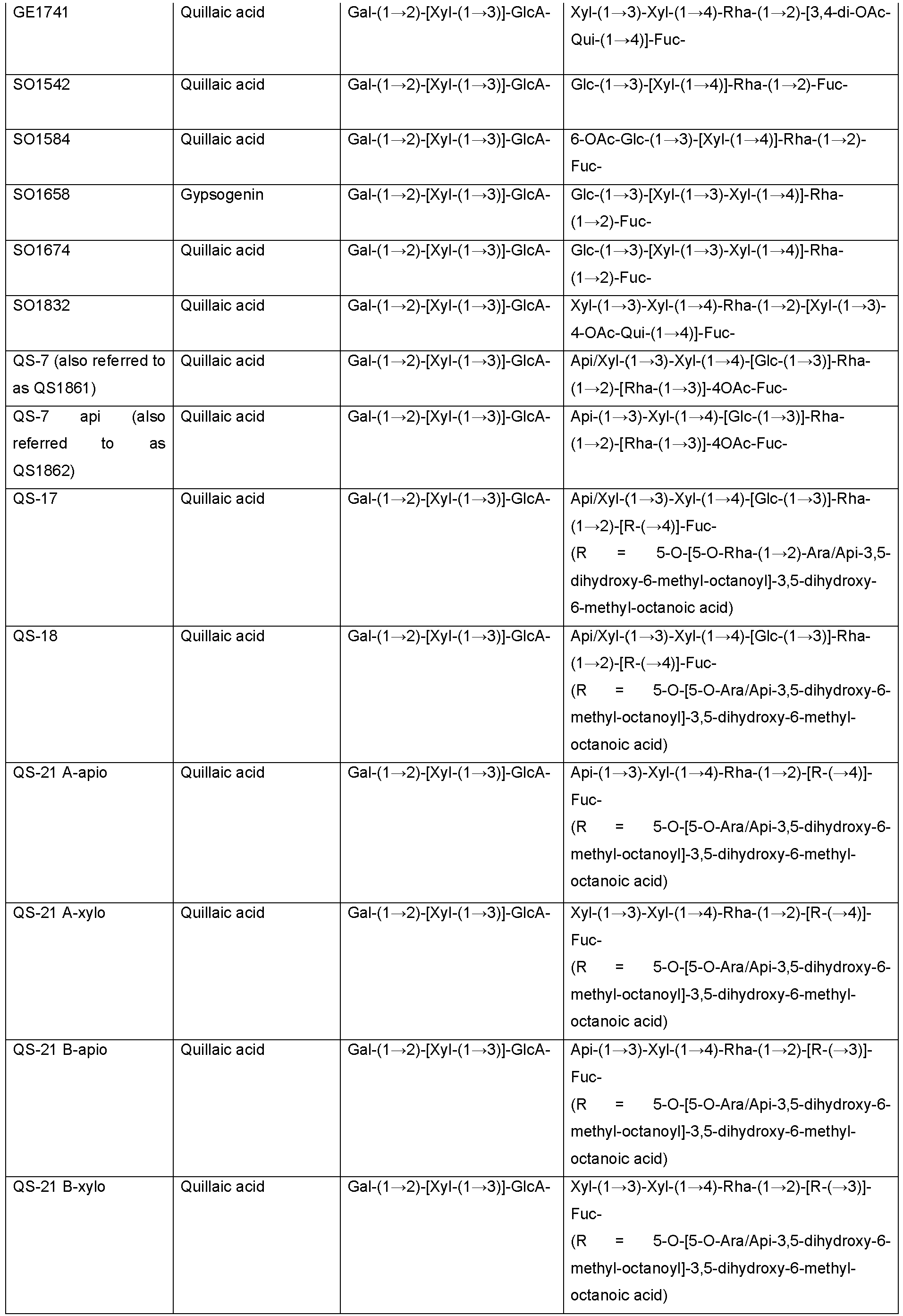

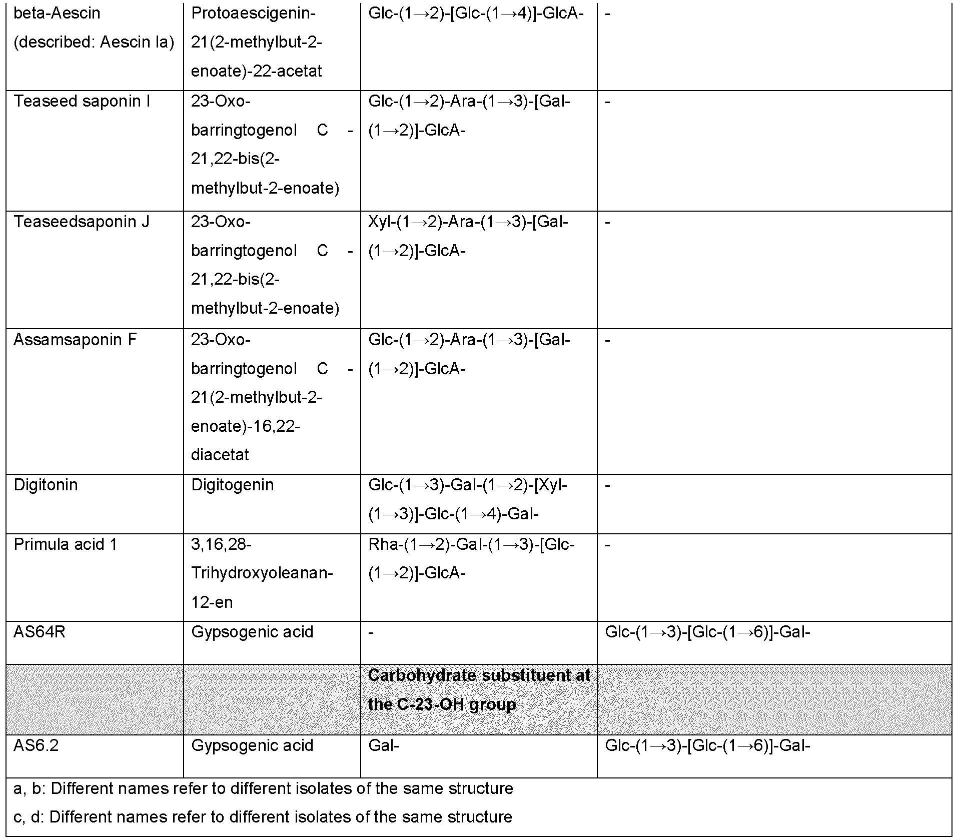

**TABLE A2 - ADCs which were previously investigated in the human clinical setting, and subsequently retracted from further clinical investigation**

| **Drug Name** | **Indication** | **Target** | **Last Development Stage** |
|---|---|---|---|
| Monoclonal Antibody Conjugate to Target EGFR for Oncology | Oncology | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Discovery |
| Affilutin | Multiple Myeloma (Kahler Disease) | | Discovery |
| IMGN-779 | Myelodys-plastic Syndrome | Cells Expressing Myeloid Cell Surface Antigen CD33 (Sialic Acid Binding Ig Like Lectin 3 or gp67 or CD33) | IND/CTA Filed |
| Neuradiab | Non-Hodgkin Lymphoma | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase I |
| IMGN-779 | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I |
| AGS-67E | Acute Myelocytic Leukemia (AML, Acute Myeloblas-tic Leukemia) | Cells Expressing Leukocyte Antigen CD37 (Tetraspanin 26 or CD37) | Phase I |
| AGS-67E | Hairy Cell Leukemia; Non-Hodgkin Lymphoma; Refractory Chronic Lymphocy-tic Leukemia (CLL); Relapsed Chronic Lymphocy-tic Leukemia (CLL); T-Cell Leukemia | Cells Expressing Leukocyte Antigen CD37 (Tetraspanin 26 or CD37) | Phase I |
| ASG-15ME | Metastatic Transitional (Urothelial) Tract Cancer | Cells Expressing SLIT And NTRK Like Protein 6 (SLITRK6) | Phase I |
| vandortuzumab vedotin | Metastatic Hormone Refractory (Castration Resistant, Androgen-Independent) Prostate Cancer | Cells Expressing Metalloreductase STEAP1 (Six Transmembrane Epithelial Antigen Of The Prostate 1 or STEAP1 or EC 1.16.1.) | Phase I |
| CDX-014 | Ovarian Cancer | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 or T Cell Membrane Protein 1 or CD365 or HAVCR1) | Phase I |
| AGS-16M18 | Liver Cancer; Renal Cell Carcinoma | | Phase I |
| vorsetuzumab mafodotin | Non-Hodgkin Lymphoma; Renal Cell Carcinoma | Cells Expressing CD70 Antigen (CD27 Ligand or Tumor Necrosis Factor Ligand Superfamily Member 7 or CD70) | Phase I |
| denintuzumab mafodotin | Acute Lymphocy-tic Leukemia (ALL, Acute Lympho-blastic Leukemia); B-Cell Non-Hodgkin Lymphoma; Burkitt Lymphoma; Lympho-blastic Lymphoma; Mantle Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| SGN-CD70A | Diffuse Large B-Cell Lymphoma; Follicular Lymphoma; Mantle Cell Lymphoma; Metastatic Renal Cell Carcinoma; Non-Hodgkin Lymphoma | Cells Expressing CD70 Antigen (CD27 Ligand or Tumor Necrosis Factor Ligand Superfamily Member 7 or CD70) | Phase I |
| RG-7636 | Metastatic Melanoma | Endothelin B Receptor (Endothelin Receptor Non Selective Type or EDNRB) | Phase I |
| SC-006 | Metastatic Colorectal Cancer | | Phase I |
| MM-310 | Breast Cancer; Endome-trial Cancer; Esophageal Cancer; Gastric Cancer; Gastroeso-phageal (GE) Junction Carcino-mas; Head And Neck Cancer Squamous Cell Carcinoma; Non-Small Cell Lung Cancer; Ovarian Cancer; Pancreatic Ductal Adenocar-cinoma; Prostate Cancer; Small-Cell Lung Cancer; Soft Tissue Sarcoma; Solid Tumor; Transitional Cell Carcinoma (Urothelial Cell Carcinoma) | Ephrin Type A Receptor 2 (Epithelial Cell Kinase or Tyrosine Protein Kinase Receptor ECK or EPHA2 or EC 2.7.10.1) | Phase I |
| PF-06647263 | Metastatic Breast Cancer; Ovarian Cancer | Cells Expressing Ephrin A4 (EPH Related Receptor Tyrosine Kinase Ligand 4 or EFNA4) | Phase I |
| PF-06263507 | Solid Tumor | Cells Expressing Trophoblast Glycoprotein (M6P1 or 5T4 Oncofetal Antigen or 5T4 Oncofetal Trophoblast Glycoprotein or Wnt Activated Inhibitory Factor 1 or TPBG) | Phase I |
| PF-06650808 | Metastatic Breast Cancer; Non-Small Cell Lung Cancer; Ovarian Cancer | Cells Expressing Neurogenic Locus Notch Homolog Protein 3 (NOTCH3) | Phase I |
| XMT-1522 | Breast Cancer; Gastric Cancer; Non-Small Cell Lung Cancer | Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1); Tubulin | Phase I |
| AMG-595 | Anaplastic Astrocyto-ma; Recurrent Glioblasto-ma Multiforme (GBM) | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I |
| pinatuzumab vedotin | Chronic Lymphocytic Leukemia (CLL) | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I |
| cantuzumab ravtansine | Colorectal Cancer; Non-Small Cell Lung Cancer; Pancreatic Cancer; Solid Tumor | | Phase I |
| AVE-9633 | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase I |
| **BIWI-1⁽¹⁾** | Breast Cancer; Carcino-mas; Esophageal Cancer; Head And Neck Cancer Squamous Cell Carcinoma | Cells Expressing CD44 Antigen (CDw44 or Epican or Extracellular Matrix Receptor III or GP90 Lymphocyte Homing/Adhesion Receptor or HUTCH I or Heparan Sulfate Proteoglycan or Hermes Antigen or Hyaluronate Receptor or Phagocytic Glycoprotein 1 or CD44) | Phase I |
| RG-7882 | Epithelial Ovarian Cancer; Fallopian Tube Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I |
| ASG-5ME | Adenocar-cinoma; Hormone Refractory (Castration Resistant, Androgen-Indepen-dent) Prostate Cancer; Metastatic Adenocar-cinoma of The Pancreas | Cells Expressing Choline Transporter Like Protein 4 (Solute Carrier Family 44 Member 4 or SLC44A4) | Phase I |
| DCDS-0780A | B-Cell Non-Hodgkin Lymphoma | | Phase I |
| SC-004 | Endome-trial Cancer; Epithelial Ovarian Cancer; Fallopian Tube Cancer; Peritoneal Cancer | | Phase I |
| RG-7600 | Ovarian Cancer; Pancreatic Ductal Adenocar-cinoma | | Phase I |
| sofituzumab vedotin | Epithelial Ovarian Cancer; Fallopian Tube Cancer; Ovarian Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I |
| IMGN-289 | Breast Cancer; Esophageal Cancer; Gastric Cancer; Head And Neck Cancer Squamous Cell Carcinoma; Non-Small Cell Lung Cancer; Solid Tumor | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I |
| SAR-428926 | Breast Cancer; Colorectal Cancer; Gastric Cancer; Non-Small Cell Lung Cancer; Ovarian Cancer; Prostate Cancer; Solid Tumor | Cells Expressing Lysosome Associated Membrane Glycoprotein 1 (CD107 Antigen Like Family Member A or CD107a or LAMP1) | Phase I |
| SGNCD-19B | B-Cell Non-Hodgkin Lymphoma; Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| SGNCD-123A | Refractory Acute Myeloid Leukemia; Relapsed Acute Myeloid Leukemia | Cells Expressing Interleukin 3 Receptor Subunit Alpha (CD123 or IL3RA) | Phase I |
| SGNCD-352A | Refractory Multiple Myeloma; Relapsed Multiple Myeloma | Cells Expressing SLAM Family Member 6 (Activating NK Receptor or NK T B Antigen or CD352 or SLAMF6) | Phase I |
| RG-7841 | Breast Cancer; Non-Small Cell Lung Cancer; Solid Tumor | Cells Expressing Lymphocyte Antigen 6E (Retinoic Acid Induced Gene E Protein or Stem Cell Antigen 2 or Thymic Shared Antigen 1 or LY6E) | Phase I |
| IMGN-388 | Solid Tumor | Cells Expressing Integrin Alpha V (Vitronectin Receptor Subunit Alpha or CD51 or ITGAV) | Phase I |
| lorvotuzumab mertansine | Refractory Multiple Myeloma; Relapsed Multiple Myeloma | Cells Expressing Neural Cell Adhesion Molecule 1 (Antigen Recognized By Monoclonal Antibody 5.1H11 or CD56 or NCAM1) | Phase I |
| lorvotuzumab mertansine | Neuroendo-crine Carcinoma; Neuroendo-crine Tumors; Non-Small Cell Lung Cancer; Ovarian Cancer; Skin Cancer | Cells Expressing Neural Cell Adhesion Molecule 1 (Antigen Recognized By Monoclonal Antibody 5.1H11 or CD56 or NCAM1) | Phase I |
| BAY-794620 | Lung Cancer; Solid Tumor | Cells Expressing Carbonic Anhydrase 9 (Carbonate Dehydratase IX or pMW1 or Membrane Antigen MN or P54/58N or Renal Cell Carcinoma Associated Antigen G250 or CA9 or EC 4.2.1.1) | Phase I |
| RG-7598 | Refractory Multiple Myeloma; Relapsed Multiple Myeloma | | Phase I |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| **ADCT-502⁽¹⁾** | Bladder Cancer; Breast Cancer; Esophageal Cancer; Gastric Cancer; Non-Small Cell Lung Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase I |
| AMG-172 | Renal Cell Carcinoma | Cells Expressing CD70 Antigen (CD27 Ligand or Tumor Necrosis Factor Ligand Superfamily Member 7 or CD70) | Phase I |
| ImmuRAIT-LL2 | B-Cell Non-Hodgkin Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I/II |
| indusatumab vedotin | Adenocar-cinoma Of The Gastroe-sophageal Junction; Gastric Cancer | Cells Expressing Heat Stable Enterotoxin Receptor (Guanylyl Cyclase C or or Intestinal Guanylate Cyclase or GUCY2C or EC 4.6.1.2) | Phase I/II |
| clivatuzumab tetraxetan | Pancreatic Cancer | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase I/II |
| **depatuxizumab mafodotin⁽²⁾** | Recurrent Malignant Glioma | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I/II |
| CDX-014 | Metastatic Renal Cell Carcinoma; Papillary Renal Cell Carcinoma | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 or T Cell Membrane Protein 1 or CD365 or HAVCR1) | Phase I/II |
| **vadastuximab talirine⁽¹⁾** | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I/II |
| vadastuximab talirine | Myelodys-plastic Syndrome | Cells Expressing Myeloid Cell Surface Antigen CD33 (Sialic Acid Binding Ig Like Lectin 3 or gp67 or CD33) | Phase I/II |
| MLN-2704 | Metastatic Hormone Refractory (Castration Resistant, Androgen-Indepen-dent) Prostate Cancer | Cells Expressing Glutamate Carboxypeptidase 2 (Folate Hydrolase 1 or Prostate Specific Membrane Antigen or PSMA or Pteroylpoly Gamma Glutamate Carboxypeptidase or Cell Growth Inhibiting Gene 27 Protein or FOLH1 or EC 3.4.17.21) | Phase I/II |
| Oncolysin B | AIDS - Related Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I/II |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| coltuximab ravtansine | Acute Lymphocy-tic Leukemia (ALL, Acute Lympho-blastic Leukemia) | Cells Expressing B Lymphocyte Antigen CD19 (B Lymphocyte Surface Antigen B4 or Differentiation Antigen CD19 or T Cell Surface Antigen Leu 12 or CD19) | Phase II |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| **indusatumab vedotin⁽²⁾** | Adenocar-cinoma Of The Gastroe-sophageal Junction; Gastric Cancer; Metastatic Adenocar-cinoma of The Pancreas | Cells Expressing Heat Stable Enterotoxin Receptor (Guanylyl Cyclase C or or Intestinal Guanylate Cyclase or GUCY2C or EC 4.6.1.2) | Phase II |
| depatuxizumab mafodotin | Squamous Non-Small Cell Lung Cancer | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II |
| **depatuxizumab mafodotin⁽²⁾** | Anaplastic Astrocyto-ma; Anaplastic Oligoastro-cytoma; Gliosar-coma; High-Grade Glioma; Oligodendroglioma; Pediatric Diffuse Intrinsic Pontine Glioma; Recurrent Glioblastoma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II |
| lifastuzumab vedotin | Non-Small Cell Lung Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II |
| lifastuzumab vedotin | Ovarian Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II |
| Bismab-A | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase II |
| denintuzumab mafodotin | Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| **Avicidin⁽¹⁾** | Colorectal Cancer; Prostate Cancer | Cells Expressing Epithelial Cell Adhesion Molecule (Adenocarcinoma Associated Antigen or Cell Surface Glycoprotein Trop 1 or Epithelial Cell Surface Antigen or Epithelial Glycoprotein 314 or KS 1/4 Antigen or KSA or Tumor Associated Calcium Signal Transducer 1 or CD326 or EPCAM) | Phase II |
| pinatuzumab vedotin | Diffuse Large B-Cell Lymphoma; Follicular Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase II |
| SGN-15 | Metastatic Breast Cancer; Non-Small Cell Lung Cancer; Ovarian Cancer; Prostate Cancer | Cells Expressing Lewis Y Antigen (CD174) | Phase II |
| cantuzumab ravtansine | Gastric Cancer; Gastroe-sophageal (GE) Junction Carcino-mas | | Phase II |
| ASP-6183 | Ovarian Cancer | | Phase II |
| SAR-566658 | Metastatic Breast Cancer | Cells Expressing Sialoglycotope CA6 Antigen | Phase II |
| Oncolysin S | Small-Cell Lung Cancer | Cells Expressing Neural Cell Adhesion Molecule 1 (Antigen Recognized By Monoclonal Antibody 5.1H11 or CD56 or NCAM1) | Phase II |
| lorvotuzumab mertansine | Small-Cell Lung Cancer | Cells Expressing Neural Cell Adhesion Molecule 1 (Antigen Recognized By Monoclonal Antibody 5.1H11 or CD56 or NCAM1) | Phase II |
| glembatumumab vedotin | Metastatic Melanoma; Metastatic Uveal Melanoma; Osteosar-coma; Squamous Non-Small Cell Lung Cancer | Cells Expressing Transmembrane Glycoprotein NMB (Transmembrane Glycoprotein HGFIN or GPNMB) | Phase II |
| MM-302 | Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase II/III |
| Neuradiab | Brain Cancer; Glioblasto-ma Multiforme (GBM) | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase III |
| clivatuzumab tetraxetan | Metastatic Adenocar-cinoma of The Pancreas | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase III |
| **depatuxizumab mafodotin⁽²⁾** | Glioblasto-ma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase III |
| **vadastuximab talirine⁽¹⁾** | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase III |
| **glembatumuma b vedotin⁽²⁾** | Metastatic Breast Cancer | Cells Expressing Transmembrane Glycoprotein NMB (Transmembrane Glycoprotein HGFIN or GPNMB) | Phase III |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase III |
| ImmuRAIT-LL2 | B-Cell Leukemia | Cells Expressing B Cell Receptor CD22 (B Lymphocyte Cell Adhesion Molecule or Sialic Acid Binding Ig Like Lectin 2 or T Cell Surface Antigen Leu 14 or CD22) | Preclinical |
| indusatumab vedotin | Metastatic Colorectal Cancer | Cells Expressing Heat Stable Enterotoxin Receptor (Guanylyl Cyclase C or or Intestinal Guanylate Cyclase or GUCY2C or EC 4.6.1.2) | Preclinical |
| ASG-15ME | Lung Cancer | Cells Expressing SLIT And NTRK Like Protein 6 (SLITRK6) | Preclinical |
| HTI-1511 | Bile Duct Cancer (Cholangiocarcinoma) ; Breast Cancer; Colorectal Cancer; Non-Small Cell Lung Cancer | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Preclinical |
| ZW-33 | Gastric Cancer; Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical |
| ZW-33 | Ovarian Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical |
| SGNCD-352A | Non-Hodgkin Lymphoma | Cells Expressing SLAM Family Member 6 (Activating NK Receptor or NK T B Antigen or CD352 or SLAMF6) | Preclinical |
| HuMax-CD74-ADC | Oncology | Cells Expressing HLA Class II Histocompatibility Antigen Gamma Chain (HLA DR Antigens Associated Invariant Chain or la Antigen Associated Invariant Chain or p33 or CD74) | Preclinical |
| sacituzumab govitecan | Pancreatic Ductal Adenocar-cinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Adenocar-cinoma; Cervical Cancer; Colorectal Cancer; Endome-trial Cancer; Epithelial Ovarian Cancer; Esophageal Cancer; Follicular Thyroid Cancer; Gastric Cancer; Glioblasto-ma Multiforme (GBM); Head And Neck Cancer Squamous Cell Carcinoma; Hepato-cellular Carcinoma; Kidney Cancer (Renal Cell Cancer); Metastatic Hormone Refractory (Castration Resistant, Androgen-Indepen-dent) Prostate Cancer; Metastatic Transitional (Urothelial) Tract Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Hepato-cellular Carcinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Metastatic Breast Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Non-Small Cell Lung Cancer; Small-Cell Lung Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Metastatic Breast Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| (1) Discontinued due to adverse events | | | |
| (2) Discontinued due to lack of efficacy | | | |

**TABLE A3 - ADCs that reached phase III clinical development**

| **Drug Name** | **Indication** | **Development Stage** | **Last Development Stage** | **Reason for Discontinuation** |
|---|---|---|---|---|
| trastuzumab emtansine | Gastric Cancer | Marketed | Phase II/III | Unspecified |
| MM-302 | Metastatic Breast Cancer | Discontinued | Phase II/III | Business/Strategic Decision |
| trastuzumab emtansine | Metastatic Breast Cancer | Marketed | Phase III | Unspecified |
| trastuzumab emtansine | Gastric Cancer | Marketed | Phase III | Unspecified |
| ibritumomab tiuxetan | Diffuse Large B-Cell Lymphoma | Marketed | Phase III | |
| inotuzumab ozogamicin | Follicular Lymphoma | Marketed | Phase III | |
| inotuzumab ozogamicin | Diffuse Large B-Cell Lymphoma; Non-Hodgkin Lymphoma | Marketed | Phase III | Lack of Efficacy |
| rovalpituzumab tesirine | Small-Cell Lung Cancer | Phase III | Phase III | |
| rovalpituzumab tesirine | Small-Cell Lung Cancer | Phase III | Phase III | |
| Neuradiab | Brain Cancer; Glioblastoma Multiforme (GBM) | Inactive | Phase III | Unspecified |
| clivatuzumab tetraxetan | Metastatic Adenocarcinoma of The Pancreas | Inactive | Phase III | Unspecified |
| depatuxizumab mafodotin | Glioblastoma Multiforme (GBM) | Inactive | Phase III | Lack of Efficacy |
| vadastuximab talirine | Acute Myelocytic Leukemia (AML, Acute Myeloblastic Leukemia) | Discontinued | Phase III | Adverse Events |
| glembatumumab vedotin | Metastatic Breast Cancer | Discontinued | Phase III | Lack of Efficacy |
| Oncolysin B | B-Cell Leukemia; Lymphoma | Discontinued | Phase III | Business/Strategic Decision |

| **TABLE A4.** Tumor-specific cell-surface receptor targets which can be targeted by immunoglobulins according to the invention, and antibodies that can be used for the ADCs and the antibodies provided with a saponin, and the ADCs provided with a saponin, of the present invention (not presented as a limitation; further immunoglobulins are equally suitable for the invention) | |
|---|---|
| **Target cell-surface receptor** | **Example monoclonal antibodies** |
| HER2 | anti-HER2 monoclonal antibody such as trastuzumab and pertuzumab |
| CD20 | anti-CD20 monoclonal antibody such as rituximab, ofatumumab, tositumomab and ibritumomab |
| CA125 | anti-CA125 monoclonal antibody such as oregovomab |
| EpCAM (17-1A) | anti-EpCAM (17-1A) monoclonal antibody such as edrecolomab |
| EGFR | anti-EGFR monoclonal antibody such as cetuximab, panitumumab and nimotuzumab |
| CD30 | anti-CD30 monoclonal antibody such brentuximab |
| CD33 | anti-CD33 monoclonal antibody such as gemtuzumab and huMy9-6 |
| vascular integrin alpha-v beta-3 | anti-vascular integrin alpha-v beta-3 monoclonal antibody such as etaracizumab |
| CD52 | anti-CD52 monoclonal antibody such as alemtuzumab |
| CD22 | anti-CD22 monoclonal antibody such as epratuzumab |
| CEA | anti-CEA monoclonal antibody such as labetuzumab |
| CD44v6 | anti-CD44v6 monoclonal antibody such as bivatuzumab |
| FAP | anti- FAP monoclonal antibody such as sibrotuzumab |
| CD19 | anti-CD19 monoclonal antibody such as huB4 |
| CanAg | anti-CanAg monoclonal antibody such as huC242 |
| CD56 | anti-CD56 monoclonal antibody such huN901 |
| CD38 | anti-CD38 monoclonal antibody such as daratumumab |
| CA6 | anti-CA6 monoclonal antibody such as DS6 |
| IGF-IR | anti-IGF-IR monoclonal antibody such as cixutumumab and 3B7 |
| integrin | anti-integrin monoclonal antibody such as CNTO 95 |
| syndecan-1 | anti-syndecan-1 monoclonal antibody such as B-B4 |

**Table A5: RIPs from plants***

| **Plant Family** | **Plant Species** | **Proteins** | **Classification** |
|---|---|---|---|
| Adoxaceae | Sambucus ebulus L. | Ebulitin α, Ebulitin β, Ebulitin γ | RIP 1 |
| | | Ebulin f, Ebulin l, Ebulin r1, Ebulin r2, SEA | RIP 2 |
| | | SEAll, SELfd, SELId, SELIm | lectin |
| | Sambucus nigra L. | α-Nigritin, β-Nigritin, γ-Nigritin, Nigritin f1, Nigritin f2 | RIP 1 |
| | | basic Nigrin b, Nigrin b = SNA-V, Nigrin f = SNA-Vf, Nigrin 11, Nigrin l2, Nigrin s, SNA-I, SNA-I', SNA-If, SNAflu-I, SNLRP1, SNLRP2 | RIP 2 |
| | | SNA-Id, SNA-Im, SNA-II, SNA-III, SNA-IV = SNA-IVf, SNA-IVI, SNApol-I, SNApol-II, TrSNA-I, TrSNA-lf | lectin |
| | Sambucus racemosa L. | basic racemosin b, SRA | RIP 2 |
| | | SRLbm = SRAbm | lectin |
| | | SSA = SSA-b-1, Sieboldin-b = SSA-b-2 | RIP 2 |
| | Sambucus sieboldiana (Miq.) Blume ex Graebn. | SSA-b-3, SSA-b-4 | lectin |
| Aizoaceae | Mesembryanthe-mum crystallinum L. | RIP1 | RIP 1 |
| Amaranthaceae | Amaranthus caudatus L. | Amaranthin = ACA | lectin |
| | Amaranthus cruentus L. | ACL | lectin |
| | Amaranthus hypochondriacus L. [Syn.: Amaranthus leucocarpus S. Watson] | A. leucocarpus lectin | lectin |
| | Amaranthus mangostanus L. | Amaramangin | RIP 1 |
| | Amaranthus tricolor L. | AAP-27 | RIP 1 |
| | Amaranthus viridis L. | Amaranthin | RIP 1 |
| | Beta vulgaris L. | Beetin-27 = BE27, Beetin-29 = BE29, Betavulgin | RIP 1 |
| | Celosia argentea L. [Syn.: Celosia cristata L.] | CCP-25, CCP-27 | RIP 1 |
| | Chenopodium album L. | CAP30 | RIP 1 |
| | Spinacia oleracea L. | SoRIP1 = BP31 | RIP 1 |
| | | SoRIP2 | RIP 1 candidate |
| Araliaceae | Aralia elata (Miq.) Seem. | Aralin | RIP 2 |
| | Panax ginseng C.A.Mey | Panaxagin | peculiar RIP 1 candidate/RNase |
| | Panax quinquefolius L. | Quinqueginsin | peculiar RIP 1 candidate/RNase |
| Asparagaceae | Asparagus officinalis L. | Asparin 1, Asparin 2 | RIP 1 |
| | Drimia maritima (L.) Stearn [Syn.: Charybdis maritima (L.) Speta] | Charybdin | RIP 1 |
| | Muscari armeniacum Leichtlin ex Baker | Musarmin 1, Musarmin 2, Musarmin 3, Musarmin 4 | RIP 1 |
| | Polygonatum multiflorum (L.) All. | PMRIPm, PMRIPt | RIP 2 |
| | Yucca gloriosa var. tristis Carrière [Syn.: Yucca recurvifolia Salisb.] | Yucca leaf protein = YLP | RIP 1 |
| Basellaceae | Basella rubra L. | Basella RIP 2a, Basella RIP 2b, Basella RIP 3 | RIP 1 |
| Caryophyllaceae | Agrostemma githago L. | Agrostin 2, Agrostin 5, Agrostin 6, Agrostin | RIP 1 |
| | Dianthus barbatus L. | Dianthin 29 | RIP 1 |
| | Dianthus caryophyllus L. | Dianthin 30, Dianthin 32 | RIP 1 |
| | Dianthus chinensis L. [Syn.: Dianthus sinensis Link] | D. sinensis RIP | RIP 1 |
| | Gypsophila elegans M.Bieb. | Gypsophilin | RIP 1 |
| | Silene chalcedonica (L.) E.H.L.Krause [Syn.: Lychnis chalcedonica L.] | Lychnin | RIP 1 |
| | Silene glaucifolia Lag. [Syn.: Petrocoptis glaucifolia (Lag.) Boiss.] | Petroglaucin 1, Petroglaucin 2 | RIP 1 |
| | Silene laxipruinosa Mayol & Rosselló [Syn.: Petrocoptis grandiflora Rothm.] | Petrograndin | RIP 1 |
| | Saponaria ocymoides L. | Ocymoidin | RIP 1 |
| | Saponaria officinalis L. | Saporin-L1 = SO-L1, Saporin-L2 = SO-L2, Saporin-L3 = SO-L3, Saporin-I = SO-I = SO-4, Saporin-R1 = SO-R1, Saporin-R2 = SO-R2, Saporin-R3 = SO-R3, SO3a, SO3b, Saporin-S5 = Saporin 5 = SO-S5, Saporin-S6 = Saporin 6 = SO-6 = SO-S6, Saporin-S8 = SO-S8, Saporin-S9 = Saporin 9 = SO-S9, SAP-C, SAP-S | RIP 1 |
| | Myosoton aquaticum (L.) Moench [Syn.: Stellaria aquatica (L.) Scop.] | Stellarin | RIP 1 |
| | Stellaria media (L.) Vill. | RIP Q3 | RIP 1 |
| | Vaccaria hispanica (Mill.) Rauschert [Syn.: Vaccaria pyramidata Medik.] | Pyramidatin | RIP 1 |
| Cucurbitaceae | Benincasa hispida (Thunb.) Cogn. | Hispin | RIP 1 |
| | | α-benincasin, β-benincasin | sRIP 1 |
| | Bryonia cretica subsp. dioica (Jacq.) Tutin. [Syn.: Bryonia dioica L.] | Bryodin 1 = BD1, Bryodin 2, Bryodin-L, Bryodin-R | RIP 1 |
| | | BDA | lectin/ **RIP** 2 like |
| | Citrullus colocynthis (L.) Schrad. | Colocin 1, Colocin 2 | RIP 1 |
| | Cucurbita foetidissima Kunth | Foetidissimin | peculiar RIP 2 |
| | | Foetidissimin II | RIP 2 |
| | Cucumis ficifolius A.Rich. [Syn.: Cucumis figarei Delile ex Naudin] | Cucumis figarei RIP = CF-RIP | RIP 1 candidate |
| | Cucurbita maxima Duchesne | Cucurmoschin | sRIP 1 candidate |
| | Cucurbita moschata Duchesne [Syn.: Cucurbita moschata (Duchesne ex Lam.) Duchesne ex Poir.] | Cucurmosin, Cucurmosin 2, C. moschata RIP, Moschatin, PRIP 1, PRIP 2 | RIP 1 |
| | | α-moschin, β-moschin | sRIP 1 candidate |
| | Cucurbita pepo L. | Pepocin | RIP 1 |
| | Cucurbita pepo var. texana (Scheele) D.S.Decker [Syn.: Cucurbita texana (Scheele) A. Gray] | Texanin | RIP 1 |
| | Gynostemma pentaphyllum (Thunb.) Makino | Gynostemmin | RIP 1 |
| | Lagenaria siceraria (Molina) Standl. | Lagenin | RIP 1 candidate |
| | Luffa acutangula (L.) Roxb. | Luffaculin-1, Luffaculin-2 | RIP 1 |
| | | Luffangulin | sRIP 1 |
| | | Luffa acutangula fruit lectin | lectin |
| | Luffa cylindrica (L.) M.Roem [Syn.: Luffa aegyptiaca Mill.] | Luffin, Luffin-a, Luffin-b, α-luffin, β-luffin, LRIP | RIP 1 |
| | | Luffacylin, Luffin P1 | sRIP 1 |
| | | Luffin-S, LuffinS(1), LuffinS(2) = luffin S2, LuffinS(3) | sRIP 1 candidate |
| | Marah oreganus (Torr. & A. Gray) Howell | MOR-I, MOR-II | RIP 1 |
| | Momordica balsamina L. | Balsamin, MbRIP-1, Momordin II | RIP 1 |
| | Momordica charantia L. | MAP 30, α-momorcharin = α-MC = α-MMC, β-momorcharin = β-MC = β-MMC, δ-momorcharin = δ-MMC, Momordin, Momordin = Momordica charantia inhibitor, Momordin II, Momordin-a, Momordin-b | RIP 1 |
| | | γ-momorcharin = γ-MMC, Charantin | sRIP 1 |
| | | RIP 1 candidate | RIP 1 candidate |
| | | MCL = M. charantia lectin, anti-H Lectin, Momordica agglutinin, Momordin, protein fraction 1, protein fraction 2 | lectin |
| | | MCL = Momordica charantia seed lectin = Momordica charantia lectin, MCL1 | RIP 2 |
| | Momordica cochinchinensis Spreng. | Cochinin B, Momorcochin, Momorcochin-S | RIP 1 |
| | Siraitia grosvenorii (Swingle) C.Jeffrey ex A.M.Lu & Zhi Y.Zhang [Syn.: Momordica grosvenorii Swingle] | Momorgrosvin | RIP 1 |
| | Sechium edule (Jacq.) Sw. | Sechiumin | RIP 1 |
| | | Sechium edule fruit lectin | lectin |
| | Trichosanthes anguina L. | Trichoanguin | RIP 1 |
| | | SGSL | lectin/ **RIP** 2 like |
| | Trichosanthes cordata Roxb. | TCA-I, TCA-II | lectin |
| | Trichosanthes cucumerina L. | TCSL | lectin/ RIP 2 candidate |
| | Trichosanthes cucumeroides (Ser.) Maxim. | β-trichosanthin = β-TCS | RIP 1 |
| | Trichosanthes kirilowii Maxim. | α-kirilowin, β-kirilowin, TAP 29, TK-35, Trichobitacin, Trichokirin, Trichomislin = TCM, Trichosanthin = Trichosanthes antiviral protein = TAP = TCS = α-trichosanthin = α-TCS = GLQ223, Trichosanthin, β-trichosanthin = β-TCS, γ-trichosanthin = γ-TCS | RIP 1 |
| | | Trichokirin S1, S-Trichokirin, Trichosanthrip | sRIP 1 |
| | | TKL-1 = Trichosanthes kirilowii lectin-1 | lectin/ RIP 2 candidate |
| | | TK-I, TK-II, TK-III, Trichosanthes kirilowii lectin | lectin |
| | Trichosanthes kirilowii Maximovicz var. japonica (Miquel) Kitamura | Karasurin-A, Karasurin-B, Karasurin-C | RIP 1 |
| | Trichosanthes lepiniate | Trichomaglin | RIP 1 |
| | Trichosanthes dioica Roxb. | TDSL | lectin/ RIP 2 candidate |
| | Trichosanthes sp. Bac Kan 8-98 | Trichobakin | RIP 1 |
| Cupressaceae | Thuja occidentalis L. | Arborvitae RIP | RIP candidate |
| Euphorbiaceae | Croton tiglium L. | Crotin I | RIP 1 candidate |
| | | Crotin 2 | RIP 1 |
| | Euphorbia characias L. | E. characias lectin | lectin |
| | Suregada multiflora (A.Juss.) Baill. [Syn.: Gelonium multiflorum A.Juss.] | Gelonin = GAP 31 | RIP 1 |
| | Hura Crepitans L. | Hura crepitans RIP, Hura crepitans RIP-5 | RIP 1 |
| | | Hura crepitans latex lectin | RIP 2 |
| | | Crepitin, Hurin, Hura crepitans seed lectin | lectin |
| | Jatropha curcas L. | Curcin, Curcin 2, Curcin-L, Jc-SCRIP | RIP 1 |
| | Manihot palmata Müll. Arg. | Mapalmin | RIP 1 |
| | Manihot esculenta Crantz. [Syn.: Manihot utilissima Pohl] | Manutin 1, Manutin 2 | RIP 1 |
| | Ricinus communis L. | Ricin = crystalline Ricin = Ricin D, Ricin E, RCA = Ricinus communis agglutinin = RCAI = RCA120 = R. communis hemagglutinin = RCB-PHA I, RCAII = RCA60 = RCB-PHA II | RIP 2 |
| | Ricinus communis, USA | Ricin 1, Ricin 2, Ricin 3 | RIP 2 |
| | Ricinus communis, India | Ricin I, Ricin II, Ricin III | RIP 2 |
| | Ricinus sanguienus, France | Ricin₁₁, Ricin₁₂, Ricin₂ | RIP 2 |
| Fabaceae | Abrus precatorius L. | Abrin, Abrin-a = Abrin C = Abrin-III, Abrin-b, Abrin-c = Abrin A = Abrin-I, Abrin-d, Abrin-II, APA = Abrus precatorius agglutinin = Abrus lectin = AAG, APA-I, APA-II | RIP 2 |
| | Abrus pulchellus Thwaites | Pulchellin, Pulchellin PI, Pulchellin PII, Pulchellin PIII | RIP 2 |
| | Pisum sativum subsp. sativum L. [Syn.: Pisum sativum var. arvense (L.) Poir.] | α-pisavin, β-pisavin | RIP 1 |
| | Pisum sativum var. macrocarpon | Sativin | RIP 1 candidate |
| Iridaceae | Iris hollandica var. Professor Blaauw | IrisRIP = IRIP, IrisRIPA1, IrisRIPA2, IrisRIP.A3 | RIP 1 |
| | | IRA, IRAb, IRAr | RIP 2 |
| Lamiaceae | Clerodendrum aculeatum (L.) Schltdl. | CA-SRI | RIP 1 candidate |
| | Clerodendrum inerme (L.) Gaertn. | CIP-29 | RIP 1 |
| | | CIP-34 | RIP 1 candidate |
| | Leonurus japonicus Houtt. | Leonurin | RIP candidate |
| Lauraceae | Cinnamomum bodinieri H. Lév. | Bodinierin | RIP 2 |
| | Cinnamomum camphora (L.) J.Presl | Camphorin | RIP 1 |
| | | Cinnamomin, Cinnamomin 1, Cinnamomin 2, Cinnamomin 3 | RIP 2 |
| | | Cinphorin | sRIP 2 |
| | Cinnamomum parthenoxylon (Jack) Meisn. [Syn.: Cinnamomum porrectum (Roxb.) Kosterm.] | Porrectin | RIP 2 |
| Malvaceae | Abelmoschus esculentus (L.) Moench | Abelesculin | RIP 1 |
| Nyctaginaceae | Boerhaavia diffusa L. | Boerhaavia inhibitor | RIP 1 candidate |
| | Bougainvillea spectabilis Willd. | BAP I, Bouganin = Bougainvillea RIP I | RIP 1 |
| | Bougainvillea × buttiana cv. Enid Lancester | BBP-24, BBP-28 | RIP 1 |
| | Bougainvillea × buttiana cv. Mahara | BBAP1 | RIP 1 |
| | Mirabilis expansa (Ruiz & Pav.) Standl. | ME1, ME2 | RIP 1 |
| | **Mirabilis jalapa L.** | MAP, MAP-2, MAP-3, MAP-4, MAP-S | RIP 1 |
| Olacaceae | Malania oleifera Chun & S. K. Lee | Malanin | lectin/ RIP 2 candidate |
| | Ximenia americana L. | Riproximin = Rpx, Rpx-I, Rpx-II | RIP 2 |
| Passifloraceae | Adenia digitata (Harv.) Engl. | Modeccin = Modeccin 4B, Modeccin 6B | RIP 2 |
| | Adenia ellenbeckii Harms | A. ellenbeckii lectin | RIP 2 candidate |
| | Adenia fruticosa Burtt Davy | A. fruticosa lectin | lectin |
| | Adenia glauca Schinz | A. glauca lectin | RIP 2 candidate |
| | Adenia goetzei Harms (unresolved name) | A. goetzei lectin | RIP 2 |
| | Adenia keramanthus Harms | A. keramanthus lectin | RIP 2 candidate |
| | Adenia lanceolata Engl. | Lanceolin | RIP 2 |
| | Adenia racemosa W. J. de Wilde | A. racemosa lectin | lectin |
| | Adenia spinosa Burtt Davy | A. spinosa lectin | RIP 2 candidate |
| | Adenia stenodactyla Harms | Stenodactylin | RIP 2 |
| | Adenia venenata Forssk. | A. venenata lectin | RIP 2 candidate |
| | Adenia volkensii Harms | Volkensin | RIP 2 |
| Phytolaccaceae | Phytolacca americana L. | α-PAP, PAP = Phytolacca americana protein = pokeweed antiviral protein, PAP-I, PAP-II, PAP-III, PAP-C, PAP-H, PAP-R, PAP-S, PAP-S1, PAP-S2 | RIP 1 |
| | Phytolacca dioica L. | Diocin 1, Diocin 2, PD-L1, PD-L2, PD-L3, PD-L4, PD-S1, PD-S2, PD-S3 | RIP 1 |
| | Phytolacca dodecandra L'Hér. | Dodecandrin, Dodecandrin C | RIP 1 |
| | Phytolacca heterotepala H. Walter | Heterotepalin 4, Heterotepalin 5b | RIP 1 |
| | Phytolacca insularis Nakai | Insularin = PIP = Phytolacca insularis antiviral protein, PIP2 = P. insularis antiviral protein 2 | RIP 1 |
| Poaceae | Hordeum vulgare L. | Barley toxin = Barley translation inhibitor = Barley Protein Synthesis Inhibitor = BPSI = RIP 30, Barley toxin I = Barley translation inhibitor I, Barley toxin II = Barley translation inhibitor II = Barley Protein Synthesis Inhibitor II = BPSI II, Barley toxin III = Barley translation inhibitor III, JIP60 | RIP 1 |
| | Oryza sativa L. | Oryza sativa RIP | RIP 1 |
| | Secale cereale L. | RPSI | RIP 1 |
| | Triticum aestivum L. | Tritin, Tritin 1, Tritin 2, Tritin 3, Tritin-S, Tritin-L | RIP 1 |
| | Zea mays L. | b-32 = maize RIP = maize proRIP1, Maize proRIP2 | RIP 3/ peculiar RIP 1 |
| Ranunculaceae | Eranthis hyemalis (L.) Salisb. | EHL | RIP 2 |
| Santalaceae | Phoradendron californicum Nutt. | PCL | RIP 2 |
| | Viscum album L. (Himalayan mistletoe) | HmRip, HmRip 1, HmRip 2, HmRip 3, HmRip 4 | RIP 2 |
| | Viscum album L. (European mistletoe) | ML-I = Mistletoe lectin I = Viscumin = Eu-ML = EML-1 = VAA-I, ML-II = Mistletoe lectin II = VAA-II, ML-III = Mistletoe lectin III = VAA-III | RIP 2 |
| | Viscum articulatum Burm. f. | Articulatin-D | RIP 2 |
| | Viscum coloratum (Kom.) Nakai [Syn.: Viscum album subsp. coloratum Kom.] | KML, KML-C, KML-IIL, KML-IIU, VCA | RIP 2 |
| Solanaceae | Nicotiana tabacum L. | CIP31 | RIP-like protein |
| | | TRIP | RIP 1 candidate |
| Thymelaeaceae | Phaleria macrocarpa (Scheff.) Boerl. | P. macrocarpa RIP | RIP candidate |
| * Schrot J, Weng A, Melzig MF, et al. Ribosome-inactivating and related proteins. Toxins (Basel). 2015 May 8;7(5):1556-615. | | | |

The invention is further illustrated by the following examples, which should not be interpreted as limiting the present invention in any way. Those examples which do not relate to a scaffold as defined in the claims are for reference only.

### EXAMPLES

### EXAMPLE A - TREATING A MAMMALIAN TUMOR-BEARING ANIMAL WITH A CONJUGATE OF THE INVENTION IN COMBINATION WITH AN ADC RESULTS IN SURVIVAL AND TUMOR REGRESSION

Female Balb/c nude mice were injected subcutaneously with a suspension of human A431 tumor cells. Under the skin of the mice, a human epidermal carcinoma developed in the xenograft animal tumor model. After injection of the tumor cells, the xenograft tumor was allowed to develop to a size of approximately 170-180 mm³. The A431 tumor cells have the following characteristics: high EGFR expressors, medium CD71 expressors, low HER2 expressors.

In Table A, the results of the treatment of control mice and tumor-bearing mice are presented. Tumor-bearing mice were treated with the indicated antibodies directed to either human Her2/neu, human EGFR, or human CD71, which are cell-surface receptors on the xenograft tumor. Cetuximab was covalently conjugated with saponin SO1861. The SO1861 was first provided with the linker EMCH (N-ε-maleimidocaproic acid hydrazide), which EMCH is a maleimide-and-hydrazide crosslinker for covalently conjugating sulfhydryls (reduced cysteines of the antibody)) to carbonyls (aldehyde or ketones; here the carbonyl of the aldehyde at position C-23 of the saponin). The saponin-EMCH was covalently coupled to reduced cysteines of the Cetuximab, forming a covalent thio-ether bond between the EMCH and the cysteine side chain. The ADCs trastuzumab-saporin (covalent conjugate) and anti-CD71 mAb (OKT-9, IgG) - saporin (covalent conjugate) were tested for their tumor-attacking efficacy in the mice, measured as tumor volume in time after start of the treatment with the ADCs. The dose of the ADCs was sub-optimal in the tumor model. That is to say, from previous experiments, it was established at which sub-optimal dose of the ADCs no tumor-regression or arrest of tumor growth would be observable.

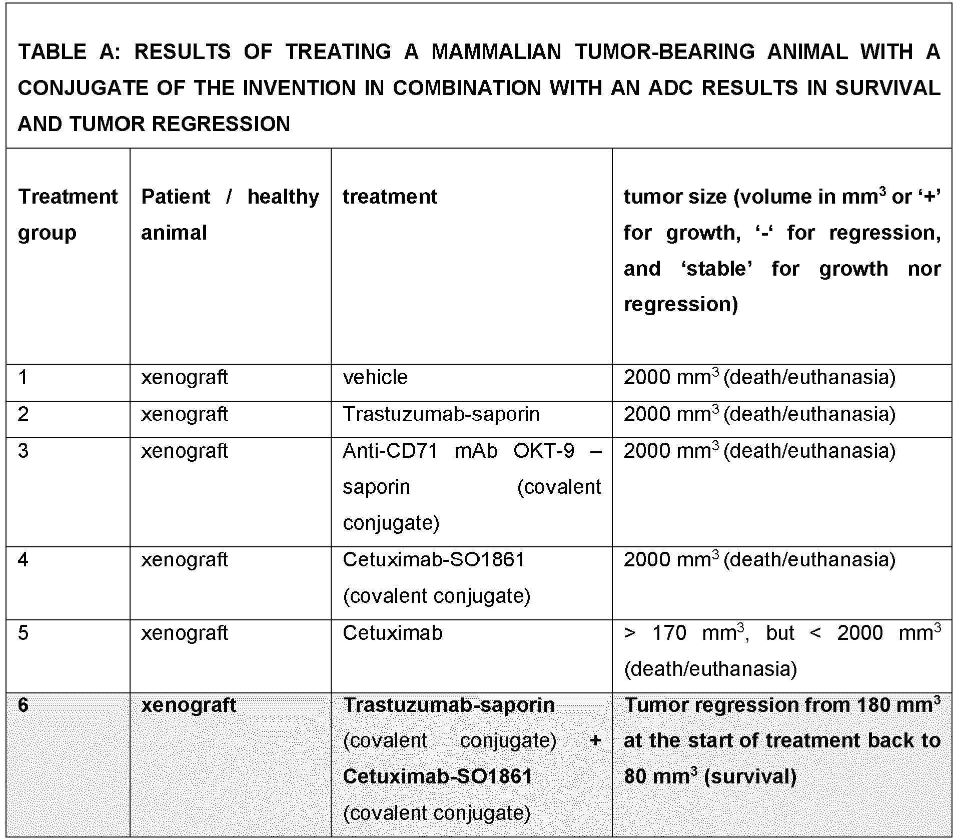

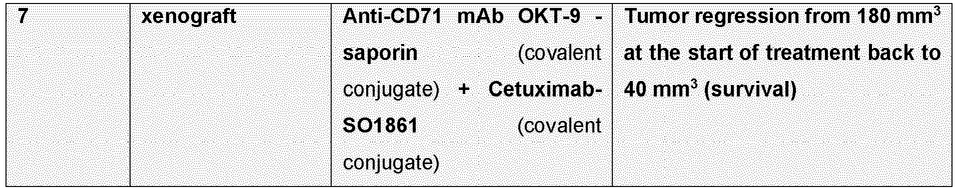

These results demonstrate that the combination therapy of an ADC at a dose which is ineffective when treatment of tumor-bearing mice with the ADC alone is considered (tumor growths, death of the mice is not prevented (euthanasia)), with a conjugate of the invention consisting of a tumor-cell specific receptor targeting antibody covalently bound to a saponin, i.e. SO1861, the covalent conjugate administered to the mice suffering from cancer, at a non-effective dose when administered alone (tumor growths, death of the mice is not prevented (euthanasia)), provides an efficient and efficacious treatment regimen, expressed as tumors in regression and prolonged survival of the treated animals (beyond the duration of the experiment). The sub-optimal dose of ADC combined with a covalently bound saponin-comprising conjugate of the invention which has no anti-tumor activity when administered alone, thus provide for an effective treatment option for cancer patients, wherein a relative low dose of the ADC is efficacious. A lower dose of ADC bears the promise of less risk for adverse events, or even no side effects at all. In addition, the stimulatory effect of the saponin-bearing conjugate of the invention when the efficacy of the ADC is considered, shows that ADCs which previously have proven to lack efficacy when tumor patient treatment is concerned, may gain renewed attention and value, since ADC efficacy is improved in combination therapy setting, as the current example demonstrated. Reference is made to Table A2 and Table A3, summarizing ADCs which were previously investigated in the human clinical setting, but then were for some ADCs retracted from further clinical investigation. Especially the ADCs for which clinical development was terminated due to observed lack of efficacy and/or due to occurrence of unacceptable adverse event are ADCs which may gain renewed value for cancer patients when combined with a covalently bound saponin-comprising conjugate of the invention, such as the cetuximab-saponin tested.

### EXAMPLE B - saponins mixture of Quillaja saponaria comprising QS-21, with endosomal/lysosomal escape enhancing activity

Scheme I displays the common molecular structure of a series of QS-21 saponins (in part adapted from: Conrado Pedebos, Laércio Pol-Fachin, Ramon Pons, Cilaine V. Teixeira Hugo Verli, Atomic Model and Micelle Dynamics of QS-21 Saponin, Molecules 2014, 19, 3744-3760). A mixture of water-soluble saponins obtained from Quillaja saponaria (Sigma-Aldrich, product No. S4521; Roth, Item No. 6857; InvivoGen, product 'Quil-A') may be applied in the endosomal/lysosomal escape enhancing conjugate, composition, combination of the invention, based on endosomal/lysosomal escape enhancing properties of at least one individual saponin present in the mixture, e.g. QS-21, or based on a combination of two or more of the saponins comprised by the mixture, such as QS-21 and QS-7.

The inventors demonstrated that the mixture of saponins from Quillaja saponaria at 2,5 microgram/ml dose was capable of enhancing endosomal escape of dianthin, as tested with mammalian tumor cells in a cell-based bioassay. The effector moiety exposed to the cells was dianthin covalently coupled to the ligand EGF: EGF-dianthin. Cells tested were tumor cell lines HeLa for free saponins, and A431, MDA-MB-468, CaSki and A2058 for testing the saponins when covalently coupled to cetuximab.

### Example 1

A trifunctional linker scaffold was designed and produced with specific chemical end groups (DBCO, TCO) for conjugation (labile, (L) conjugation) with on one arm an SO1861 molecule and on the other arm an antisense HSP27BNA oligo nucleotide (targeting and inducing degradation of the onco-target *hsp27* mRNA in cancer cells) to produce SO1861-L-trifunctional linker-L-HSP27BNA (Figure 16). SO1861-L-trifunctional linker-L-HSP27BNA was conjugated with its the third arm (maleimide) to the cysteine residues (Cys) anti-EGFR antibody, cetuximab (cetuximab-Cys-(SO1861-L-trifunctional linker-L-HSP27BNA)⁴).

This scaffold comprising conjugate was tested in a A431 xenograph 'nude' mouse tumor model for EGFR-mediated tumor targeted gene silencing activity. Dosings started at day 12 when tumors reached ~170mm³ in size and tumor samples were collected at 72h after the first dosing and analysed for HSP27 gene expression compared to cellular control mRNA expression (reference genes). This revealed that 1 dosing of 25mg/kg cetuximab-Cys-(SO1861-L-trifunctional linker-L-HSP27BNA)^{3,7} resulted in a 40% reduction in HSP27 gene expression in the tumors compared to single dosing of cetuximab-(Cys-L-SO1861)^{3,8} or cetuximab-(Lys-L-HSP27BNA)⁴ mono therapies (Figure 1). Compared to the vehicle control tumors a reduction of 25% gene silencing was observed. This shows and enables that conjugated SO1861 efficiently can induce targeted delivery of therapeutic oligo nucleotides in tumors, *in vivo.*

To further strengthen this, cetuximab-Cys-(SO1861-L-trifunctional linker-L-HSP27BNA DAR4)⁴ was tested for enhanced HSP27 gene silencing in EGFR expressing (A431) *, in vitro* as illustrated in Figure 2. Cetuximab-Cys-(SO1861-L-trifunctional linker-L-HSP27BNA)^{3,7} efficiently induces HSP27 gene silencing in A431 cells compared to Cetuximab-(Lys-L-HSP27BNA)⁴ or Cetuximab-(Cys-L-SO1861)^{3,8} alone (Figure 2).

### Example 2

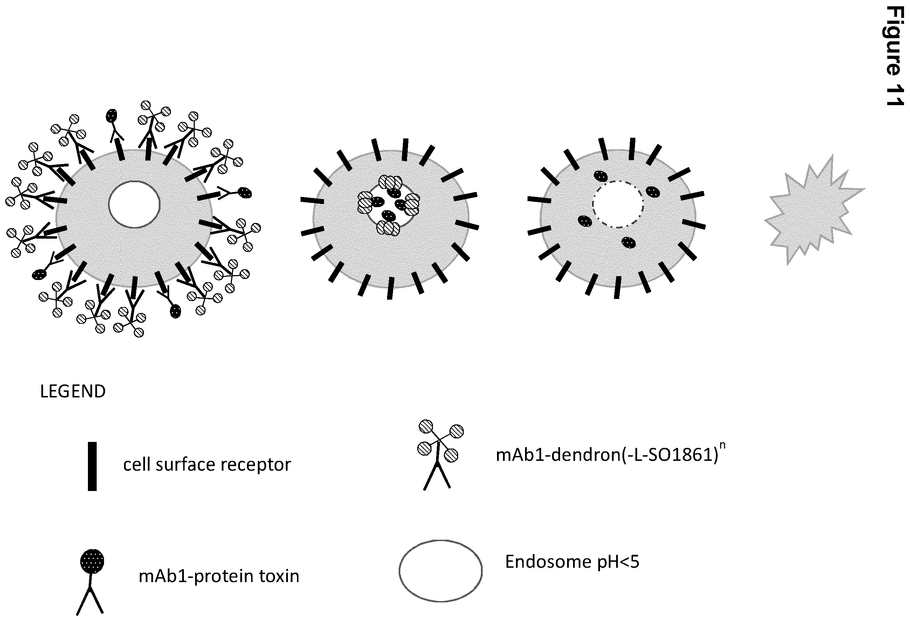

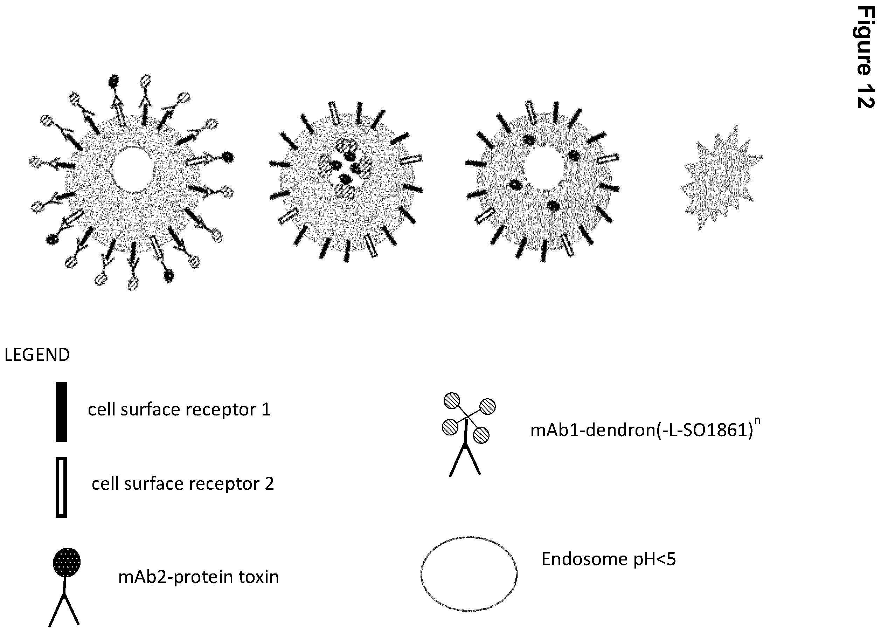

1 target 2-components system is the combination treatment of mAb1-(dendron(SO1861)ⁿ)ⁿ and mAb1-effector as illustrated in Figure 11 and whereas the 2 target 2-component system is the combination of mAb1-(dendron(SO1861)ⁿ)ⁿ + mAb2-effector as illustrated in Figure 12.

Dendron(-L-SO1861)⁴ was conjugated to the anti-EGFR antibody, cetuximab via cysteine residues (Cys) conjugation with a DAR3,9, cetuximab-Cys-(dendron(-L-S01861)⁴)^{3,9} and tested for enhanced cell killing activity in combination with an anti-EGFR antibody-protein toxin conjugate (cetuximab-saporin) in EGFR expressing cells (MDA-MB-468). Cetuximab-Cys-(dendron(-L-SO1861)⁴)^{3,9} + 10 pM cetuximab-saporin efficiently induces toxin-mediated cell killing in high EGFR expressing cells, whereas this was not induced by Cetuximab-Cys-(dendron(-L-SO1861)⁴)^{3,9} or cetuximab (equivalent) + 10pM cetuximab-saporin or cetuximab (Figure 3A). Similar experiments in cells that express low levels of EGFR (HeLa) revealed no activity of Cetuximab-Cys-(dendron(-L-SO1861)⁴)^{3,9} (Figure 3C) indicating that in the absence of sufficient EGFR receptor expression, effective intracellular SO1861 concentrations are not reached (threshold) to induce endosomal protein toxin escape and toxin-mediated cell killing.

Next, dendron(-L-SO1861)⁴ was conjugated to the anti-HER2 antibody, trastuzumab via cysteine conjugation (Cys) with a DAR4, trastuzumab-Cys-(dendron(-L-SO1861)⁴)⁴ and tested for enhanced cell killing activity in combination with an anti-HER2 antibody-protein toxin conjugate (trastuzumab-saporin) in HER2 expressing cells (SK-BR-3). trastuzumab-Cys-(dendron(-L-SO1861)⁴)⁴ + 50 pM trastuzumab-saporin efficiently induce toxin-mediated cell killing, whereas this was not induced by trastuzumab-Cys-(dendron(-L-SO1861)⁴)⁴ or trastuzumab (equivalent) + 50nM trastuzumab-saporin or trastuzumab (Figure 3B). Similar experiments in cells that express low levels of HER2 (JIMT-1) revealed no activity of Trastuzumab-Cys-(dendron(-L-SO1861)⁴)⁴ (Figure 3D) indicating that in the absence of sufficient HER2 receptor expression, effective intracellular SO1861 concentrations are not reached (threshold) to induce endosomal protein toxin escape and toxin-mediated cell killing.

Next, Cetuximab-Cys-(dendron(-L-SO1861)⁴)^{3,9} or Cetuximab-Lys-(dendron(-L-SO1861)⁴)^{4,4} (Lys=dendron(-L-SO1861)⁴ conjugated to lysines of antibody) was tested in combination with 10 pM CD71mab-saporin in a 2 target 2 components system in EGFR⁺⁺/CD71⁺ cells (MDA-MB-468). This showed for both conjugates a strong enhancement of the cell killing activity, whereas this was not induced by Cetuximab-Cys-(dendron(-L-SO1861)⁴)^{3,9} or Cetuximab-Lys-(dendron(-L-SO1861)⁴)^{4,4} or cetuximab (equivalent) + 10 pM CD71mab-saporin or cetuximab (Figure 4A). Similar experiments in cells that express lower levels of EGFR (CaSKi, EGFR⁺/CD71⁺) revealed reduced activity for both cetuximab-Cys-(dendron(-L-SO1861)⁴)^{3,9} or cetuximab-Lys-(dendron(-L-SO1861)⁴)^{4,4} (Figure 4C ) compared to the activity in high expressors (Figure 4A) indicating that in cells with lower EGFR receptor expression levels, the effective intracellular SO1861 concentrations is lower resulting in reduced toxin-mediated cell killing activity.

Same experiment was performed with trastuzumab-Cys-(dendron(-L-SO1861)⁴)⁴ or trastuzumab-Lys-(dendron(-L-SO1861)⁴)^{4,7} in combination with CD71mab-saporin on HER2⁺⁺/CD71⁺ (SK-BR-3) cell lines revealing strong cell killing activity compared to the controls (Figure 4B). When trastuzumab-Cys-(dendron(-L-SO1861)⁴)⁴ or trastuzumab-Lys-(dendron(-L-SO1861)⁴)^{4,7} was tested on HER2^{+/-}/CD71⁺ (JIMT-1) in combination with 10 pM CD71mab-saporin no cell killing activity could be observed indicating that in the absence of sufficient HER2 receptor expression, effective intracellular SO1861 concentrations are not reached (threshold) to induce endosomal protein toxin escape and toxin-mediated cell killing.

Next, trastuzumab-Cys-(dendron(-L-SO1861)⁴)⁴ + trastuzumab-emtansine (T-DM1, antibody-small molecule toxin conjugate) was tested for enhanced cell killing activity in HER2 expressing cells (SK-BR-3). No enhanced cell killing was observed with this combination, compared to T-DM1 alone or T-DM1 + equivalent trastuzumab, since the endosomal membrane forms no barrier for small molecules to reach the cytoplasm. (Figure 5).

### Example 3

### Materials and methods

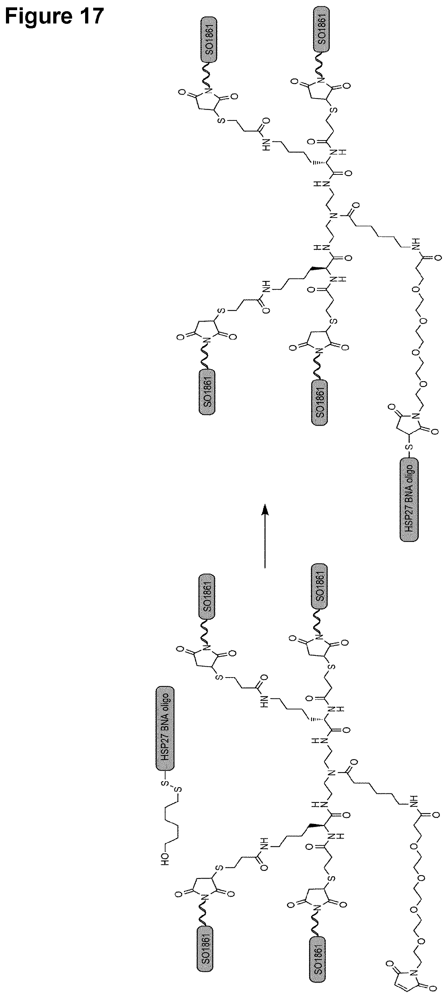

### dendron(SO1861)₄-BNA oligo synthesis (Figure 17)

HSP27BNA oligo disulfide (1.1 mg, 0.187 µmol) was dissolved in 20 mM NH₄HCO₃ with 1.0 mM TCEP (500 µL) and the mixture was shaken for 1 min and left standing at room temperature. After 1 hour the reaction mixture was filtered by using a centrifugal filter with a molecular weight cut-off of 3000 Da (14000 × g for 30 min). The residue solution was diluted with 20 mM NH₄HCO₃ with 1.0 mM TCEP (500 µL) and the resulting mixture was filtered again under the same conditions described above. The residue solution was diluted with 20 mM NH₄HCO₃/acetonitrile (3:1, v/v, 1.0 mL) and the resulting mixture was added to dendron(SO1861)4-maleimide1 (3.54 mg, 0.375 µmol) (Figure 17) . The reaction mixture was shaken for 1 min and left standing at room temperature. After 10 min the reaction mixture was subjected to preparative LC-MS.^{4A} Fractions corresponding to the product were immediately pooled together, frozen and lyophilized overnight to give the title compound (1.25 mg, 85%) as a white fluffy solid. Purity based on LC-MS 94%
LRMS (m/z): 1896 [M-8]⁸⁻, 2167 [M-7]⁷⁻
LC-MS r.t. (min): 3.77^{6B}

### results

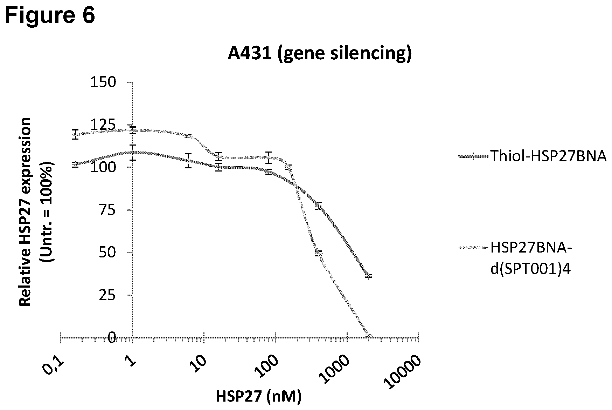

HSP27BNA oligo, (antisense BNA oligo targeting the mRNA transcript of the cancer target, heat shock protein 27 (HSP27BNA)) was conjugated to a dendron(-L-SO1861)⁴ (HSP27BNA-dendron(-L-SO1861)⁴, Figure 17) and co-administrated to A431 cancer cells. As readout, gene silencing of HSP27 mRNA in A431 cells was determined. This revealed that HSP27BNA-dendron(-L-SO1861)⁴ treatment resulted in an improvement of HSP27 gene silencing activity compared to the HSP27BNA alone (Figure 6).

### Example 4

### Methods

### SO1861 releasing assay

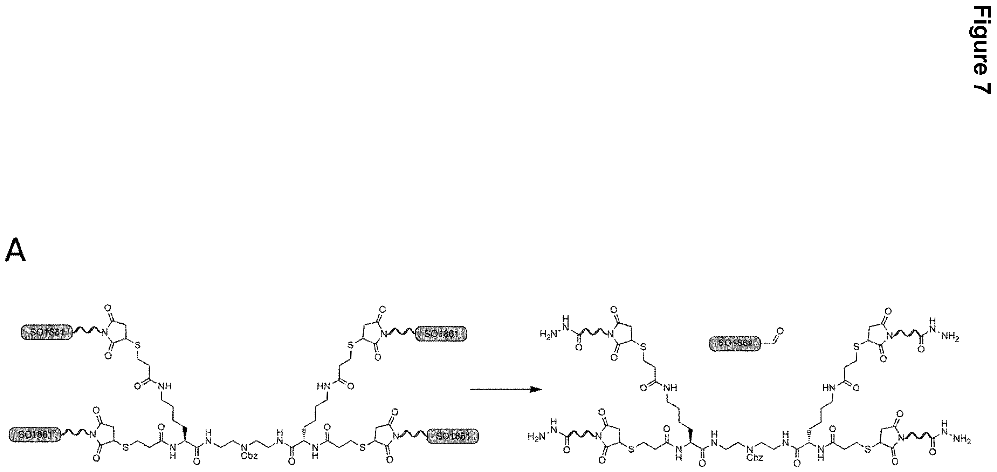

To dendron(SO1861)₄-Cbz (0.05 mg) (Figure 7A) was added 50 µL of solution containing water/acetonitrile/TFA (1.00 mL/1.00 mL/4 drops). The reaction mixture was shaken for 1 min and left standing at room temperature. The SO1861 release was followed over time by using UPLC-MS.⁴

### Results

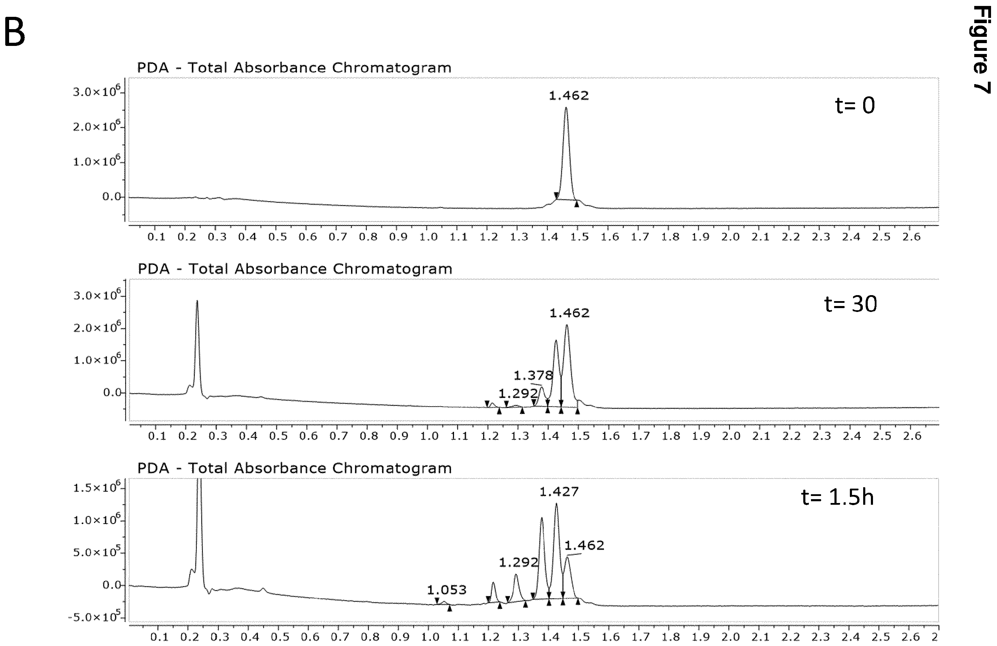

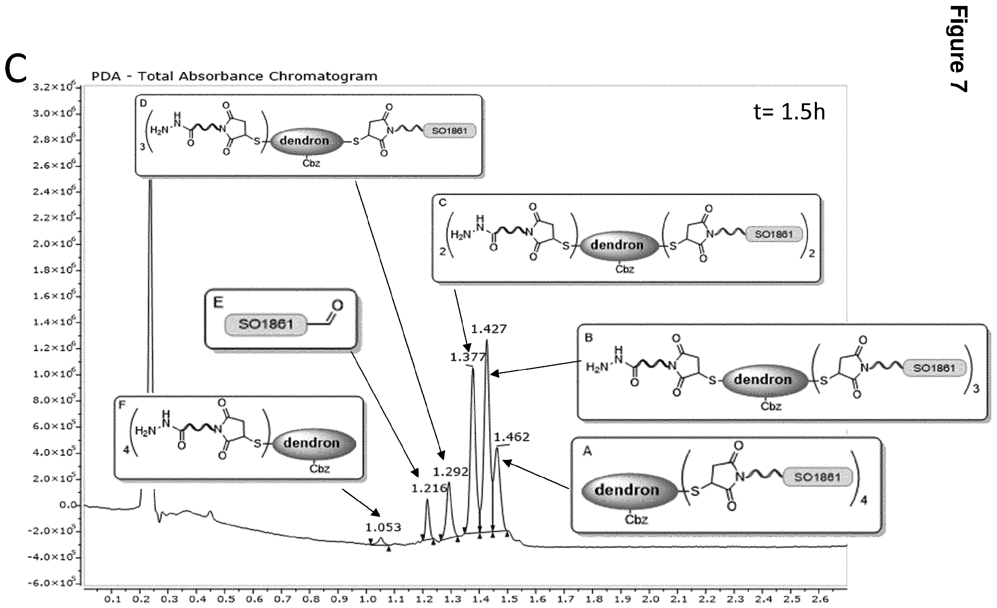

The release efficiency of the SO1861 molecules from the dendron(-L-SO1861)⁴ (Figure 7A) under acid conditions has been determined (Figure 7A). Figure 7B shows the UPLC UV-traces (PDA) of dendron(-L-SO1861)⁴ itself (top), reaction mixture after 30 min (middle) and the reaction mixture after 1.5 hours (bottom). Figure 7C shows the interpretation of the observed m/z values by LRMS. The following m/z values with corresponding UV r.t. (min) were observed: r.t. = 1.46, 2282 [M-4]⁴⁻ (A, dendron(-L-SO1861)⁴); r.t. = 1.43, 2427 [M-3]³⁻ (B, dendron(-L-SO1861)³); r.t. = 1.38, 1812 [M-3]³⁻ (C, dendron(-L-SO1861)² ); r.t. = 1.29, 1797 [M+2]²⁺ (D, dendron-L-SO1861); r.t. = 1.22, 1862 [M-1]¹⁻ (E, SO1861); r.t. = 1.05, 1747/1769 [M+1/M+23]¹⁺ (F, dendron-).

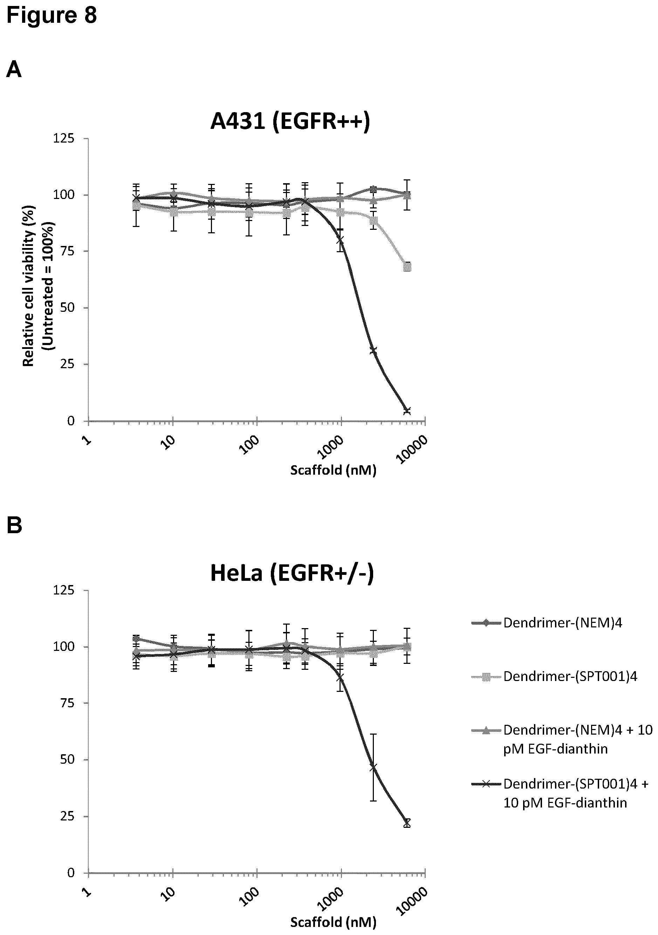

Next, dendron(-L-SO1861)⁴ was tested for enhanced delivery of a targeted toxin, EGFdianthin on EGFR expressing cells (A431 and HeLa). This shows that dendron(L-SO1861)⁴ + 10 pM EGFdianthin can induce enhanced toxin-mediated cell killing, whereas the 'naked' dendron (Dendron(NEM)⁴) or dendron(-L-SO1861)⁴ or Dendron(NEM)⁴ + 10 pM EGFdianthin is not showing enhanced cell killing at these concentrations (Figure 8A, 8B).

### Example 5

### dendron(-L-SO1861)" synthesis (Figure 13, 14, 15)

### materials and methods

### Abbreviations

- DCM: dichloromethane
- DIPEA: *N,N*-diisopropylethylamine
- DMF: *N,N*-dimethylformamide
- EDCI.HCI: 3-((Ethylimino)methyleneamino)-N,N-dimethylpropan-1-aminium chloride
- EMCH.TFA: N-(ε-maleimidocaproic acid) hydrazide, trifluoroacetic acid salt
- min: minutes
- r.t.: retention time
- TCEP: tris(2-carboxyethyl)phosphine hydrochloride
- Temp: temperature
- TFA: trifluoroacetic acid
- THF: tetrahydrofuran

### Analytical methods

### LC-MS method 1, ¹

Apparatus: Agilent 1200 Bin. Pump: G1312A, degasser; autosampler, ColCom, DAD: Agilent G1316A, 210, 220 and 220-320 nm, PDA: 210-320 nm, MSD: Agilent LC/MSD G6130B ESI, pos/neg 100-1000; ELSD Alltech 3300 gas flow 1.5 ml/min, gas temp: 40°C; column: Waters XSelect^{™} CSH C18, 30×2.1mm, 3.5µm, Temp: 35 °C, Flow: 1 mL/min, Gradient: t₀ = 5% A, t_{1.6min} = 98% A, t₃ₘᵢₙ = 98% A, Posttime: 1.3 min, Eluent A: 0.1% formic acid in acetonitrile, Eluent B: 0.1% formic acid in water.

### LC-MS method 2, ²

Apparatus: Agilent 1260 Bin. Pump: G7112B, Multisampler, Column Comp, DAD: Agilent G7115A, 210, 220 and 220-320 nm, PDA: 210-320 nm, MSD: Agilent LC/MSD G6130B ESI, mass ranges depending on the molecular weight of the product:
^{A}pos/neg 100-1000
^{B}pos/neg 100-1400
; ELSD Alltech 3300 gas flow 1.5 ml/min, gas temp: 40°C; column: Waters XSelect^{™} C18, 30×2.1mm, 3.5µm, Temp: 40 °C, Flow: 1 mL/min, Gradient: t₀ = 5% A, t_{1.6min} = 98% A, t₃ₘᵢₙ = 98% A, Posttime: 1.3 min, Eluent A: 0.1% formic acid in acetonitrile, Eluent B: 0.1% formic acid in water.

### LC-MS method 3, ³

Apparatus: Waters IClass; Bin. Pump: UPIBSM, SM: UPISMFTN with SO; UPCMA, PDA: UPPDATC, 210-320 nm, SQD: ACQ-SQD2 ESI, pos/neg 800-1500; ELSD: gaspressure 40 psi, drift tube temp: 50°C; column: Waters XSelect^{™} CSH C18, 50×2.1mm, 2.5µm Temp: 25°C, Flow: 0.6 mL/min, Gradient: t₀ = 5% A, t_{2.0min} = 98% A, t_{2.7min} = 98% A, Posttime: 0.3 min, Eluent A: acetonitrile, Eluent B: 10 mM ammonium bicarbonate in water (pH=9.5).

### LC-MS method 4, ⁴

Apparatus: Waters IClass; Bin. Pump: UPIBSM, SM: UPISMFTN with SO; UPCMA, PDA: UPPDATC, 210-320 nm, SQD: ACQ-SQD2 ESI, pos/neg 1500-2500; ELSD: gaspressure 40 psi, drift tube temp: 50°C; column: Waters XSelect^{™} CSH C18, 50×2.1mm, 2.5µm Temp: 25°C, Flow: 0.6 mL/min, Gradient: t₀ = 15% A, t_{2.0min} = 60% A, t_{2.7min} = 98% A, Posttime: 0.3 min, Eluent A: acetonitrile, Eluent B: 10 mM ammonium bicarbonate in water (pH=9.5).

### LC-MS method 5, ⁵

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product: ^{A}pos/neg 1500-2500
^{B}neg 2000-3000
; ELSD: gaspressure 40 psi, drift tube temp: 50°C; column: Acquity C18, 50×2.1mm, 1.7µm Temp: 60°C, Flow: 0.6 mL/min, Gradient: t₀ = 2% A, t_{5.0min} = 50% A, t_{6.0min} = 98% A, Posttime: 1.0 min, Eluent A: acetonitrile, Eluent B: 10 mM ammonium bicarbonate in water (pH=9.5).

### Preparative methods

### Preparative MP-LC method 1, ¹

Instrument type: Reveleris^{™} prep MPLC; column: Waters XSelect^{™} CSH C18 (145×25 mm, 10µ); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient: t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B; Detection UV: 210, 225, 285 nm.

### Preparative MP-LC method 2, ²

Instrument type: Reveleris^{™} prep MPLC; Column: Phenomenex LUNA C18(3) (150×25 mm, 10µ); Flow: 40 mL/min; Column temp: room temperature; Eluent A: 0.1% (v/v) Formic acid in water, Eluent B: 0.1% (v/v) Formic acid in acetonitrile; Gradient: t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B; Detection UV : 210, 225, 285 nm.

### Preparative LC-MS method 1, ³

MS instrument type: Agilent Technologies G6130B Quadrupole; HPLC instrument type: Agilent Technologies 1290 preparative LC; Column: Waters XSelect^{™} CSH (C18, 100×30mm, 10µ); Flow: 25 ml/min; Column temp: room temperature; Eluent A: 100% acetonitrile; Eluent B: 10 mM ammonium bicarbonate in water pH=9.0; lin. gradient depending on the polarity of the product:
^{A}t₀ = 20% A, t₂ₘᵢₙ = 20% A, t_{8.5min} = 60% A, t₁₀ₘᵢₙ = 100% A, t₁₃ₘᵢₙ = 100% A
^{B}t₀ = 5% A, t₂ₘᵢₙ = 5% A, t_{8.5min} = 40% A, t₁₀ₘᵢₙ = 100% A, t₁₃ₘᵢₙ = 100% A
^{C}t₀ = 10% A, t₂ₘᵢₙ = 10% A, t_{8.5min} = 50% A, t₁₀ₘᵢₙ = 100% A, t₁₃ₘᵢₙ = 100% A
; Detection: DAD (220-320 nm); Detection: MSD (ESI pos/neg) mass range: 100 - 800; Fraction collection based on DAD.

### Preparative LC-MS method 2, ⁴

MS instrument type: Agilent Technologies G6130B Quadrupole; HPLC instrument type: Agilent Technologies 1290 preparative LC; column: Waters XBridge Shield (C18, 150×19mm, 5µ); Flow: 25 ml/min; Column temp: room temperature; Eluent A: 100% acetonitrile; Eluent B: 10 mM ammonium bicarbonate in water pH=9.0; lin. gradient: t₀ = 5% A, t_{2.5min} = 5% A, t₁₁ₘᵢₙ = 40% A, t₁₃ₘᵢₙ = 100% A, t₁₇ₘᵢₙ = 100% A; Detection: DAD (220-320 nm); Detection: MSD (ESI pos/neg) mass range: 100 - 800; Fraction collection based DAD

### Flash chromatography

Grace Reveleris X2^{®} C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50bar (725psi); Detection: UV 200-400nm, combination of up to 4 UV signals and scan of entire UV range, ELSD; Column sizes: 4-330g on instrument, luer type, 750g up to 3000g with optional holder.

### SO1861-EMCH synthesis (Figure 13)

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC.¹ Fractions corresponding to the product were immediately pooled together, frozen and lyophilized overnight to give the title compound (120 mg, 90%) as a white fluffy solid. Purity based on LC-MS 96%.
LRMS (m/z): 2069 [M-1]¹⁻
LC-MS r.t. (min): 1.08⁴

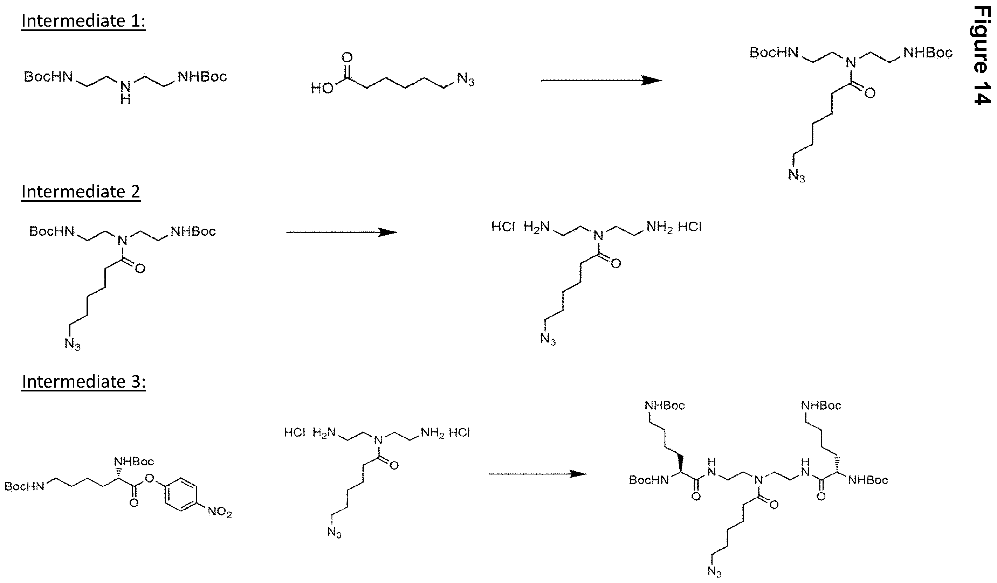

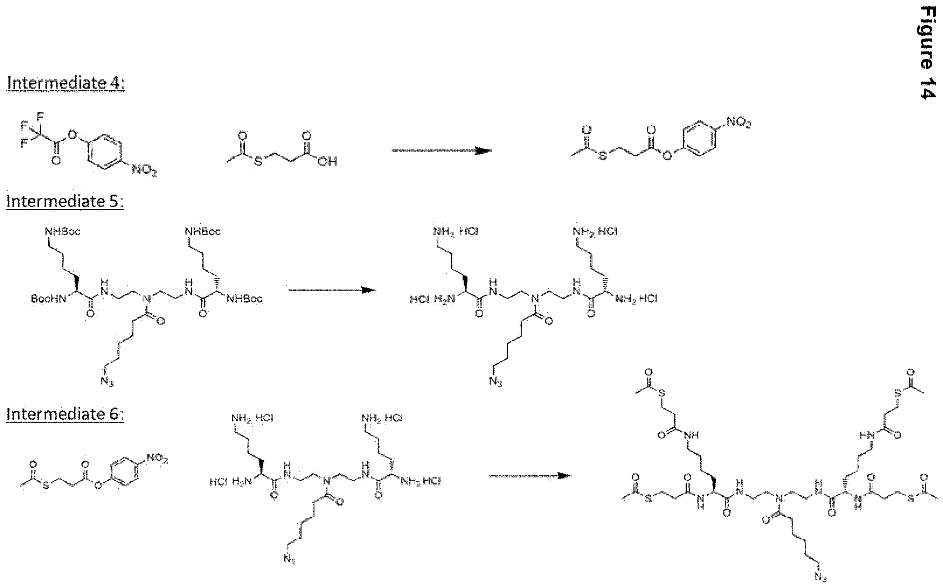

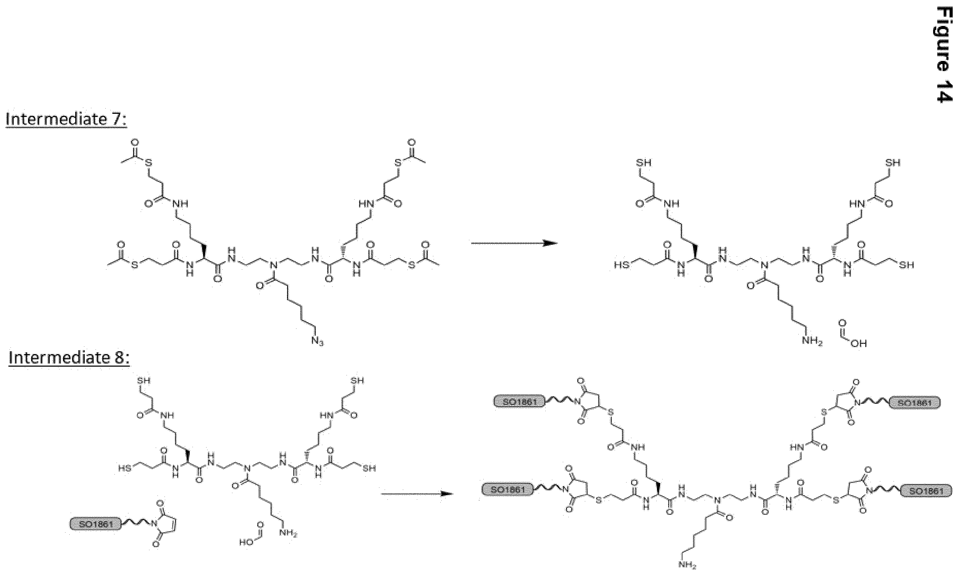

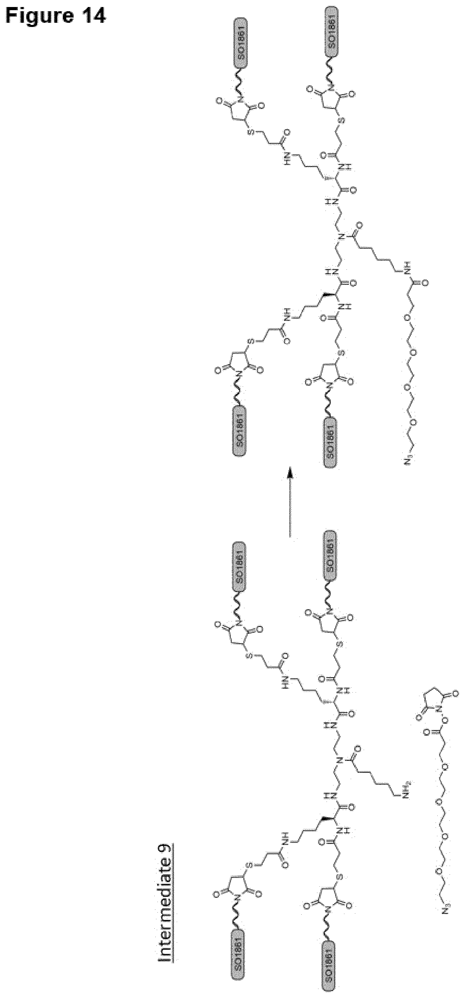

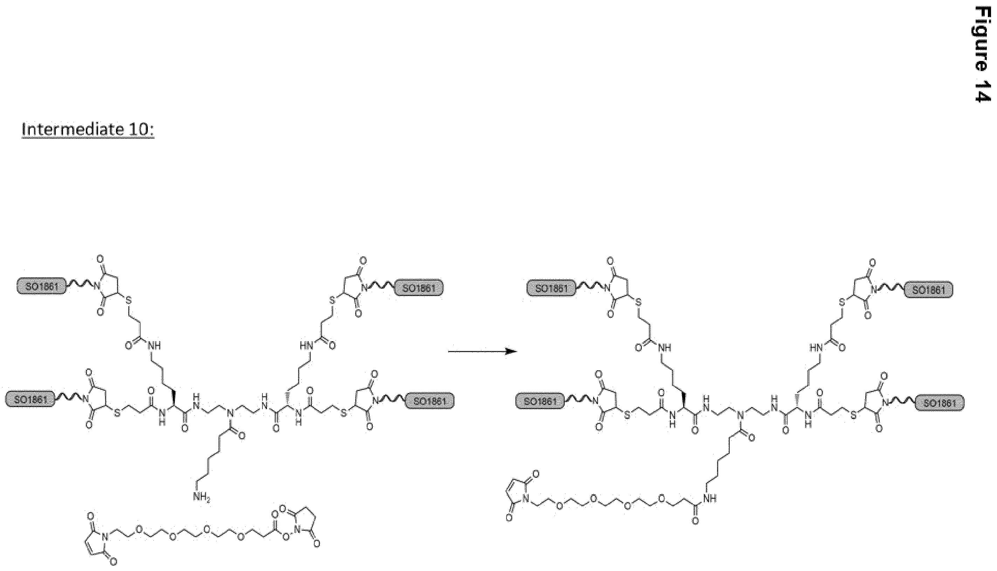

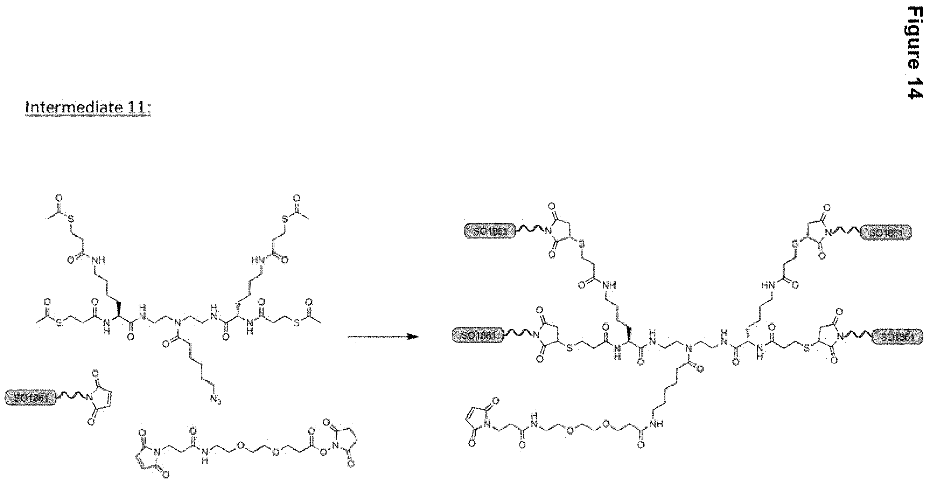

### Dendron(-L-SO1861)⁴ synthesis (Figure 14)

### Intermediate 1:

### di-tert-butyl (((6-azidohexanoyl)azanediyl)bis(ethane-2,1-diyl))dicarbamate

6-azidohexanoic acid (0.943 g, 6.00 mmol), EDCI.HCI (1.21 g, 6.30 mmol) and Oxyma Pure (0.938 g, 6.60 mmol) were dissolved in DMF (10.0 mL) and the mixture was stirred for 5 min. Next a solution of di-tert-butyl (azanediylbis(ethane-2,1-diyl))dicarbamate (1.82 g, 6.00 mmol) in DMF (5.00 mL) was added and the reaction mixture was stirred at room temperature. After 5 hours the reaction mixture was evaporated *in vacuo* and the residue was dissolved in ethyl acetate (50 mL). The resulting solution was washed with 1N potassium bisulphate solution (50 mL), saturated sodium bicarbonate solution (2 × 50 mL) and brine (50 mL), dried over Na₂SO₄, filtered and evaporated *in vacuo.* The residue was purified by flash chromatography (ethyl acetate - heptane gradient, 10:90 rising to 100:0) to give the title compound (2.67 g, 100%) as a white solid. Purity based on LC-MS 98%.
LRMS (m/z): 287/343/465 [M-155/M-99/M+23]¹⁺
LC-MS r.t. (min): 2.02^{2A}

### Intermediate 2:

### N,N-bis(2-aminoethyl)-6-azidohexanamide dihydrochloride

To di-tert-butyl (((6-azidohexanoyl)azanediyl)bis(ethane-2,1-diyl))dicarbamate (2.66 g, 6.00 mmol) was added HCI in isopropanol (5-6 N, 20.0 mL, 110 mmol) and the reaction mixture was stirred at room temperature. After 4 hours, the reaction mixture was evaporated *in vacuo* and the resulting crude product was co-evaporated with DCM (3 × 20 mL) to give the crude title product (1.49 g, 79%) as a white solid.

### LRMS (m/z): 243 [M+1]¹⁺

### Intermediate 3:

### tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate

To a solution of N,N-bis(2-aminoethyl)-6-azidohexanamide dihydrochloride (1.19 g, 3.76 mmol) in DMF (30.0 mL) and DIPEA (2.62 mL, 15.1 mmol) was added Boc-Lys(Boc)-ONp (3.69 g, 7.90 mmol) and the mixture was stirred at room temperature overnight. The reaction mixture was evaporated *in vacuo* and the residue was dissolved in ethyl acetate (100 mL). The resulting solution was washed with 1N potassium bisulphate solution (100 mL) and saturated sodium bicarbonate solution (5 × 100 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (3.07 g, 91%) as a slightly yellowish solid. Purity based on LC-MS 94%.
LRMS (m/z): 800/900/922 [M-99/M+1/M+23]¹⁺
LC-MS r.t. (min): 2.17^{2A}

### Intermediate 4:

### 4-nitrophenyl 3-(acetylthio)propanoate

4-Nitrophenyl trifluoroacetate (5.17 g, 22.0 mmol) and 3-(Acetylthio)propionic Acid (2.96 g, 20.0 mmol) were dissolved in DCM (50.0 mL). Next, DIPEA (6.97 mL, 40.0 mmol) was added and the reaction mixture was stirred at room temperature overnight. The reaction mixture was evaporated *in vacuo* and the residue was dissolved in ethyl acetate (50 mL). The resulting solution was washed with 1N potassium bisulphate solution (50 mL), saturated sodium bicarbonate solution (5 × 50 mL) and brine (50 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (4.90 g, 91%) as a slightly yellowish solid. Purity based on LC-MS 99%.
LRMS (m/z): 292 [M+23]¹⁺
LC-MS r.t. (min): 1.94^{2A}

### Intermediate 5:

### (S)-2,6-diamino-N-(2-(6-azido-N-(2-((S)-2,6-diaminohexanamido)ethyl)hexanamido)ethyl)hexanamide tetrahydrochloride

tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate (1.80 g, 2.00 mmol) was dissolved in HCI in isopropanol (5-6N, 50.0 ml, 275 mmol) and the reaction mixture was stirred at room temperature overnight. The reaction mixture was evaporated *in vacuo* and the resulting crude product was co-evaporated with DCM (3 × 20 mL) to give the crude title product as a white solid.
LRMS (m/z): 250 [M+2]²⁺, 500 [M+1]¹⁺

### Intermediate 6:

### (2S)-2,6-bis[3-(acetylsulfanyl)propanamido]-N-[2-(6-azido-N-{2-[(2S)-2,6-bis[3-(acetylsulfanyl)propanamido]hexanamido]ethyl}hexanamido)ethyl]hexanamide

To a solution of (S)-2,6-diamino-N-(2-(6-azido-N-(2-((S)-2,6- diaminohexanamido)ethyl)hexanamido) ethyl)hexanamide tetrahydrochloride (1.29 g, 2.00 mmol) in DMF (30 mL) and DIPEA (3.48 mL, 20.0 mmol) was added 4-nitrophenyl 3-(acetylthio)propanoate (2.26 g, 8.40 mmol) and the reaction mixture was stirred at room temperature over the weekend. The reaction mixture was evaporated *in vacuo* and the residue was dissolved in DCM/methanol (95:5, v/v, 100 mL). The resulting solution was washed with 1N potassium bisulphate solution (100 mL), 1 N sodium hydroxide solution (3 × 100 mL) and brine (100 mL), dried over Na₂SO₄, filtered and evaporated *in vacuo.* The residue was purified by flash chromatography (DCM - methanol/DCM (1/9, v/v) gradient 100:0 rising to 0:100) to give the title product (1.33 g, 65%) as a white solid. On LC-MS an impurity (15%) was found with m/z values corresponding to the product with one deprotected thioacetate group. The impurity was formed during or after work-up. Purity based on LC-MS 85%.
LRMS (m/z): 510 [M+2]²⁺, 1019/1041 [M+1/M+23]¹⁺
LC-MS r.t. (min): 1.86^{2B}

### Intermediate 7:

### N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate

Scaffold 2 (102 mg, 0.100 mmol) was dissolved in methanol (1.00 ml). Next, a freshly prepared 1 N Sodium hydroxide solution (0.440 ml, 0.440 mmol) was added and the reaction mixture was stirred at room temperature. After 30 min a 1.0 M trimethylphosphine solution in THF (0.500 ml, 0.500 mmol) was added and the resulting mixture was stirred at room temperature. After 30 min the reaction mixture was evaporated *in vacuo* and co-evaporated with methanol (2 × 10 mL). The residue was dissolved in a mixture of methanol/water (9:1, v/v, 1.00 mL) and the resulting solution was subjected to preparative MP-LC.² Fractions corresponding to the product were immediately pooled together, frozen and lyophilized overnight to give the title compound (75.6 mg, 87%) as a colorless sticky oil. Purity based on LC-MS 96%.
LRMS (m/z): 513 [M+2]²⁺, 825 [M+1]¹⁺
LC-MS r.t. (min): 1.42^{2A}

### Intermediate 8:

### dendron(-L-SO1861)⁴-amine

N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate (2.73 mg, 3.13 µmol) was dissolved in a mixture of 20 mM NH₄HCO₃ with 0.5 mM TCEP/acetonitrile (3:1, v/v, 3.00 mL). Next, SO1861-EMCH (29.2 mg, 0.014 mmol) was added and the reaction mixture was stirred at room temperature. After 1.5 hours the reaction mixture was subjected to preparative LC-MS.^{3B} Fractions corresponding to the product were immediately pooled together, frozen and lyophilized overnight to give the title compound (12.3 mg, 43%) as a white fluffy solid. Purity based on LC-MS 97%.
LRMS (m/z): 1517 [M-6]⁶⁻, 1821 [M-5]⁵⁻, 2276 [M-4]⁴⁻
LC-MS r.t. (min): 4.39^{5A}

### Intermediate 9:

### dendron(-L-SO1861)⁴-azide

Dendron(SO1861)₄-amine (6.81 mg, 0.748 µmol) and 2,5-dioxopyrrolidin-1-yl 1-azido-3,6,9,12-tetraoxapentadecan-15-oate (2.90 mg, 7.48 µmol) were dissolved in DMF(1.00 mL). Next, DIPEA (1.302 µL, 7.48 µmol) was added and the mixture was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (5.86 mg, 84%) as a white fluffy solid. Purity based on LC-MS 90%.
LRMS (m/z): 2344 [M-4]⁴⁻
LC-MS r.t. (min): 4.78^{5B}

### Intermediate 10:

### dendron(-L-SO1861)⁴-maleimide1

Dendron(SO1861)₄-amine (8.12 mg, 0.891 µmol) and 2,5-dioxopyrrolidin-1-yl 1-(2,5-dioxo-2,5-dihydro-1H-pyrrol-1-yl)-3,6,9,12-tetraoxapentadecan-15-oate (3.94 mg, 8.91 µmol) were dissolved in DMF(1.00 mL). Next, DIPEA (1.55 µL, 8.91 µmol) was added and the mixture was shaken for 1 min and left standing at room temperature. After 3 hours the reaction mixture was subjected to preparative LC-MS.^{3C} Fractions corresponding to the product were immediately pooled together, frozen and lyophilized overnight to give the title compound (6.76 mg, 80%) as a white fluffy solid. Purity based on LC-MS 66%.
LRMS (m/z): 2358 [M-4]⁴⁻
LC-MS r.t. (min): 2.13^{6C}

### Intermediate 11:

### dendron(-L-SO1861)⁴-maleimide2

Scaffold 2 (5.10 mg, 5.00 µmol) was dissolved in methanol (100 µL). Next, a freshly prepared 1 N Sodium hydroxide solution (22.0 µL, 22.0 µmol) was added and the mixture was shaken for 1 min and left standing at room temperature. After 30 min a 1.0 M trimethylphosphine solution in THF (25.0 µL, 25.0 µmol) was added and the resulting mixture was shaken for 1 min and left standing at room temperature. After 30 min the reaction mixture was evaporated *in vacuo* and co-evaporated with methanol (2 × 5 mL). The resulting residue was dissolved in a mixture of 20 mM NH₄HCO₃ with 0.5 mM TCEP/acetonitrile (3:1, v/v, 3.242 mL). From this solution, directly, 1000 µL was added to SO1861-EMCH (14.4 mg, 6.94 µmol, 4.5 equiv. compared to the scaffold) and the mixture was shaken for 1 min and left standing at room temperature. After 10 min the reaction mixture was lyophilized overnight. To the resulting residue 2,5-Dioxopyrrolidin-1-yl 3-(2-(2-(3-(2,5-dioxo-2h-pyrrol-1(5h)-yl)propanamido)ethoxy)ethoxy)propanoate (5.84 mg, 0.014 mmol) and DMF (1.00 mL) were added. Next, DIPEA (2.39 µL, 0.014 mmol) was added and the suspension was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (10.9 mg, 85%) as a white fluffy solid. Purity based on LC-MS 80%.
LRMS (m/z): 2354 [M-4]⁴⁻
LC-MS r.t. (min): 4.16^{5B}

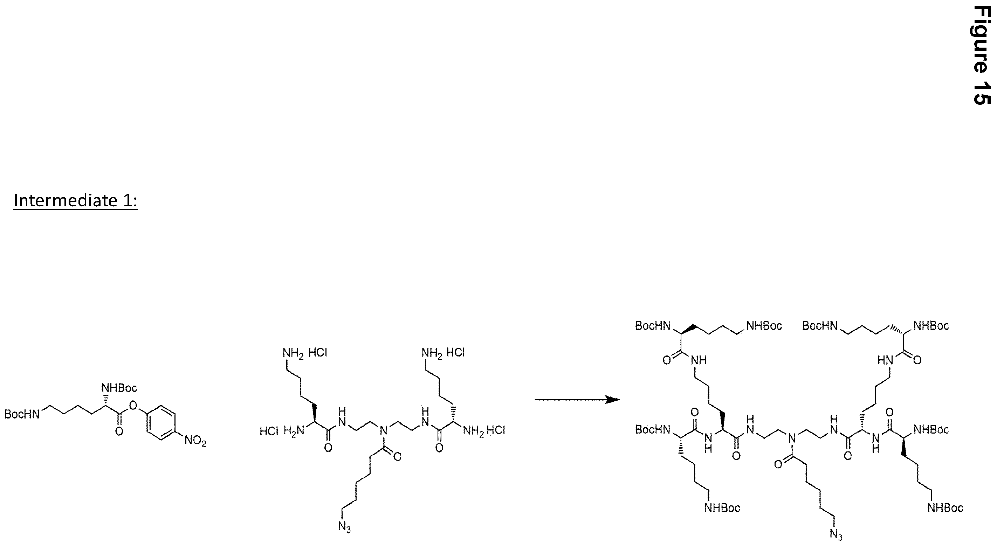

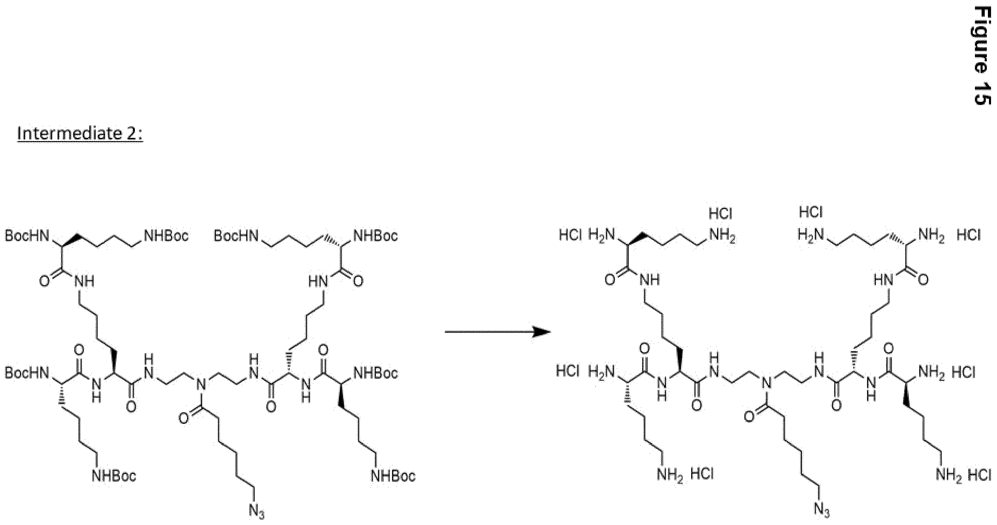

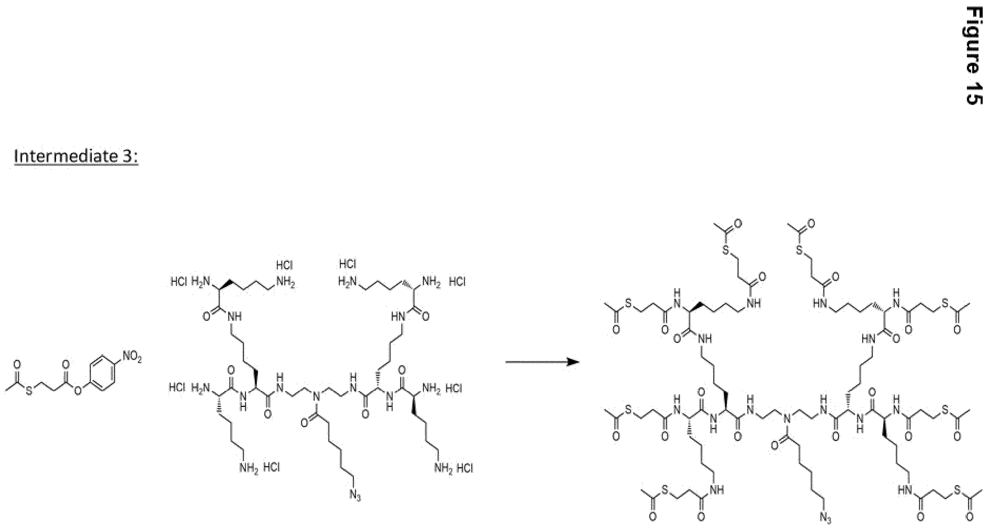

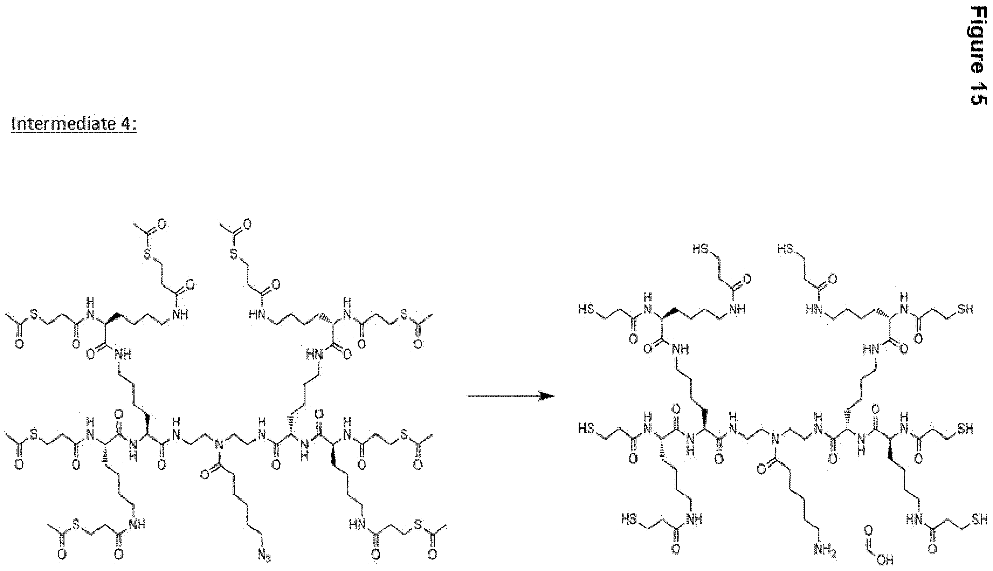

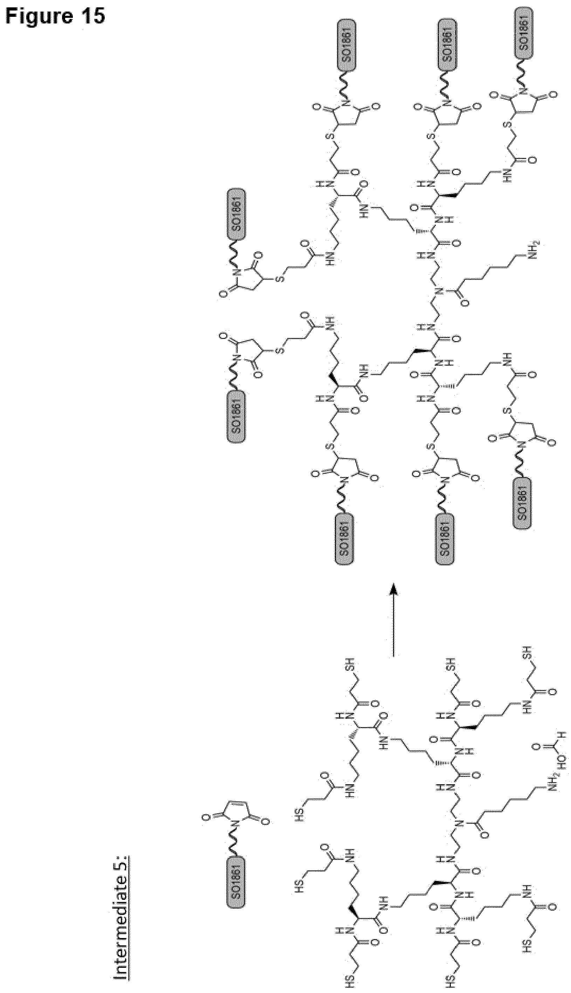

### Dendron(-L-SO1861)⁸synthesis (Figure 15)

### Intermediate 1:

### tert-butyl N-[(1S)-1-{[(1S)-1-{[2-(6-azido-N-{2-[(2S)-2,6-bis[(2S)-2,6-bis({[(tert-butoxy)carbonyl]amino})hexanamido]hexanamido]ethyl}hexanamido)ethyl]carbamoyl}-5-[(2S)-2,6-bis({[(tert-butoxy)carbonyl]amino})hexanamido]pentyl]carbamoyl}-5-{[(tert-butoxy)carbonyl]amino}pentyl]carbamate

(S)-2,6-diamino-N-(2-(6-azido-N-(2-((S)-2,6-diaminohexanamido)ethyl)hexanamido)ethyl)hexanamide tetrahydrochloride (964 mg, 1.50 mmol) was dissolved in DMF (25.0 mL) and triethylamine (2.08 mL, 15.0 mmol). Next, Boc-Lys(Boc)-ONp (3.36 g, 7.18 mmol) was added and the reaction mixture was stirred at room temperature overnight. The reaction mixture was evaporated *in vacuo* and the residue was purified by flash chromatography (DCM - methanol/DCM (1/9, v/v) gradient 100:0 rising to 0:100) to give the title product (2.71 g, 100%) as a white solid. Purity based on LC-MS 97%.
LRMS (m/z): 807 [M-198]²⁺
LC-MS r.t. (min): 2.35^{2B}

### Intermediate 2:

### (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride

Intermediate 1 (2.71 g, 1.50 mmol) was dissolved in HCI in isopropanol (5-6N, 25.0 ml, 138 mmol) and the reaction mixture was stirred at room temperature overnight. Next, the reaction mixture was evaporated *in vacuo* and the resulting crude product was co-evaporated with DCM (3 × 20 mL) to give the crude title product as a white solid.
LRMS (m/z): 203/254 [M-200/M+4]⁴⁺, 338 [M+3]³⁺, 507 [M+2]²⁺, 1012 [M+1]¹⁺

### Intermediate 3:

### (2S)-2,6-bis[3-(acetylsulfanyl)propanamido]-N-[(1S)-1-{[2-(6-azido-N-{2-[(2S)-2,6-bis[(2S)-2,6-bis[3-(acetylsulfanyl)propanamido]hexanamido]hexanamido]ethyl}hexanamido)ethyl]carbamoyl}-5-[(2S)-2,6-bis[3-(acetylsulfanyl)propanamido]hexanamido]pentyl]hexanamide

To (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride (300 mg, 0.230 mmol) was added DMF (20.0 mL), triethylamine (320 µl, 2.30 mmol) and 4-nitrophenyl 3-(acetylthio)propanoate (595 mg, 2.21 mmol). The resulting suspension was sonicated at 60 °C for 30 min and left stirring at room temperature overnight. The reaction mixture was evaporated *in vacuo* and the residue was purified by first performing flash chromatography (DCM - methanol/DCM (1/9, v/v) gradient 100:0 rising to 0:100), followed by preparative MP-LC² to give the title product (70 mg, 15%) as a white solid. Purity based on LC-MS 100%.
LRMS (m/z): 685 [M+3]³⁺
LC-MS r.t. (min): 1.91^{2A}

### Intermediate 4:

### (2S)-N-[(15)-1-{[2-(6-amino-N-{2-[(25)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate

Scaffold 4 (10.0 mg, 4.87 µmol) was dissolved methanol (200 µL). Next, a freshly prepared 1 N Sodium hydroxide solution (42.9 µL, 0.043 mmol) was added and the resulting mixture was shaken for 1 min and left standing at room temperature. After 30 min a 1.0 M trimethylphosphine solution in THF (24.4 µL, 0.024 mmol) was added and the resulting mixture was shaken for 1 min and left standing at room temperature. After 30 min the reaction mixture was diluted with water (1 mL) and directly subjected to preparative MP-LC.² Fractions corresponding to the product were immediately pooled together, frozen and lyophilized overnight to give the title compound (4.02 mg, 48%) as a white fluffy solid.
LRMS (m/z): 564 [M+3]³⁺, 846 [M+2]²⁺
LC-MS r.t. (min): 1.54^{2C}

### Intermediate 5:

### dendron(-L-SO1861)⁸-amine

Scaffold 5 (0.52 mg, 0.299 µmol) and SO1861-EMCH (29.2 mg, 0.014 mmol) were dissolved in a mixture of 20 mM NH₄HCO₃ with 0.5 mM TCEP/acetonitrile (3:1, v/v, 1.00 mL) and the resulting mixture was shaken for 1 min and left standing at room temperature. After 30 min TCEP (0.30 mg, 1.05 µmol) was added and the reaction mixture was shaken for 1 min. Next, the mixture was directly subjected to preparative LC-MS.^{3B} Fractions corresponding to the product were immediately pooled together, frozen and lyophilized overnight to give the title compound (2.17 mg, 40%) as a white fluffy solid. Purity based on LC-MS 97%.
LRMS (m/z): 2282 [M-8]⁸⁻, 2607 [M-7]⁷⁻
LC-MS r.t. (min): 4.41^{5A}

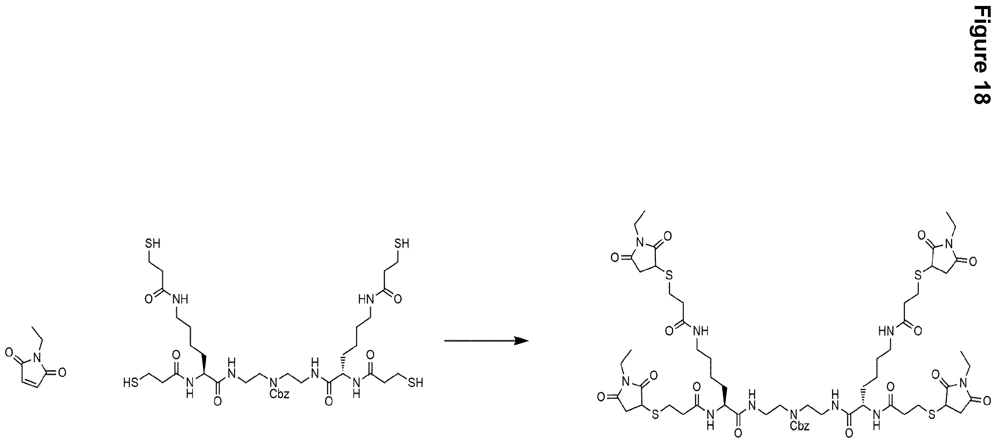

### Dendron(NEM)⁴synthesis (Figure 18)

To benzyl bis(2-((S)-2,6-bis(3-mercaptopropanamido)hexanamido)ethyl)carbamate (1.69 mg, 2.00 µmol) and N-Ethylmaleimide (1.05 mg, 8.40 µmol) was added a mixture of 20 mM NH₄HCO₃/acetonitrile (3:1, v/v, 2.00 mL) and the reaction mixture was stirred at room temperature. After 2 hours, the reaction mixture was lyophilized overnight. The resulting residue was purified by using preparative LC-MS^{3A} to give the title compound (1.53 mg, 57%) as a white fluffy solid. Purity based on LC-MS 98%.
LRMS (m/z): 1346 [M+1]¹⁺
LC-MS r.t. (min): 1.43^{3A}

### Example 6

### SO1861-trifunctional linker-BNAoligo synthesis and tumor sample gene silencing analysis (Figure 16)

### Materials and methods

### Trifunctional linker

Trifunctional linker (DBCO, TCO, maleimide) was ordered at Bio-Synthesis Inc. (Lewisville, Texas).

### HSP27BNA oligo

### HSP27BNA(-thiol) oligos (sequence 5'-GGCacagccagtgGCG-3') (Zhang et al., 2011) were ordered at Bio-synthesis Inc. (Lewisville, Texas)

### Intermediate 1:

### SO1861-azide

To SO1861 60 mg, 0.032 mmol)) and 1-azido-3,6,9,12-tetraoxapentadecane-15-hydrazide (39.3 mg, 0.129 mmol) was added methanol (extra dry, 1.00 mL) and TFA (9.86 µl, 0.129 mmol) and the reaction mixture was shaken for 1 min and left standing at room temperature. After 2 hours the reaction mixture was subjected to preparative MP-LC.¹ Fractions corresponding to the product were immediately pooled together, frozen and lyophilized overnight to give the title compound (58.4 mg, 84%) as a white fluffy solid. Purity based on LC-MS 100%.
LRMS (m/z): 2150 [M-1]¹⁻
LC-MS r.t. (min): 1.10^{3B}

### Intermediate 2:

### SO1861-trifunctional linker

SO1861-azide (45 mg, 0.021 mmol) and trifunctional linker (26.5 mg, 0.022 mmol) were dissolved in DMF (2.50 mL) and the resulting mixture was shaken for 1 min and left standing at room temperature. After 30 min the reaction mixture was subjected to preparative LC-MS.^{3C} Fractions corresponding to the product were immediately pooled together, frozen and lyophilized overnight to give the title compound (58.4 mg, 84%) as a white fluffy solid. Purity based on LC-MS 89%.
LRMS (m/z): 1677 [M-2]²⁻
LC-MS r.t. (min): 2.54^{6A}

### Intermediate 3:

### (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

To 1-(4-formylbenzamido)-N-(4-(6-methyl-1,2,4,5-tetrazin-3-yl)benzyl)-3,6,9,12-tetraoxapentadecan-15-amide (28.0 mg, 0.048 mmol) and EMCH.TFA (24.5 mg, 0.072 mmol) was added methanol (extra dry, 2.00 mL) and TFA (11.1 µL, 0.145 mmol) and the reaction mixture stirred at 50 °C. After 30 min the reaction mixture was evaporated *in vacuo* and the resulting residue was purified by MP-LC.¹ Fractions corresponding to the product were immediately pooled together, frozen and lyophilized overnight to give the title compound (33.4 mg, 88%) as a bright purple fluffy solid. Purity based on LC-MS 92%.
LRMS (m/z): 394 [M+2]²⁺, 789 [M+1]¹⁺
LC-MS r.t. (min): 1.28^{7A}

### Intermediate 4:

### methyltetrazine-BNA oligo

To HSP27 BNA oligo disulfide (70.0 mg, 0.012 mmol) was dissolved in 20 mM NH₄HCO₃ (20.0 mL). Next, TCEP (14.3 mg, 0.050 mmol) was added and the reaction mixture was shaken for 1 min and left standing at room temperature. The reaction mixture was filtered by using a centrifugal filter with a molecular weight cut-off of 3000 Da (5000 × g for 30 min). Next, a solution of 20 mM NH₄HCO₃ with 2.5 mM TCEP (20.0 mL) was added to the residue solution and the resulting mixture was filtered again under the same conditions described above. The residue solution was diluted with 20 mM NH₄HCO₃ (30.0 mL) and the resulting mixture was added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (14.8 mg, 18.8 µmol) in acetonitrile (10.0 mL). The reaction mixture was shaken for 1 min and left standing at room temperature. After 30 min the reaction mixture was frozen and lyophilized over the weekend to yield the crude title product as a pink fluffy solid. To the crude product was added a solution of 20 mM NH₄HCO₃ (20.0 mL) and the resulting suspension was filtered over a 0.45 µm syringe filter. The filtrate was filtered using a centrifugal filter with the same conditions as described above. Next, again a solution of 20 mM NH₄HCO₃ (20.0 mL) was added to the residue solution and the resulting mixture was again filtered by using a centrifugal filter with the same conditions described above. The residue solution was diluted with 20 mM NH₄HCO₃ (20.0 mL) and the resulting mixture was lyophilized overnight to yield the title product (90.0 mg, 115%) as a pink fluffy solid. Purity based on LC-MS 91%.
LRMS (m/z): 1631 [M-4]⁴⁻, 2174 [M-3]³⁻
LC-MS r.t. (min): 0.73^{7B}

### Intermediate 5:

### SO1861-trifunctional linker-BNA oligo

Methyltetrazine-BNA oligo (90.0 mg, 0.014 mmol) and SO1861-trifunctional linker (48.6 mg, 0.014 mmol) were dissolved in a mixture of water/acetonitrile (4:1, v/v, 12.0 mL). The reaction mixture was shaken for 1 min and left standing at room temperature. After 15 min the mixture was subjected to preparative LC-MS.^{4A} Fractions corresponding to the product were immediately pooled together, frozen and lyophilized overnight to give the title compound (82.0 mg, 60%) as a white fluffy solid. Purity based on LC-MS 92% (2 peaks with both m/z values corresponding to the title compound).
LRMS (m/z): 1641 [M-6]⁶⁻, 1970 [M-5]⁵⁻
LC-MS r.t. (min): 3.24 and 3.40^{6B}

### Intermediate 1:

### SO1861-azide

To SO1861 60 mg, 0.032 mmol)) and 1-azido-3,6,9,12-tetraoxapentadecane-15-hydrazide (39.3 mg, 0.129 mmol) was added methanol (extra dry, 1.00 mL) and TFA (9.86 µl, 0.129 mmol) and the reaction mixture was shaken for 1 min and left standing at room temperature. After 2 hours the reaction mixture was subjected to preparative MP-LC.¹ Fractions corresponding to the product were immediately pooled together, frozen and lyophilized overnight to give the title compound (58.4 mg, 84%) as a white fluffy solid. Purity based on LC-MS 100%.
LRMS (m/z): 2150 [M-1]¹⁻
LC-MS r.t. (min): 1.10^{3B}

### Intermediate 2:

### SO1861-trifunctional linker

SO1861-azide (45 mg, 0.021 mmol) and trifunctional linker (26.5 mg, 0.022 mmol) were dissolved in DMF (2.50 mL) and the resulting mixture was shaken for 1 min and left standing at room temperature. After 30 min the reaction mixture was subjected to preparative LC-MS.^{3C} Fractions corresponding to the product were immediately pooled together, frozen and lyophilized overnight to give the title compound (58.4 mg, 84%) as a white fluffy solid. Purity based on LC-MS 89%.
LRMS (m/z): 1677 [M-2]²⁻
LC-MS r.t. (min): 2.54^{6A}

### Intermediate 3:

### (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

To 1-(4-formylbenzamido)-N-(4-(6-methyl-1,2,4,5-tetrazin-3-yl)benzyl)-3,6,9,12-tetraoxapentadecan-15-amide (28.0 mg, 0.048 mmol) and EMCH.TFA (24.5 mg, 0.072 mmol) was added methanol (extra dry, 2.00 mL) and TFA (11.1 µL, 0.145 mmol) and the reaction mixture stirred at 50 °C. After 30 min the reaction mixture was evaporated *in vacuo* and the resulting residue was purified by MP-LC.¹ Fractions corresponding to the product were immediately pooled together, frozen and lyophilized overnight to give the title compound (33.4 mg, 88%) as a bright purple fluffy solid. Purity based on LC-MS 92%.
LRMS (m/z): 394 [M+2]²⁺, 789 [M+1]¹⁺
LC-MS r.t. (min): 1.28^{7A}

### Intermediate 4:

### methyltetrazine-BNA oligo

To HSP27 BNA oligo disulfide (70.0 mg, 0.012 mmol) was dissolved in 20 mM NH₄HCO₃ (20.0 mL). Next, TCEP (14.3 mg, 0.050 mmol) was added and the reaction mixture was shaken for 1 min and left standing at room temperature. The reaction mixture was filtered by using a centrifugal filter with a molecular weight cut-off of 3000 Da (5000 × g for 30 min). Next, a solution of 20 mM NH₄HCO₃ with 2.5 mM TCEP (20.0 mL) was added to the residue solution and the resulting mixture was filtered again under the same conditions described above. The residue solution was diluted with 20 mM NH₄HCO₃ (30.0 mL) and the resulting mixture was added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (14.8 mg, 18.8 µmol) in acetonitrile (10.0 mL). The reaction mixture was shaken for 1 min and left standing at room temperature. After 30 min the reaction mixture was frozen and lyophilized over the weekend to yield the crude title product as a pink fluffy solid. To the crude product was added a solution of 20 mM NH₄HCO₃ (20.0 mL) and the resulting suspension was filtered over a 0.45 µm syringe filter. The filtrate was filtered using a centrifugal filter with the same conditions as described above. Next, again a solution of 20 mM NH₄HCO₃ (20.0 mL) was added to the residue solution and the resulting mixture was again filtered by using a centrifugal filter with the same conditions described above. The residue solution was diluted with 20 mM NH₄HCO₃ (20.0 mL) and the resulting mixture was lyophilized overnight to yield the title product (90.0 mg, 115%) as a pink fluffy solid. Purity based on LC-MS 91%.
LRMS (m/z): 1631 [M-4]⁴⁻, 2174 [M-3]³⁻
LC-MS r.t. (min): 0.73^{7B}

### Intermediate 5:

### SO1861-trifunctional linker-BNA oligo

Methyltetrazine-BNA oligo (90.0 mg, 0.014 mmol) and SO1861-trifunctional linker (48.6 mg, 0.014 mmol) were dissolved in a mixture of water/acetonitrile (4:1, v/v, 12.0 mL). The reaction mixture was shaken for 1 min and left standing at room temperature. After 15 min the mixture was subjected to preparative LC-MS.^{4A} Fractions corresponding to the product were immediately pooled together, frozen and lyophilized overnight to give the title compound (82.0 mg, 60%) as a white fluffy solid. Purity based on LC-MS 92% (2 peaks with both m/z values corresponding to the title compound).
LRMS (m/z): 1641 [M-6]⁶⁻, 1970 [M-5]⁵⁻
LC-MS r.t. (min): 3.24 and 3.40^{6B}

### RNA isolation and gene expression analyses of tumor samples

Total RNA was isolated from tumors using TriZol (Thermo Fisher) according to the manufacturer's instructions. Isolated RNA was resuspended in nuclease-free water (NFW). RNA samples were checked for their RNA integrity on the gel. To prepare cDNA, first 100 ng total RNA was mixed with Random Hexamers (Qiagen; final concentration 2 µM) in a final volume of 12.5 µl in NFW, denatured for 5min at 70°C and immediately cooled on ice. Next, 7.5 µl of a cDNA synthesis mix was added, consisting of 4 µl 5xRT Buffer (Promega), 0.4 µl 25mM dNTPs (Promega), 1 µl 200 U/µL MMLV RT-enzyme (Promega), 0.5 µL 40 U/µL RNAse Inhibitor (Promega) and 1.6 µL NFW. The following cDNA synthesis protocol was used: 1) 10 minutes 25°C 2) 60 minutes 37°C 3) 5 minutes 85°C 4) ∞ 4°C

For a single qPCR reaction the following mix was prepared: 1 µL cDNA, 0.05 µL forward primer (250 µM), 0.05 µL reverse primer (250 µM), 8.9 µl LNFW, 10 µl SYBR Green (Bio-Rad). The following qPCR protocol was used: 1 cycle: 5 minutes 95°C, 40 cycles: 15s 95°C + 30s 60°C.

HSP27 gene expression was calculated using 2^{-(Ct}_{HSP27}^{-GEOMEAN(Ct}_{ref1}^{;Ct}_{ref2} ^{;Ct}_{ref3}^{;Ct}_{ref4})), where ref1, ref2, ref3 and ref4 are the reference genes IMMT, EIF2S2, GUSB and UBC for the analysis of the tumors. Two reference genes were chosen based on the performance of a GeNORM analysis among nine reference genes tested to choose the most ideal and stable reference gene for this tumor samples. To do so, qPCR results were imported in Qbase+ software program by which two quality measures are calculated: the coefficient of variation of the normalized reference gene expression levels (V); and the geNorm stability M-value (M)1. A reference gene with an M<0.2 and a V<0.15 is considered very stable. Based on this analysis IMMT and EIF2S2 were chosen as the most stable reference genes. However, UBC and GUSB were also added to the group of reference genes to further enhance the accuracy of the normalization. Each sample was analyzed as technical triplicate on a CFX96 Real-Time qPCR machine (Bio-Rad).

Primers used in qPCR are shown below in Table B1.

**Table B1. Primers**

| Gene | Primer | Sequence (5'-3') |
|---|---|---|
| UBC | Forward | CAGCCGGGATTTGGGTCG |
| | Reverse | CACGAAGATCTGCATTGTCAAGT |
| GUSB | Forward | TGCGTAGGGACAAGAACCAC |
| | Reverse | GGGAGGGGTCCAAGGATTTG |
| IMMT | Forward | AACCCACACCTGCACTTTCA |
| | Reverse | TTTTCCTGTTGTGCAAGGCG |
| EIF2S2 | Forward | CCACAAGTCGTCCGAGTAGG |
| | Reverse | GGAGATGTTTGGGCTGACGA |
| HSP27 | Forward | |
| | Reverse | |

### Example 7

### Antibody-(SO1861-L-trifunctional linker-L-HSP27) (Cys)

### Antibody-dendron

### Antibody-saporin

### T-DM1

### Materials and Methods

Trastuzumab (Tras, Herceptin^{®}, Roche), Cetuximab (Cet, Erbitux^{®}, Merck KGaA), Tris(2-carboxyethyl)phosphine hydrochloride (TCEP, 98 %, Sigma-Aldrich), 5,5'-Dithiobis(2-nitrobenzoic acid) (DTNB, Ellman's reagent, 99%, Sigma-Aldrich), Zeba^{™} Spin Desalting Columns (2 mL, Thermo-Fisher), NuPAGE^{™} 4-12% Bis-Tris Protein Gels (Thermo-Fisher), NuPAGE^{™} MES SDS Running Buffer (Thermo-Fisher), Novex^{™} Sharp Pre-stained Protein Standard (Thermo-Fisher), PageBlue^{™} Protein Staining Solution (Thermo-Fischer), Pierce^{™} BCA Protein Assay Kit (Thermo-Fisher), N-Ethylmaleimide (NEM, 98 %, Sigma-Aldrich), 1,4-Dithiothreitol (DTT, 98 %, Sigma-Aldrich), Sephadex G25 (GE Healthcare), isopropyl alcohol (IPA, 99.6 %, VWR), Tris(hydroxymethyl)aminomethane (Tris, 99%, Sigma-Aldrich), Tris(hydroxymethyl)aminomethane hydrochloride (Tris.HCL, Sigma-Aldrich), L-Histidine (99%, Sigma-Aldrich), D-(+)-Trehalose dehydrate (99%, Sigma-Aldrich), Polyethylene glycol sorbitan monolaurate (TWEEN 20, Sigma-Aldrich), DPBS - Dulbecco's Phosphate-Buffered Saline (Thermo-Fisher), Guanidine hydrochloride (99%, Sigma-Aldrich), Ethylenediaminetetraacetic acid disodium salt dihydrate (EDTA-Na₂, 99 %, Sigma-Aldrich), sterile filters 0.2 µm (Sartorius), succinimidyl 4-(N-maleimidomethyl)cyclohexane-1-carboxylate (SMCC, Thermo-Fisher), Dianthin-Cys (Dia-Cys, Dianthin mutant with a single C-terminal cysteine function, Proteogenix), Vivaspin T4 and T15 concentrator (Sartorius), Superdex 200PG (GE Healthcare), Tetra(ethylene glycol) succinimidyl 3-(2-pyridyldithio)propionate (PEG₄-SPDP, Thermo-Fisher), HSP27 Oligonucleotide (Biosynthesis).

### Methods

### UV-Vis

Absorbance measurements were performed on a Perkin Elmer Lambda 25 UV-Vis or on a Thermo NanoDrop ND-2000 spectrophotometer in the spectral range of 200-750 nm.

Concentrations were determined using a Thermo Nanodrop 2000 or Lambda 25 spectrometer using the following parameters: $Trastuzumab {OD}_{280} = 1.5 {\left(mg/ml\right)}^{- 1} {cm}^{- 1}$ $Cetuximab {OD}_{280} = 1.4 {\left(mg/ml\right)}^{- 1} {cm}^{- 1}$ $HSP 27 Oligonucleotide {OD}_{260} = 153 , 000 {M}^{- 1} {cm}^{- 1} ; {Rz}_{260 : 280} = 1.819$ $Dia - Cys {OD}_{280} = 0.57 {\left(mg/ml\right)}^{- 1} {cm}^{- 1}$ ${PEG}_{4} - SPDP \left(PDT\right) {OD}_{343} = 8 , 080 {M}^{- 1} {cm}^{- 1}$ $SAMSA - Fluorescein {OD}_{495} = 64 , 500 {M}^{- 1} {cm}^{- 1} ; {Rz}_{280 : 495} = 0.232$ $Ellmans \left(TNB\right) {OD}_{412} = 14 , 150 {M}^{- 1} {cm}^{- 1}$

### Size Exclusion Chromatography

Size exclusion chromatography (SEC) was carried out on an AKTA purifier. Samples were analyzed by SEC using either a Biosep SEC-S3000 column or an Sephadex G50M column (10 x 40 cm) eluting with TBS/ isopropyl alcohol solution (85:15 v/v). Sample purities were determined by integration of the antibody sample peak with respect to the trace aggregate peaks.

### Ellman's assay

### Antibody-dendron(-L-SO1861)⁴ (Cys and Lys)

### Trastuzumab-(L-d(SO1861)₄)₄ and Cetuximab-(L-d(SO1861)₄)₄ synthesis with a DAR4 (FBR706 STB22/9-10)

Trastuzumab and Cetuximab are referred hereafter as "Ab". Ab was conjugated to dendritic SO1861-EMCH [d(SO1861)₄-EMCH] via a labile (L) Tetra(ethylene glycol) succinimidyl 3-(2-pyridyldithio)propionate (PEG₄-SPDP) linker. The procedure is exemplary described for Cetuximab-L-(d(SO1861)₄)₄:
Cetuximab was desalted into DPBS pH 7.5 buffer and then normalized to 2.50 mg/ml. To an aliquot of Ab (9.19 mg, 61 nmol) was added an aliquot of freshly prepared PEG₄-SPDP solution (5.0 mg/ml, 6.70 mole equivalents, 411 nmol), the mixture vortexed briefly then incubated for 60 minutes at 20 °C with roller-mixing. After incubation, the reaction was quenched with the addition of glycine (20 mg/ml, 7.7 µl), then the SPDP moiety reduced *in situ* by the addition of TCEP (5.0 mg/ml, 4.0 mole equivalents per SPDP, 1.64 µmol). This mixture was roller-mixed for 15 minutes at 20 °C with roller-mixing. The resulting Ab-SH was purified by gel filtration using a zeba spin desalting column into TBS pH 7.5. The Ab-SH was characterized by UV-vis analysis and Ellman's assay (SH to Ab ratio = 5.4). To the bulk Ab-SH (7.41 mg, 1.93 mg/ml, 49 nmol) was added an aliquot of freshly prepared d(SO1861)₄-EMCH solution in DMSO (10 mg/ml, 8.0 mole equivalents per Ab, 0.4 µmol, 3.16 mg, 0.32 ml), the mixture vortexed briefly then incubated overnight at 20 °C. Besides the conjugation reaction, two aliquots of the desalted Ab-SH (0.25 mg, 1.67 nmol) were removed prior to conjugation, and were reacted with NEM (8.0 mole equivalents per Ab, 13.3 nmol, 6.7 µl of a 0.25 mg/ml solution) or TBS pH 7.5 buffer (6.7 µl) overnight at 20 °C, as positive and negative controls, respectively. After incubation for 18 hours (prior to addition of NEM), the crude conjugate mixture was centrifuged briefly and 100 µl aliquot removed for analysis by UV-vis and alongside positive and negative controls were characterized by Ellman's assay to obtain d(SO1861)₄ incorporation. To the bulk Ab - d(SO1861)₄ mixture was added an aliquot of freshly prepared NEM solution (2.5 mg/ml, 5.0 mole equivalents, 0.25 µmol) and the mixture purified by 1.6 × 30 cm Sephadex G50 column eluting with DPBS pH 7.5 to give purified Cetuximab-(L-d(SO1861)₄)₄ conjugate. The product was filtered to 0.2 µm to clarify and then concentrated carefully to ca. 3 mg/ml using a vivaspin T15 concentrator (3,000 g, 5 minute intervals, 5 °C) to give the final Cetuximab-(L-d(SO1861)₄)₄ conjugate. Yield: 4.41 mg, 48% (1.64 mg/ml). d(SO1861)4 to Ab ratio = 4.4 (see table B2).

**Table B2.**

| Batch | Ab feed (mg) | PEG₄-SPDP mol equivalents | d(SO1861)₄-EMCH mol equivalents | Obtained DAR | Purity by analytical SEC | Yield |
|---|---|---|---|---|---|---|
| Trastuzumab-(L-d(SO1861)₄)₄ | 9.0 | 6.81 | 8 | 4.7 | 99.2 % | 2.34 mg (26 %) |
| Cetuximab-(L-d(SO1861)₄)₄ | 9.2 | 6.7 | 8 | 4.4 | 96.7 % | 4.41 mg (48 %) |

### Antibody-L-HSP27BNA (Cys)

### Trastuzumab-(L-HSP27)₄, Cetuximab-(L-HSP27)₄, PEG₄-SPDP with a DAR4 and Cetuximab-(L-HSP27)₂ synthesis via PEG₄-SPDP with a DAR2

Trastuzumab, Cetuximab, Trastuzumab-L-SO1861, Cetuximab-L-SO1861 are referred hereafter as "Ab". Ab was conjugated to HSP27 via a labile (L) Tetra(ethylene glycol) succinimidyl 3-(2-pyridyldithio)propionate (PEG₄-SPDP) linker.

HSP27 (2.7mg, 470 nmol, 6.10 mg/ml) was reacted with TCEP (10 mole equivalents, 4.7 µmol, 1.34 mg, 50 mg/ml) for 30 minutes at 20 °C with roller mixing. After, the oligo-SH was purified by PD10 G25 desalting column eluting into TBS pH 7.5 and used promptly. Oligo-SH was obtained (2.48 mg, 90%, 1.24 mg/ml, SH to oligo ratio = 0.8)

Trastuzumab-(L-SO1861)₄ (1.3 mg, 8.7 nmol, 2.50 mg/ml) was reacted with an aliquot of freshly prepared PEG₄-SPDP solution (9.26 mole equivalents, 80.3 nmol, 45 µg) in DMSO (1 mg/ml) for 60 minutes at 20 °C with roller mixing. After, the reaction was quenched with glycine (15.1 µl of 2 mg/ml freshly prepared solution in TBS pH 7.5) and then desalted via zeba desalting column eluting with TBS pH 7.5. An aliquot of the resulting Tras-(L-SO1861)-(L-PEG₄-SPDP) was taken out and tested by UV-Vis analysis. SPDP incorporation was determined using TCEP to liberate pyridiyl-2-thione (PDT) and by UV-vis analysis at 343 nm (SPDP to Ab ratio: 4). The remaining Tras-(L-SO1861)-(L-PEG₄-SPDP) was reacted with an aliquot of freshly prepared HSP27 oligonucleotide (oligo-SH) (8 mole equivalents, 54.8 nmol, 0.32 mg, 1.24 mg/ml) and incubated overnight at 20 °C with roller mixing. After 17 hours, the conjugate was analysed by UV-vis analysis to ascertain incorporation of HSP27 by displacement of pyridiyl-2-thione (PDT) at 343 nm. The crude conjugate was purified using a 1.6 × 33 cm Sephadex G50 column eluting with DPBS pH 7.5. The resulting Trastuzumab-(L-SO1861)4-(L-HSP27)4 was obtained as a single fraction. Yield: 0.47 mg, 45% (0.49 mg/ml), HSP27 to Ab ratio = 3.5 (see Table B3).

### All Yields: STB17/1-8 needed

**Table B3. Summarized reaction conditions and results**

| **Batch** | **PEG₄**-**SPDP mol equivalents** | **HSP27 (oligo-SH) mol equivalents** | **Obtained DAR** | **Purity by analytical SEC (%)** | **Yield (%)** |
|---|---|---|---|---|---|
| **Tras-(L-SO1861)₄**-**(L-HSP27)₄** | 9.26 | 8 | 3.5 | 85.1 | 45 |
| **Tras-(L-HSP27)₄** | 6.81 | 8 | 4.4 | 96.0 | n.d. |
| **Cet-(L-SO1861)₄-( L-HSP27)₄** | 7.21 | 8 | 3.8 | 80.8 | n.d. |
| **Cet- (L-HSP27)₄** | 6.70 | 8 | 3.9 | 93.9 | n.d. |
| **Cet-(L-SO1861)₄-( L-HSP27)₂** | 3.34 | 3.6 | 1.8 | 76.2 | 81 |
| **Cet- (L-HSP27)₂** | 2.3 | 3.6 | 1.5 | 94.9 | 87 |

### Example 8

### Materials and methods

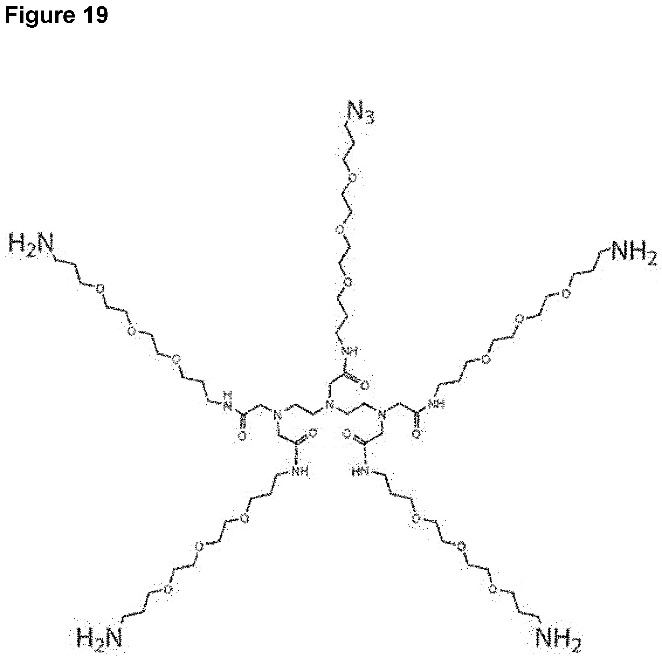

In our current work, we investigated a model scaffold consisting of four molecular arms for saponin binding via a Schiff base (imine) and one arm for click chemistry. The polymeric structure (Figure 19) is a pentavalent polyethylene glycol-based dendrimer of the first generation (i.e. number of repeated branching cycles) that was purchased from Iris Biotech GmbH (Marktredwitz, Germany). The saponin (in this example SA1641) was purified from a saponin composite raw extract from Gypsophila species called Saponinum album obtained from Merck (Darmstadt, Germany). The powdered raw extract (2.5 g) was hydrolyzed in water (100 mL) with sodium hydroxide (0.2 g). The solution was stirred for 20 h at 40 °C and then supplemented with glacial acetic acid until pH 5.0 was reached. To remove tannins, the solution was shaken in a separatory funnel with 30 mL butanol. The aqueous phase was recaptured and butanol extraction repeated two times. The butanol phases were supplemented with anhydrous sodium sulfate, filtered and pooled. Butanol was evaporated and the remaining saponin powder resolved in 20% methanol to a final concentration of 30 mg/mL. After short sonication, different saponins were separated by high performance liquid chromatography (HPLC). Tubes (excluding column) were rinsed with warm water (40 °C) at a flow of 1.5 mL/min and then including Eurospher RP-C18-column (5 µm, 250 × 8 mm) with isopropanol (100%). Saponins were applied to the column and eluted with a methanol gradient (20% methanol to 70% methanol within 30 min at 1.5 mL/min in water supplemented with 0.01% trifluoroacetic acid followed by 70% methanol for further 60 min) (Sama et al, 2018). Aliquots of the fractions were analyzed for their SA1641 content by electrospray ionization mass spectrometry (ESI-MS). Fractions containing pure SA1641 were pooled and methanol evaporated. The aqueous solution was frozen as a thin film in a rotating round-bottom flask by use of dry ice. After storage for 16 h at - 80 °C, the sample was lyophilized. To produce the scaffold as defined in the invention, the polymeric structure (0.2 mM) and SA1641 (3.2 mM) were solved in water (approx. pH 8) and equal volumes mixed and shaken for 24 h at 26 °C. Then sodium cyanoborohydride (NaCNBH₃; 0,1 M) was added in 4-fold molar excess referred to SA1641 and the sample incubated for further 24 h. The structure was then verified by ultra performance liquid chromatography (UPLC)/ESI-MS. The samples were applied to a RP-C4-column and eluted with a methanol gradient (25% methanol to 80% methanol within 15 min in water supplemented with 0.01% trifluoroacetic acid followed by 80% methanol for further 10 min). The fractions were analyzed by use of LockSpray^{™} that is an ion source designed specifically for exact mass measurement with electrospray ionization using LC-time-of-flight (LC-TOF) mass spectrometers from Waters Corporation.

### Results

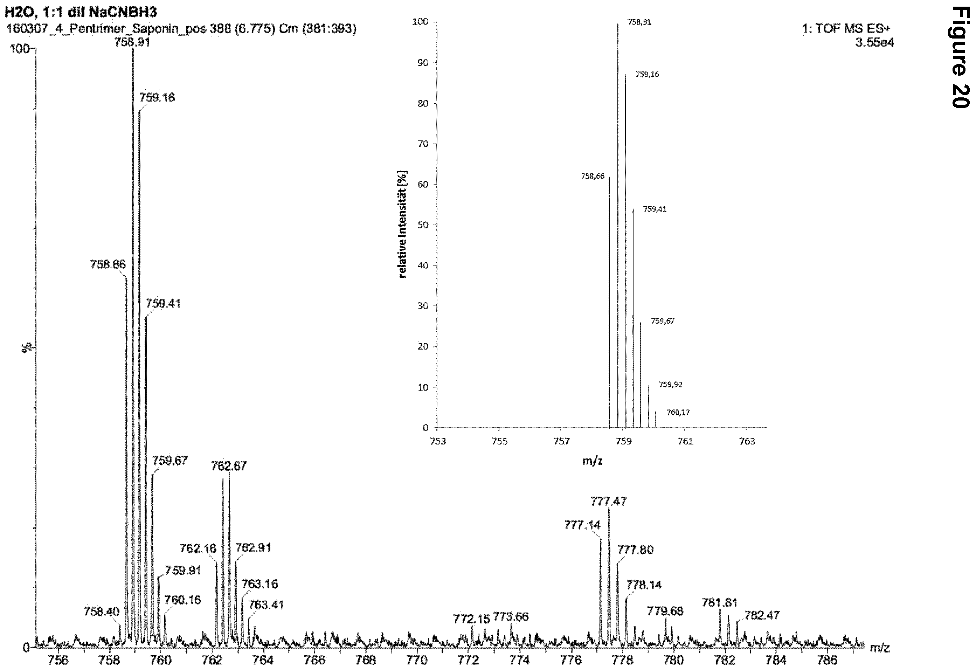

The inset of Figure 20 shows the theoretically expected mass spectrum obtained from a calculation with the isotope pattern calculator enviPat Web 2.0. The pattern considers the charge of the molecule and the natural occurrence of isotopes, which is the reason that more than one peek is expected for a single substance. The experimental data (Figure 20) obtained by UPLC/ESI-MS show almost exactly the same peaks at *m*/*z* 758-760 with same intensity as predicted, thus proving successful SA1641 coupling to the polymeric structure.

### Example 9

### Materials and methods

As an example for a pharmaceutical active substance, we used the targeted toxin dianthin-Epidermal Growth Factor (dianthin-EGF). The plasmid His-dianthin-EGF-pET11d (Weng et al, 2009) (100 ng) was added to 20 µL Escherichia coli Rosetta^{™} 2 (DE3) pLysS Competent Cells (Novagen, San Diego, CA, USA). Cells were transformed by a heat-shock (30 min on ice, 90 s at 42 °C and 1 min on ice). Thereafter, 300 µL lysogeny broth (LB) was added and the suspension incubated for 1 h at 37 °C while shaking at 200 rpm. A preheated lysogeny broth agar plate with 50 µg/mL ampicillin was inoculated with 100 µl bacteria suspension and the plate incubated overnight at 37 °C. Lysogeny broth (3 mL) with 50 µg/mL ampicillin was inoculated with a colony from the plate and the bacteria were incubated for 8 h at 37 °C and 200 rpm. The suspension (50 µL) was added to 500 mL of lysogeny broth with 50 µg/mL ampicillin and incubated overnight at 37 °C and 200 rpm. Subsequently, the volume was scaled-up to 2.0 L and bacteria grew under the same conditions until an optical density at wavelength 600 nm of 0.9 was reached. Thereafter, protein expression was induced by the addition of isopropyl β-D-1-thiogalactopyranoside (IPTG) at a final concentration of 1 mM. Protein expression lasted for 3 h at 37 °C and 200 rpm. Finally, the bacterial suspension was centrifuged at 5,000 × *g* and 4 °C for 5 min, resuspended in 20 mL PBS (137 mM NaCl, 2.7 mM KCI, 8.1 mM Na₂HPO₄, 1.47 mM KH₂PO₄) and stored at -20 °C until use. For purification, bacterial suspensions were thawed and lysed by sonication. Lysates were centrifuged (15,800 × g, 4 °C, 30 min) and imidazole added to a final concentration of 20 mM. The supernatant was incubated with 2 mL of Ni-nitrilotriacetic acid agarose under continuous shaking for 30 min at 4 °C in the presence of 20 mM imidazole. Subsequently, the material was poured into a 20-mL-column and washed three times with 10 mL wash buffer (50 mM NaH₂PO₄, 300 mM NaCl, 20 mM imidazole) and dianthin-EGF eluted by 10-mL-portions of increasing concentrations of imidazole (31, 65, 125 and 250 mM) in wash buffer. Eluate fractions (2 mL) were dialyzed overnight at 4 °C against 2.0 L PBS. Desalted dianthin-EGF was concentrated by an Amicon^{®} Ultra-15 (10 kDa) and the protein concentration quantified.

To introduce a suitable click chemistry group into dianthin-EGF, alkyne-PEG₅-N-hydroxysuccinimidyl ester in 8-fold molar excess referred to dianthin-EGF was solved in dimethyl sulfoxide and added to 9 volumes of dianthin-EGF (1 mg in 0.2 M NaH₂PO₄/Na₂HPO₄, pH 8). After incubation at room temperature for 4 h, non-bound alkyne was separated by use of a PD10 column (GE-Healthcare, Freiburg, Germany). Click chemistry with the polymeric structure was conducted by copper(I)-catalyzed alkyne-azide cycloaddition. Alkyne-dianthin-EGF (0.02 mM), dendrimer (0.05 mM), CuSO₄ (0.1 mM), tris(3-hydroxypropyltriazolylmethyl)amine (0.5 mM) and sodium ascorbate (5 mM) were incubated under gentle agitation for 1 h at room temperature in 0.1 M NaH₂PO₄/Na₂HPO₄, pH 8. Low molecular mass substances were then separated using a PD10 column.

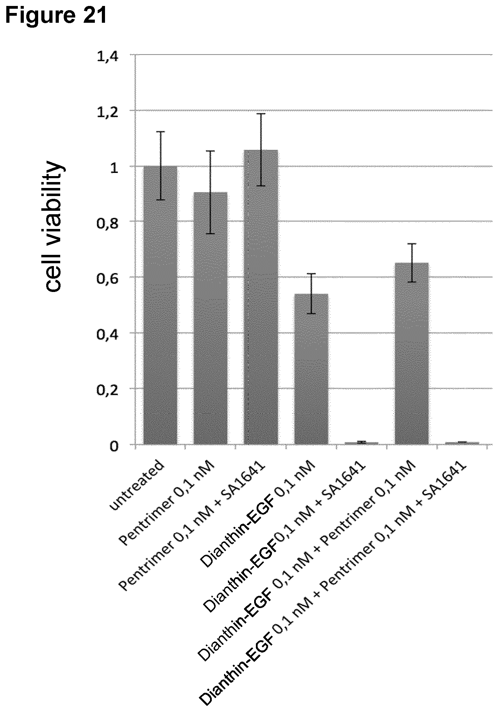

To test the efficacy of the invention, we conducted a viability assay with HER14 cells. These cells are fibroblasts stably transfected with the human epidermal growth factor receptor and therefore target cells for the targeted toxin dianthin-EGF. HER14 cells (2,000 cells/100 µL/well) were seeded into wells of 96-well-cell culture plates and incubated for 24 h in DMEM medium supplemented with 10% fetal calf serum and 1% penicillin/streptomycin at 37 °C, 5% CO₂ and 98% humidity. The different test substances (see results and Figure 21) were then added in triplicates in a volume of 25 µL and supplemented with further 25 µL of medium. After an incubation of 72 h, 30 µL 3-(4,5-dimethylthiazol-2-yl)-2,5-diphenyltetrazolium bromide (0.5 mg/mL in water) was added per well and incubated for 2 h. Thereafter, the medium was carefully removed and replaced by an aqueous solution containing 10% (v/v) isopropanol, 5% (w/v) sodium dodecyl sulfate and 400 mM HCI, and incubated for 5 min. Solubilized formazan was photometrically quantitated at 570 nM in a microplate reader (Spectra MAX 340 PC, Molecular Devices, Sunnyvale, CA, USA). Untreated cells were normalized to 1 and all samples referred to the untreated control. Significance was determined by unpaired two-sample t-tests.

### Results

The polymeric structure, in the example a pentameric dendrimer (pentrimer), does not have any cytotoxic effect on the target cells, neither in absence nor in presence of SA1641 (Figure 21, column 2 and 3). In the absence of the scaffold, the targeted toxin (dianthin-EGF) shows half maximal toxicity at a concentration of 0.1 nM (column 4). In the presence of SA1641 the same concentration results in death of all cells indicating the general ability of SA1641 to act as an enhancer of the endosomal escape (column 5). The presence of the polymeric structure does not affect the toxicity of dianthin-EGF neither in the presence nor in the absence of SA1641 (columns 6 and 7), indicating that the scaffold does not affect the toxicity of dianthin-EGF. To couple the model polymeric structure via click chemistry to the example pharmaceutically active substance of dianthin-EGF, the substance had to be coupled with an alkyne group before.. A manufacturer of a pharmaceutically active substance can introduce the click position during synthesis directly into the substance at a position of his choice where the activity of the substance remains unaffected. There was no additional loss of activity when clicking the alkyne-modified pharmaceutically active substance to the polymeric structure indicating that the polymeric structure itself was not toxic.

### Example 10

### Materials

The following chemicals were used as purchased: methanol (MeOH, LiChrosolv, Merck), N-ε-maleimidocaproic acid hydrazide (EMCH, 95%, TCI Chemicals), trifluoroacetic acid (TFA, 99.8%, Carl Roth), 2-mercaptoethanol (98%, Sigma-Aldrich), poly(amidoamine) (PAMAM dendrimer, ethylenediamine core, generation 5.0 solution, Sigma-Aldrich), cyanine 3 carboxylic acid (Cy3-COOH, 95%, Lumiprobe), 1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide (HATU, 97%, Sigma-Aldrich), bovine serum albumin fraction V (BSA, Carl Roth), dimethylsulfoxide (DMSO, 99%, Carl Roth), 2-lminothiolane hydrochloride (98%, Sigma-Aldrich), rhodamine b (RhodB, 95%, Merck), Dulbecco's phosphate buffered saline (PBS, Gibco), hydrochloric acid (HCI, 37%, Merck), NHS-PEG₁₃-DBCO (Click Chemistry Tools), Alexa Fluor^{™} 488 5-TFP (Thermo-Fischer), azido-PEG3-SS-NHS (Conju-Probe), sodium cyanoborohydride (NaCNBH₃, 95 %, Sigma-Aldrich), ammonium persulfate (APS, 98%, Sigma-Aldrich), N,N,N',N'-tetramethylethylenediamine (TMEDA, 99 %, Sigma-Aldrich), customized peptide SESDDAMFCDAMDESDSK (95%, PeptideSynthetics), azido-dPEG₁₂-NHS (95%, Quanta Biodesign), PFd-G4-Azide-NH-BOC Dendron (G4-dendron, 95%, Polymer Factory), Cyanin5-DBCO (Cy5-DBCO, 95%, Lumiprobe), Chloroform (CHCl₃, 99.5 %, Sigma), Amicon Ultra 0.5 mL centrifugal filters (3 kDa MWCO, Sigma), mPEG-SCM (mPEG₂ₖ-NHS, 95.6%, Creative PEG Works), Amicon Ultra 15 mL centrifugal filters (10 kDa MWCO, Sigma).

### Methods

### MALDI-TOF-MS

MALDI-TOF spectra were recorded on a MALDI-Mass Spectrometer (Bruker Ultrafex III). Typically, the sample dissolved in MilliQ water in nanomolar to micromolar range was spotted on the target (MTP 384 target plate polished steel T F, Bruker Daltons) using either super-DHB (99%, Fluka) or sinapinic acid (SA, 99%, Sigma-Aldrich) as the matrix dissolved in acetonitrile (MADLI-TOF-MS tested, Sigma) / 0.1% TFA (7:3 v/v) via the dried-droplet-method. PepMix (Peptide Calibration Standard, Bruker Daltons) or ProteMass (Protein Calibration Standard, Sigma-Aldrich) served as calibration standards. RP mode refers to reflector positive mode. RN mode refers to reflector negative mode. LP mode refers to linear positive mode.

### H-NMR

¹H NMR analysis was performed using a Bruker 400 MHz NMR spectrometer. The sample preparation, in which 2 mg of sample had been dissolved in 0.8 mL of methanol-*D₄* (99%, Deutero), was performed 24 h prior to the measurement.

### UV-Vis

UV-Vis measurements were performed on a NanoDrop ND-1000 spectrophotometer in the spectral range of 200-750 nm.

### Size Exclusion Chromatography

Size exclusion chromatography (SEC) was performed with Sephadex G 25 Superfine from GE Healthcare and on prepacked PD10 columns (GE Healthcare, Sephadex G 25 M). The material was activated by swelling in the respective eluent prior to performing chromatography.

### Dialysis

Regenerated cellulose membranes: MWCO = 1 and 2 kDa (Spectra/Por), and MWCO = 12-14 kDa (Carl Roth) were used to perform dialysis. Typically, dialysis was carried out for 24 h with 1 L of solvent that was exchanged after first 6 h of the process.

### Lyophilization

Freeze-drying was performed on an Alpha 1-2 LD plus (Martin Christ Gefriertrocknungsanlagen GmbH). Typically, samples were frozen with liquid nitrogen and placed into the freeze-dryer at high vacuum.

### SO1861-EMCH synthesis

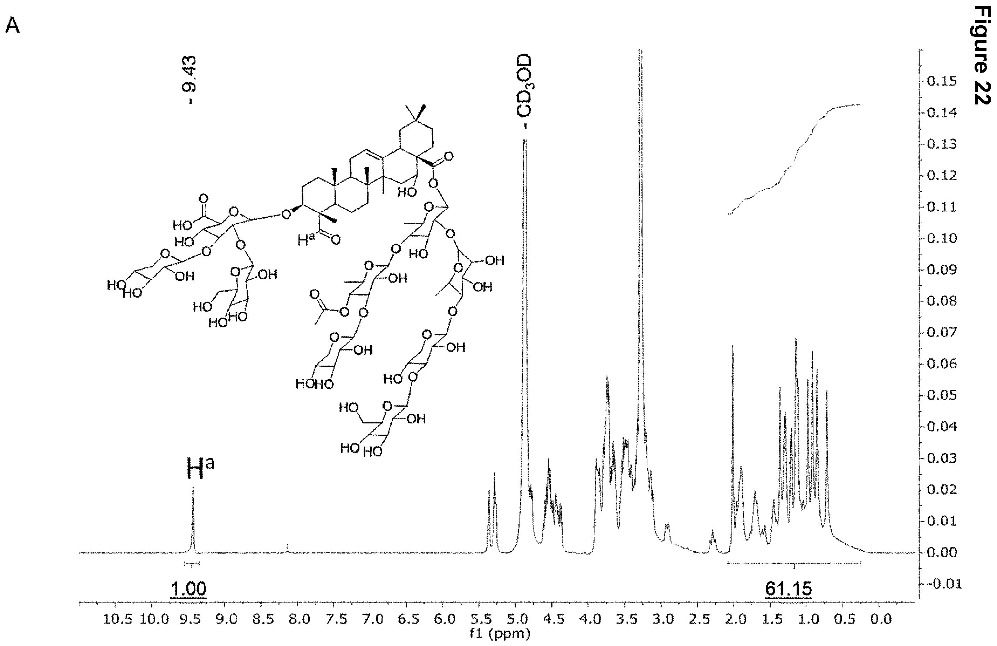

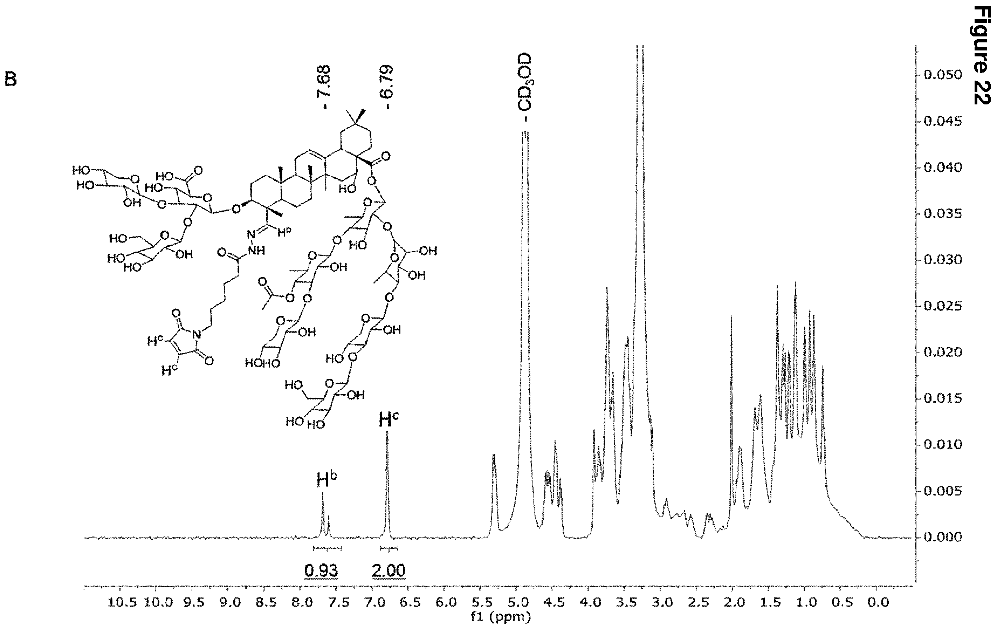

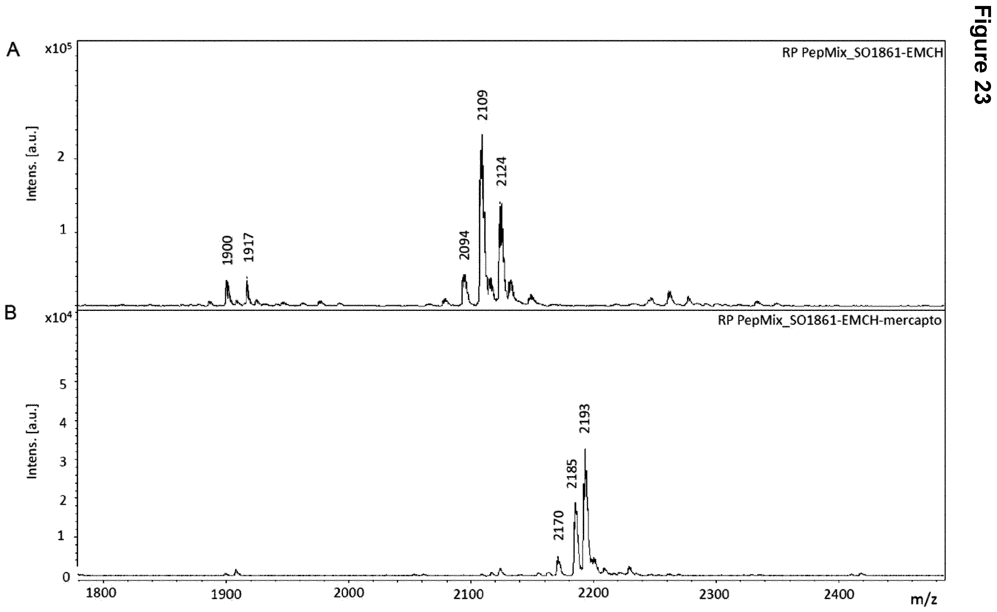

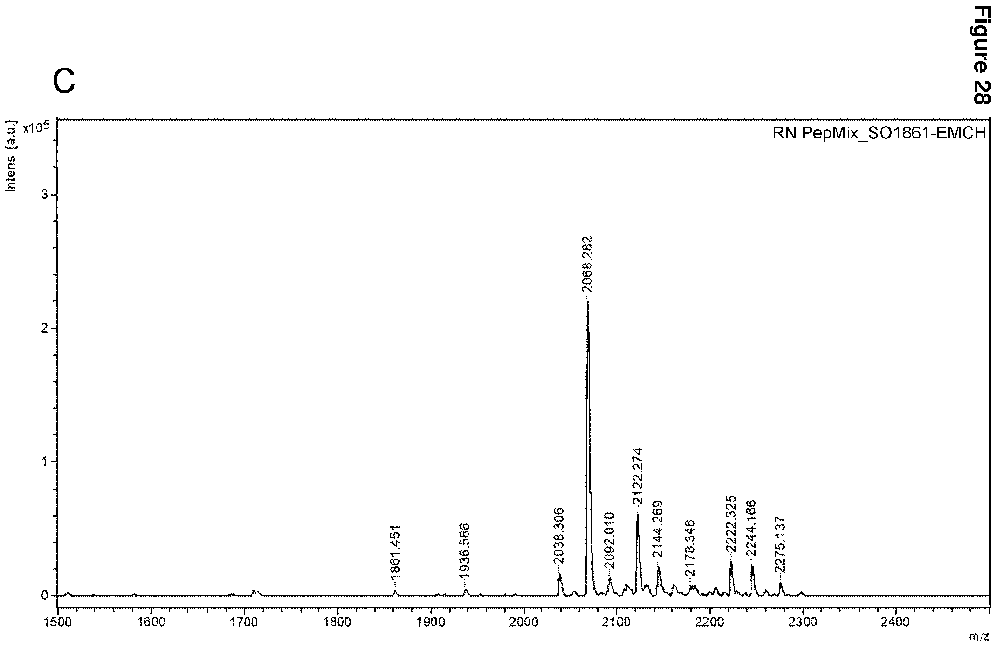

SO1861 from Saponaria officinalis L (59 mg, 31.7 µmol) and EMCH (301 mg, 888 µmol) were placed in a round flask with stirrer and dissolved in 13 mL methanol. TFA (400 µL, cat.) was added to the solution and the reaction mixture was stirred for 3 h at 800 rpm and room temperature on a RCT B magnetic stirrer (IKA Labortechnik). After stirring for 3 h, the mix was diluted either with MilliQ water or PBS and dialyzed extensively for 24 h against either with MilliQ water or PBS using regenerated cellulose membrane tubes (Spectra/Por 7) with a MWCO of 1 kDa. After dialysis, the solution was lyophilized to obtain a white powder. Yield 62.4 mg (95 %). Dried aliquots were further used for characterization via ¹H NMR and MALDI-TOF-MS.
¹H NMR (400 MHz, methanol-*D*₄) (Figure 22 A, SO1861): δ = 0.50-5.50 (m, saponin triterpenoid and sugar backbone protons), 9.43 (1H, s, aldehyde proton of saponin, H^{a}).
¹H NMR (400 MHz, methanol-*D*₄) (Figure 22 B. SO1861-EMCH, PBS workup): δ = 0.50-5.50 (m, saponin triterpenoid and sugar backbone protons), 6.79 (2 H, s, maleimide protons, H^{c}), 7.62-7.68 (1 H, m, hydrazone proton, H^{b}).
MALDI-TOF-MS (RP mode) (Figure 23 A): *m*/*z* 2124 Da ([M+K]⁺, saponin-EMCH), m/z 2109 Da ([M+K]⁺, SO1861-EMCH), *m*/*z* 2094 Da ([M+Na]⁺, SO1861-EMCH)
MALDI-TOF-MS (RN mode) (Figure 28 C): *m*/*z* 2275 Da ([M-H]⁻, saponin-EMCH conjugate), 2244 Da ([M-H]⁻, saponin-EMCH conjugate), 2222 Da ([M-H]⁻, saponin-EMCH conjugate), 2178 Da ([M-H]⁻, saponin-EMCH conjugate), 2144 Da ([M-H]⁻, saponin-EMCH conjugate), 2122 Da ([M-H]⁻, saponin-EMCH conjugate), 2092 Da ([M-H]⁻, saponin-EMCH conjugate), 2070 Da ([M-H]⁻, SO1861-EMCH), 2038 Da ([M-H]⁻, SO1832-EMCH), 1936 Da ([M-H]⁻, SO1730-EMCH), 1861 Da ([M-H]⁻, SO1861).

### SO1861-EMCH-mercaptoethanol

To SO1861-EMCH (0.1 mg, 48 nmol) 200 µL mercaptoethanol (18 mg, 230 µmol) was added and the solution was shaken for 1 h at 800 rpm and room temperature on a ThermoMixer C (Eppendorf). After shaking for 1 h, the solution was diluted with methanol and dialyzed extensively for 4 h against methanol using regenerated cellulose membrane tubes (Spectra/Por 7) with a MWCO of 1 kDa. After dialysis, an aliquot was taken out and analyzed via MALDI-TOF-MS.

MALDI-TOF-MS (Figure 23B) (RP mode): *m*/*z* 2193 Da ([M+K]⁺, SO1861-EMCH-mercaptoethanol), m/z 2185 Da ([M+K]⁺, SO1861-EMCH-mercaptoethanol), *m*/*z* 2170 Da ([M+Na]⁺, SO1861-EMCH-mercaptoethanol).

### BSA-SO1861 synthesis

2-iminothiolane (231 µg, 1.1 µmol) dissolved in 47 µL PBS was added to a BSA-RhodB solution (10 mg, 0.15 µmol) in 200 µL PBS and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (1 mg, 0.5 µmol) dissolved in 100 µL PBS was added to the collected BSA-SH fraction. The reaction mixture was shaken for 12 h at 800 rpm and room temperature on a ThermoMixer C (Eppendorf). After shaking for 12 h the BSA-SO1861 concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined.

MALDI-TOF-MS (Figure 15 A) (LP mode): *m*/*z* 74.2 kDa ([M+H]⁺, BSA-SO1861 with 4 SO1861 attached), 72.2 kDa ([M+H]⁺, BSA-SO1861 with 3 SO1861 attached), 70.2 kDa ([M+H]⁺, BSA-SO1861 with 2 SO1861 attached), 37.0 kDa ([M+H]²⁺, BSA-SO1861 with 4 SO1861 attached), 35.9 kDa ([M+H]²⁺, BSA-SO1861 with 3 SO1861 attached), 34.7 kDa ([M+H]²⁺, BSA-SO1861 with 2 SO1861 attached).

### Cy3-PAMAM

720 µL PAMAM dissolved in methanol (30 mg, 1.04 µmol) was placed into a 250 mL round flask and methanol was removed via a rotary evaporator (20 mbar, 60 °C). Remaining PAMAM was dissolved in 9 mL DMSO. HATU (7.6 mg, 20 µmol) dissolved in 0.5 mL DMSO was added to a Cy3-COOH (0.6 mg, 1.2 µmol) solution in DMSO and the mix was shaken for 1 h at 800 rpm at room temperature on a ThermoMixer C (Eppendorf). After shaking for 1 h, the HATU-Cy3 solution was added to the stirring PAMAM solution and the reaction mix was stirred for 12 h at room temperature. After stirring for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the volume of the conjugate solution was reduced via a rotary evaporator (20 mbar, 60 °C) and the concentrated conjugate solution was run through a Sephadex G25 superfine size exclusion column (16 mL column volume). The first fraction was collected and lyophilized to obtain the viscous pink PAMAM-Cy3 conjugate. PAMAM-Cy3 conjugate formation was confirmed by chromatography on thin layer chromatography (methanol/water, v/v 1:1), and the appearance of a faster band on a Sephadex G 25 superfine column. Yield 21.3 mg (63 %). The dye per PAMAM molar ratio determined by UV-Vis spectrophotometry was 0.43.

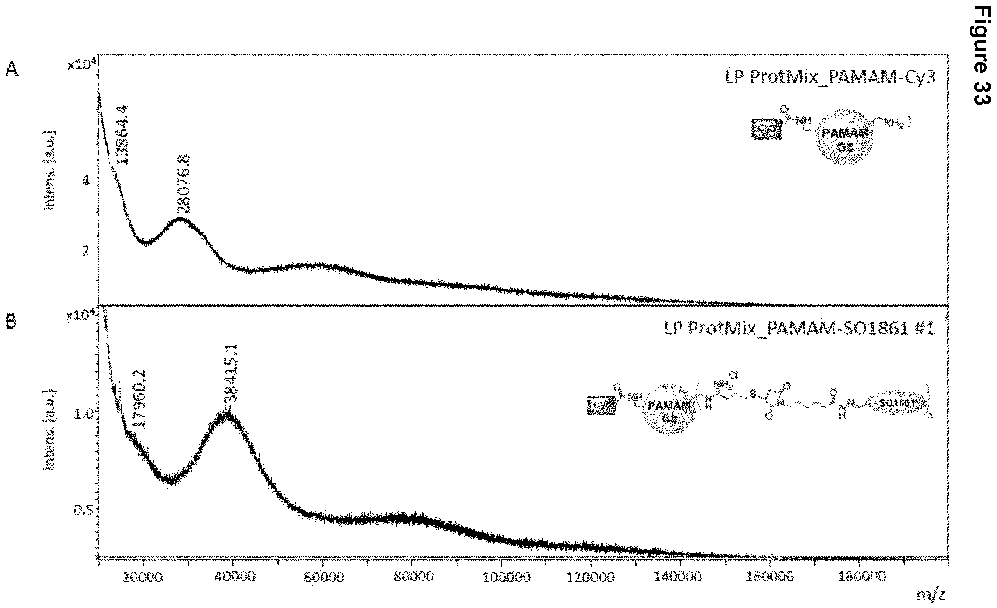

MALDI-TOF-MS (Figure 33 A) (LP mode): *m*/*z* 28.0 kDa ([M+H]⁺, Cy3-PAMAM).

### Cy3-PAMAM-SO1861 synthesis

Procedure is described exemplary for Cy3-PAMAM-(SO1861)₅. 2-iminothiolane (1 mg, 6.7 µmol) dissolved in 250 µL MilliQ water was added to a PAMAM-Cy3 solution (0.5 mg, 17 nmol) in 125 µL MilliQ water and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (176 µg, 85 nmol) dissolved in 40 µL MilliQ water was added to the collected Cy3-PAMAM-SH fraction. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12 - 14 kDa. After dialysis, the Cy3-PAMAM-SO1861 solution was concentrated using centrifugal filtration at 4000 rpm (15 °C) via Amicon Ultra 15 filters with a MWCO of 3 kDa. The conjugate was stored as solution in the fridge and aliquots were taken for analysis. Yield: 0.5 mg (75%).

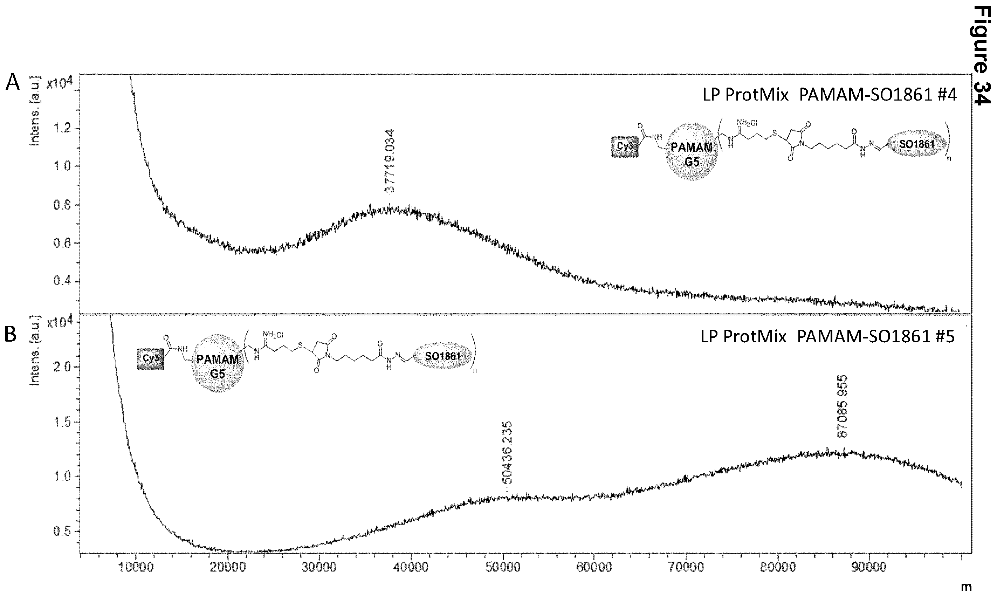

MALDI-TOF-MS spectra are illustrated in Figures 33 B-D, and Figure 34. MALDI-TOF-MS of Cy3-PAMAM-(SO1861)₆ (Figure 33 B) (LP mode): *m*/*z* 38.4 kDa ([M+H]⁺, Cy3-PAMAM-SO1861), 17.9 kDa ([M+H]²⁺, Cy3-PAMAM-SO1861).

The synthesis of Cy3-PAMAM-(SO1861)₅, Cy3-PAMAM-(SO1861)₁₃, Cy3-PAMAM-(SO1861)₅₁, and Cy3-PAMAM-(SO1861)₂₇, has been performed via the above described methodology but differ in the feed equivalents of the starting materials 2-iminothiolane and SO1861-EMCH. The respective feed equivalents of the starting materials and the respective mass of the conjugates are highlighted in Table 1.

**Table 1. Reaction parameter for Cy3-PAMAM-SO1861 synthesis.**

| **2-iminothiolane feed equivalents to Cy3-PAMAM** | **SO1861-EMCH feed equivalents to Cy3-PAMAM** | **Mass of conjugate via MALDI-TOF-MS** | **SO1861 molecules attached per PAMAM** | **Resulting conjugate** |
|---|---|---|---|---|
| 384 | 6 | 38.7 kDa | ~5 | Cy3-PAMAM-(SO1861)₆, Figure 33 B |
| 384 | 20 | 53.9 kDa | ~13 | Cy3-PAMAM-(SO1861)₁₃, Figure 33 C |
| 384 | 57 | 133.9 kDa | ~51 | Cy3-PAMAM-(SO1861)₅₁, Figure 33 D |
| 8 | 5 | 37.7 kDa | ~5 | Cy3-PAMAM-(SO1861)₅, Figure 34 A |
| 32 | 30 | 87.0 kDa | ~27 | Cy3-PAMAM-(SO1861)₂₇, Figure 34 B |

### Cy3-PAMAM-NC-SO1861 synthesis

Cy3-PAMAM (0.5 mg, 18 nmol), SO1861 (2.3 mg, 1.24 µmol), and HATU (64.6 mg, 170 µmol) were dissolved separately in 200 µL DMSO. SO1861 and HATU solutions were mixed and shaken for 20 min at 800 rpm and room temperature on a ThermoMixer C (Eppendorf). After shaking for 20 min, Cy3-PAMAM solution was added to the shaking SO1861-HATU solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The Cy3-PAMAM-NC-(SO1861)₁₇ conjugate was stored as solution in the fridge and aliquots were taken for analysis. Yield: 0.77 mg (69%).

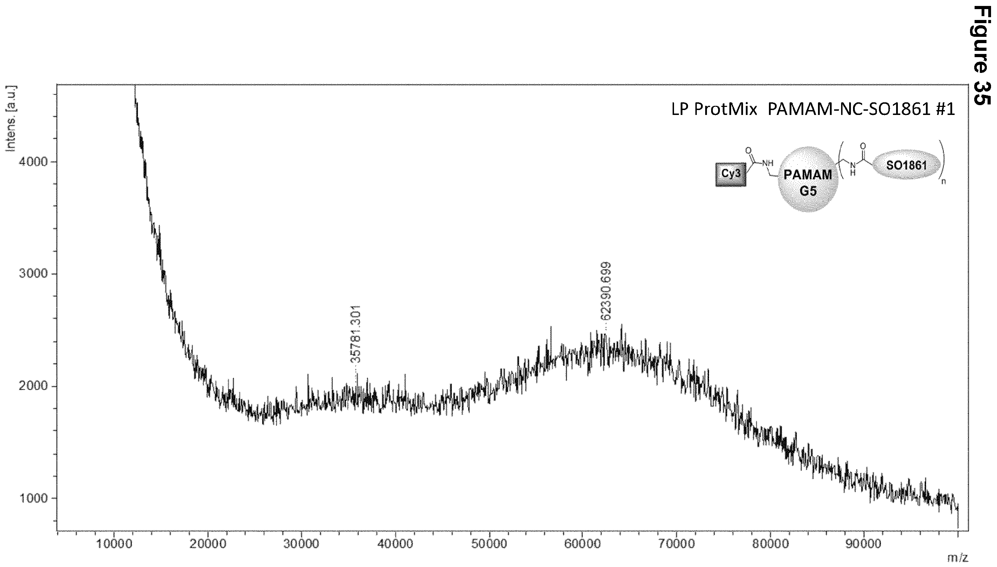

MALDI-TOF-MS (Figure 35) (LP mode): *m*/*z* 62.3 kDa ([M+H]⁺, Cy3-PAMAM-NC-SO1861), 35.7 kDa ([M+H]²⁺, Cy3-PAMAM-NC-SO1861).

### G4-dendron dye labeling and deprotection

PFd-G4-Azide-NH-BOC (G4-dendron) (9.75 mg, 2.11 µmol) was placed into a 2 mL reaction tube (Eppendorf) and dissolved in 200 µL DMSO. 100 µL of a Cy5-DBCO solution in DMSO (1.72 µmol * mL^{- 1}, 170 nmol) was added to the G4-dendron solution and the mix was shaken for 12 hours at room temperature and 800 rpm on a ThermoMixer C (Eppendorf). After shaking for 12 h, the reaction mix was diluted with MilliQ water and dialyzed extensively for 24 h against MilliQ water using regenerated cellulose membrane tubes (Spectra/Por 7) with a MWCO of 1 kDa. After dialysis, the solution was lyophilized to obtain a blue powder. The crude product was used as obtained from lyophilization for the deprotection step.

Partially Cy5 labeled lyophilized G4-dendron was dissolved in 12 mL CHCl₃ in 50 mL round flask with stirrer. 12 mL TFA was added and the reaction mix was stirred for 3 h at 800 rpm and room temperature on a RCT B magnetic stirrer (IKA Labortechnik). After stirring for 3 h, the solvent was removed under reduced pressure (50 °C, 30 mbar) on a rotary evaporator (Heidolph WB 2000). After evaporation, the batch was dissolved in MilliQ water and run through a PD10 size exclusion column. G4-dendron conjugate formation was confirmed by chromatography on thin layer chromatography (methanol/water, v/v 1:1), and the appearance of a faster band on a PD10 column. Obtained fraction of size exclusion chromatography was lyophilized to obtain a blue powder.

Yield 5.7 mg (93 %). The dye per G4-dendron molar ratio determined by UV-Vis spectrophotometry was 0.012.

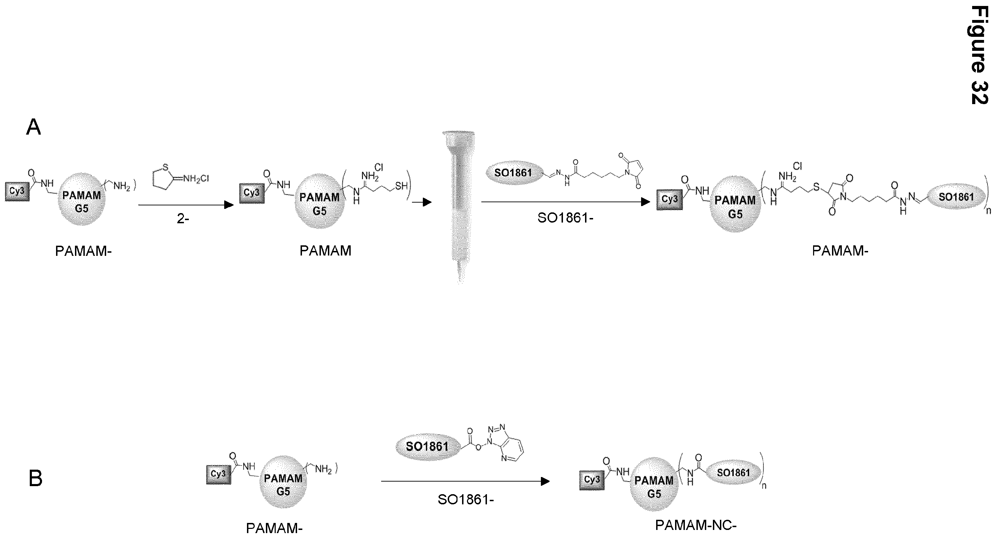

MALDI-TOF-MS (Figure 32 B) (RP mode): *m*/*z* 3956 Da ([M+Na]⁺, Cy5-G4-dendron + PF₆⁻ counterion), 3820 Da ([M+Na]⁺, Cy5-G4-dendron - PF₆⁻ counterion), 3617 Da ([M+H]⁺, G4-dendron impurity), 3017 ([M+H]⁺, G4-dendron).

### G4-dendron-SO1861 synthesis

Procedure is described exemplary for the lowest G4-dendron to SO1861-EMCH ratio. 2-iminothiolane (2.65 mg, 19.2 µmol) dissolved in 300 µL MilliQ water was added to a partially Cy5 labeled G4-dendron solution (0.577 mg, 192 nmol) in 252 µL MilliQ water and the mix was shaken for 40 min at 800 rpm and room temperature on a ThermoMixer C (Eppendorf). After shaking for 40 min, the reaction mix was immediately run through a PD10 size exclusion column and SO1861-EMCH (1.19 mg, 575 nmol) dissolved in 100 µL MilliQ water was added to the collected G4-dendron-SH fraction. The reaction mixture was shaken for 12 h at 800 rpm and room temperature on a ThermoMixer C (Eppendorf). After shaking for 12 h, the reaction mix was concentrated via centrifugal filtration using Amicon Ultra centrifugal filters (3 kDa MWCO). The conjugate was stored as solution in the fridge and aliquots were taken for analysis. Yield: 90 nmol (47%).

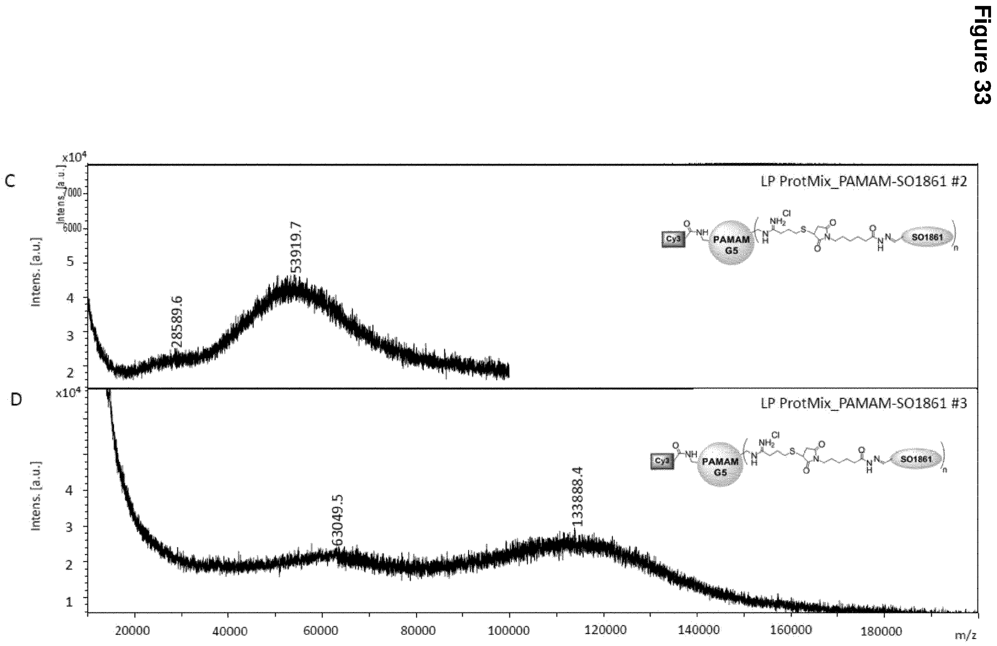

MALDI-TOF-MS spectra are illustrated in Figure 33. MALDI-TOF-MS of G4-dendron-SO1861 (Figure 33 C) (LP mode): *m*/*z* 10.19 kDa ([M+H]⁺, Cy5-G4-dendron-[SO1861]₃), 9.27 kDa ([M+H]⁺, G4-dendron-[SO1861]₃), 7.92 kDa ([M+H]⁺, Cy5-G4-dendron-[SO1861]₂), 7.14 kDa ([M+H]⁺, G4-dendron-[SO1861]₂), 5.86 kDa ([M+H]⁺, Cy5-G4-dendron-[SO1861]₁), 5.07 kDa ([M+H]⁺, G4-dendron-[SO1861]₁).

The synthesis of other G4-dendron-(SO1861)ₙ conjugates has been performed via the above described methodology but differs in the feed equivalents of the starting material SO1861-EMCH. The respective feed equivalents of the starting materials and the respective mass of the conjugates are highlighted in Table 2.

**Table 2. Reaction parameter for G4-dendron-SO1861 synthesis.**

| **2-Iminothiolane feed equivalents to G4-dendron** | **SO1861-EMCH feed equivalents to G4-dendron** | **Mass of conjugates via MALDI-TOF-MS** | **SO1861 molecules attached per G4-dendron** | **Resulting MS spectrum** |
|---|---|---|---|---|
| 100 | 3 | 5.07 - 10.18 kDa | ~ 1 -3 | Figure 49 C |
| 100 | 10 | 5.07 - 11.64 kDa | ~ 1 - 4 | Figure 49 B |
| 100 | 22 | 6.20 - 22.02 kDa | ~ 1 - 9 | Figure49 A |

### PAMAM thiolation

Procedure is described exemplary for the highest PAMAM to 2-iminothiolane ratio. To a PAMAM (333 µg, 12.8 nmol) solution dissolved in 30 µL methanol 2-iminothiolane (0.53 mg, 3.84 µmol) dissolved in 128 µL MilliQ water was added. The reaction mixture was shaken for 12 h at 800 rpm and room temperature on a ThermoMixer C (Eppendorf). After shaking for 12 h, the reaction mix was washed 4 times with MilliQ water via centrifugal filtration using Amicon Ultra centrifugal filters (3 kDa MWCO) at 15 °C and 13500 rpm. After washing the sample was lyophilized to obtain a white solid. Yield was not determined.

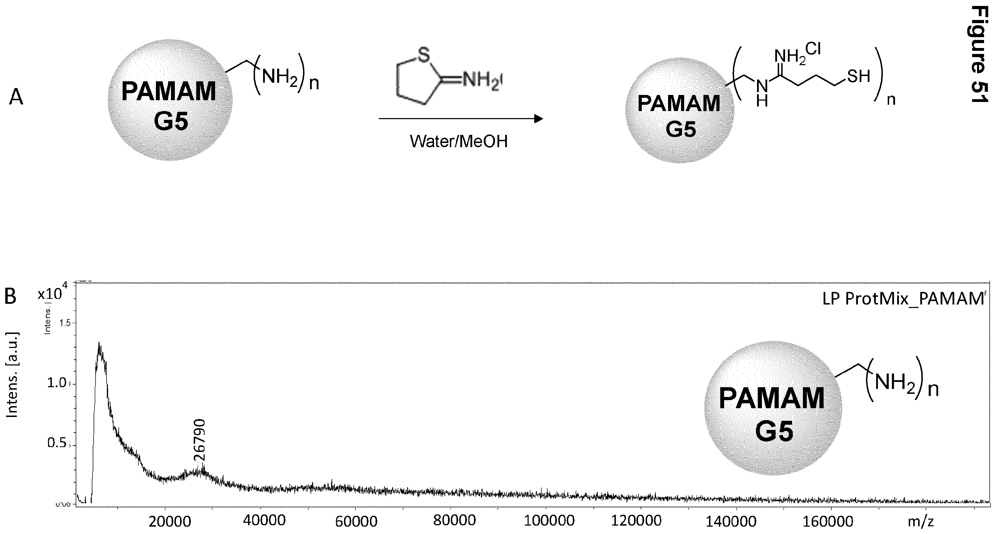

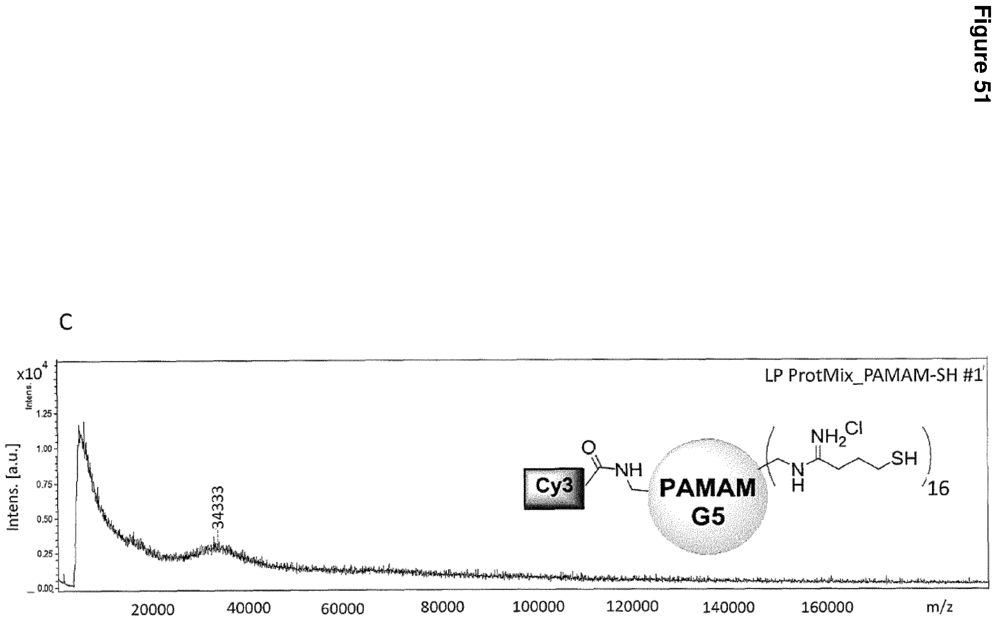

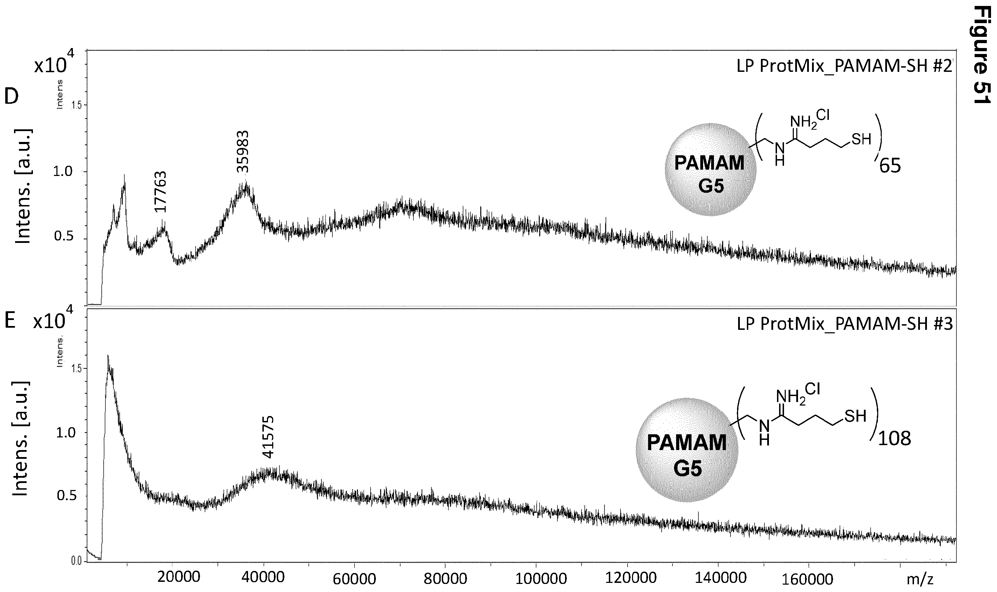

MALDI-TOF-MS spectra are illustrated in Figure 51. MALDI-TOF-MS of PAMAM-(SH)₁₀₈ (Figure 51 C) (LP mode): *m*/*z* 41.5 kDa ([M+H]⁺, PAMAM-[SH]₁₀₈).

The synthesis of other PAMAM-iminothiolane conjugates has been performed via the above described methodology but differs in the feed equivalents of the starting material 2-iminothiolane. For the lowest 2-iminothiolane feed reaction Cy3-PAMAM has been used.

The respective feed equivalents of the starting materials and the respective mass of the conjugates are highlighted in Table 3.

**Table 3. Reaction parameter for PAMAM-SH synthesis.**

| **2-iminothiolane feed equivalents to PAMAM** | **Mass of conjugates via MALDI-TOF-MS** | **Iminothiolane molecules attached per PAMAM** | **Resulting MS spectrum** |
|---|---|---|---|
| 50 | 34.4 kDa | ~ 16 | Fig. 51 C |
| 100 | 35.9 kDa | ~ 65 | Fig. 51 D |
| 300 | 41.5 kDa | ~108 | Fig. 51 E |

### PAMAM PEGylation

Procedure is described exemplary for the lowest PAMAM to mPEG₂ₖ ratio. To a PAMAM (333 µg, 12.8 nmol) solution dissolved in 10 µL DMSO mPEG₂ₖ-NHS (0.268 mg, 128 nmol) dissolved in 13 µL DMSO was added. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the batch was concentrated via centrifugal filtration using Amicon Ultra 15 mL centrifugal filters (10 kDa MWCO). The concentrated batch was run through a PD10 size exclusion column followed by lyophilization to obtain a white fluffy powder. Yield was not determined.

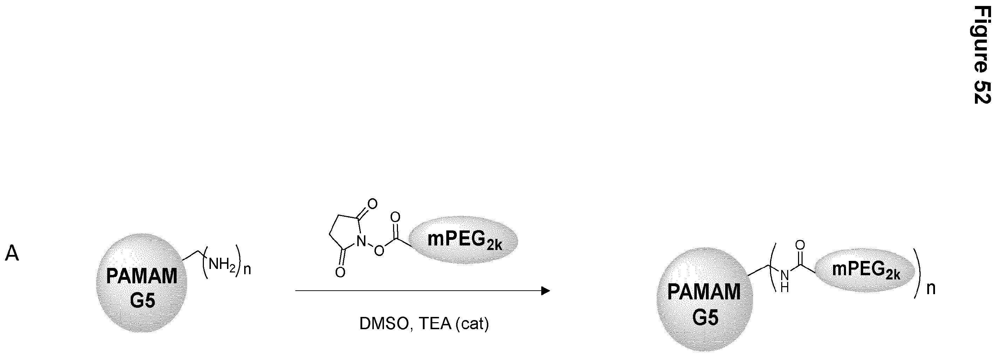

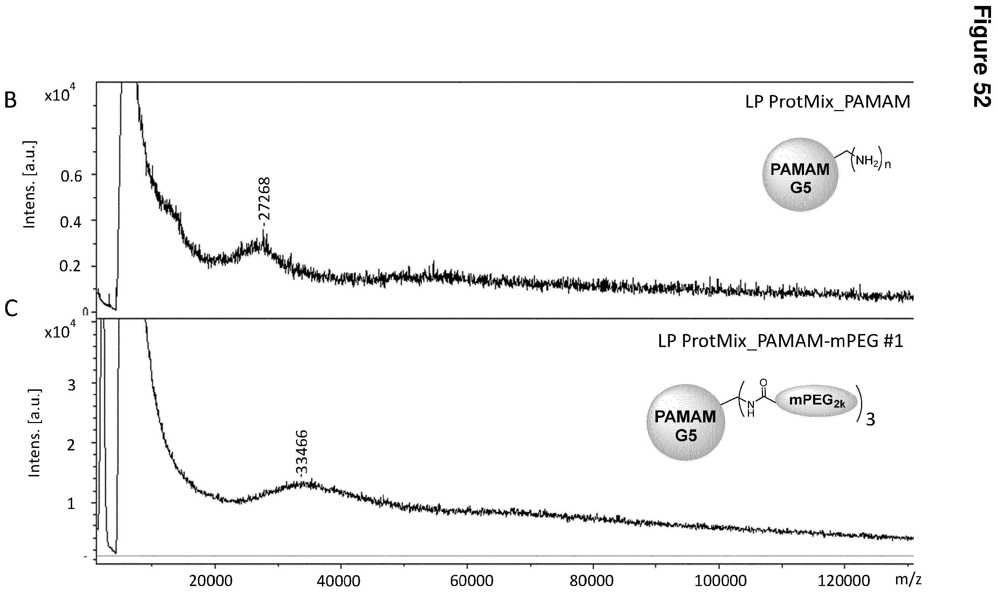

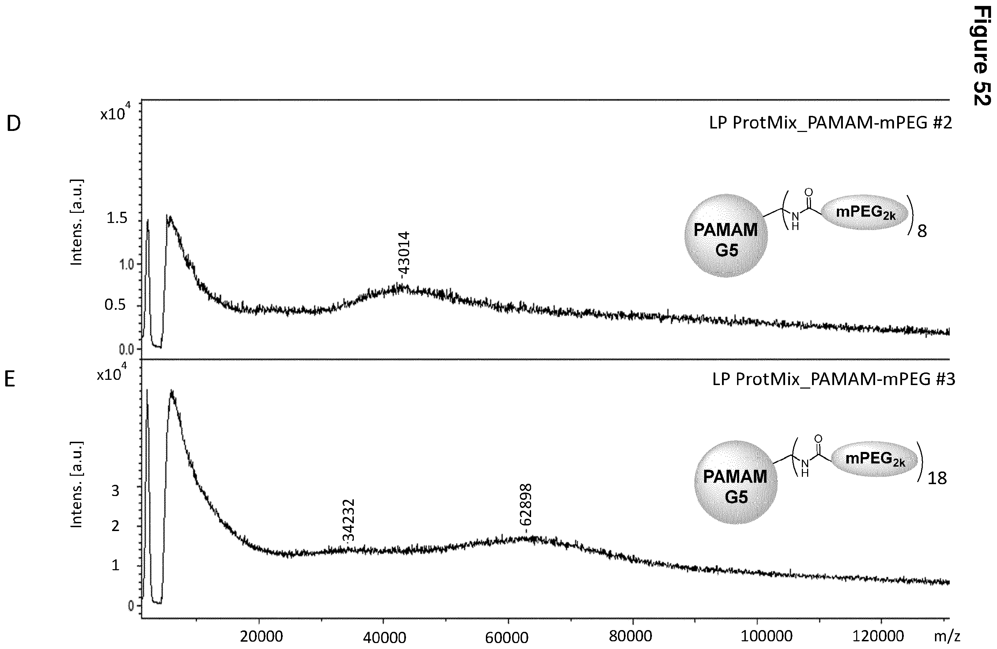

MALDI-TOF-MS spectra are illustrated in Figure 52. MALDI-TOF-MS of PAMAM-(mPEG₂ₖ)₃ (Figure 52 C) (LP mode): *m*/*z* 33.46 kDa ([M+H]⁺, PAMAM-[mPEG₂ₖ]₃).

The synthesis of other PAMAM-mPEG₂ₖ conjugates has been performed via the above described methodology but differs in the feed equivalents of the starting material mPEG₂ₖ-NHS. The respective feed equivalents of the starting materials and the respective mass of the conjugates are highlighted in Table 4.

**Table 4. Reaction parameter for PAMAM-mPEG₂ₖ synthesis.**

| **mPEG₂ₖ-NHS feed equivalents to PAMAM** | **Mass of conjugates via MALDI-TOF-MS** | **mPEG₂ₖ molecules attached per PAMAM** | **Resulting MS spectrum** |
|---|---|---|---|
| 10 | 28.5 kDa | ~ 3 | Figure 52 C |
| 20 | 43.0 kDa | ~ 8 | Figure 52 D |
| 100 | 62.8 kDa | ~ 18 | Figure 52 E |

### Cy3-PAMAM-SO1861-DBCO synthesis

Procedure is described exemplary for Cy3-PAMAM-(SO1861)₂₇-(DBCO)₁₀. Cy3-PAMAM-(SO1861)₂₇ (0.41 mg, 4.71 nmol) was freeze-fried and dissolved in 100 µL DMSO. DBCO-PEG₁₃-NHS ester (0.197 mg, 188 nmol) dissolved in DMSO was added to the Cy3-PAMAM-SO1861 solution and the mixture was shaken at 800 rpm and room temperature on a ThermoMixer C (Eppendorf). After shaking for 3 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-SO1861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%).

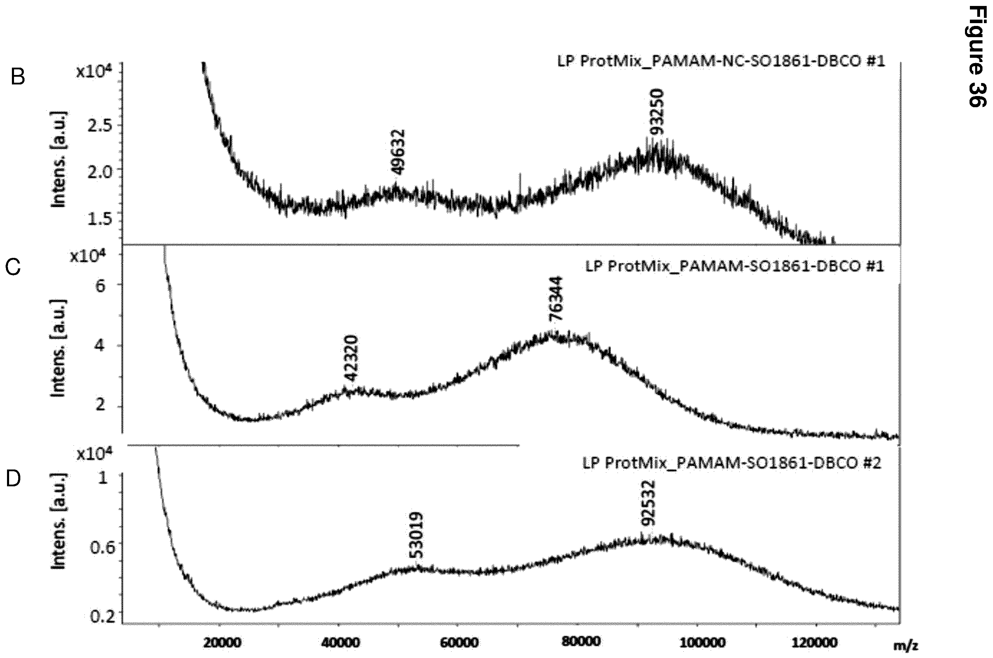

MALDI-TOF-MS (Figure 36 D) (LP mode): *m*/*z* 92.5 kDa ([M+H]⁺, Cy3-PAMAM-SO1861-DBCO), 53.0 kDa ([M+H]²⁺, Cy3-PAMAM-SO1861-DBCO).

The synthesis of Cy3-PAMAM-(SO1861)₅-(DBCO)₃₈, and Cy3-PAMAM-(SO1861)₂₇-(DBCO)₁₀, have been performed via the above described methodology. The respective feed equivalents of the starting material and the respective mass of the conjugates are highlighted in Table 5.

**Table 5. Reaction parameter for Cy3-PAMAM-SO1861-DBCO synthesis.**

| **Used Cy3-PAMAM-saponin batch** | **DBCO-PEG13-NHS feed equivalents** | **Mass via MALDI-TOF-MS** | **DBCO molecules attached per PAMAM** | **Resulting conjugate** |
|---|---|---|---|---|
| Cy3-PAMAM-(SO1861)₅ | 40 | 76.3 kDa | ~38 | Cy3-PAMAM-(SO1861)₅-(DBCO)₃₈, Figure 36 C |
| Cy3-PAMAM-(SO1861)₂₇ | 40 | 92.5 kDa | ~10 | Cy3-PAMAM-(SO1861)₂₇-(DBCO)₁₀, Figure 36 D |

### Cy3-PAMAM-NC-SO1861-DBCO synthesis

Cy3-PAMAM-NC-(SO1861)₁₇ (0.3 mg, 4.8 nmol) was freeze-fried and dissolved in 100 µL DMSO. DBCO-PEG₁₃-NHS ester (0.202 mg, 194 nmol) dissolved in DMSO was added to the Cy3-PAMAM-NC-SO1861 solution and the mixture was shaken at 800 rpm and room temperature on a ThermoMixer C (Eppendorf). After shaking for 3 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-SO1861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%). Mass spectrometry indicates the conjugation of 30 DBCO moieties per PAMAM molecule.

MALDI-TOF-MS (Figure 36 B) (LP mode): *m*/*z* 93.2 kDa ([M+H]⁺, Cy3-PAMAM-NC-SO1861-DBCO), 49.6 kDa ([M+H]²⁺, Cy3-PAMAM-NC-SO1861-DBCO).

### EGFDianthin and dianthin expression

Plasmid-DNA (His-dianthin-EGF-pET11d or His-dianthin-pET11d) [20] was transformed into chemically competent Escherichia coli NiCo21 (DE3) (New England Biolabs^{®}, Inc.) and grown in 3 mL lysogeny broth supplemented with 50 µg/mL ampicillin at 37 °C for 5 h at 200 rpm. These bacteria were used to inoculate 500 mL lysogeny broth supplemented with 50 µg/mL ampicillin for overnight culture at 37 °C. Subsequently, the culture volume was scaled up to 2 L and bacteria were grown until an optical density (A600) of 0.9. Protein expression was induced by the addition of isopropyl β-D-1-thiogalactopyranoside (IPTG) at a final concentration of 1 mM. Cells were further grown for 3 h at 37 °C and 200 rpm. After centrifugation (5 min, 5,000 g, 4 °C) cell pellets were resuspended in 20 mL phosphate buffered saline (Dulbecco's phosphate-buffered saline (PBS) with Ca²⁺ and Mg²⁺, pH 7.4) and stored at -20 °C. After thawing, proteins were released by ultrasound device (Branson Sonifier 250, G. Heinemann). The solution was centrifuged (15,800 × g, 30 min, 4 °C) and adjusted to 20 mM imidazole concentration. The construct contained an N-terminal His-tag and was purified by nickel nitrilotriacetic acid chromatography (Ni-NTA Agarose, Qiagen, Hilden, Germany). After elution with imidazole (20-250 mM) the eluates were analyzed by sodium dodecyl sulfate polyacrylamide gel electrophoresis (SDS-PAGE) (12%). Fractions containing dianthin-EGF or dianthin were dialyzed against 2 L chitin binding domain buffer (20 mM tris(hydroxymethyl)-aminomethane/HCl, 500 mM NaCl, 1 mM EDTA, 0.1% Tween-20, pH 8.0) at 4 °C. Further purification by chitin column affinity chromatography served to remove bacterial proteins with binding activity for Ni-NTA agarose. After elution with chitin binding domain buffer, the fractions were analyzed by SDS-PAGE (12%). Fractions containing dianthin-EGF or dianthin were dialyzed against 5 L PBS at 4 °C. Purified proteins were concentrated by Amicon centrifugal filter devices (10 kDa, Millipore, Eschborn, Germany). The protein concentration was determined by a bicinchoninic acid assay (Pierce, Rockford, USA).

### Dianthin-EGF-Alexa488 synthesis

Dianthin-EGF (240 µg, 6.7 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin-EGF sodium carbonate solution was placed into a 2 mL reaction tube and Alexa Fluor^{™} 488 5-TFP (50 µg, 56 nmol) dissolved in 10 µL DMSO was added to the protein solution. The mix was shaken at 800 rpm and room temperature on a ThermoMixer C (Eppendorf) for 80 min. After shaking, the mix was run through a Sephadex G25 M size exclusion column (GE Healthcare, PD10 column). The dianthin-EGF-Alexa488 conjugate was stored in solution in 0.1 M sodium carbonate buffer at pH 9 in the fridge and aliquots were taken for analysis. Yield: 210 µg (85%).

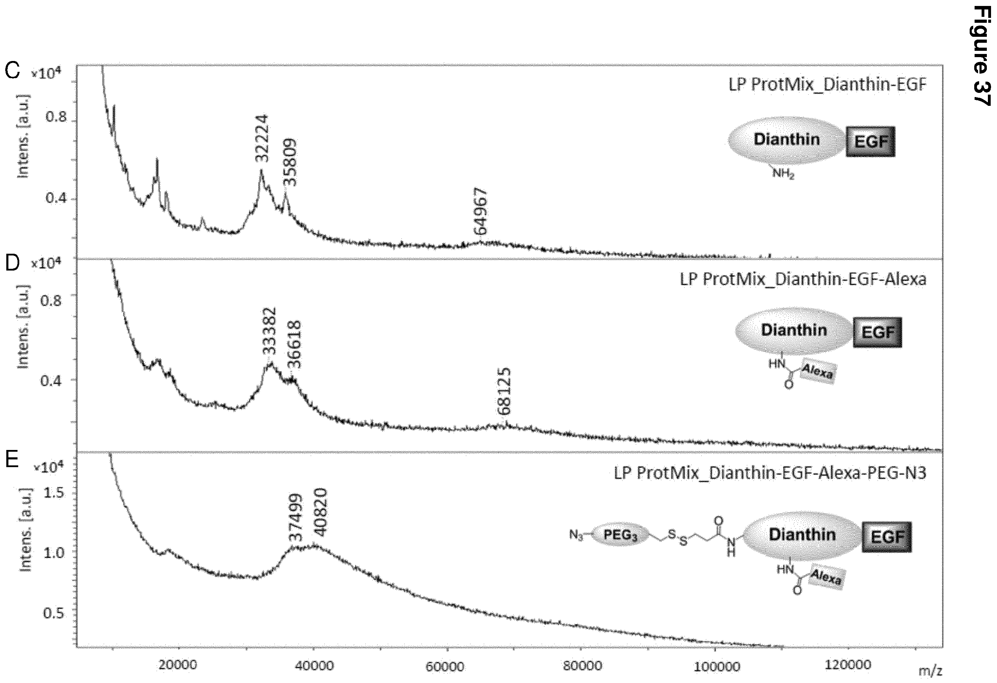

MALDI-TOF-MS (Figure 37 D) (LP mode): *m*/*z* 36.8 kDa ([M+H]⁺, dianthin-EGF-Alexa488), *m*/*z* 33.6 kDa ([M+H]⁺, dianthin-EGF-Alexa488), 18.8 kDa ([M+H]²⁺, dianthin-EGF-Alexa488), 16.6 kDa ([M+H]²⁺, dianthin-EGF-Alexa488).

### Dianthin-Alexa488 synthesis

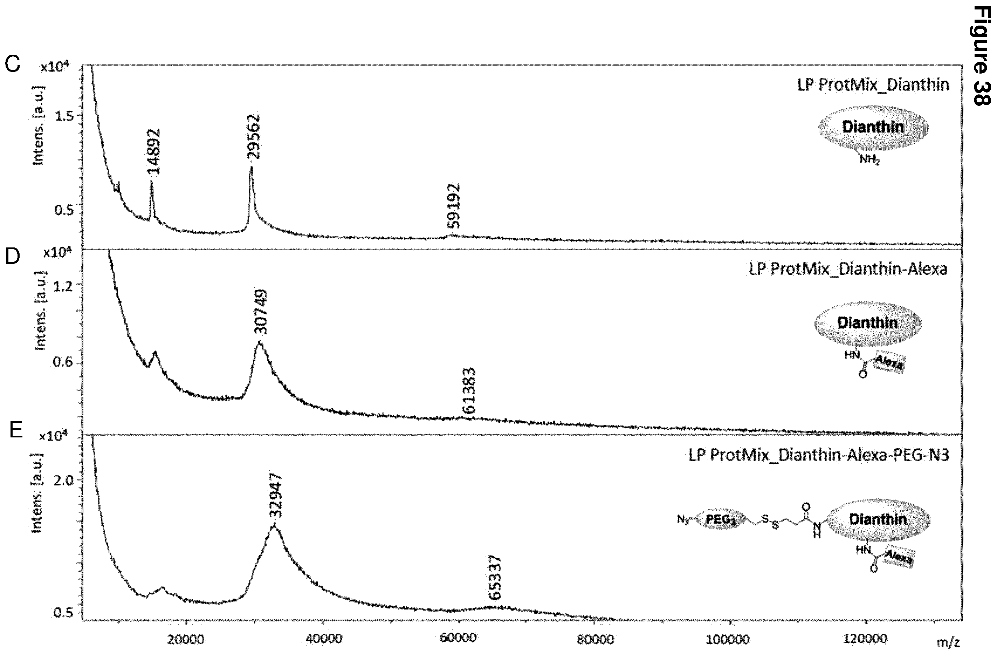

Dianthin (184 µg, 6.2 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin sodium carbonate solution was placed into a 2 mL reaction tube and Alexa Fluor^{™} 488 5-TFP (16.7 µg, 19 nmol) dissolved in 3.5 µL DMSO was added to the protein solution. The mix was shaken at 800 rpm and room temperature on a ThermoMixer C (Eppendorf) for 80 min. After shaking, the mix was run through a Sephadex G25 M size exclusion column (GE Healthcare, PD 10 column). The dianthin-Alexa488 conjugate was stored in solution in 0.1 M sodium carbonate buffer at pH 9 in the fridge and aliquots were taken for analysis. Yield: not determined MALDI-TOF-MS (Figure 38 D) (LP mode): *m*/*z* 30.7 kDa ([M+H]⁺, dianthin-Alexa488).

### Dianthin-EGF-Alexa488-S-S-PEG-N₃, and Dianthin-EGF-Alexa488-PEG₁₂-N₃ synthesis

Procedure is described exemplary for dianthin-EGF-Alexa488-S-S-PEG-N₃. Dianthin-EGF-Alexa488 (70 µg, 1.9 nmol) sodium carbonate solution was placed into a 2 mL reaction tube and azido-PEG₃-S-S-NHS (120 µg, 272 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.

Yield: 54 µg (70%).

MALDI-TOF-MS (Figure 37 E) (LP mode): *m*/*z* 40.8 kDa ([M+H]⁺, dianthin-EGF-Alexa488-S-S-PEG-N₃), *m*/*z* 37.5 kDa ([M+H]⁺, dianthin-EGF-Alexa488-S-S-PEG-N₃).

The synthesis of dianthin-EGF-Alexa488-S-S-PEG-N₃, and dianthin-EGF-Alexa488-PEG₁₂-N₃ have been performed via the above described methodology but differed in the used azido-PEG linker. The respective azido-PEG linker, their feed equivalents, and the respective mass of the conjugates are highlighted in Table 6.

**Table 6. Reaction parameter for dianthin-EGF-Alexa488-PEG-N₃ synthesis**

| **Used toxin batch** | **Azido-PEG linker used** | **Azido-PEG linker feed equivalents** | **Mass of conjugate via MALDI-TOF-MS** | **Resulting conjugate** |
|---|---|---|---|---|
| Dianthin-EGF-Alexa488 | Azido-PEG₃-S-S-NHS | 135 | 40.8 kDa | Dianthin-EGF-Alexa488-S-S-PEG₃-N₃ |
| Dianthin-EGF-Alexa488 | Azido-PEG₁₂-NHS | 135 | 43.3 kDa | Dianthin-EGF-Alexa488-PEG₁₂-N₃ |

### Dianthin-Alexa488-S-S-PEG-N₃

Dianthin-Alexa488 (24.5 µg, 0.8 nmol) sodium carbonate solution was placed into a 2 mL reaction tube and azido-PEG₃-S-S-NHS (34 µg, 78 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.

Yield: 10.3 µg (39%).

MALDI-TOF-MS (Figure 38 E) (LP mode): *m*/*z* 32.9 kDa ([M+H]⁺, dianthin-Alexa488-S-S-PEG-N₃).

### Cy3-PAMAM-Saponin-Toxin conjugate synthesis

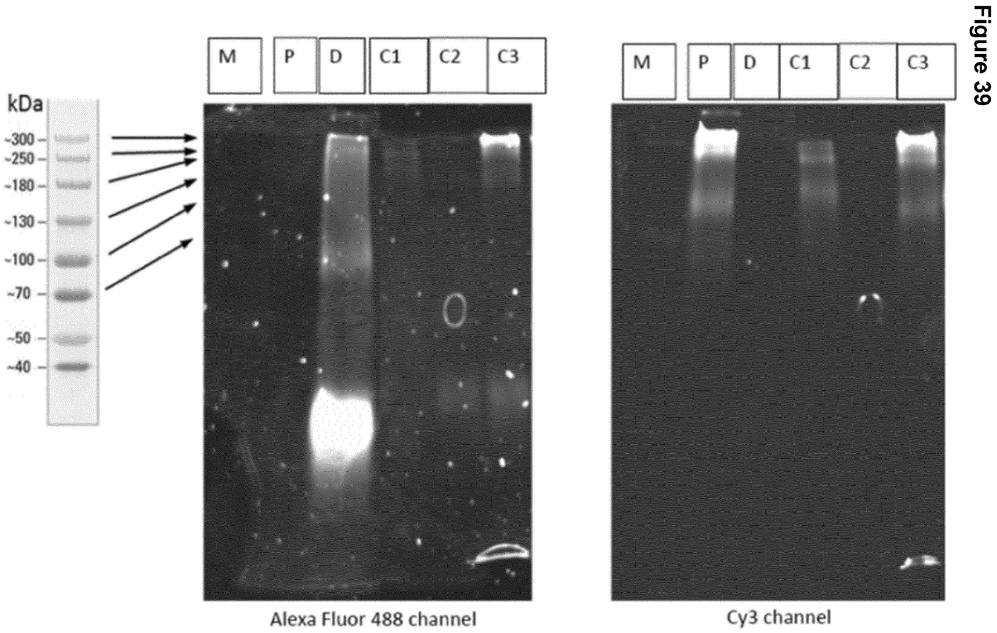

Procedure is described exemplary for Cy3-PAMAM-(SO1861)₂₇-DBCO. Cy3-PAMAM-(SO1861)₂₇-DBCO (17 µg, 0.184 nmol) solution in MilliQ water was mixed with a dianthin-EGF-Alexa488-SS-PEG3-N₃ (3.6 µg, 0.089 nmol) solution in PBS in a 1.5 mL reaction tube and the reaction mix was shaken at 800 rpm and 15 °C on a ThermoMixer C (Eppendorf) for 2 h. After shaking, small aliquots were taken out for analysis via SDS-PAGE and fluorescence imaging on a Molecular Imager^{®} VersaDoc^{™} MP 4000 imaging system (Bio-Rad) (Figure 39).

The synthesis of Cy3-PAMAM-(SO1861)₅-S-S-Dianthin-EGF-Alexa488, Cy3-PAMAM-(SO1861)₂₇-S-S-Dianthin-EGF-Alexa488, Cy3-PAMAM-NC-(SO1861)₁₇-S-S-Dianthin-EGF- Alexa488, Cy3-PAMAM-NC-(SO1861)₁₇-S-S-Dianthin-Alexa488, and Cy3-PAMAM-NC-(SO1861)₁₇-Dianthin-EGF-Alexa488, have been performed via the above described methodology but differ in the used PAMAM-saponin-DBCO batch, the used azido-toxin batch, and their feed equivalents. The respective feed equivalents of the starting materials are highlighted in Table 7.

**Table 7. Reaction parameter for Cy3-PAMAM-saponin-toxin synthesis.**

| **PAMAM-saponin-DBCO batch used** | **PAMAM-saponin-DBCO feed equivalents** | **Azido-toxin batch used** | **Azido-toxin feed equivalents** | **Resulting conjugate** |
|---|---|---|---|---|
| Cy3-PAMAM-(SO1861)₅-(DBCO)₃₈ | 3 | Dianthin-EGF-Alexa488-S-S-PEG₃-N₃ | 1 | Cy3-PAMAM-(SO1861)₅-S-S-Dianthin-EGF-Alexa488 |
| Cy3-PAMAM-(SO1861)₂₇-(DBCO)₁₀ | 2.1 | Dianthin-EGF-Alexa488-S-S-PEG₃-N₃ | 1 | Cy3-PAMAM-(SO1861)₂₇-S-S-Dianthin-EGF-Alexa488 |
| Cy3-PAMAM-NC-(SO1861)₁₇-(DBCO)₃₀ | 2.3 | Dianthin-EGF-Alexa488-S-S-PEG₃-N₃ | 1 | Cy3-PAMAM-NC-(SO1861)₁₇-S-S-Dianthin-EGF-Alexa488 |
| Cy3-PAMAM-NC-(SO1861)₁₇-(DBCO)₃₀ | 7.1 | Dianthin-Alexa488-S-S-PEG₃-N₃ | 1 | Cy3-PAMAM-NC-(SO1861)₁₇-S-S-Dianthin-Alexa488 |
| Cy3-PAMAM-NC-(SO1861)₁₇-(DBCO)₃₀ | 2.3 | Dianthin-EGF-Alexa488-PEG₁₂-N₃ | 1 | Cy3-PAMAM-NC-(SO1861)₁₇-Dianthin-EGF-Alexa488 |

### Cy3-PAMAM-NC-SO1861 synthesis via reductive amination

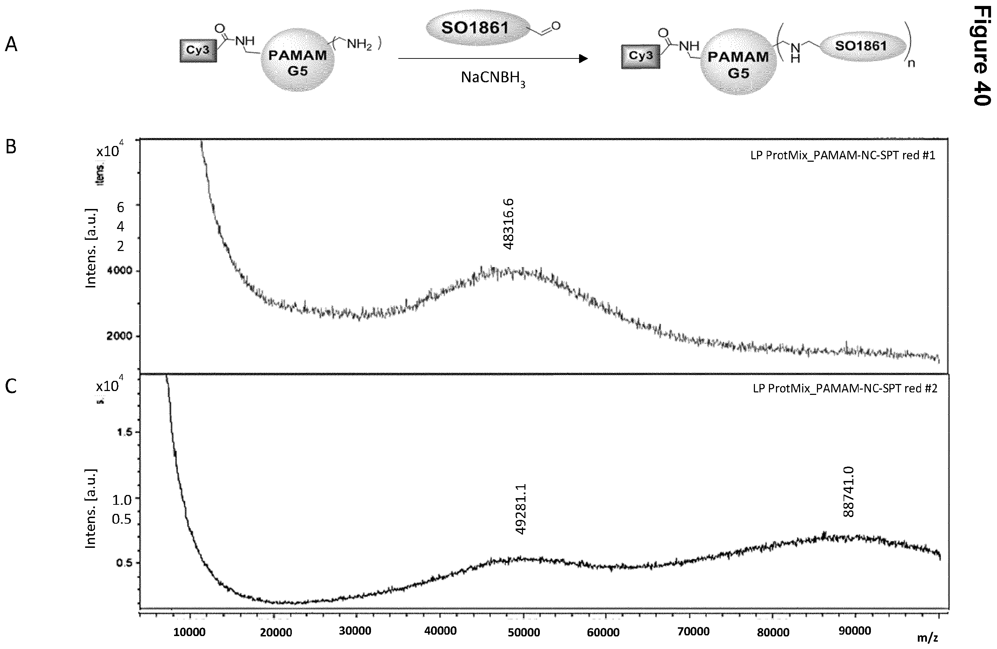

Cy3-PAMAM (0.19 mg, 13 nmol) and SO1861 (0.73 mg, 0.39 µmol) were dissolved separately in 200 µL 0.1 M acetate buffer at pH 5. SO1861 and Cy3-PAMAM solutions were mixed and shaken for 20 min at 800 rpm and room temperature on a ThermoMixer C (Eppendorf). After shaking for 20 min, NaCNBH₃ (5 mg, 81 µmol) was added to the shaking reaction solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined
MALDI-TOF-MS (Figure 40 B, C) (LP mode): *m*/*z* 88.7 kDa ([M+H]⁺, Cy3-PAMAM-NC-SO1861), 49.2 kDa ([M+H]²⁺, Cy3-PAMAM-NC-SO1861).

The synthesis of Cy3-PAMAM-NC-(SO1861)₃₀, and Cy3-PAMAM-NC-(SO1861)₁₀, have been performed via the above described methodology but differed in the time after which the reducing agent NaCNBH₃ was added to the reaction batch. The respective time of the NaCNBH₃ addition and the respective mass of the conjugates are highlighted in Table 8.

**Table 8. Reaction parameter Cy3-PAMAM-NC-SO1861 synthesis via reductive amination.**

| **Time of shaking reaction mix before NaCNBH₃ addition** | **Mass via MALDI-TOF-MS** | **SO1861 molecules attached per PAMAM** | **Resulting conjugate** |
|---|---|---|---|
| 20 min | 88.8 kDa | ~30 | Cy3-PAMAM-NC-(SO1861)₃₀, Figure 40 C |
| 12 h | 48.0 kDa | ~10 | Cy3-PAMAM-NC-(SO1861)₁₀, Figure 40 B |

### Poly(SO1861) synthesis

SO1861-EMCH (0.13 mg, 63 nmol) was dissolved in 30 µL degased MilliQ water. APS (0.2 µg, 0.8 nmol) dissolved in **4** µL degased MilliQ water was added to the SO1861-EMCH solution and the solution was placed into a ThermoMixer C (Eppendorf) at 60 °C. Then, TMEDA (cat., 0.5 µL) was added to the mix and the mix was shaken at 800 rpm and 60 °C on a ThermoMixer C (Eppendorf) for 2 h. After 2 h, a small aliquot was taken out for analysis via mass spectrometry.

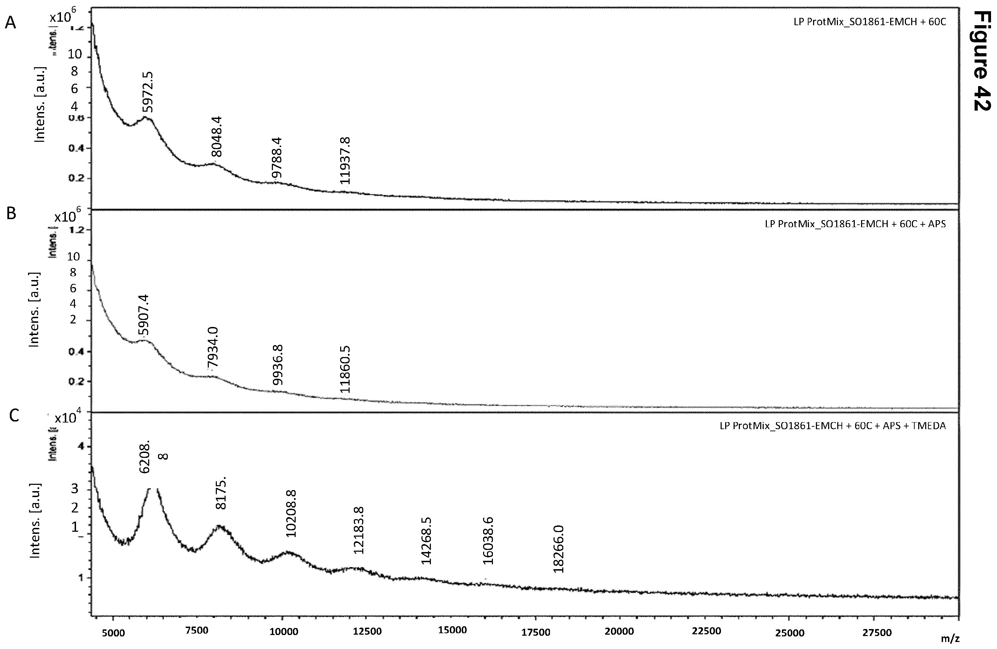

MALDI-TOF-MS (Figure 42 C) (LP mode): *m*/*z* 18.2 kDa ([M+H]⁺, poly(SO1861)₉), 16.0 kDa ([M+H]⁺, poly(SO1861)₈), 14.2 kDa ([M+H]⁺, poly(SO1861)₇), 12.2 kDa ([M+H]⁺, poly(SO1861)₆), 10.2 kDa ([M+H]⁺, poly(SO1861)₅), 8.2 kDa ([M+H]⁺, poly(SO1861)₄), 6.2 kDa ([M+H]⁺, poly(SO1861)₃).

### SO1861-EMCH peptide coupling

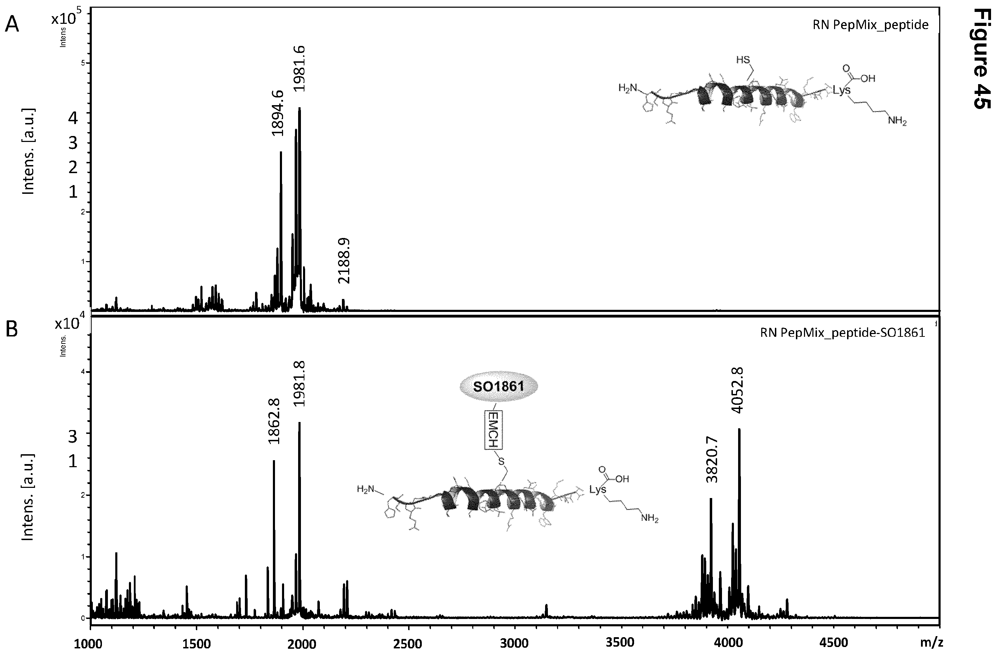

Customized peptide with the sequence SESDDAMFCDAMDESDSK (0.6 mg, 0.3 µmol) and SO1861-EMCH (0.8 mg, 0.39 µmol) were dissolved separately in 200 µL PBS. SO1861-EMCH and peptide solutions were mixed and shaken for 12 h at 800 rpm and room temperature on a ThermoMixer C (Eppendorf). After shaking small aliquots were taken out for analysis. Yield: not determined
MALDI-TOF-MS (Figure 45 B) (RN mode): *m*/*z* 4.05 kDa ([M+H]⁻, peptide-SO1861), 3.92 kDa ([M+H]⁻, peptide-SO1730), 1.98 kDa ([M+H]⁻, peptide), 1.86 kDa ([M+H]⁻, SO1861).

### Results

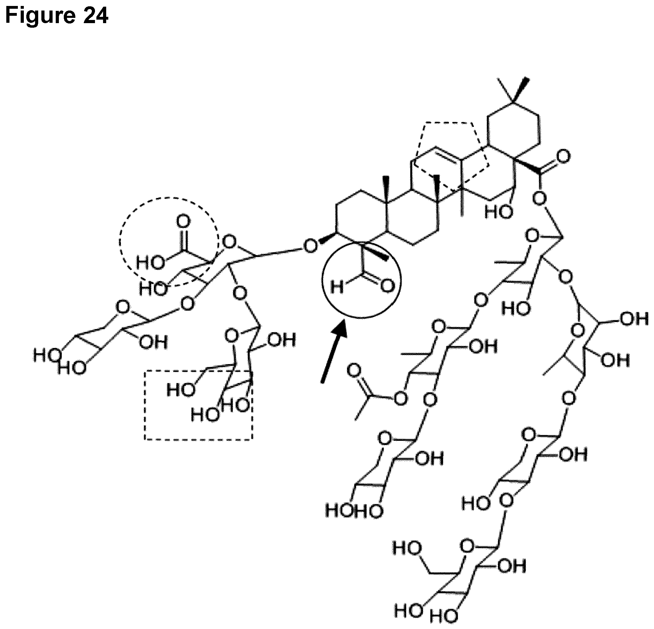

Considering available chemical groups for conjugation reactions to the SO1861 molecule, four chemical groups have been identified. The alcohols and diols of the sugar residues, the aldehyde group on the triterpenoid backbone, the carboxylic acid on one of the sugar residues (glucuronic acid), and the alkene group on the triterpenoid backbone as highlighted in Figure 24.

In view of the pros and cons of each identified chemical group (Table 9), the aldehyde and alcohol groups are best suitable for reversible conjugation reactions, while the alkene and the carboxylic acid (glucuronic acid) are the groups best suitable for irreversible / stable conjugation reactions. The aldehyde group within the molecule structure of SO1861, however, is the most suitable for reversible conjugation reactions over the alcohols. On the one hand, because there is only one aldehyde present in the structure that allows chemoselective reactions. On the other hand, because the aldehyde can perform reversible conjugation reactions with a variety of chemical groups such as amines, hydrazides, and hydroxylamines forming acid-cleavable moieties like imines, hydrazones, and oximes. This factor enables a freedom of choice over the chemical group for the desired reversible conjugation reaction. Contrary, the alcohols are good candidates for reversible conjugation reaction via the formation of acetals and ketals as well, but lack in chemoselectivity since they are present in a large quantity on the glycosidic structure.

For the formation of an irreversible and stable bond the carboxylic acid is the most suitable since it can form amides and esters with the common tools used in peptide chemistry (e.g. reaction with amines via carbodiimide mediated amide formation).

**Table 9. Functional groups that are available for saponin conjugation reactions**

| **Functional Group** | **Pros** | **Cons** |
|---|---|---|
| **Alcohol (Diols)** | - Suitable for reversible acetal/ketal formation | - Acetal/ketal formation without chemoselectivity |
| | - Suitable for ester formations with activated carboxylic acids | - Ester formation without chemoselectivity |
| **Aldehyde** | - Suitable for chemoselective reversible hydrazone formation with hydrazides | |
| | - Suitable for chemoselective reversible imine formation with amines | - Not suitable for acetal formation in the presence of unprotected saponin sugar diols |
| | - Suitable for chemoselective reversible oxime formation with hydroxylamines | |
| **Alkene** | - Suitable for chemoselective irreversible radical reactions | - Not suitable for reversible conjugation reactions |
| | | - Not suitable for reactions involving a hydrogenation step |
| **Carboxylic acid** | - Suitable for chemoselective amide / ester formation with amines and alcohols after activation | - Not suitable for reversible conjugation reactions under mild conditions |

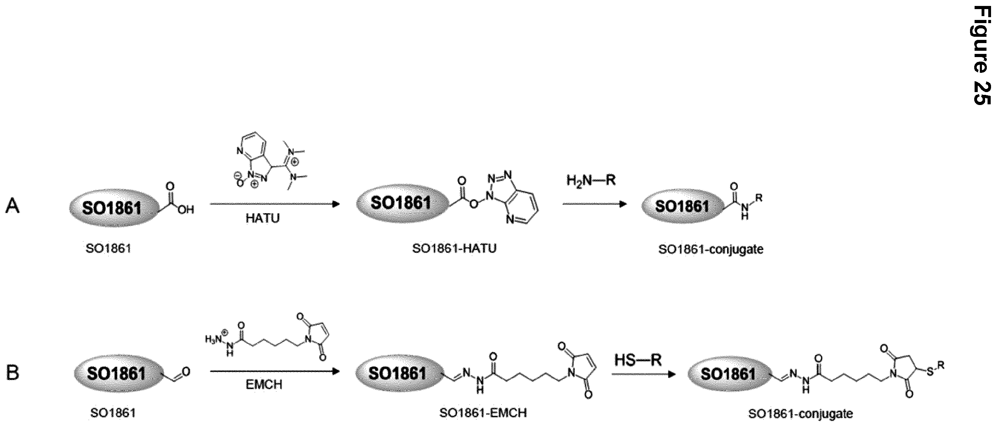

Thus, for the development of an endosomal escape enhancing saponin (such as SO1861) a methodology has been established that allows the generation of a non-cleavable and cleavable 'ready to conjugate' saponins (Figure 25) using the most suitable chemical groups present on SO1861.

For producing non-cleavable 'ready to conjugate' saponins the carboxylic group of SO1861 is activated via a reagent used in peptide coupling chemistry to generate an active ester (e.g. 1-[Bis(dimethylamino)methylene]-1H-1,2,3-triazolo[4,5-b]pyridinium 3-oxide hexafluorophosphate, HATU). The resulting active ester of SO1861 is able to react with amines forming stable amide bonded conjugates (Figure 25 A).

For producing cleavable 'ready to conjugate' saponins the aldehyde group of SO1861 is reacted with an EMCH (ε-maleimidocaproic acid hydrazide) linker. The hydrazide group of EMCH forms an acid cleavable hydrazone bond with the aldehyde of SO1861. At the same time the EMCH linker presents a maleimide group that is thiol (sulfhydryl group) reactive and thus can be conjugated to thiols (Figure 25 B).

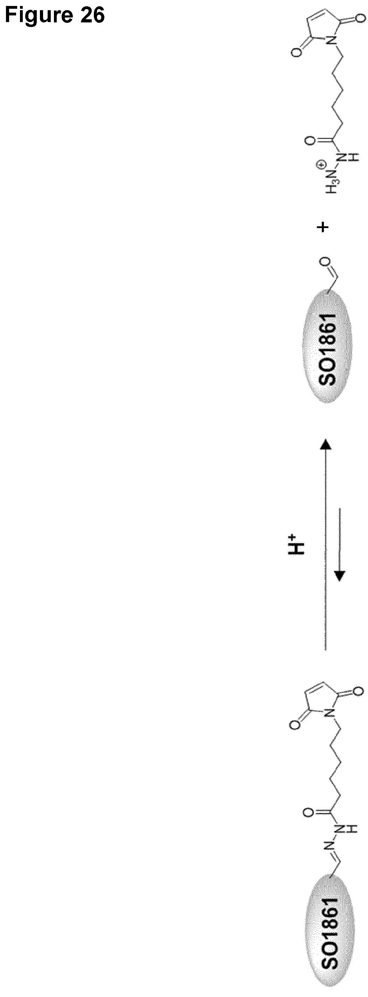

The maleimide group of SO1861-EMCH performs a rapid and specific Michael addition reaction with thiols and thiol bearing polymeric structures when carried out in a pH range of 6.5-7.5 (Figure 25 B). In addition, the acid sensitive hydrazone linkage between the SO1861 and EMCH can be utilized to perform saponin release from a scaffold in acidic environment (Figure 26). Thus, the EMCH linker fulfills both the need for a pH cleavable strategy and a conjugation strategy to a polymeric structure.

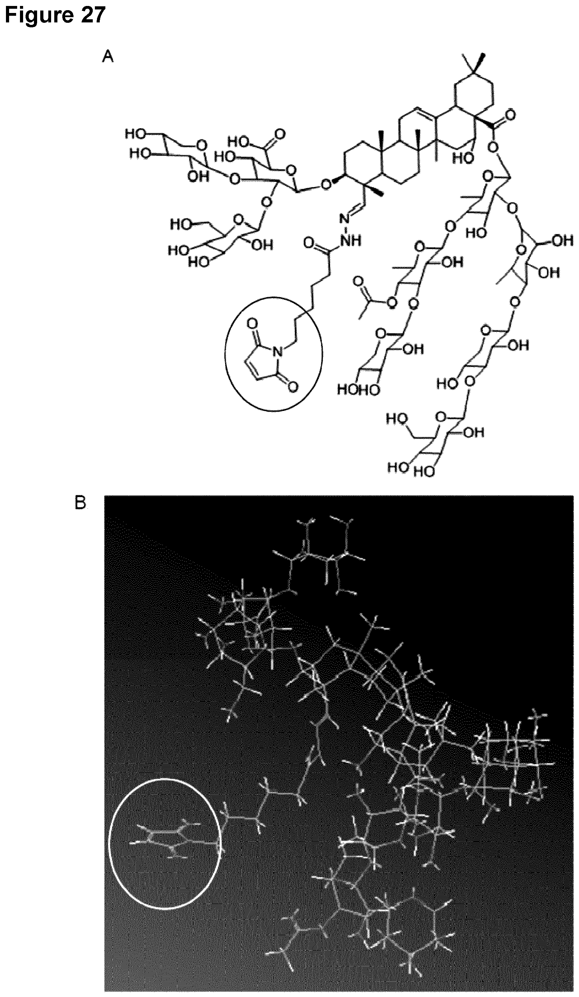

Regarding an ideal EMCH spacer length for conjugation to a polymeric structure, computer simulation (PerkinElmer, ChemBio3D, Ver. 13.0.0.3015) shows that the maleimide group on SO1861-EMCH is located at the periphery of the molecule and thus should be accessible for thiol bearing polymeric structures (Figure 27).

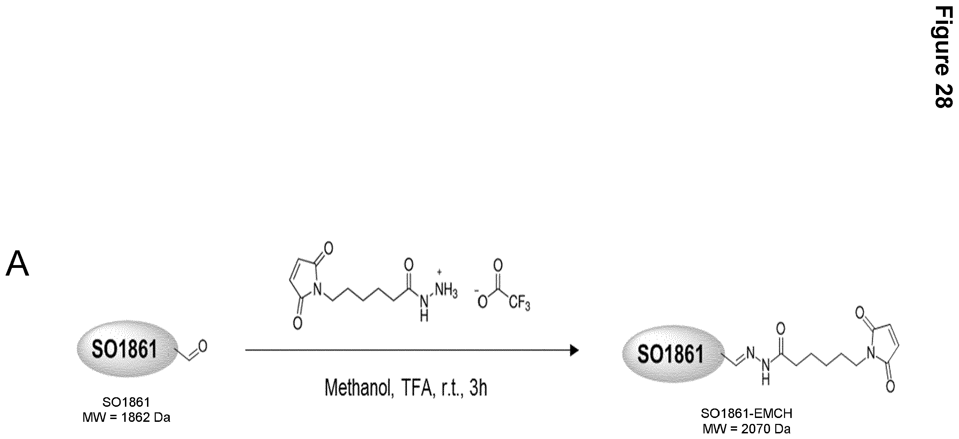

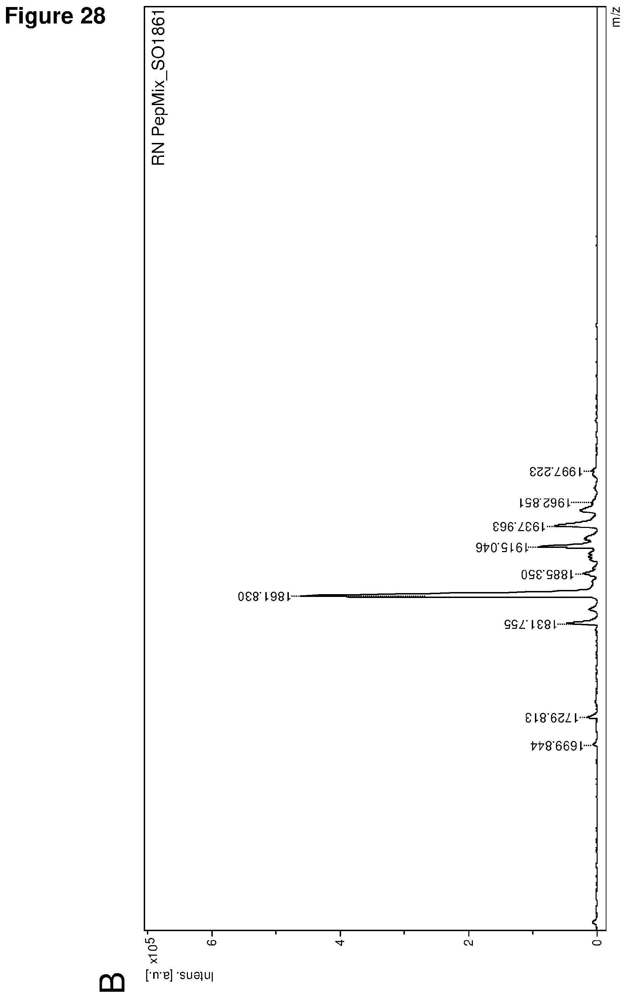

To synthesize the SO1861-EMCH, a strategy has been developed that allows the conversion of the aldehyde group on the SO1861 to EMCH (Figure 28 A). The SO1861-EMCH conjugate was isolated and successfully characterized via nuclear magnetic resonance spectroscopy (see materials and methods section, Figure 22B) and matrix-assisted laser desorption/ionization time-of-flight mass spectrometry (MALDI-TOF-MS) as shown in Figure 28 B and 28 C, and Figure 23 A.

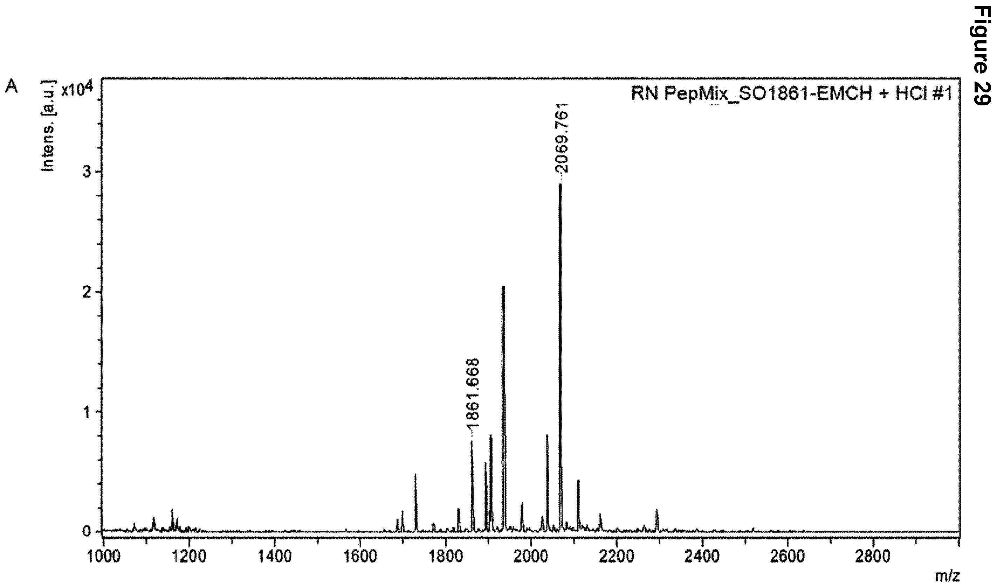

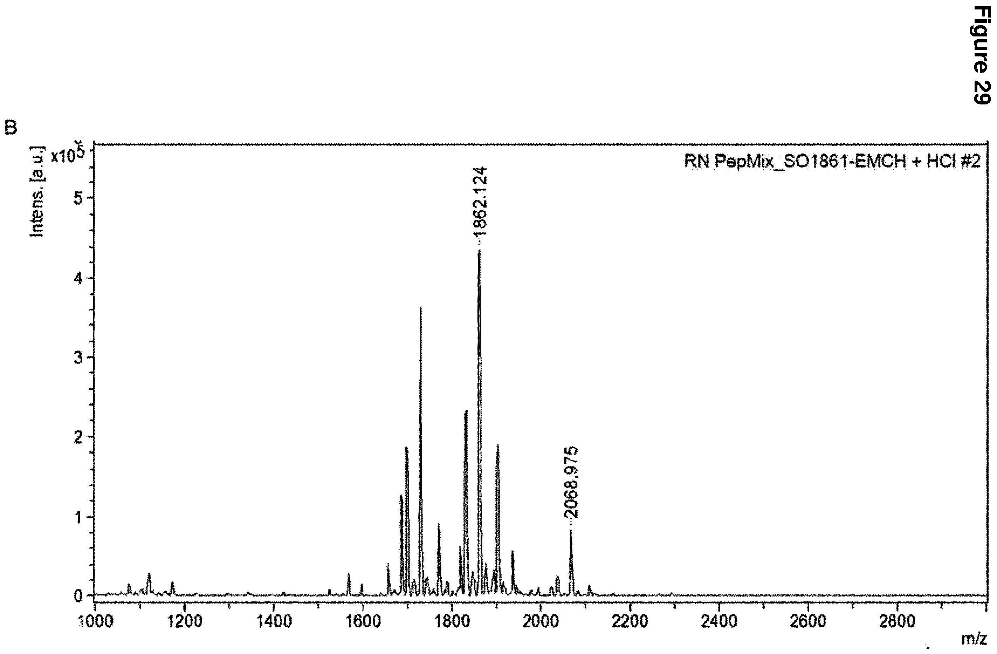

For testing the pH dependent hydrolysis of the hydrazone bond, SO1861-EMCH was dissolved in an HCl solution at pH 3 and MALDI-TOF-MS spectra were recorded at two different points in time (Figure 29). As shown on Figure 29 A and 29 B, a clear decreasing tendency of the peak at m/z 2070 Da that corresponds to SO1861-EMCH is visible in Figure 29 B. Since SO1861 is generated during hydrolysis, an increase of the peak at *m*/*z* 1861 Da was recorded that accompanied the decreasing tendency at *m*/*z* 2070 Da. These results show that the hydrazone bond is responsive towards hydrolysis and gets cleaved even if it is attached on SO1861.

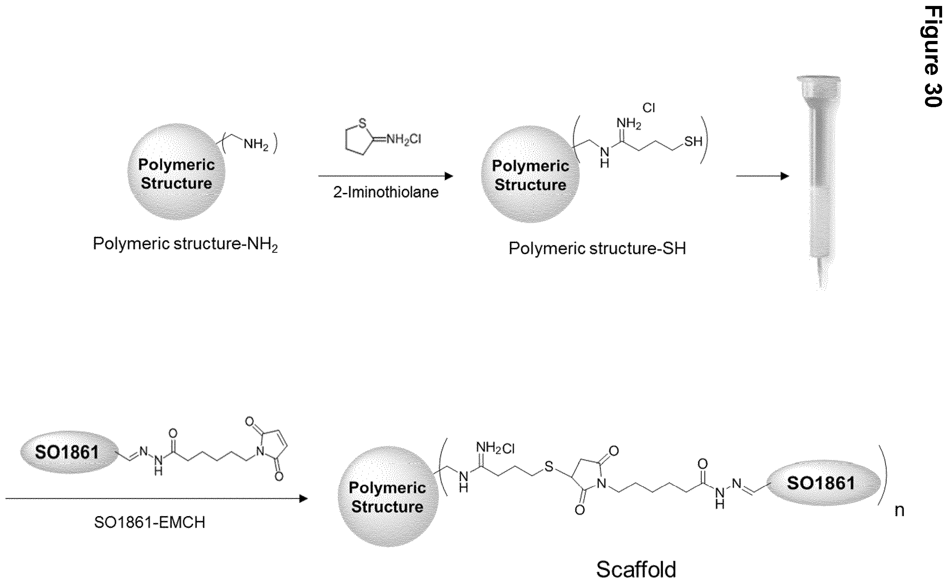

In order to conjugate SO1861-EMCH to a polymeric structure, accessible amines of the polymeric structure are converted into thiols with the aid of the agent 2-iminothiolane. The generated free thiols on the polymeric structure act then as the nucleophile for the thiol-ene Michael-type addition to the maleimide group of SO1861-EMCH (Figure 30). This developed methodology is suitable for the conjugation of SO1861-EMCH to any available polymeric structure that obtains accessible amine groups and allows furthermore a control over the number of conjugated SO1861 molecules depending on the polymeric structure, respectively.

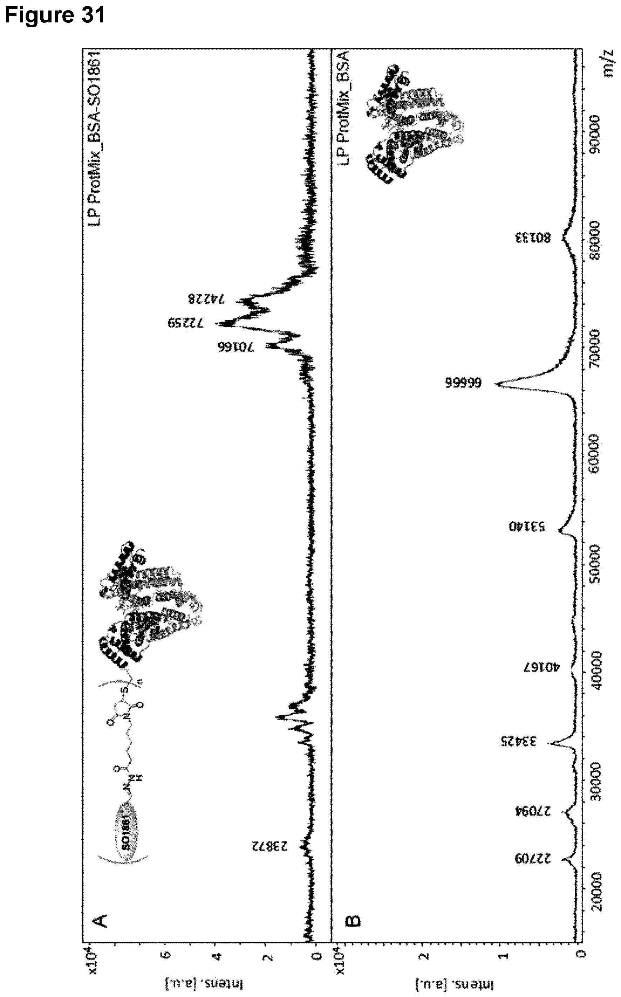

First proof of concept for conjugation of 'ready-to conjugate saponins' to a polymeric structure was obtained by use of the amine of a protein (poly amino acid scaffold example), bovine serum albumin (BSA). After conjugation, mass spectrometry obtained the corresponding peaks of BSA-SO1861 at *m*/*z* ~ 70, ~72, and ~ 74 kDa (Figure 31 A). In comparison with the detected mass of BSA with *m*/*z* 66 kDa (Figure 31 B), the obtained masses of BSA-SO1861 correspond to a mixture of BSA-SO1861 conjugates consisting of 2, 3, and 4 SO1861 molecules per BSA.

Next proof of concept for conjugation of 'ready-to conjugate saponins' to a polymeric structure was obtained by the use of the amine bearing generation 5 (G5) dendrimer poly(amidoamine) (PAMAM with covalently coupled red-fluorescent dye (Cy3)). PAMAM-Cy3 was utilized as the polymeric structure for the conjugation to both SO1861-EMCH and SO1861-HATU and served as a model for conjugation of SO1861 to a polymeric structure (Figure 32).

All accessible amine groups of Cy3-PAMAM were converted into thiols using a 3 fold excess of 2-iminothiolane per Cy3-PAMAM amines followed by the reaction with SO1861-EMCH. Three different feed equivalents (5, 20 and 57) of SO1861-EMCH were used for the three reaction batches. After reaction, the recorded masses of the Cy3-PAMAM-SO1861 conjugates at MALDI-TOF-MS show an increment of the corresponding masses with increasing the SO1861-EMCH feed (Figure 33). The three different feeds corresponded to an obtained mass of *m*/*z* 38.4 kDa, *m*/*z* 53.9 kDa and *m*/*z* 133.8 kDa for the Cy3-PAMAM-SO1861 conjugates that correspond to 6, 13 and 51 SO1861 molecules attached per PAMAM dendrimer as shown on Figure 33 B-D.

In another reaction, only a certain number of PAMAM amines were converted into thiols prior to reaction with SO1861-EMCH. Here, two different feed equivalents of 2-Iminothiolane (8 and 32) and two different feed equivalents of SO1861-EMCH (5 and 30) were used, respectively. After reaction, the respective spectra of the Cy3-PAMAM-SO1861 conjugates at MALDI-TOF-MS show peaks at *m*/*z* 37.7 kDa (5 feed equivalents of SO1861-EMCH) and at *m*/*z* 87.0 kDa (30 feed equivalents of SO1861-EMCH) as shown in Figure 34. The obtained masses at *m*/*z* 37.7 kDa and *m*/*z* 87.0 kDa correspond to Cy3-PAMAM-SO1861 conjugates with 5 and 30 SO1861 molecules attached and demonstrate that with this method almost all feed of SO1861-EMCH were conjugated.

For the generation of a non-pH-cleavable saponin conjugate the carboxylic acid of SO1861 was activated with HATU and then reacted with the amines of Cy3-PAMAM forming a pH stable amide bound between Cy3-PAMAM and SO1861. The resulting mass of the conjugate was detected via MALDI-TOF-MS with a mass of *m*/*z* 62.3 kDa that corresponds to Cy3-PAMAM-NC-SO1861 (NC = non-cleavable) conjugate with 17.5 SO1861 molecules attached per PAMAM (Figure 32 B, Figure 35).

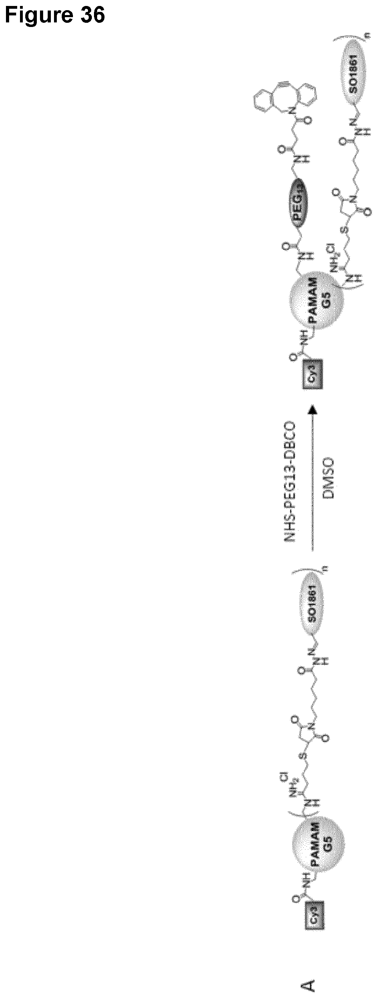

Next, the saponin conjugated scaffolds were conjugated to linking points for a possible conjugation to targeted therapeutics (e.g. targeted toxins) via the so-called strain-promoted alkyne-azide cycloaddition (SPAAC, click chemistry) to obtain a functionalized scaffold. For this reaction, Cy3-PAMAM-SO1861 (Figure 36 C, D) and Cy3-PAMAM-NC-SO1861 (Figure 36 B) were conjugated to a heterobifunctional NHS-PEG₁₃-DBCO linker that generated a strained alkyne on the conjugates' surface (Figure 36 A). The NHS (N Hydroxysuccinimide) moiety of the linker reacted with remaining amines of the PAMAM-saponin conjugates forming an amide bond between the scaffold and the linker. The resulting DBCO (dibenzocyclooctyne) moiety on the conjugates is able to perform SPAAC with corresponding azides on the targeted therapeutics.

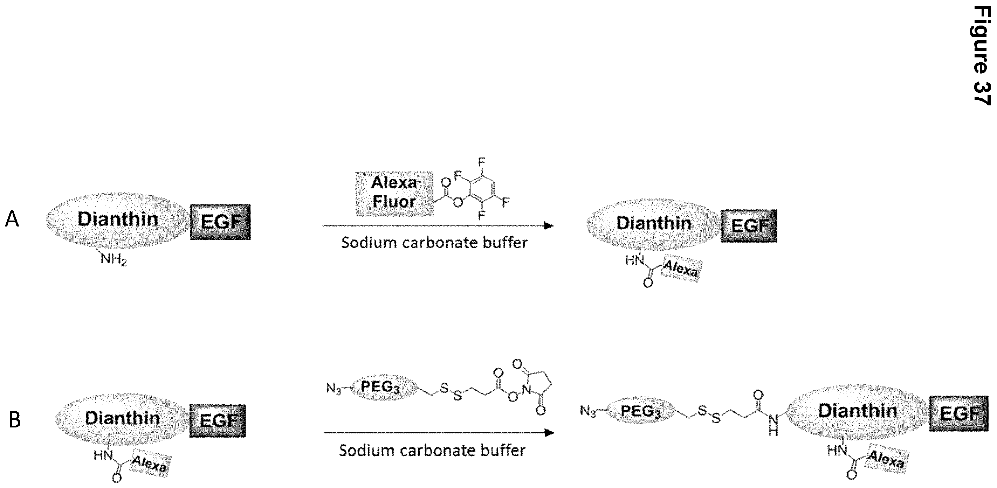

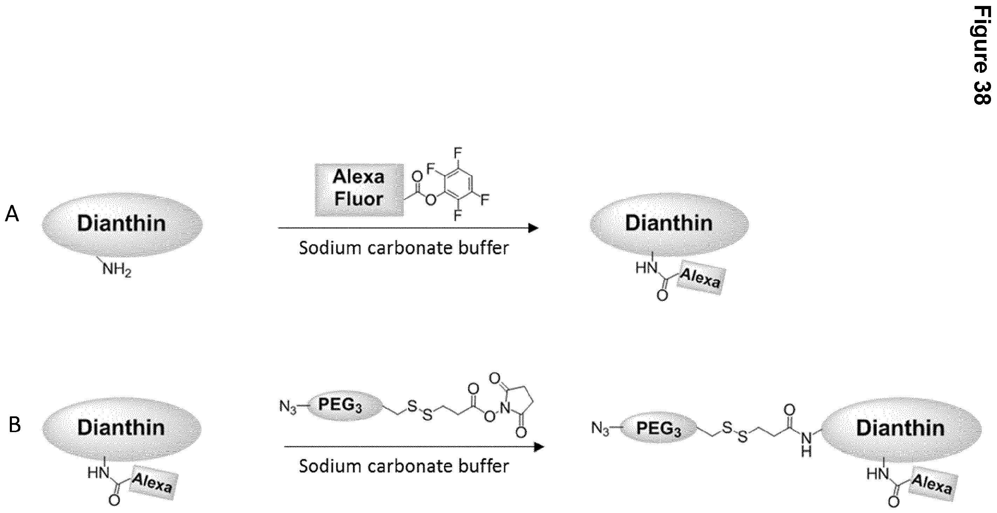

Dianthin-EGF served as a model targeted toxin and dianthin served as a non-targeted toxin. Both toxins were labeled with Alexa Fluor^{™} 488 using the tetrafluorophenyl ester (TFP) derivative of the dye. The dye labeled proteins were then conjugated to a heterobifunctional NHS-SS-PEG₃-azide linker to obtain the corresponding chemical moiety for the SPAAC to the PAMAM-saponin conjugates. Maldi-TOF-MS measurements showed that one Alexa Fluor^{™} 488 dye and 9 NHS-SS-PEG3-azide molecules were attached per dianthin-EGF molecule (Figure 37, Figure 38). Furthermore, Alexa Fluor^{™} 488 labeled dianthin-EGF was conjugated to a heterobifunctional NHS-PEG₁₂-azide linker that lacked the disulfide bond and would thus generate a non-toxin-cleavable construct.

The Cy3-PAMAM-NC-SO1861-DBCO and Cy3-PAMAM-SO1861-DBCO conjugates were reacted with Alexa Fluor^{™} 488 labeled azido-toxins to perform a strain-promoted alkyne-azide cycloaddition. The conjugation between the reacting agents was indicated via gel electrophoresis and the co-localization of the fluorescent signals of Cy3 that is only attached on the PAMAM polymer and Alexa Fluor^{™} 488 that is only attached on the toxins on a polyacrylamide gel after gel electrophoresis (Figure 39).

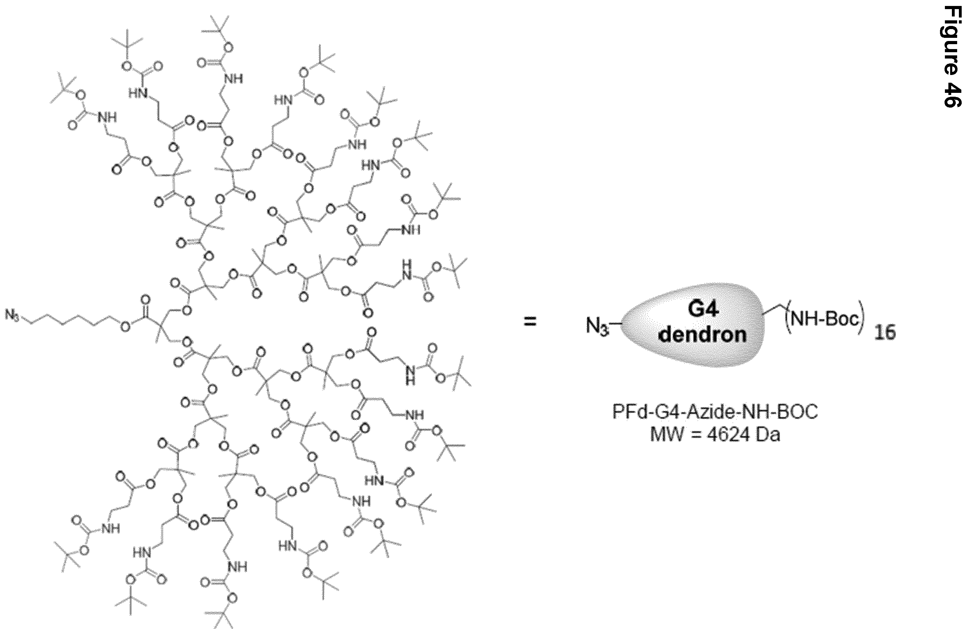

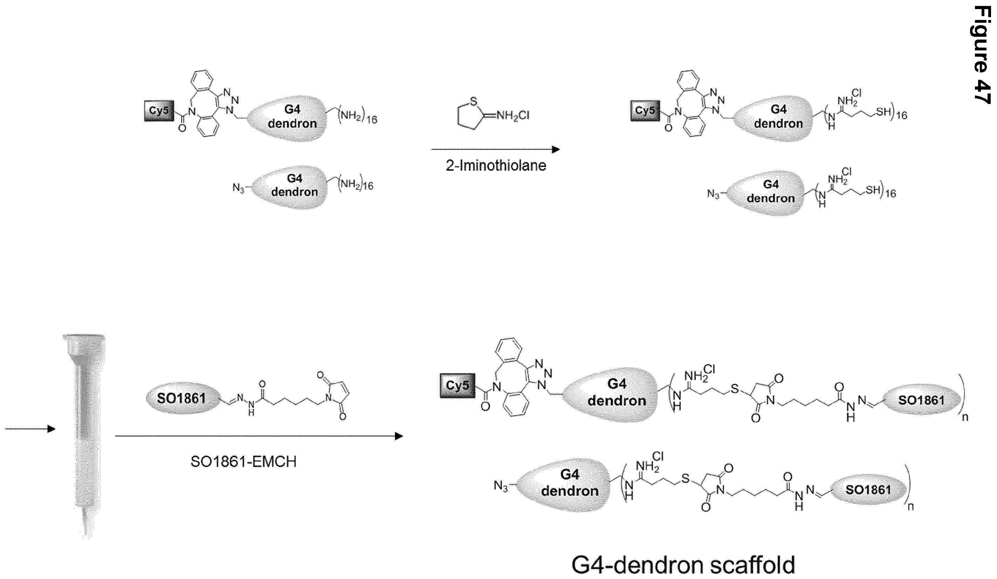

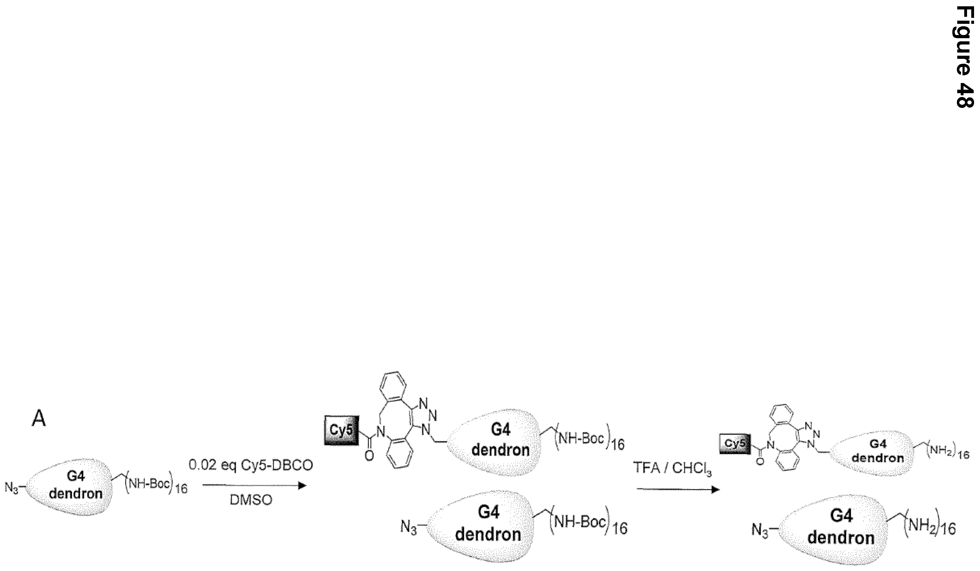

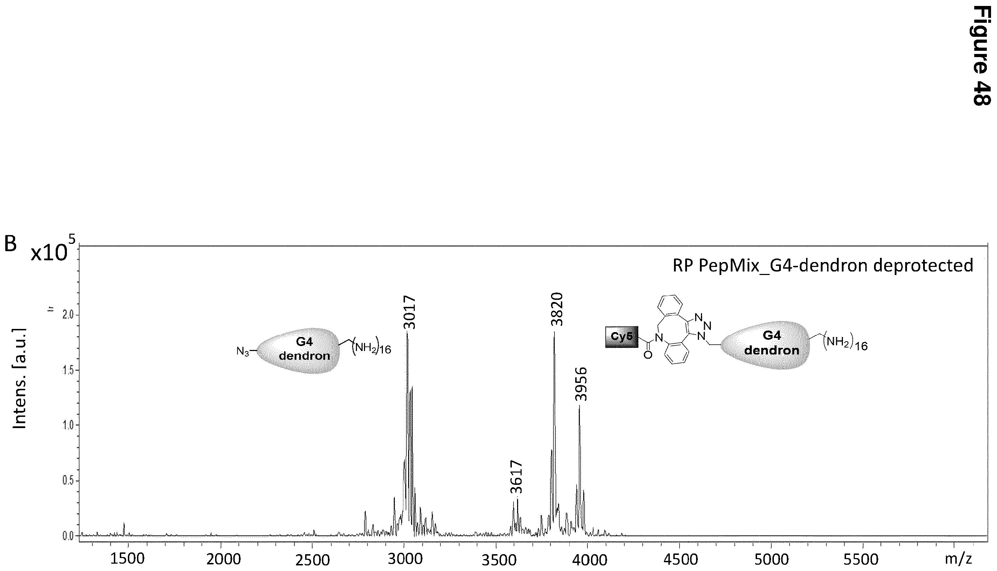

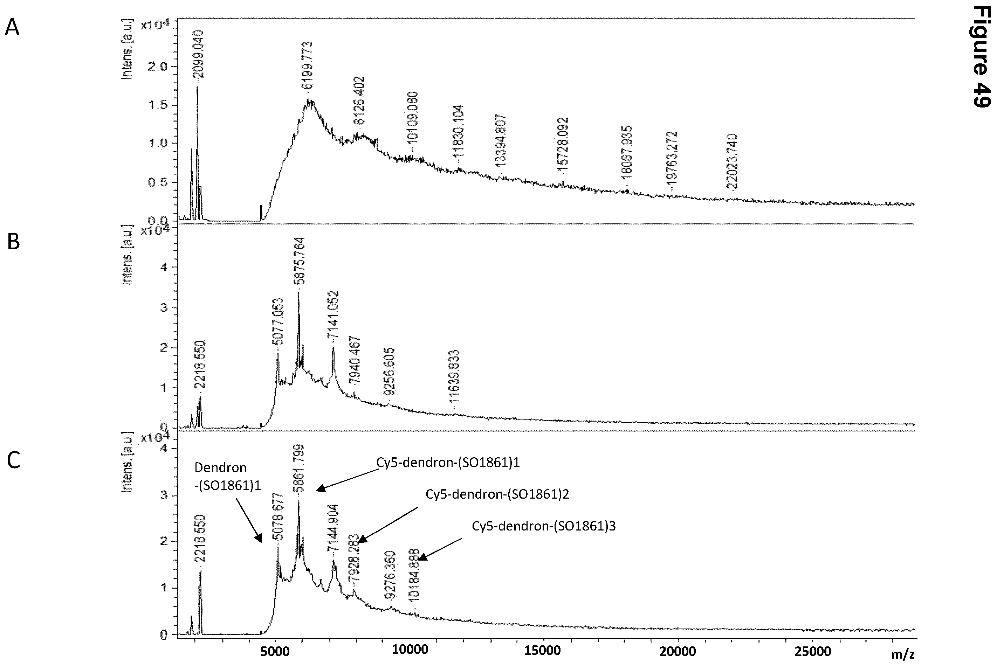

As an alternative polymeric structure to the PAMAM dendrimer, a G4-dendron (PFd-G4-Azide-NH-BOC, Polymer Factory) with 16 functional amino end groups and an azido group at the focal point was utilized for the conjugation to SO1861 (Figure 46). The advantage of using a dendron over a dendrimer is the focal point that the dendron structure is exhibiting. This focal point allows the direct conjugation to a targeted toxin without the need of its post-modification with orthogonal click functions (Figure 47). As shown in Figure 47, the dendron underwent the same methodology as described for the PAMAM dendrimer. Briefly, after partial dye labeling and deprotection (Figure 48), the amino groups of the dendron were converted into thiols using the thiolating reagent 2-iminothiolane followed by conjugation to SO1861-EMCH. For the conjugation to SO1861-EMCH three different feed equivalents of SO1861-EMCH were used. The dendron-SO1861 conjugates were analyzed via MALDI-TOF-MS. As expected, the conjugate species of 1 and 2 SO1861 molecules per dendron molecule were obtained when low SO1861-EMCH feed equivalents of 3 and 10 were used (Figure 49 B, C). Higher dendron-SO1861 conjugate species of up to 9 SO1861 molecules per dendron were obtained (Figure 49 A) when using a feed equivalent of 22 SO1861-EMCH molecules per dendron molecule. In further experiments, the saponin functionalized dendron will be conjugated to targeted toxins over its focal point to yield a functionalized scaffold and will be evaluated biologically.

The previous examples demonstrate that a methodology has been developed that allows the conjugation of a determined amount of SO1861 molecules or other endosomal escape enhancer molecules to a polymeric structure for enhanced cytoplasmic delivery of therapeutic substances such as targeted toxins.

To further test other conjugation methodologies of SO1861 to a polymeric structure, the reductive amination pathway was used. For this, the aldehyde group of SO1861 was directly conjugated to PAMAM amines forming an imine bound. The imine bond formation was followed a reductive amination step through the addition of the reductive agent sodium cyanoborohydride forming a pH-stable amine bond between SO1861 and PAMAM (Figure 40 A). Similar to the EMCH and HATU approach, this methodology allows a control over the number of conjugated saponins per polymer as shown on Figure 40 B, C. Here, PAMAM-saponin conjugates were produced which obtained a number of 10 (Figure 40 B) and 30 (Figure 40 C) SO1861 molecules per PAMAM.

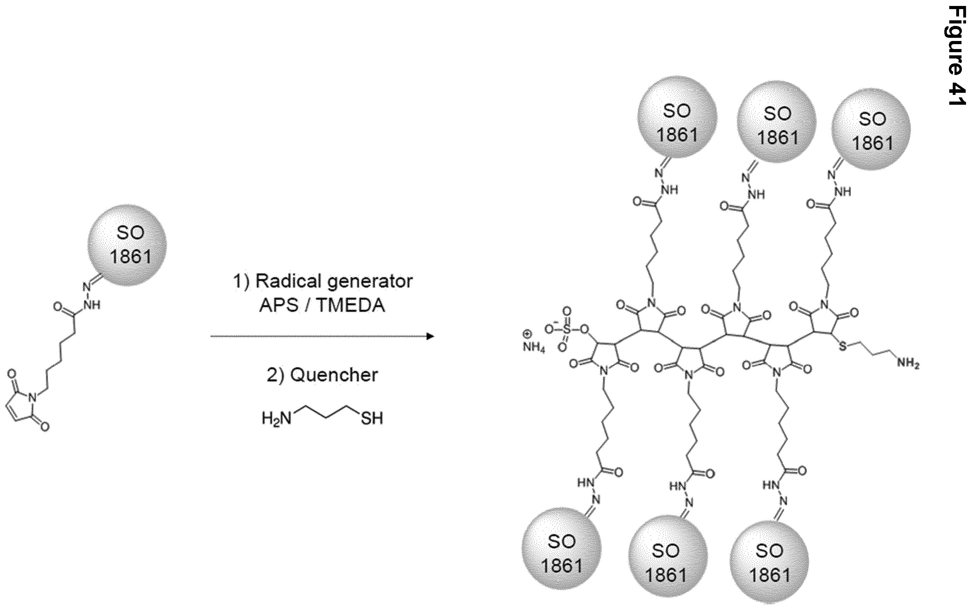

Another approach for the development of a SO1861 scaffold among the discussed polymer, and protein approach is the poly(SO1861) approach. The idea of this approach is to generate a polymer that consists of SO1861 molecules only, with pH sensitive cleavable bonds that release the SO1861. In addition, the poly(SO1861) should be able to perform conjugation reactions to toxins and biopolymers. The main goal with this approach is to keep it as simple and cost effective as possible. Since a protocol for the generation of acid cleavable SO1861 has been developed already (SO1861-EMCH approach) it would be interesting to see if it is possible to polymerize the SO1861-EMCH through simple addition of a polymerization initiator without further modifying the SO1861 or identifying other conjugation sites on the SO1861 molecule. In the past, several papers have discussed the polymerization of maleimide groups by using radical initiators which attack the double bond of the maleimide group and thus initiate a radical polymerization along the double bonds of the maleimides (29-31). Since SO1861-EMCH reveals a maleimide group in its structure this group could potentially be explored for radical polymerization reactions to yield a poly(SO1861) with acid cleavable function. If the polymerization reaction has a reasonable reaction time the generated SO1861 polymers could be quenched with a radical quencher that not only quenches the reaction but also generates a functional group for toxin or biopolymer conjugation. Such a reaction scheme is illustrated in Figure 41. Here, the system of ammonium persulfate (APS) and tetramethylethylenediamine (TMEDA) is shown in an exemplary way as radical generator and aminopropanethiol serves as a model radical quencher. Using aminopropanethiol as a quencher exemplary, the generated amine group could be specifically further modified to a click-able group or being used to directly conjugate the poly(SO1861) to a toxin.

In free radical polymerization the reaction conditions have a huge influence on the polymer properties and the reaction outcome. For instance, temperature, monomer concentration, and initiator concentration play a major role for forming the polymer and have to be fine-tuned according to the desired polymer properties. As radical polymerizations are usually carried out at temperatures above 50 °C, the first reactions have been performed at a temperature of 60 °C. It was interesting to see if the SO1861-EMCH polymerization can be initiated spontaneously and if APS and TMEDA would have an influence on the polymerization degree. Thus, three reactions have been carried out, using the same SO1861-EMCH concentration, but differ in their APS / TMEDA composition. In the first reaction only the SO1861-EMCH was heated up to 60 °C for 3 h, while the second reaction contained SO1861-EMCH and APS, and the third reaction contained SO1861-EMCH, APS, and TMEDA. (For these experiments the same amount and concentration of starting materials have been used which are mentioned in the Materials and Methods section "Poly(SO1861) synthesis"). The batches have been analyzed via MALDI-TOF-MS as shown on Figure 42 A-C. Interestingly it has been shown that SO1861-EMCH started to form oligomers consisting of 2, 3, 4, 5, and 6 SO1861 molecules spontaneously when heated up to 60 °C (Figure 42 A). The addition of 11⁻³ equivalents APS at the same temperature had no influence on this trend (Figure 42 B). When using the initiator system of APS / TMEDA, however, SO1861 oligomers of up to 9 SO1861 molecules with a molecular weight of 18.2 kDa could be detected (Figure 42 C). In addition, the obtained peaks for the oligomers seemed much bigger in comparison with the peaks in Figure 42 A and 42 B, indicating a higher consumption of SO1861-EMCH for this reaction.

To further fine-tune the reaction conditions, other initiators such as azo-initiators like 2,2'-azobis[2-methyl-N-(2-hydroxyethyl)propionamide] and azobisisobutyronitrile will be tested, as well as other polymerization techniques such as controlled radical polymerization (atom-transfer radical-polymerization, reversible addition-fragmentation chain transfer, etc). Moreover, another hydrazide linker as a substitute for EMCH could be considered which obtains a functional group (such as an acryl or acrolyol residue) that is more suitable for radical polymerization than the maleimide group.

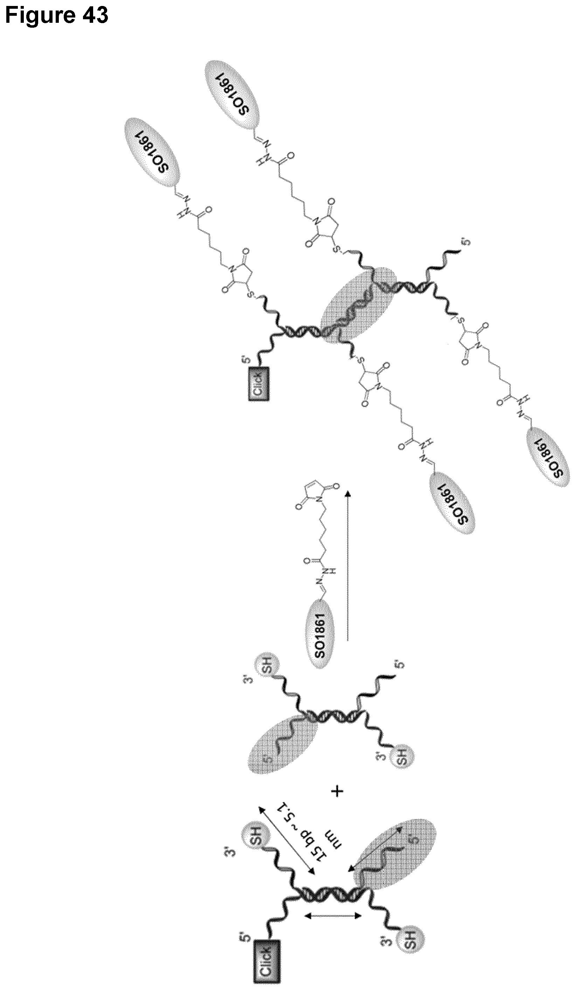

Another approach for the development of a SO1861 scaffold is the DNA approach. The idea of this approach is to utilize the concept of the so-called DNA-origami ( Kolb et al, 2004; Bird et al, 1988). DNA-origami as the polymeric or assembled polymeric structure to conjugate saponins to it, can offer several inherent advantages including stability, scalability, and precise control of the final size and shape of the resulting DNA-saponin scaffold. Since these DNA nanocarriers are comprised of natural DNA, they are biocompatible and do not show toxicity to living cells, and can ease the release of cargo from internal cellular compartments. The multivalency of such a structure can further allow fine-tuning targeting capabilities and high capacity for a variety of payloads such as fluorophores and toxins. Thus, in this approach DNA strands are identified that offer chemical functional groups on the 3' and 5' endings respectively, and that are able to hybridize only in certain wanted areas of the sequence that allow a control over the final shape of the construct. The chemical groups should be utilized to couple saponins, for instance though a thiol-ene reaction between the already developed SO1861-EMCH and a thiol group on one of the 3' and 5' DNA strands. The complementary DNA strand can offer a click function group that can be used for coupling to a targeted toxin. The concept is illustrated in Figure 43.

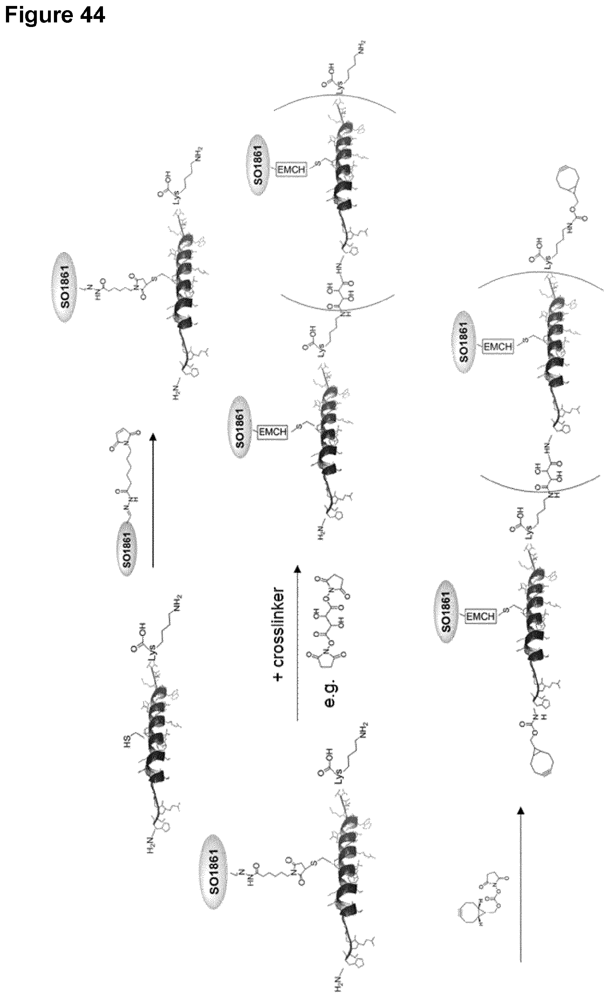

A similar approach is imaginable by using a specific peptide sequence instead of DNA strands that is able to bind and release saponins and that can be polymerized forming a large poly(peptide)-like structure. In this approach, a peptide sequence has been identified and purchased that has a length fitting the calculated size of a SO1861-EMCH molecule, that offers a cysteine residue in the middle of the sequence, and that obtains an amine group at both the N-terminus and C-terminus. The cysteine residue can be utilized to conjugate SO1861-EMCH via a thiol-ene reaction of the maleimide group of SO1861-EMCH and the thiol group of the cysteine residue. The two amine groups can be utilized to polymerize the peptide-SO1861 conjugate with a suitable crosslinker as shown on Figure 44.

Conjugation studies have shown that the conjugation of SO1861-EMCH to the customized peptide (sequence: SESDDAMFCDAMDESDSK) was successful. The peptide that bears a maleimide reactive cysteine in the middle of the sequence and its conjugation to SO1861-EMCH was analyzed via MALDI-TOF-MS (Figure 45). The MALDI-TOF-MS spectra shows the expected peak for the peptide-SO1861 conjugate at *m*/*z* 4053 Da and an additional peak at *m*/*z* 3821 Da which is the peptide-SO1861 conjugate of the corresponding saponin-EMCH of SO1730. As SO1861-EMCH has been used in slight excess (1.3 equivalents) and no SO1861-EMCH peak was detected after reaction, it can be assumed that the conjugation was quantitative. For starting first polymerization reactions of the peptide-SO1861, disuccinimidyl tartrate will be utilized as the amine reactive cross-linker.

### Example 4

### Cell viability assay

HeLa cells were seeded in DMEM (PAN-Biotech GmbH) supplemented with 10% fetal bovine serum (PAN-Biotech GmbH) and 1% penicillin/streptomycin (PAN-Biotech GmbH), in a 96 well plate at 5,000 c/w in 100 µL/well and incubated overnight at 37 °C and 5% CO₂. The next day 20x concentrated stocks of the PAMAM, PAMAM-conjugates, G4-dendron (prepared in Example 3) or chloroquine (Sigma Aldrich) samples were prepared in DMEM. The media was removed from the cell culture plate and replaced by 160 µL culture media, followed by the addition of 10 µL sample/well (from the 20x concentrated stocks) and a 45 min incubation at 37 °C. During this incubation the SO1861 concentration curve was prepared. The SO1861 master stock was heated for 10 min at 50 °C, while shaking at 1,250 rpm. Followed by 15 sec sonication and a brief re-heating at 50 °C for 1 min, while shaking at 1,250 rpm. Subsequently a serial dilution of SO1861 was prepared in PBS. The SO1861 concentration curve was prepared as 10x concentrated stock, from which 20 µL was added/well. After a 15 min incubation at 37 °C, 10 µL dianthin-EGF (prepared in Example 2) diluted in DMEM to 30 pM) or DMEM containing an equal amount of PBS was added/well, to obtain a final dianthin-EGF concentration of 1.5 pM as well as the indicated SO1861 and the different polymeric structures or chloroquine concentrations in a final volume of 200 µL/well.

After treatment the cells were incubated for 72 hr at 37 °C before the cell viability was determined by a MTS-assay, performed according to the manufacturer's instruction (CellTiter 96^{®} AQueous One Solution Cell Proliferation Assay, Promega). Briefly, the MTS solution was diluted 20× in DMEM without phenol red (PAN-Biotech GmbH) supplemented with 10% FBS. The cells were washed once with 200 µL/PBS well, after which 100 µL diluted MTS solution was added/well. The plate was incubated for approximately 20-30 minutes at 37 °C. Subsequently, the OD at 492 nm was measured on a Thermo Scientific Multiskan FC plate reader (Thermo Scientific). For quantification the background signal of 'medium only' wells was subtracted from all other wells, before the cell viability percentage of treated/untreated cells was calculated, by dividing the background corrected signal of treated wells over the background corrected signal of the untreated wells (x 100).

### FACS analysis

HeLa cells were seeded in DMEM (PAN-Biotech GmbH) supplemented with 10% fetal calf serum (PAN-Biotech GmbH) and 1% penicillin/streptomycin (PAN-Biotech GmbH), at 500,000 c/plate in 10 cm dishes and incubated for 48 hrs (5% CO₂, 37 °C), until a confluency of 90% was reached. Next, the cells were trypsinized (TryplE Express, Gibco Thermo Scientific) to single cells. 0.75 x 10⁶ Cells were transferred to a 15 mL falcon tube and centrifuged (1,400 rpm, 3 min). The supernatant was discarded while leaving the cell pellet submerged. The pellet was dissociated by gentle tapping the falcon tube on a vortex shaker and the cells were washed with 4 mL cold PBS (Mg²⁺ and Ca²⁺ free, 2% FBS). After washing the cells were resuspended in 3 mL cold PBS (Mg²⁺ and Ca²⁺ free, 2% FBS) and divided equally over 3 round bottom FACS tubes (1 mL/tube). The cells were centrifuged again and resuspended in 200 µL cold PBS (Mg²⁺ and Ca²⁺ free, 2% FBS) or 200 µL antibody solution; containing 5 µL antibody in 195 µL cold PBS (Mg²⁺ and Ca²⁺ free, 2% FBS). APC Mouse IgG1, κ Isotype Ctrl FC (#400122, Biolegend) was used as isotype control, and APC anti-human EGFR (#352906, Biolegend) was used to stain the EGFR receptor. Samples were incubated for 30 min at 4 °C on a tube roller mixer. Afterwards, the cells were washed 3x with cold PBS (Mg²⁺ and Ca²⁺ free, 2% FBS) and fixated for 20 min at room temperature using a 2% PFA solution in PBS. Cells were washed 2x with cold PBS, and resuspended in 250-350 µL cold PBS for FACS analysis. Samples were analyzed with a BD FACSCanto II flow cytometry system (BD Biosciences) and FlowJo software.

### Results

Previously it has been described that the amino groups in amine containing polymeric structures such as PAMAM and PEI (polyethylenimine) are able to block the acidification of the endosomes via the their intrinsic H⁺ buffering capacity. Since the endosomal escape enhancing properties of SO1861 are only exposed at low endosomal pH (< pH5), the scaffold or functionalized scaffold should not contain chemical groups that are able to interfere in acidification of the endosomes and thus block the activity of SO1861.

The amine containing polymeric structures of a G5 PAMAM (128 primary amines as well as approximately 126 tertiary amines) and G4-dendron (16 primary amines) were tested, in order to determine if these molecules inhibit the endosomal escape enhancing capacity of SO1861. Coadministration experiments of PAMAM (native or thiolated) or dendron (native) in combination with dianthin-EGF and various SO1861 concentrations on HeLa cells were performed. As control for the inhibition of endosomal acidification chloroquine was used.

HeLa cells show sufficient EGFR cell surface levels (Figure 50 A). It is observed that both 'native' PAMAM and chloroquine inhibit the SO1861-mediated endosomal escape of the targeted toxin and subsequent cell killing in Hela cells (Figure 50 B). PAMAM at 500 nM inhibits even to an equal extent as the endosomal acidification inhibitor chloroquine, while 667 nM dendron has no effect at all. To further address if the inhibitory activity of the 'native' PAMAM is due to the availability of amino groups in PAMAM, the primary amino groups of PAMAM were partially thiolated through reaction with 2-iminothiolane (Figure 51), resulting in 16 of 128 (Figure 51 C), 65/128 (Figure 51 D), and 108/128 (Figure 51 E) blocked primary amines. It is observed that thiolation of 65 and 108 primary amines overcomes the inhibition of SO1861-mediated endosomal escape, whereas thiolation of up to 16 amines groups still shows the inhibitory effects of SO1861-mediated endosomal escape of the targeted toxin (Figure 50 C). The primary amino groups of PAMAM were also partially PEGylated through a reaction with mPEG2k-NHS (Figure 52), resulting in 3 of 128 (Figure 52 C), 8/128 (Figure 52 D), and 18/128 (Figure 52 E) blocked primary amines. Blocking only 3 primary amines by PEGylation is already sufficient to reverse the inhibition of SO1861-mediated endosomal escape (Figure 51 D). The shielding effect of PEGylation most likely extends beyond the small number of amines that are converted, as PEGylation is known to introduce a hydration layer that can shield off an entire molecule, if a sufficient level is reached.

These results demonstrate that the presence of a certain number of free amino groups on polymeric structures, such as PAMAM, can block endosomal acidification and thus inhibit the endosomal escape activity of SO1861 or other glycosides. When the number of amino groups is lower, as shown for the G4-dendron, or if the amino groups have been shielded, such as thiolation or PEGylation, the inhibitory effect is reversed.

### REFERENCES

Weng, A.; Thakur, M.; Beceren-Braun, F.; Bachran, D.; Bachran, C.; Riese, S.B.; Jenett-Siems, K.; Gilabert-Oriol, R.; Melzig, M.F.; Fuchs, H. The toxin component of targeted anti-tumor toxins determines their efficacy increase by saponins. Molecular oncology 2012, 6, 323-332. saponinum album in a synergistic way. Journal of immunotherapy 2009, 32, 713-725.
Sama, S.; Jerz, G.; Schmieder, P.; Joseph, J.F.; Melzig, M.F.; Weng, A. Plant derived triterpenes from Gypsophila elegans M.Bieb. enable non-toxic delivery of gene loaded nanoplexes. Journal of Biotechnology 2018, 284, 131-139
Kolb, H.C.; Finn, M.G.; Sharpless, K.B. Click Chemistry: Diverse Chemical Function from a Few Good Reactions. Angewandte Chemie 2001, 40, 2004-2021.
Bird, R.E.; Hardmann, K.D.; Jacobson, J.W.; Johnson, S.; Kaufman, B.M.; Lee, S.M.; Lee, T.; Pope, S.H.; Riordan, G.S.; Whitlow, M. Single-chain antigen-binding proteins. Science 1988, 242, 423-426.
Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333

## Claims

1. Scaffold suitable for covalently binding at least one biologically active molecule to a carrier molecule,
wherein the carrier molecule comprises or consists of any of a proteinaceous molecule, a protein, a peptide, a nucleic acid, an oligonucleotide, a lipid, a fat, a fatty acid, a nanoparticle, and a carbohydrate;
wherein the scaffold consists of a polymeric or oligomeric structure with at least one of said biologically active molecules covalently bound to said polymeric or oligomeric structure, wherein the scaffold further comprises a first chemical group for covalently coupling of the scaffold to the carrier molecule;
wherein the polymeric or oligomeric structure is selected from the group consisting of:
• poly- or oligo(amines), such as polyethylenimine and poly(amidoamine),
• polyethylene glycols,
• poly- or oligo(esters), such as poly(lactids),
• poly(lactams),
• polylactide-co-glycolide copolymers,
• poly- or oligosaccharides, such as cyclodextrin and polydextrose,
• poly- or oligo(amino acids), such as proteins, peptides and polylysine, and
• DNA oligomers or polymers, RNA polymers, stabilized RNA polymers and PNA (peptide nucleic acid) polymers;
wherein the at least one biologically active molecule is a bisdesmosidic triterpene saponin belonging to the type of a 12,13-dehydrooleanane with an aldehyde function in position C-23, and
wherein the aldehyde function in position C-23 is covalently coupled to the polymeric or oligomeric structure via a hydrazone bond.

2. Scaffold according to claim 1, wherein the at least one bisdesmosidic triterpene saponin belonging to the type of a 12,13-dehydrooleanane with an aldehyde function in position C-23 has a molecular mass of 3.000 Dalton or less.

3. Scaffold according to claim 1 or 2, wherein the at least one bisdesmosidic triterpene saponin belonging to the type of a 12,13-dehydrooleanane with an aldehyde function in position C-23 is an amphiphilic molecule.

4. Scaffold according to any one of the claims 1-3, wherein the bisdesmosidic triterpene saponin belonging to the type of a 12,13-dehydrooleanane with an aldehyde function in position C-23 is a single specific molecule or is a mixture of different molecules, when more than one biologically active molecules are covalently bound to the polymeric or oligomeric structure comprised by the scaffold.

5. Scaffold according to any one of the claims 1-4, wherein the at least one bisdesmosidic triterpene saponin belonging to the type of a 12,13-dehydrooleanane with an aldehyde function in position C-23 comprises a glucuronic acid function in a carbohydrate substituent at the C-3beta-OH group of the saponin.

6. Scaffold according to any one of claim 1 - 5, wherein the at least one bisdesmosidic triterpene saponin belonging to the type of a 12,13-dehydrooleanane with an aldehyde function in position C-23 is:
- a saponin that can be isolated from a *Gypsophila* species and/or a *Saponaria* species and/or an *Agrostemma* species and/or a *Quillaja* species such as *Quillaja saponaria,*
or
- a single specific saponin or a mixture of two or more different saponins, such as one or more of the saponins SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, or any of their stereomers and/or any combinations thereof,
or
- SO1861 and/or GE1741 and/or SA1641 and/or QS-21 and/or saponin with a quillaic acid aglycon core, a Gal-(1→2)-[Xyl-(1→3)]-GlcA carbohydrate substituent at the C-3beta-OH group and a Glc-(1→3)-Xyl-(1→4)-Rha-(1→2)-[Xyl-(1→3)-4-OAc-Qui-(1→4)]-Fuc carbohydrate substituent at the C-28-OH group, and/or is 3-O-beta-D-galactopyranosyl-(1→2)-[beta-D-xylopyranosyl-(1→3)]-beta-D-glucuronopyranosyl quillaic acid 28-O-beta-D-glucopyranosyl-(1→3)-beta-D-xylopyranosyl-(1→4)- alpha-L-rhamnopyranosyl-(1→2)-[beta-D-xylopyranosyl-(1→3)-4-OAc-beta-D-quinovopyranosyl-(1→4)]-beta-D-fucopyranoside,
or
- SO1861 and/or QS-21.

7. Scaffold according to any one of claim 1 - 6, wherein:
- the at least one bisdesmosidic triterpene saponin belonging to the type of a 12,13-dehydrooleanane with an aldehyde function in position C-23 has a molecular mass of at least 1.500 Dalton and comprises optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units, optionally containing at least one acetyl residue such as two acetyl residues and/or optionally comprising deoxy carbohydrates and/or optionally comprising quinovose and/or optionally comprising glucose and/or optionally comprising 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(1→2)-Ara/Api-3,5-dihydroxy-6-methyl-octanoyl]-3,5-dihydroxy-6-methyl-octanoic acid bound to a carbohydrate via an ester bond,
or
- the at least one bisdesmosidic triterpene saponin belonging to the type of a 12,13-dehydrooleanane with an aldehyde function in position C-23 is QS-21 or any one or more of QS-21A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, protonated QS1861 (QS1862), Quil-A.

8. Scaffold according to any one of claims 1 - 7, wherein the aldehyde function in position C-23 of the at least one saponin is covalently coupled to linker N-ε-maleimidocaproic acid hydrazide, which linker is covalently coupled via a thio-ether bond to a sulfhydryl group in the polymeric or oligomeric structure of the scaffold, wherein the sulfhydryl group is preferably a sulfhydryl group of a cysteine.

9. Scaffold according to any one of claim 1 - 8, wherein the chemical group for covalently coupling of the scaffold to the carrier molecule is a click chemistry group, preferably a tetrazine, an azide, an alkene or an alkyne, or a cyclic derivative of any of these groups, more prefererably an azide.

10. Scaffold according to any one of claim 1 - 9, wherein the scaffold is a tri-functional linker comprising a second chemical group with at least one bisdesmosidic triterpene saponin belonging to the type of a 12,13-dehydrooleanane with an aldehyde function in position C-23 covalently bound thereto, comprising a third chemical group for covalent binding to a molecule and comprising the first chemical group for covalent binding to the carrier,
and/or
wherein the at least one bisdesmosidic triterpene saponin belonging to the type of a 12,13-dehydrooleanane with an aldehyde function in position C-23 is a defined number of glycoside molecules or a defined range of glycoside molecules, preferably a defined number of glycoside molecules or a defined range of glycoside molecules selected from 1-128 or at least 2, 3, 4, 5, 6, 8, 10, 16, 32, 64 or 128 glycoside molecules, or any number of glycoside molecules therein between.

11. Scaffold according to any one of claims 1 - 10, wherein the polymeric or oligomeric structure is selected from the group consisting of:
• poly- or oligo(amines), such as polyethylenimine and poly(amidoamine),
• polyethylene glycols,
• poly- or oligo(esters), such as poly(lactids), and
• poly- or oligo(amino acids), such as proteins, peptides and polylysine.

12. Scaffold according to any one of claims 1 - 11, wherein the polymeric or oligomeric structure is selected from the group consisting of:
• a pentavalent polyethylene glycol-based dendrimer of the following structure: 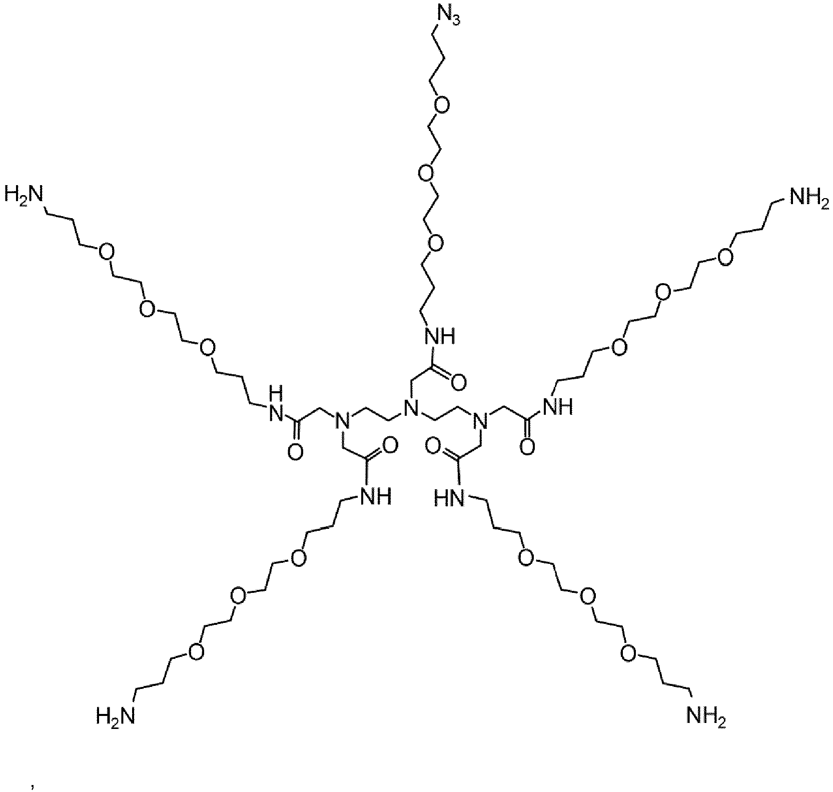
• a generation 5 (G5) poly(amidoamine) dendrimer having an ethylenediamine core which has been derivatised with 2-iminothiolane,
• a polyester dendron with 16 functional amino end groups and an azido group at the focal point, which in its NH-BOC protected form corresponds to the following structure: 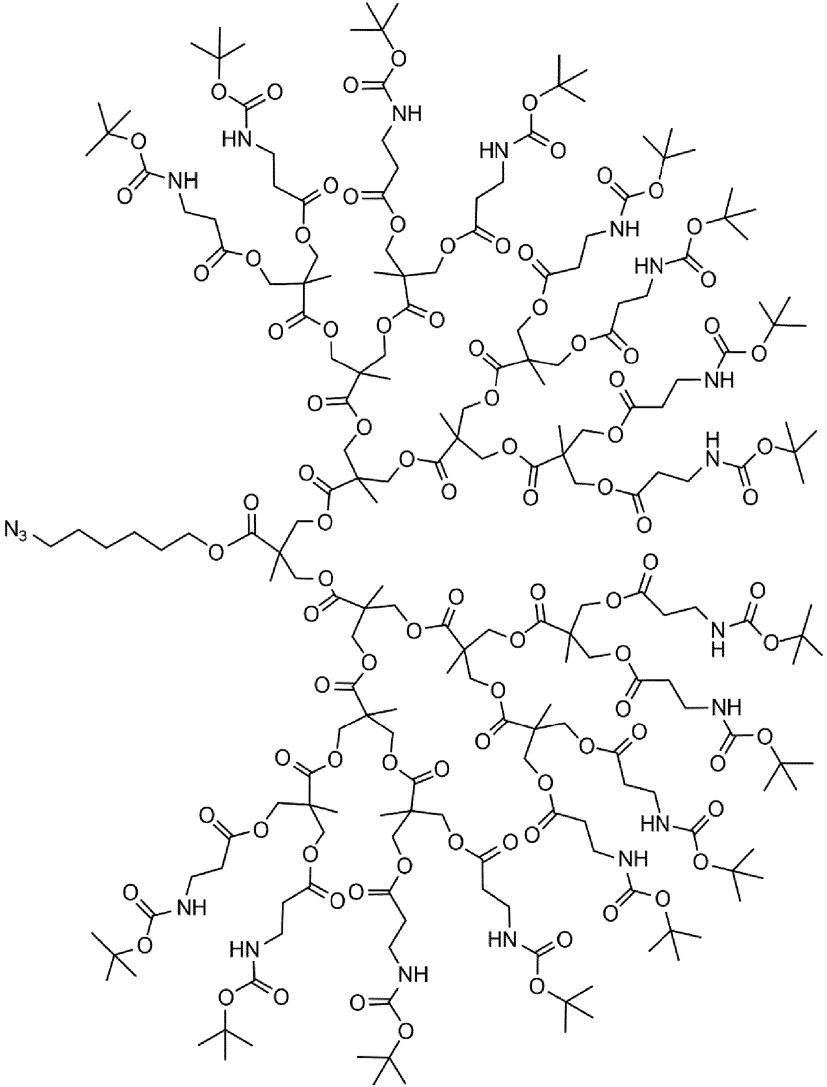 , and which has been derivatised with 2-iminothiolane,
• bovine serum albumin (BSA), and
• a peptide with the sequence SESDDAMFCDAMDESDSK.

13. Scaffold according to any one of the claims 1-12, wherein the carrier molecule comprises or consists of an immunoglobulin, at least one binding domain of an immunoglobulin and/or at least one binding fragment of an immunoglobulin, or comprises or consists of at least one non-proteinaceous ligand and/or at least one proteinaceous ligand for binding to a cell-surface molecule such as EGF or a cytokine,
or
wherein the carrier molecule comprises or consists of an antibody, an IgG, a molecule comprising or consisting of a Vhh domain or Vh domain, a Fab, an scFv, an Fv, a dAb, an F(ab)₂, Fcab fragment.

14. Scaffold according to any one of the claims 1-13, wherein the carrier molecule comprises or consists of at least one binding domain and/or at least one binding fragment for binding to a cell-surface receptor such as a tumor-cell specific cell-surface receptor selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably selected from CD71, EGFR, HER2.

15. Scaffold according to any one of the claims 1-14, wherein the carrier molecule comprises or consists of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, or at least one tumor-cell receptor binding-fragment thereof and/or at least one tumor-cell receptor binding-domain thereof,
or
wherein the carrier molecule comprises or consists of any one of cetuximab or trastuzumab or OKT-9, or at least one tumor-cell receptor binding-fragment thereof, and/or at least one tumor-cell receptor binding-domain thereof, such as at least one tumor-cell specific receptor binding-fragment thereof and/or at least one tumor-cell specific receptor binding-domain thereof.

16. Scaffold according to any one of the claims 1-15, wherein the scaffold is suitable for forming a covalent bond with the carrier molecule, said covalent bond preferably involving a cysteine side-chain of the carrier molecule and/or a lysine side-chain of the carrier molecule when the carrier molecule comprises at least a cysteine and/or a lysine.

17. Scaffold according to any one of claims 1-16, wherein the carrier molecule comprises or consists of at least one effector molecule, or wherein the carrier further comprises at least one effector molecule when dependent on any of claims 13-16, wherein the effector molecule is at least one of an active pharmaceutical substance, such as any one or more of a payload, a toxin, a drug, a polypeptide, an oligonucleotide, a nucleic acid, a xeno nucleic acid, an enzyme such as urease and Cre-recombinase, a protein toxin, and a ribosome-inactivating protein,
and
wherein the protein toxin preferably comprises or consists of any one or more of a protein toxin selected from Table A5 and/or a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin,
and/or wherein the oligonucleotide, the xeno nucleic acid or the nucleic acid preferably comprises or consists of any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, preferably a BNA, for example a BNA for silencing HSP27 protein expression,
and/or
wherein the effector molecule preferably comprises or consists of at least one payload, preferably selected from any one or more of a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

18. Scaffold according to any one of claims 1-16, wherein the carrier molecule comprises or consists of a covalently linked combination of an effector molecule and a monoclonal antibody, preferably selected from Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3.

19. Method for producing a scaffold suitable for covalently binding at least one bisdesmosidic triterpene saponin belonging to the type of a 12,13-dehydrooleanane with an aldehyde function in position C-23 to a carrier molecule, the method comprising:
a) providing a polymeric or oligomeric structure comprising a first chemical group for covalently coupling of the polymeric structure or the oligomeric structure to the carrier molecule and comprising at least one of a second chemical group different from the first chemical group, wherein each second chemical group is for covalently coupling one of the at least one bisdesmosidic triterpene saponins belonging to the type of a 12,13-dehydrooleanane with an aldehyde function in position C-23 to the oligomeric or polymeric structure; and
b) covalently coupling at least one bisdesmosidic triterpene saponin belonging to the type of a 12,13-dehydrooleanane with an aldehyde function in position C-23 to said polymeric or oligomeric structure via the second chemical group(s), wherein the bisdesmosidic triterpene saponin(s) belonging to the type of a 12,13-dehydrooleanane with an aldehyde function in position C-23 is/are any one of the bisdesmosidic triterpene saponins belonging to the type of a 12,13-dehydrooleanane with an aldehyde function in position C-23 of claims 1-7, more preferably SO1861 and/or GE1741 and/or SA1641 and/or QS-21,
wherein the polymeric or oligomeric structure is selected from the group consisting of:
• poly- or oligo(amines), such as polyethylenimine and poly(amidoamine),
• polyethylene glycols,
• poly- or oligo(esters), such as poly(lactids),
• poly(lactams),
• polylactide-co-glycolide copolymers,
• poly- or oligosaccharides, such as cyclodextrin and polydextrose,
• poly- or oligo(amino acids), such as proteins, peptides and polylysine, and
• DNA oligomers or polymers, RNA polymers, stabilized RNA polymers and PNA (peptide nucleic acid) polymers; and
wherein the aldehyde function in position C-23 is covalently coupled to the polymeric or oligomeric structure via a hydrazone bond,
therewith providing the scaffold.

20. Method for producing a scaffold covalently bound to a carrier molecule, the scaffold comprising at least one covalently bound bisdesmosidic triterpene saponin belonging to the type of a 12,13-dehydrooleanane with an aldehyde function in position C-23, the method comprising:
a) providing a scaffold comprising at least one bisdesmosidic triterpene saponin belonging to the type of a 12,13-dehydrooleanane with an aldehyde function in position C-23 covalently bound to a polymeric or oligomeric structure in said scaffold, preferably providing a scaffold according to any one of claims 1 - 18 or the scaffold obtainable by the method of claim 19 or the scaffold obtained with the method of claim 19; and
b) covalently coupling the scaffold of a) to a carrier molecule according to any one of the claims -13-18,
therewith providing the scaffold covalently bound to a carrier molecule, the scaffold comprising at least one covalently bound bisdesmosidic triterpene saponin belonging to the type of a 12,13-dehydrooleanane with an aldehyde function in position C-23,
wherein the polymeric or oligomeric structure is selected from the group consisting of:
• poly- or oligo(amines), such as polyethylenimine and poly(amidoamine),
• polyethylene glycols,
• poly- or oligo(esters), such as poly(lactids),
• poly(lactams),
• polylactide-co-glycolide copolymers,
• poly- or oligosaccharides, such as cyclodextrin and polydextrose,
• poly- or oligo(amino acids), such as proteins, peptides and polylysine, and
• DNA oligomers or polymers, RNA polymers, stabilized RNA polymers and PNA (peptide nucleic acid) polymers; and
wherein the aldehyde function in position C-23 is covalently coupled to the polymeric or oligomeric structure via a hydrazone bond.

## Patentansprüche

1. Gerüst, das zur kovalenten Bindung mindestens eines biologisch aktiven Moleküls an ein Trägermolekül geeignet ist,
wobei das Trägermolekül ein beliebiges von einem proteinhaltigen Molekül, einem Protein, einem Peptid, einer Nukleinsäure, einem Oligonukleotid, einem Lipid, einem Fett, einer Fettsäure, einem Nanopartikel und einem Kohlenhydrat umfasst oder daraus besteht;
wobei das Gerüst aus einer polymeren oder oligomeren Struktur besteht, wobei mindestens eines der genannten biologisch aktiven Moleküle kovalent an die polymere oder oligomere Struktur gebunden ist, wobei das Gerüst weiter eine erste chemische Gruppe zum kovalenten Koppeln des Gerüsts an das Trägermolekül umfasst;
wobei die polymere oder oligomere Struktur ausgewählt ist aus der Gruppe bestehend aus:
• Poly- oder Oligo(aminen), wie Polyethylenimin und Poly(amidoamin),
• Polyethylenglykolen,
• Poly- oder Oligo(estern), wie Poly(lactiden),
• Poly(lactamen),
• Polylactid-co-Glycolid-Copolymeren,
• Poly- oder Oligosacchariden, wie Cyclodextrin und Polydextrose,
• Poly- oder Oligo(aminosäuren), wie Proteinen, Peptiden und Polylysin, und
• DNA-Oligomeren oder -Polymeren, RNA-Polymeren, stabilisierten RNA-Polymeren und PNA (Peptid-Nukleinsäure)-Polymeren;
wobei das mindestens eine biologisch aktive Molekül ein bisdesmosidisches Triterpensaponin ist, das zum Typ eines 12,13-Dehydrooleanans mit einer Aldehydfunktion in Position C-23 gehört, und
wobei die Aldehydfunktion in Position C-23 über eine Hydrazonbindung kovalent an die polymere oder oligomere Struktur gekoppelt ist.

2. Gerüst nach Anspruch 1, wobei das mindestens eine bisdesmosidische Triterpensaponin, das zum Typ eines 12,13-Dehydrooleanans mit einer Aldehydfunktion in Position C-23 gehört, eine Molekülmasse von 3.000 Dalton oder weniger aufweist.

3. Gerüst nach Anspruch 1 oder 2, wobei das mindestens eine bisdesmosidische Triterpensaponin, das zum Typ eines 12,13-Dehydrooleanans mit einer Aldehydfunktion in Position C-23 gehört, ein amphiphiles Molekül ist.

4. Gerüst nach einem der Ansprüche 1 bis 3, wobei das bisdesmosidische Triterpensaponin, das zum Typ eines 12,13-Dehydrooleanans mit einer Aldehydfunktion in Position C-23 gehört, ein einzelnes spezifisches Molekül ist oder ein Gemisch aus verschiedenen Molekülen ist, wenn mehr als ein biologisch aktives Molekül kovalent an die von dem Gerüst umfasste polymere oder oligomere Struktur gebunden ist.

5. Gerüst nach einem der Ansprüche 1-4, wobei das mindestens eine bisdesmosidische Triterpensaponin, das zum Typ eines 12,13-Dehydrooleanans mit einer Aldehydfunktion in Position C-23 gehört, eine Glucuronsäurefunktion in einem Kohlenhydratsubstituenten an der C-3beta-OH-Gruppe des Saponins umfasst.

6. Gerüst nach einem der Ansprüche 1-5, wobei das mindestens eine bisdesmosidische Triterpensaponin, das zum Typ eines 12,13-Dehydrooleanans mit einer Aldehydfunktion in Position C-23 gehört, Folgendes ist:
- ein Saponin, das aus einer Gypsophila-Spezies und/oder einer *Saponaria-* Spezies und/oder einer *Agrostemma-* Spezies und/oder einer Quillaja-Spezies wie *Quillaja saponaria* isoliert werden kann,
oder
- ein einzelnes spezifisches Saponin oder ein Gemisch aus zwei oder mehreren verschiedenen Saponinen, wie beispielsweise eines oder mehrere der
Saponine SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, Gypsosid A, AG1, AG2, SO1542, SO1584, SO1658, SO1674, SO1832 oder eines ihrer Stereomere und/oder beliebige Kombinationen davon,
oder
- SO1861 und/oder GE1741 und/oder SA1641 und/oder QS-21 und/oder Saponin mit einem Quillajasäure-Aglykonkern, einem Gal-(1→2)-[Xyl-(1→3)]-GlcA-Kohlenhydratsubstituenten an der C-3beta-OH-Gruppe und einem Glc-(1→3)-Xyl-(1→4)-Rha-(1→2)-[Xyl-(1→3)-4-OAc-Qui-(1→4)]-Fuc-Kohlenhydratsubstituenten an der C-28-OH-Gruppe, und/oder 3-O-beta-D-galactopyranosyl-(1→2)-[beta-D-xylopyranosyl-(1→3)]-beta-D-Glucuronopyranosylquillaja-säure 28-O-beta-D-glucopyranosyl-(1→3)-beta-D-xylopyranosyl-(1→4)-alpha-L-rhamno-pyranosyl-(1→2)-[beta-D-xylopyranosyl-(1→3)-4-OAc-beta-D-quinovopyranosyl-(1→4)]-beta-D-fucopyranosid ist,
oder
- SO1861 und/oder QS-21.

7. Gerüst nach einem der Ansprüche 1-6, wobei:
- das mindestens eine bisdesmosidische Triterpensaponin, das zum Typ eines 12,13-Dehydrooleanans mit einer Aldehydfunktion in Position C-23 gehört, eine Molekülmasse von mindestens 1.500 Dalton aufweist und optional eine Hydroxylgruppe an der Position C-16 umfasst, mit einer ersten verzweigten Kohlenhydrat-Seitenkette an der C-3-Position, wobei die erste verzweigte Kohlenhydrat-Seitenkette optional Glucuronsäure enthält, wobei das Saponin eine Estergruppe mit einer zweiten verzweigten Kohlenhydrat-Seitenkette an der C-28-Position enthält, wobei die zweite verzweigte Kohlenhydratkette vorzugsweise mindestens vier Kohlenhydrateinheiten umfasst, optional mindestens einen Acetylrest, wie beispielsweise zwei Acetylreste, enthält und/oder optional Desoxy-Kohlenhydrate umfasst und/oder optional Chinovose umfasst und/oder optional Glucose umfasst und/oder optional 4-Methoxyzimtsäure umfasst und/oder optional 5-O-[5-O-Ara/Api-3,5-dihydroxy-6-methyl-octanoyl]-3,5-dihydroxy-6-methyl-octansäure umfasst und/oder optional 5-O-[5-O-Rha-(1→2)-Ara/Api-3,5-dihydroxy-6-methyl-octanoyl]-3,5-dihydroxy-6-methyl-octansäure, über eine Esterbindung gebunden an ein Kohlenhydrat, umfasst,
oder
- das mindestens eine bisdesmosidische Triterpensaponin, das zum Typ eines 12,13-Dehydrooleanans mit einer Aldehydfunktion in Position C-23 gehört, QS-21 oder eines oder mehrere von QS-21 A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, protoniertem QS1861 (QS1862), Quil-A ist.

8. Gerüst nach einem der Ansprüche 1-7, wobei die Aldehydfunktion in Position C-23 des mindestens einen Saponins kovalent an den Linker N-ε-Maleimidocapronsäurehydrazid gekoppelt ist, wobei der Linker über eine Thioetherbindung kovalent an eine Sulfhydryl-gruppe in der polymeren oder oligomeren Struktur des Gerüsts gekoppelt ist, wobei die Sulfhydrylgruppe vorzugsweise eine Sulfhydrylgruppe eines Cysteins ist.

9. Gerüst nach einem der Ansprüche 1-8, wobei die chemische Gruppe zur kovalenten Kopplung des Gerüsts an das Trägermolekül eine Click-Chemie-Gruppe, vorzugsweise ein Tetrazin, ein Azid, ein Alken oder ein Alkin oder ein cyclisches Derivat einer dieser Gruppen ist, insbesondere ein Azid.

10. Gerüst nach einem der Ansprüche 1-9, wobei das Gerüst ein trifunktioneller Linker ist, umfassend eine zweite chemische Gruppe mit mindestens einem bisdesmosidischen Triterpensaponin, das zum Typ eines 12,13-Dehydrooleanans mit einer in Position C-23 kovalent daran gebundenen Aldehydfunktion gehört, umfassend eine dritte chemische Gruppe zur kovalenten Bindung an ein Molekül und umfassend die erste chemische Gruppe zur kovalenten Bindung an den Träger,
und/oder
wobei das mindestens eine bisdesmosidische Triterpensaponin, das zum Typ eines 12,13-Dehydrooleanans mit einer Aldehydfunktion in Position C-23 gehört, eine definierte Anzahl von Glykosidmolekülen oder ein definierter Bereich von Glykosidmolekülen ist, vorzugsweise eine definierte Anzahl von Glykosidmolekülen oder ein definierter Bereich von Glykosid-molekülen, ausgewählt aus 1-128 oder mindestens 2, 3, 4, 5, 6, 8, 10, 16, 32, 64 oder 128 Glykosidmolekülen, oder eine beliebige Anzahl von Glykosidmolekülen dazwischen ist.

11. Gerüst nach einem der Ansprüche 1-10, wobei die polymere oder oligomere Struktur ausgewählt ist aus der Gruppe bestehend aus:
• Poly- oder Oligo(aminen), wie Polyethylenimin und Poly(amidoamin),
• Polyethylenglykolen,
• Poly- oder Oligo(estern), wie Poly(lactiden), und
• Poly- oder Oligo(aminosäuren), wie Proteinen, Peptiden und Polylysin.

12. Gerüst nach einem der Ansprüche 1-11, wobei die polymere oder oligomere Struktur ausgewählt ist aus der Gruppe bestehend aus:
• einem fünfwertigen Dendrimer auf Basis von Polyethylenglykol mit der folgenden Struktur: 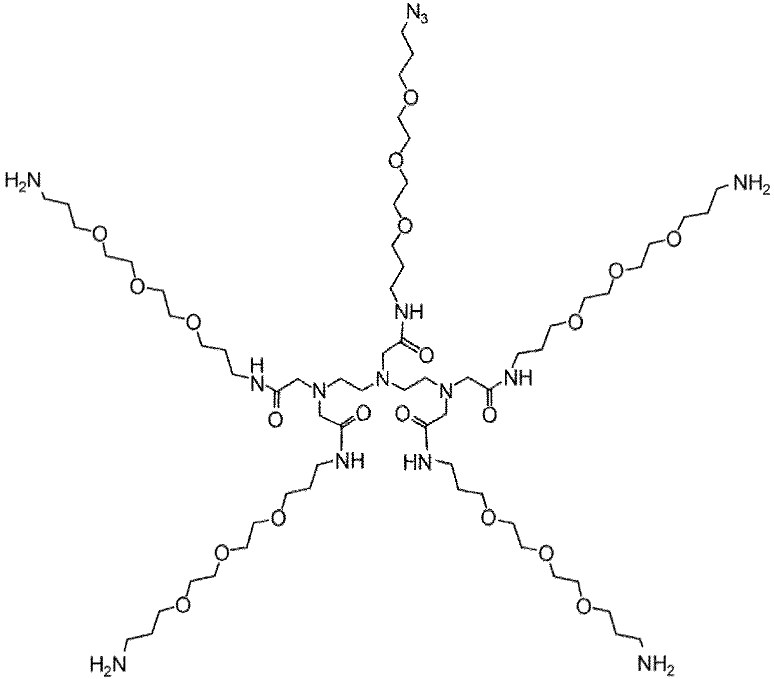
• einem Poly(amidoamin)-Dendrimer der Generation 5 (G5) mit einem Ethylendiamin-Kern, das mit 2-Iminothiolan derivatisiert wurde,
• einem Polyester-Dendron mit 16 funktionellen Aminoendgruppen und einer Azidogruppe im Zentrum, das in seiner NH-BOC-geschützten Form der folgenden Struktur entspricht: 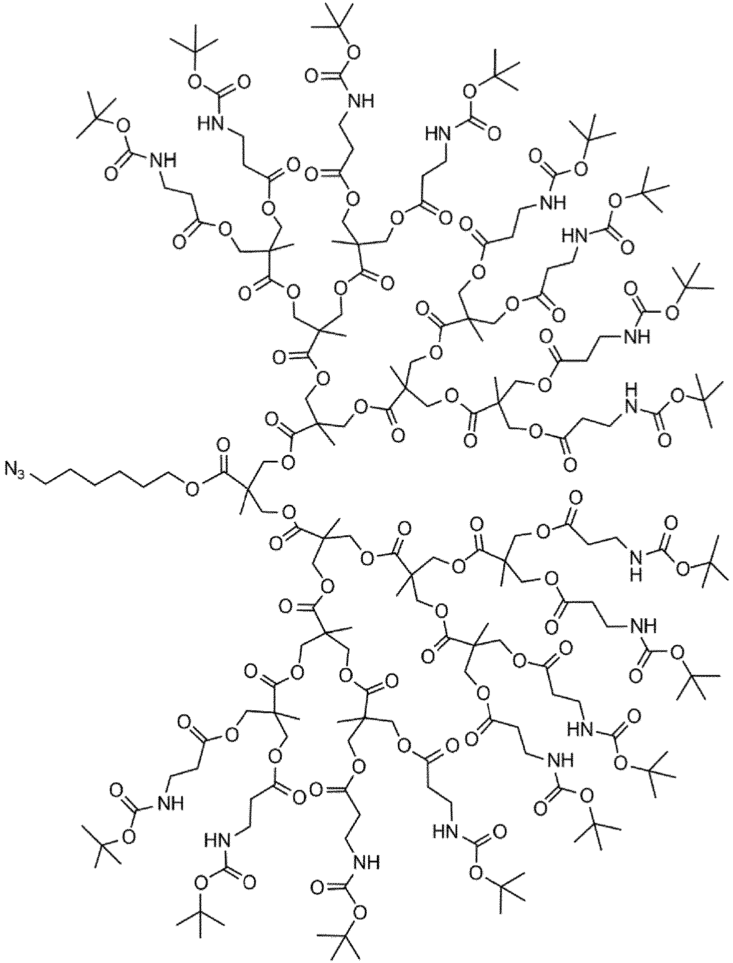 , und das mit 2-Iminothiolan derivatisiert wurde,
• Rinderserumalbumin (BSA) und
• einem Peptid mit der Sequenz SESDDAMFCDAMDESDSK.

13. Gerüst nach einem der Ansprüche 1-12,
wobei das Trägermolekül ein Immunglobulin, mindestens eine Bindungsdomäne eines Immunglobulins und/oder mindestens ein Bindungsfragment eines Immunglobulins umfasst oder daraus besteht oder mindestens einen nicht-proteinhaltigen Liganden und/oder mindestens einen proteinhaltigen Liganden zur Bindung an ein Zelloberflächenmolekül wie EGF oder ein Zytokin umfasst oder daraus besteht,
oder
wobei das Trägermolekül einen Antikörper, ein IgG, ein Molekül das eine Vhh-Domäne oder Vh-Domäne umfasst oder daraus besteht, ein Fab, ein scFv, ein Fv, ein dAb, ein F(ab)₂, ein Fcab-Fragment umfasst oder daraus besteht.

14. Gerüst nach einem der Ansprüche 1-13, wobei das Trägermolekül mindestens eine Bindungsdomäne und/oder mindestens ein Bindungsfragment zur Bindung an einen Zelloberflächenrezeptor umfasst oder daraus besteht, wie beispielsweise einen Tumorzellspezifischen Zelloberflächenrezeptor, ausgewählt aus CD71, CA125, EpCAM(17-1A), CD52, CEA, CD44v6, FAP, EGF-IR, Integrin, Syndecan-1, vaskulärem Integrin alpha-V beta-3, HER2, EGFR, CD20, CD22, Folatrezeptor 1, CD146, CD56, CD19, CD138, CD27L-Rezeptor, PSMA, CanAg, Integrin-alphaV, CA6, CD33, Mesothelin, Cripto, CD3, CD30, CD239, CD70, CD123, CD352, DLL3, CD25, EphrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, vorzugsweise ausgewählt aus CD71, EGFR, HER2.

15. Gerüst nach einem der Ansprüche 1-14, wobei das Trägermolekül ein beliebiges von Cetuximab, Daratumumab, Gemtuzumab, Trastuzumab, Panitumumab, Brentuximab, Inotuzumab, Moxetumomab, Polatuzumab, Obinutuzumab, OKT-9-anti-CD71-monoklonalem Antikörper vom IgG-Typ, Pertuzumab, Rituximab, Ofatumumab, Herceptin, Alemtuzumab, Pinatuzumab, OKT-10-anti-CD38-monoklonalem Antikörper, einem Antikörper aus Tabelle A2 oder Tabelle A3 oder Tabelle A4 oder mindestens einem Tumorzellrezeptor-bindenden Fragment davon und/oder mindestens einer Tumorzellrezeptor-bindenden Domäne davon umfasst oder daraus besteht,
oder
wobei das Trägermolekül ein beliebiges von Cetuximab oder Trastuzumab oder OKT-9, oder mindestens ein Tumorzellrezeptor-bindendes Fragment davon und/oder mindestens eine Tumorzellrezeptor-bindende Domäne davon umfasst oder daraus besteht, wie mindestens ein Tumorzell-spezifisches Rezeptor-bindendes Fragment davon und/oder mindestens eine Tumorzell-spezifische Rezeptor-bindende Domäne davon.

16. Gerüst nach einem der Ansprüche 1-15, wobei das Gerüst zur Bildung einer kovalenten Bindung mit dem Trägermolekül geeignet ist, wobei die kovalente Bindung vorzugsweise eine Cystein-Seitenkette des Trägermoleküls und/oder eine Lysin-Seitenkette des Trägermoleküls umfasst, wenn das Trägermolekül mindestens ein Cystein und/oder ein Lysin umfasst.

17. Gerüst nach einem der Ansprüche 1-16, wobei das Trägermolekül mindestens ein Effektormolekül umfasst oder daraus besteht, oder wobei der Träger weiter mindestens ein Effektormolekül umfasst, wenn abhängig von einem der Ansprüche 13-16, wobei das Effektormolekül mindestens eines von einer aktiven pharmazeutischen Substanz, wie beispielsweise eines oder mehrere von einer Wirkstoffladung, einem Toxin, einem Arzneimittel, einem Polypeptid, einem Oligonukleotid, einer Nukleinsäure, einer Xenonukleinsäure, einem Enzym wie Urease und Cre-Rekombinase, einem Proteintoxin und einem Ribosom-inaktivierenden Protein ist,
und
wobei das Proteintoxin vorzugsweise eines oder mehrere von einem Proteintoxin, ausgewählt aus Tabelle A5 und/oder einem viralen Toxin wie Apoptin; einem bakteriellen Toxin wie Shiga-Toxin, Shiga-ähnlichem Toxin, Pseudomonas aeruginosa Exotoxin (PE) oder Exotoxin A von PE, vollständigem oder verkürztem Diphtherietoxin (DT), Cholera-Toxin; einem Pilztoxin wie Alpha-Sarcin; einem Pflanzentoxin, einschließlich Ribosomen-inaktivierenden Proteinen und der A-Kette von Typ-2-Ribosomen-inaktivierenden Proteinen wie Dianthin, z. B. Dianthin-30 oder Dianthin-32, Saporin, z. B. Saporin-S3 oder Saporin-S6, Bouganin oder dem gegen Bouganin immunisierten Derivat Debouganin von Bouganin, Shiga-ähnlichem Toxin A, Pokeweed-Antivirusprotein, Ricin, Ricin-A-Kette, Modeccin, Modeccin-A-Kette, Abrin, Abrin A-Kette, Volkensin, Volkensin A-Kette, Viscumin, Viscumin A-Kette; oder einem tierischen oder humanen Toxin wie Frosch-RNase oder Granzym B oder Angiogenin von Menschen oder einem Fragment oder Derivat davon umfasst oder daraus besteht; vorzugsweise ist das Proteintoxin Dianthin und/oder Saporin,
und/oder wobei das Oligonukleotid, die Xenonukleinsäure oder die Nukleinsäure vorzugsweise eines oder mehrere von einem Vektor, einem Gen, einem Zellselbstmord induzierenden Transgen, Desoxyribonukleinsäure (DNA), Ribonukleinsäure (RNA), Antisense-Oligonukleotid (ASO, AON), kurzer interferierender RNA (siRNA), Mikro-RNA (miRNA), DNA-Aptamer, RNA-Aptamer, mRNA, Minicircle-DNA, Peptidnukleinsäure (PNA), Phosphoramidat-Morpholino-Oligomer (PMO), Locked Nucleic Acid (LNA), verbrückter Nukleinsäure (BNA), 2'-Desoxy-2'-fluorarabino-Nukleinsäure (FANA), 2'-O-Methoxyethyl-RNA (MOE), 2'-O,4'-Aminoethylen verbrückter Nukleinsäure, 3'-Fluorhexitol-Nukleinsäure (FHNA), einem Plasmid, Glykol-Nukleinsäure (GNA) und Threose-Nukleinsäure (TNA) oder einem Derivat davon umfasst oder daraus besteht, vorzugsweise eine BNA, beispielsweise eine BNA zum Silencing der HSP27-Proteinexpression,
und/oder
wobei das Effektormolekül vorzugsweise mindestens eine Wirkstoffladung umfasst oder aus dieser besteht, vorzugsweise ausgewählt aus einem oder mehreren von einem auf Ribosomen abzielenden Toxin, einem auf Elongationsfaktoren abzielenden Toxin, einem auf Tubulin abzielenden Toxin, einem auf DNA abzielenden Toxin und einem auf RNA abzielenden Toxin, noch bevorzugter einem oder mehreren von Emtansin, Pasudotox, Maytansinoid-Derivat DM1, Maytansinoid-Derivat DM4, Monomethyl-Auristatin E (MMAE, Vedotin), Monomethyl-Auristatin F (MMAF, Mafodotin), einem Calicheamicin, N-Acetyl-γ-calicheamicin, einem Pyrrolobenzodiazepin (PBD)-Dimer, einem Benzodiazepin, einem CC-1065-Analogon, einem Duocarmycin, Doxorubicin, Paclitaxel, Cisplatin, Cyclophosphamid, Etoposid, Docetaxel, 5-Fluorouracil (5-FU), Mitoxantron, einem Tubulysin, einem Indolinobenzodiazepin, AZ13599185, einem Cryptophycin, Rhizoxin, Methotrexat, einem Anthracyclin, einem Camptothecin-Analogon, SN-38, DX-8951f, Exatecanmesylat, einer verkürzten Form des *Pseudomonas aeruginosa-* Exotoxins (PE38), einem Duocarmycin-Derivat, einem Amanitin, α-Amanitin, einem Spliceostatin, einem Thailanstatin, Ozogamicin, Tesirin, Amberstatin269 und Soravtansin, oder einem Derivat dieser.

18. Gerüst nach einem der Ansprüche 1-16, wobei das Trägermolekül eine kovalent gebundene Kombination aus einem Effektormolekül und einem monoklonalen Antikörper, vorzugsweise ausgewählt aus Gemtuzumab Ozogamicin, Brentuximab Vedotin, Trastuzumab Emtansin, Inotuzumab Ozogamicin, Moxetumomab Pasudotox und Polatuzumab Vedotin und einem Antikörper-Wirkstoff-Konjugat aus Tabelle A2 und Tabelle A3 umfasst oder daraus besteht.

19. Verfahren zur Herstellung eines Gerüsts, das zur kovalenten Bindung mindestens eines bisdesmosidischen Triterpensaponins, das zum Typ eines 12,13-Dehydrooleanans mit einer Aldehydfunktion in Position C-23 gehört, an ein Trägermolekül geeignet ist, wobei das Verfahren Folgendes umfasst:
a) Bereitstellen einer polymeren oder oligomeren Struktur, die eine erste chemische Gruppe zum kovalenten Koppeln der polymeren Struktur oder der oligomeren Struktur an das Trägermolekül umfasst und mindestens eine zweite chemische Gruppe umfasst, die sich von der ersten chemischen Gruppe unterscheidet, wobei jede zweite chemische Gruppe zur kovalenten Kopplung eines der mindestens einen bisdesmosidischen Triterpensaponine, die zum Typ eines 12,13-Dehydrooleanans mit einer Aldehydfunktion in Position C-23 gehören, an die oligomere oder polymere Struktur dient; und
b) kovalentes Koppeln mindestens eines bisdesmosidischen Triterpensaponins, das zum Typ eines 12,13-Dehydrooleanans mit einer Aldehydfunktion in Position C-23 gehört, an die polymere oder oligomere Struktur über die zweite(n) chemische(n) Gruppe(n), wobei das/die bisdesmosidische(n) Triterpensaponin(e), das/die zum Typ eines 12,13-Dehydrooleanans mit einer Aldehydfunktion in Position C-23 gehört/gehören, ein beliebiges der bisdesmosidischen Triterpensaponine vom Typ eines 12,13-Dehydrooleanans mit einer Aldehydfunktion in Position C-23 der Ansprüche 1-7 ist/sind, vorzugsweise SO1861 und/oder GE1741 und/oder SA1641 und/oder QS-21,
wobei die polymere oder oligomere Struktur ausgewählt ist aus der Gruppe bestehend aus:
• Poly- oder Oligo(aminen), wie Polyethylenimin und Poly(amidoamin),
• Polyethylenglykolen,
• Poly- oder Oligo(estern), wie Poly(lactiden),
• Poly(lactamen),
• Polylactid-Co-Glycolid-Copolymeren,
• Poly- oder Oligosacchariden, wie Cyclodextrin und Polydextrose,
• Poly- oder Oligo(aminosäuren), wie Proteinen, Peptiden und Polylysin, und
• DNA-Oligomeren oder -Polymeren, RNA-Polymeren, stabilisierten RNA-Polymeren und PNA (Peptid-Nukleinsäure)-Polymeren; und
wobei die Aldehydfunktion in Position C-23 über eine Hydrazonbindung kovalent an die polymere oder oligomere Struktur gekoppelt ist,
wodurch das Gerüst bereitgestellt wird.

20. Verfahren zur Herstellung eines Gerüsts, das kovalent an ein Trägermolekül gebunden ist, wobei das Gerüst mindestens ein kovalent gebundenes bisdesmosidisches Triterpensaponin umfasst, das zum Typ eines 12,13-Dehydrooleanans mit einer Aldehydfunktion in Position C-23 gehört, wobei das Verfahren Folgendes umfasst:
a) Bereitstellen eines Gerüsts, das mindestens ein bisdesmosidisches Triterpensaponin umfasst, das zum Typ eines 12,13-Dehydrooleanans mit einer Aldehydfunktion in Position C-23 gehört, das kovalent an eine polymere oder oligomere Struktur in dem Gerüst gebunden ist, vorzugsweise Bereitstellen eines Gerüsts nach einem der Ansprüche 1-18 oder des durch das Verfahren nach Anspruch 19 erhältlichen Gerüsts oder des mit dem Verfahren nach Anspruch 19 erhaltenen Gerüsts; und
b) kovalentes Koppeln des Gerüsts aus a) an ein Trägermolekül gemäß einem der Ansprüche 13-18,
wodurch das Gerüst kovalent an ein Trägermolekül gebunden bereitgestellt wird, wobei das Gerüst mindestens ein kovalent gebundenes bisdesmosidisches Triterpensaponin umfasst, das zum Typ eines 12,13-Dehydrooleanans mit einer Aldehydfunktion in Position C-23 gehört,
wobei die polymere oder oligomere Struktur ausgewählt ist aus der Gruppe bestehend aus:
• Poly- oder Oligo(aminen), wie Polyethylenimin und Poly(amidoamin),
• Polyethylenglykolen,
• Poly- oder Oligo(estern), wie Poly(lactiden),
• Poly(lactamen),
• Polylactid-co-Glycolid-Copolymeren,
• Poly- oder Oligosacchariden, wie Cyclodextrin und Polydextrose,
• Poly- oder Oligo(aminosäuren), wie Proteinen, Peptiden und Polylysin, und
• DNA-Oligomeren oder -Polymeren, RNA-Polymeren, stabilisierten RNA-Polymeren und PNA (Peptid-Nukleinsäure)-Polymeren; und
wobei die Aldehydfunktion in Position C-23 über eine Hydrazonbindung kovalent an die polymere oder oligomere Struktur gebunden ist.

## Revendications

1. Échafaudage adapté pour lier de manière covalente au moins une molécule biologiquement active à une molécule porteuse,
où la molécule porteuse comprend ou consiste en l'une quelconque parmi une molécule protéique, une protéine, un peptide, un acide nucléique, un oligonucléotide, un lipide, une graisse, un acide gras, une nanoparticule et un glucide ;
où l'échafaudage consiste en une structure polymère ou oligomère avec au moins une desdites molécules biologiquement actives liée de manière covalente à ladite structure polymère ou oligomère, où l'échafaudage comprend en outre un premier groupe chimique pour lier de manière covalente l'échafaudage à la molécule porteuse ;
où la structure polymère ou oligomère est choisie dans le groupe constitué de :
• poly- ou oligo(amines), telles que la polyéthylèneimine et la poly(amidoamine),
• polyéthylènes glycols,
• poly- ou oligo(esters), tels que les poly(lactides),
• poly(lactames),
• copolymères polylactide-co-glycolide,
• poly- ou oligosaccharides, tels que la cyclodextrine et le polydextrose,
• poly- ou oligo(acides aminés), tels que les protéines, les peptides et la polylysine, et
• oligomères ou polymères d'ADN, polymères d'ARN, polymères d'ARN stabilisés et polymères de PNA (acide nucléique peptidique) ;
où l'au moins une molécule biologiquement active est une saponine triterpénique bisdesmosidique appartenant au type d'un 12,13-déhydrooléanane avec une fonction aldéhyde en position C-23, et
où la fonction aldéhyde en position C-23 est couplée de manière covalente à la structure polymère ou oligomère via une liaison hydrazone.

2. Échafaudage selon la revendication 1, où l'au moins une saponine triterpénique bisdesmosidique appartenant au type d'un 12,13-déhydrooléanane avec une fonction aldéhyde en position C-23 a une masse moléculaire de 3 000 Dalton ou moins.

3. Échafaudage selon la revendication 1 ou 2, où l'au moins une saponine triterpénique bisdesmosidique appartenant au type d'un 12,13-déhydrooléanane avec une fonction aldéhyde en position C-23 est une molécule amphiphile.

4. Échafaudage selon l'une quelconque des revendications 1 à 3, où la saponine triterpénique bisdesmosidique appartenant au type d'un 12,13-déhydrooléanane avec une fonction aldéhyde en position C-23 est une molécule spécifique unique ou est un mélange de différentes molécules, lorsque plus d'une molécule biologiquement active est liée de manière covalente à la structure polymère ou oligomère comprise par l'échafaudage.

5. Échafaudage selon l'une quelconque des revendications 1 à 4, où l'au moins une saponine triterpénique bisdesmosidique appartenant au type d'un 12,13-déhydrooléanane avec une fonction aldéhyde en position C-23 comprend une fonction acide glucuronique dans un substituant glucidique au niveau du groupe C-3bêta-OH de la saponine.

6. Échafaudage selon l'une quelconque des revendications 1 à 5, où l'au moins une saponine triterpénique bisdesmosidique appartenant au type d'un 12,13-déhydrooléanane avec une fonction aldéhyde en position C-23 est :
- une saponine qui peut être isolée à partir d'une espèce *Gypsophila* et/ou d'une espèce *Saponaria* et/ou d'une espèce *Agrostemma* et/ou d'une espèce *Quillaja* telle que *Quillaja saponaria,*
ou
- une saponine spécifique unique ou un mélange de deux ou plusieurs saponines différentes, telles qu'une ou plusieurs des saponines SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponine, Saponinum album, QS-18, Quil-A, Gyp1, gypsoside A, AG1, AG2, S01542, S01584, S01658, S01674, S01832, ou l'un quelconque de leurs stéréoisomères et/ou toute combinaison de ceux-ci,
ou
- S01861 et/ou GE1741 et/ou SA1641 et/ou QS-21 et/ou saponine avec un noyau aglycone d'acide quillaïque, un substituant glucidique Gal-(1→2)-[Xyl-(1→3)]-GlcA au niveau du groupe C-3bêta-OH et un substituant glucidique Glc-(1→3)-Xyl-(1→4)-Rha-(1→2)-[Xyl-(1→3)-4-OAc-Qui-(1→4)]-Fuc au niveau du groupe C-28-OH, et/ou est 3-O-bêta-D-galactopyranosyle-(1→2)-[bêta-D-xylopyranosyle-(1→3)]-bêta-D-glu curonopyranoyl quillaïque acid 28-O-bêta-D-glucopyranosyle-(1→3)-bêta-D-xylopyranosyle-(1→4)-alpha-L-rha mnopyranosyle-(1→2)-[bêta-D-xylopyranosyle-(1→3)-4-OAc-bêta-D-quinovopyr anosyle-(1→4)]-bêta-D-fucopyranoside, ou
- S01861 et/ou QS-21.

7. Échafaudage selon l'une quelconque des revendications 1 à 6, où :
- l'au moins une saponine triterpénique bisdesmosidique appartenant au type d'un 12,13-déhydrooléanane avec une fonction aldéhyde en position C-23 a une masse moléculaire d'au moins 1 500 Dalton et comprend éventuellement un groupe hydroxyle en position C-16, avec une première chaîne latérale glucidique ramifiée en position C3, laquelle première chaîne latérale glucidique ramifiée contient éventuellement de l'acide glucuronique, où la saponine contient un groupe ester avec une deuxième chaîne latérale glucidique ramifiée en position C-28, laquelle deuxième chaîne glucidique ramifiée comprend de préférence au moins quatre unités glucidiques, contenant éventuellement au moins un résidu acétyle tel que deux résidus acétyle et/ou comprenant éventuellement des glucides désoxy et/ou comprenant éventuellement du quinovose et/ou comprenant éventuellement du glucose et/ou comprenant éventuellement de l'acide 4-méthoxycinnamique et/ou comprenant éventuellement de l'acide 5-O -[5-O-Ara/Api-3,5-dihydroxy-6-méthyl-octanoyl]-3,5-dihydroxy-6-méthyl-octanoïque et/ou comprenant éventuellement de l'acide 5-O-[5-O-Rha-(1→2)-Ara/Api-3,5-dihydroxy-6-méthyl-octanoyl]-3,5-dihydroxy-6-m éthyl-octanoïque lié à un glucide via une liaison ester,
ou
- l'au moins une saponine triterpénique bisdesmosidique appartenant au type d'un 12,13-déhydrooléanane avec une fonction aldéhyde en position C-23 est QS-21 ou l'une quelconque ou plusieurs parmi QS-21 A, QS-21 A-api, QS-21 A-xyl, QS-21B, QS-21 B-api, QS-21 B-xyl, QS-7-xyl, QS-7-api, QS-17-api, QS-17-xyl, QS-18, QS1861, QS1861 protoné (Q51862), Quil-A.

8. Échafaudage selon l'une quelconque des revendications 1 à 7, où la fonction aldéhyde en position C-23 de l'au moins une saponine est couplée de manière covalente à un lieur hydrazide de l'acide N-ε-maléimidocaproïque, lequel lieur est couplé de manière covalente via une liaison thioéther à un groupe sulfhydryle dans la structure polymère ou oligomère de l'échafaudage, où le groupe sulfhydryle est de préférence un groupe sulfhydryle d'une cystéine.

9. Échafaudage selon l'une quelconque des revendications 1 à 8, où le groupe chimique pour coupler de manière covalente l'échafaudage à la molécule porteuse est un groupe à chimie click, de préférence une tétrazine, un azide, un alcène ou un alcyne, ou un dérivé cyclique de l'un quelconque de ces groupes, plus préférablement un azide.

10. Échafaudage selon l'une quelconque des revendications 1 à 9, où l'échafaudage est un lieur trifonctionnel comprenant un deuxième groupe chimique avec au moins une saponine triterpénique bisdesmosidique appartenant au type d'un 12,13-déhydrooléanane avec une fonction aldéhyde en position C-23 lié de manière covalente à celui-ci, comprenant un troisième groupe chimique pour la liaison covalente à une molécule et comprenant le premier groupe chimique pour la liaison covalente au porteur,
et/ou
où l'au moins une saponine triterpénique bisdesmosidique appartenant au type d'un 12,13-déhydrooléanane avec une fonction aldéhyde en position C-23 est un nombre défini de molécules de glycoside ou une plage définie de molécules de glycoside, de préférence un nombre défini de molécules de glycoside ou une plage définie de molécules de glycoside choisies parmi 1-128 ou au moins 2, 3, 4, 5, 6, 8, 10, 16 ou 128 molécules de glycoside, ou tout nombre de molécules de glycoside compris entre ces valeurs.

11. Échafaudage selon l'une quelconque des revendications 1 à 10, où la structure polymère ou oligomère est choisie dans le groupe constitué de :
• poly- ou oligo(amines), telles que la polyéthylèneimine et la poly(amidoamine),
• polyéthylènes glycols,
• poly- ou oligo(esters), tels que les poly(lactides), et
• poly- ou oligo(acides aminés), tels que les protéines, les peptides et la polylysine.

12. Échafaudage selon l'une quelconque des revendications 1 à 11, où la structure polymère ou oligomère est choisie dans le groupe constitué de :
• un dendrimère à base de polyéthylène glycol pentavalent de structure suivante : 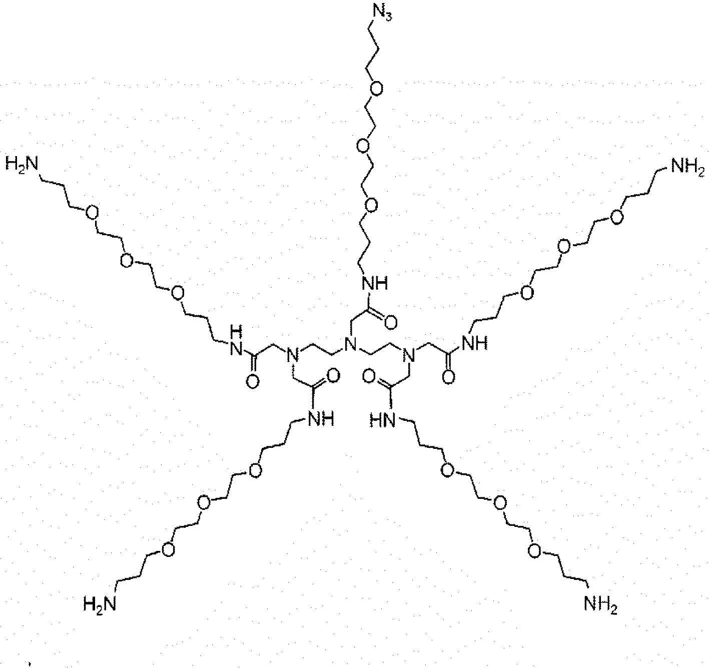
• un dendrimère poly(amidoamine) de génération 5 (G5) ayant un noyau éthylènediamine qui a été dérivé avec du 2-irminothiolane,
• un dendron polyester avec 16 groupes terminaux amino fonctionnels et un groupe azido au point focal, qui, sous sa forme protégée par NH-BOG, correspond à la structure suivante : 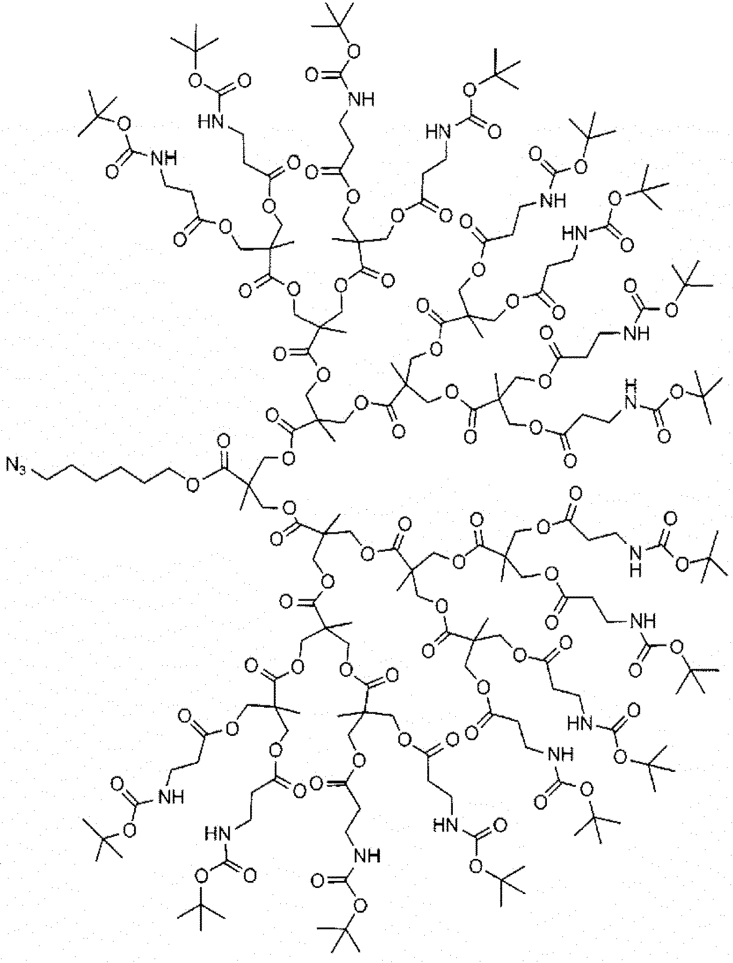 et qui a été dérivé avec du 2-iminothiolane,
• l'albumine sérique bovine (BSA), et
• un peptide ayant la séquence SESDDAMFCDAMDESDSK.

13. Échafaudage selon l'une quelconque des revendications 1 à 12,
où la molécule porteuse comprend ou consiste en une immunoglobuline, au moins un domaine de liaison d'une immunoglobuline et/ou au moins un fragment de liaison d'une immunoglobuline, ou comprend ou consiste en au moins un ligand non protéique et/ou au moins un ligand protéique pour se lier à une molécule de surface cellulaire telle que l'EGF ou une cytokine,
ou
où la molécule porteuse comprend ou consiste en un anticorps, une IgG, une molécule comprenant ou consistant en un domaine Vhh ou un domaine Vh, un Fab, un scFv, un Fv, un dAb, un F(ab)2, un fragment Fcab.

14. Échafaudage selon l'une quelconque des revendications 1 à 13, où la molécule porteuse comprend ou consiste en au moins un domaine de liaison et/ou au moins un fragment de liaison pour se lier à un récepteur de surface cellulaire tel qu'un récepteur de surface cellulaire spécifique des cellules tumorales choisi parmi CD71, CA125, EpCAM(17-1A), CD52, CEA, CD44v6, FAP, EGF-IR, intégrine, syndécane-1, intégrine vasculaire alpha-V bêta-3, HER2, EGFR, CD20, CD22, récepteur 1 du folate, CD146, CD56, CD19, CD138, récepteur CD27L, PSMA, CanAg, intégrine alphaV, CA6, CD33, mésothéline, Cripto, CD3, CD30, CD239, CD7O, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, de préférence choisi parmi CD71, EGFR, HER2.

15. Échafaudage selon l'une quelconque des revendications 1 à 14, où la molécule porteuse comprend ou consiste en l'un quelconque parmi cétuximab, daratumumab, gemtuzumab, trastuzumab, panitumumab, brentuximab, inotuzumab, moxetumomab, polatuzumab, obinutuzumab, l'anticorps monoclonal anti-CD71 OKT-9 de type IgG, pertuzumab, rituximab, ofatumumab, Herceptin, alemtuzumab, pinatuzumab, l'anticorps monoclonal anti-CD38 OKT-10, un anticorps du tableau A2 ou du tableau A3 ou du tableau A4, ou au moins un fragment de liaison au récepteur des cellules tumorales de celui-ci et/ou au moins un domaine de liaison au récepteur des cellules tumorales de celui-ci,
ou
où la molécule porteuse comprend ou consiste en l'un quelconque parmi le cétuximab ou le trastuzumab ou l'OKT-9, ou au moins un fragment de liaison au récepteur des cellules tumorales de celui-ci, et/ou au moins un domaine de liaison au récepteur des cellules tumorales de celui-ci, tel qu'au moins un fragment de liaison au récepteur spécifique des cellules tumorales de celui-ci et/ou au moins un domaine de liaison au récepteur spécifique des cellules tumorales de celui-ci.

16. Échafaudage selon l'une quelconque des revendications 1 à 15, où l'échafaudage est adapté pour former une liaison covalente avec la molécule porteuse, ladite liaison covalente impliquant de préférence une chaîne latérale cystéine de la molécule porteuse et/ou une chaîne latérale lysine de la molécule porteuse lorsque la molécule porteuse comprend au moins une cystéine et/ou une lysine.

17. Échafaudage selon l'une quelconque des revendications 1 à 16, où la molécule porteuse comprend ou consiste en au moins une molécule effectrice, ou où le porteur comprend en outre au moins une molécule effectrice lorsqu'il dépend de l'une quelconque des revendications 13 à 16, où la molécule effectrice est au moins l'une parmi une substance pharmaceutique active, telle qu'une quelconque ou plusieurs parmi une charge utile, une toxine, un médicament, un polypeptide, un oligonucléotide, un acide nucléique, un acide xénonucléique, une enzyme telle que l'uréase et la Cre-recombinase, une toxine protéique et une protéine inactivant les ribosomes,
et
où la toxine protéique comprend ou consiste de préférence en l'une quelconque ou plusieurs parmi une toxine protéique choisie dans le tableau A5 et/ou une toxine virale telle que l'apoptine ; une toxine bactérienne telle que la toxine Shiga, la toxine de type Shiga, l'exotoxine de Pseudomonas aeruginosa (PE) ou l'exotoxine A de PE, la toxine diphtérique (DT) complète ou tronquée, la toxine cholérique ; une toxine fongique telle que l'alpha-sarcine ; une toxine végétale incluant des protéines inactivant les ribosomes et la chaîne A des protéines inactivant les ribosomes de type 2 telles que la dianthine, par exemple la dianthine-30 ou la dianthine-32, la saporine, par exemple la saporine-S3 ou la saporine-S6, la bouganine ou le dérivé désimmunisé débouganine de la bouganine, la toxine de type Shiga A, la protéine antivirale de phytolaque, la ricine, la chaîne A de la ricine, la modeccine, la chaîne A de la modeccine, l'abrine, la chaîne A de l'abrine, la volkensine, la chaîne A de la volkensine, la viscumine, la chaîne A de la viscumine ; ou une toxine animale ou humaine telle que la RNase de grenouille, ou la granzyme B ou l'angiogénine provenant d'humains, ou tout fragment ou dérivé de celles-ci ; de préférence, la toxine protéique est la dianthine et/ou la saporine,
et/ou où l'oligonucléotide, l'acide nucléique xéno ou l'acide nucléique comprend de préférence ou consiste en l'un quelconque ou plusieurs parmi un vecteur, un gène, un transgène induisant le suicide cellulaire, l'acide désoxyribonucléique (ADN), l'acide ribonucléique (ARN), un oligonucléotide antisens (ASO, AON), un ARN interférent court (siARN), un microARN (miARN), un aptamère d'ADN, un aptamère d'ARN, un ARNm, un ADN mini-cercle, un acide nucléique peptidique (PNA), un oligomère morpholino phosphoramidate (PMO), un acide nucléique verrouillé (LNA), un acide nucléique ponté (BNA), un acide nucléique 2'-désoxy-2'-fluoroarabino (FANA), un ARN 2'-O-méthoxyéthyle (MOE), un acide nucléique ponté 2'-O,4'-aminoéthylène, un acide nucléique 3'-fluorohexitol (FHNA), un plasmide, un acide nucléique glycolique (GNA) et un acide nucléique thréose (TNA), ou un dérivé de ceux-ci, de préférence un BNA, par exemple un BNA pour le silençage de l'expression de la protéine HSP27,
et/ou
où la molécule effectrice comprend de préférence ou consiste en au moins une charge utile, choisie de préférence parmi l'une quelconque ou plusieurs parmi une toxine ciblant les ribosomes, une toxine ciblant les facteurs d'élongation, une toxine ciblant la tubuline, une toxine ciblant l'ADN et une toxine ciblant l'ARN, et plus préférablement l'une quelconque ou plusieurs parmi l'emtansine, le pasudotox, le dérivé de maytansinoïde DM1, le dérivé maytansinoïde de DM4, la monométhyl auristatine E (MMAE, vedotine), la monométhyl auristatine F (MMAF, mafodotine), une calichéamicine, la N-acétyl-γ-calichéamicine, un dimère de pyrrolobenzodiazépine (PBD), une benzodiazépine, un analogue de CC-1065, une duocarmycine, la doxorubicine, le paclitaxel, cisplatine, cyclophosphamide, étoposide, docétaxel, 5-fluorouracile (5-FU), mitoxantrone, une tubulysine, une indolinobenzodiazépine, AZ13599185, une cryptophycine, rhizoxine, méthotrexate, une anthracycline, un analogue de la camptothécine, SN-38, DX-8951f, mésylate d'exatecan, forme tronquée de l'exotoxine de *Pseudomonas aeruginosa* (PE38), un dérivé de la duocarmycine, une amanitine, l'α-amanitine, une spliceostatine, une thailanslatine, l'ozogamicine, la tésirine, amberstatine 269 et soravtansine, ou un dérivé de ceux-ci.

18. Échafaudage selon l'une quelconque des revendications 1 à 16, où la molécule porteuse comprend ou consiste en une combinaison liée de manière covalente d'une molécule effectrice et d'un anticorps monoclonal, de préférence choisi parmi gemtuzumab ozogamicin, brentuximab vedotin, trastuzumab emtansine, inotuzumab ozogamicine, moxetumomab pasudotox et polatuzumab vedotin, et un conjugué anticorps-médicament du tableau A2 et du tableau A3.

19. Procédé de production d'un échafaudage adapté pour lier de manière covalente au moins une saponine triterpénique bisdesmosidique appartenant au type d'un 12,13-déhydrooléanane avec une fonction aldéhyde en position C-23 à une molécule porteuse, le procédé comprenant :
a) fournir une structure polymère ou oligomère comprenant un premier groupe chimique pour coupler de manière covalente la structure polymérique ou la structure oligomérique à la molécule porteuse et comprenant au moins l'un d'un deuxième groupe chimique différent du premier groupe chimique, où chaque deuxième groupe chimique sert à coupler de manière covalente l'une des au moins une saponine triterpénique bisdesmosidique appartenant au type d'un 12,13 -déhydrooléanane avec une fonction aldéhyde en position C-23 à la structure oligomère ou polymère ; et
b) coupler de manière covalente au moins une saponine triterpénique bisdesmosidique appartenant au type d'un 12,13-déhydrooléanane avec une fonction aldéhyde en position C-23 à ladite structure polymère ou oligomère via le(s) deuxième(s) groupe(s) chimique(s), où la ou les saponines triterpéniques bisdesmosidiques appartenant au type d'un 12,13 -déhydrooléanane avec une fonction aldéhyde en position C-23 est/sont l'une quelconque des saponines triterpéniques bisdesmosidiques appartenant au type d'un 12,13-déhydrooléanane avec une fonction aldéhyde en position C-23 des revendications 1 à 7, de préférence SO1861 et/ou GE1741 et/ou SA1641 et/ou QS-21,
où la structure polymère ou oligomère est choisie dans le groupe constitué de :
• poly- ou oligo(amines), telles que la polyéthylèneimine et la poly(amidoamine),
• polyéthylènes glycols,
• poly- ou oligo(esters), tels que les poly(lactides),
• poly(lactames),
• copolymères polylactide-co-glycolide,
• poly- ou oligosaccharides, tels que la cyclodextrine et le polydextrose,
• poly- ou oligo(acides aminés), tels que les protéines, les peptides et la polylysine, et
• oligomères ou polymères d'ADN, polymères d'ARN, polymères d'ARN stabilisés et polymères de PNA (acide nucléique peptidique) ; et
où la fonction aldéhyde en position C-23 est couplée de manière covalente à la structure polymère ou oligomère via une liaison hydrazone,
fournissant ainsi l'échafaudage.

20. Procédé de production d'un échafaudage lié de manière covalente à une molécule porteuse, l'échafaudage comprenant au moins une saponine triterpénique bisdesmosidique liée de manière covalente appartenant au type d'un 12,13-déhydrooléanane avec une fonction aldéhyde en position C-23, le procédé comprenant :
a) fournir un échafaudage comprenant au moins une saponine triterpénique bisdesmosidique appartenant au type d'un 12,13-déhydrooléanane avec une fonction aldéhyde en position C -23 liée de manière covalente à une structure polymère ou oligomère dans ledit échafaudage, de préférence fournir un échafaudage selon l'une quelconque des revendications 1 à 18 ou l'échafaudage pouvant être obtenu par le procédé selon la revendication 19 ou l'échafaudage obtenu avec le procédé selon la revendication 19 ; et
b) coupler de manière covalente l'échafaudage de a) à une molécule porteuse selon l'une quelconque des revendications 13 à 18, fournissant ainsi l'échafaudage lié de manière covalente à une molécule porteuse, l'échafaudage comprenant au moins une saponine triterpénique bisdesmosidique liée de manière covalente appartenant au type d'un 12,13-déhydrooléanane avec une fonction aldéhyde en position C-23,
où la structure polymère ou oligomère est choisie dans le groupe constitué de :
• poly- ou oligo(amines), telles que la polyéthylèneimine et la poly(amidoamine),
• polyéthylènes glycols,
• poly- ou oligo(esters), tels que les poly(lactides),
• poly(lactames),
• copolymères polylactide-co-glycolide,
• poly- ou oligosaccharides, tels que la cyclodextrine et le polydextrose,
• poly- ou oligo(acides aminés), tels que les protéines, les peptides et la polylysine, et triterpénique
• oligomères ou polymères d'ADN, polymères d'ARN, polymères d'ARN stabilisés et polymères de **PNA** (acide nucléique peptidique) ; et
où la fonction aldéhyde en position C-23 est couplée de manière covalente à la structure polymère ou oligomère via une liaison hydrazone.
